# EUROPEAN PATENT APPLICATION

(11) **EP 4 071 178 A1**
(43) Date of publication of application: **12.10.2022**
(21) Application number: 20897007.9
(22) Date of filing: 04.12.2020
(51) Int. Cl.: C07K 19/00, C12N 15/62, C12P 21/02

(54) **METHOD FOR PRODUCING PEPTIDE HAVING PHYSIOLOGICAL ACTIVITY, AND PEPTIDE COMPRISING SHORT LINKER**

(30) Priority: 06.12.2019 JP 2019221040
(71) Applicant: AJINOMOTO CO., INC., Chuo-ku Tokyo 104-8315 (JP)
(72) Inventor: ITO, Kenichiro, Kawasaki-shi, Kanagawa 210-8681 (JP); MATSUDA, Yoshihiko, Kawasaki-shi, Kanagawa 210-8681 (JP); YAMADA, Naoko, Kawasaki-shi, Kanagawa 210-8681 (JP); KIKUCHI, Yoshimi, Kawasaki-shi, Kanagawa 210-8681 (JP); KONISHI, Atsushi, Kawasaki-shi, Kanagawa 210-8681 (JP); KANZAKI, Michiya, Kawasaki-shi, Kanagawa 210-8681 (JP); TAKAHASHI, Kazutoshi, Kawasaki-shi, Kanagawa 210-8681 (JP); YAMAGUCHI, Hiroki, Kawasaki-shi, Kanagawa 210-8681 (JP); FUJIWARA, Ayako, Kawasaki-shi, Kanagawa 210-8681 (JP); MIYAIRI, Kyohei, Kawasaki-shi, Kanagawa 210-8681 (JP)
(74) Representative: Hoffmann Eitle
(86) International application number: PCT/JP2020/045327
(87) International publication number: WO 2021/112249

(57) **Abstract**

Provided are: a method for producing a complicated peptide molecule, such as a dimeric peptide, in a simpler manner at lower cost compared with the conventional chemical synthesis methods; a dimeric peptide which comprises a specific short linker and has a satisfactory activity; and others.

## Description

### Technical Field

The present invention relates to a method for producing a peptide having physiological activity, a peptide comprising a short linker, and the like.

### Background Art

In recent years, biopharmaceuticals such as antibody medicine, regenerative medicine, nucleic acid medicine, and the like have been studied and actually used as modality of medicines. In addition, medium molecules, especially peptides, are attracting attention as new modality of medicines. In particular, a number of peptides having a special shape (special peptides) such as cyclic peptides have been developed, and are expected to be applied to pharmaceuticals.

Under such circumstances, the development of dimer peptides and peptide complexes is expected to accelerate in the future. For example, regarding dimer peptides, APL-2 of Apellis Pharmaceuticals has been clinically studied as a therapeutic agent for age-related macular degeneration and the like and is expected to be put into practical use.

The dimer peptide generally has a structure in which two peptide chains are linked by a short linker, and the peptide complex generally has a structure in which a peptide chain and a drug are linked by a short linker. As such a short linker, a PEG linker is mainly used.

However, in the production of dimer peptides and peptide complexes, conventional chemical synthesis methods require chemical synthesis of peptide chains, addition of PEG linkers, and purification, and many production steps are required. In addition, if the peptide is a cyclic peptide, the peptide chain must be further cyclized. Therefore, in order to produce dimer peptides or peptide complexes by conventional chemical synthesis methods, it is necessary to go through complicated steps, which is very time-consuming. Moreover, in the conventional chemical synthesis methods, the synthesis of a dimer peptide obtained by binding a first peptide and a second peptide via a PEG linker yields a product in which the C-terminus of the first peptide binds to the linker and the C-terminus of the second peptide also binds to the linker, but cannot perform efficient synthesis of dimer peptides such that the C-terminus of the first peptide binds to the linker and the N-terminus of the second peptide binds to the linker.

In addition, the PEG linker mainly used is expensive, and it cannot be said that the conventional chemical synthesis methods are economically preferable.

Published Japanese Translation of PCT International Application No. 2013-531480 describes a drug conjugate comprising a biologically active protein and a random coil polypeptide containing an amino acid sequence composed of at least 50 proline and alanine amino acid residues. Patent Literature 1 states that the random coil polypeptide provides increased blood stability of the drug conjugate.

However, Published Japanese Translation of PCT International Application No. 2013-531480 does not specifically disclose the use of the random coil polypeptide in place of a short linker such as a PEG linker in a dimer peptide or peptide complex.

Further, in order to utilize a peptide molecule such as a dimer peptide as a pharmaceutical, it is desired not only to improve the stability of the peptide molecule such as a dimer peptide but also to maintain or improve the activity. As described above, the PEG linker is mainly used as a short linker for linking two peptide chains, and the use of the PEG linker poses a manufacturing problem, and from the viewpoint of the activity of peptide molecules such as dimer peptides, more useful short linkers are eagerly desired.

### Summary of Invention

As described above, in the production of peptide molecules such as dimer peptides, the conventional chemical synthesis methods are not easy to operate and require high costs.

Therefore, an aspect of the present invention aims to provide a method for producing a peptide molecule such as a dimer peptide easily and inexpensively as compared with the conventional chemical synthesis methods.

As described above, as a short linker for linking two peptide chains, more useful short linkers are desired from the viewpoint of the activity of peptide molecules such as dimer peptides.

Therefore, an aspect of the present invention aims to provide a peptide molecule such as a dimer peptide having good activity containing a specific short linker.

The present invention may include, for example, the following aspects.
[1] A peptide molecule comprising:
   (I) a first physiologically active peptide portion;
   (II) a linker portion composed of 49 or less amino acid residues; and
   (III) a second physiologically active peptide portion located on the opposite side of the linker portion from the first physiologically active peptide portion, wherein

   the first physiologically active peptide portion and the second physiologically active peptide portion may be the same as or different from each other, and
   at least 90% of an amino acid sequence of the linker portion is composed of amino acid residues selected from alanine (A), proline (P), and serine (S).
[2] The peptide molecule according to [1] above, wherein the first physiologically active peptide portion constitutes a cyclic peptide and/or the second physiologically active peptide portion constitutes a cyclic peptide.
[3] The peptide molecule according to [1] or [2] above, wherein the first physiologically active peptide portion constitutes a cyclic peptide having a cyclic structure formed by a disulfide bond and/or the second physiologically active peptide portion constitutes a cyclic peptide having a cyclic structure formed by a disulfide bond.
[4] The peptide molecule according to any one of [1] to [3] above, wherein the first physiologically active peptide portion and the second physiologically active peptide portion each constitute a cyclic peptide that binds to a c-Met protein, and may be the same as or different from each other.
[5] The peptide molecule according to [4] above, wherein the cyclic peptide is a cyclic peptide having a cyclic structure formed by a disulfide bond, containing an amino acid sequence selected from the following (a) to (i):
   (a) CYRQFNRRTHEVWNLDC (SEQ ID NO: 1);
   (b) CRQFNRRTHEVWNLDC (SEQ ID NO: 2);
   (c) CYWYYAWDQTYKAFPC (SEQ ID NO: 3);
   (d) CWYYAWDQTYKAFPC (SEQ ID NO: 4);
   (e) CYISWNEFNSPNWRFITC (SEQ ID NO: 5);
   (f) CISWNEFNSPNWRFITC (SEQ ID NO: 6);
   (g) a peptide in which one or two amino acid residues other than a cysteine residue are substituted, deleted, or added in any of the amino acid sequences (a) to (f), and which binds to a c-Met protein;
   (h) a peptide which is composed of an amino acid sequence having 90% or more sequence identity with any of the amino acid sequences (a) to (f), but having cysteine residues at both ends, and which binds to a c-Met protein; and
   (i) a peptide in which at least one amino acid other than the cysteine residues at both ends in any of the amino acid sequences (a) to (h) is modified (the amino acid modification is phosphorylation, methylation, acetylation, adenylylation, ADP-ribosylation, or glycosylation).
[6] The peptide molecule according to any one of [1] to [3] above, wherein the first physiologically active peptide portion and the second physiologically active peptide portion each constitute a cyclic peptide that binds to an erythropoietin receptor, and may be the same as or different from each other.
[7] The peptide molecule according to [6] above, wherein the cyclic peptide is a cyclic peptide having a cyclic structure formed by a disulfide bond, containing an amino acid sequence selected from the following (j) to (n):
   (j) GGLYACHMGPMTWVCQPLRG (SEQ ID NO: 65);
   (k) CISWNEFNSPNWRFITC (SEQ ID NO: 66);
   (1) a peptide in which one or two amino acid residues other than a cysteine residue are substituted, deleted, or added in the amino acid sequence of (j) or (k), and which binds to an erythropoietin receptor;
   (m) a peptide which is composed of an amino acid sequence having 90% or more sequence identity with either the amino acid sequence of (j) or (k), but having a cysteine residue at the same position in (j) or (k), and which binds to an erythropoietin receptor; and
   (n) a peptide in which at least one amino acid other than the cysteine residue in any of the amino acid sequences (j) to (m) is modified (the amino acid modification is phosphorylation, methylation, acetylation, adenylylation, ADP-ribosylation, or glycosylation).
[8] The peptide molecule according to any one of [1] to [3] above, wherein the first physiologically active peptide portion and the second physiologically active peptide portion are each composed of a physiologically active peptide that binds to a thrombopoietin receptor, and may be the same as or different from each other.
[9] The peptide molecule according to [8] above, wherein the physiologically active peptide contains an amino acid sequence selected from the following (o) to (u):
   (o) IEGPTLRQWLAARA (SEQ ID NO: 67);
   (p) GGCADGPTLREWISFCGG (SEQ ID NO: 68);
   (q) GGCTLREWLHGGFCGG (SEQ ID NO: 69);
   (r) LAIEGPTLRQWLHGNGRDT (SEQ ID NO: 70);
   (s) a peptide in which one or two amino acid residues other than a cysteine residue are substituted, deleted, or added in any of the amino acid sequences (o) to (r), and which binds to a thrombopoietin receptor;
   (t) a peptide which is composed of an amino acid sequence having 90% or more sequence identity with any of the amino acid sequences (o) to (r), but having a cysteine residue at the same position in (p) or (q) when based on (p) or (q), and which binds to a thrombopoietin receptor; and
   (u) a peptide in which at least one amino acid other than the cysteine residue in any of the amino acid sequences (o) to (t) is modified (the amino acid modification is phosphorylation, methylation, acetylation, adenylylation, ADP-ribosylation, or glycosylation).
[10] The peptide molecule according to any one of [1] to [9] above, further comprising: a functional modification portion at a position not adjacent to the linker portion (II).
[11] The peptide molecule according to any one of [1] to [10] above, wherein
   the linker portion (II) is a first linker portion, and
   the peptide molecule further comprising:
      a second linker portion on the opposite side of the second physiologically active peptide portion from the first linker portion; and
      a third physiologically active peptide portion located on the opposite side of the second linker portion from the second physiologically active peptide portion, wherein
   the third physiologically active peptide portion may be the same as or different from the first physiologically active peptide portion and/or second physiologically active peptide portion, and
   the second linker portion is composed of 49 or less amino acid residues, and at least 90% of the amino acid sequence thereof is composed of amino acid residues selected from alanine (A), proline (P), and serine (S), which may be the same as or different from the first linker portion.
[12] The peptide molecule according to any one of [1] to [11] above, wherein
   the linker portion (II) repeatedly contains two or more amino acid sequences selected from the group consisting of AP, AAP, AS, ASP, and ASS, and if a second linker portion is present, the second linker portion repeatedly contains two or more amino acid sequences selected from the group consisting of AP, AAP, AS, ASP, and ASS.
[13] A method for producing a peptide molecule, comprising the steps of:
   producing the peptide molecule by expressing a polynucleotide in a host cell containing the polynucleotide encoding the peptide molecule or by expressing a polynucleotide encoding the peptide molecule in a cell-free expression system, wherein
   the peptide molecule contains (I) a first physiologically active peptide portion, (II) a linker portion composed of an amino acid residue, and (III) a second physiologically active peptide portion and/or an additional amino acid sequence composed of at least one amino acid residue, located on the opposite side of the linker portion from the first physiologically active peptide portion,
   the first physiologically active peptide portion and the second physiologically active peptide portion each have a molecular weight of 10,000 or less and are composed of 50 or less amino acid residues, and may be the same as or different from each other, and
   the additional amino acid sequence has a molecular weight of 10,000 or less and is composed of 50 or less amino acid residues.
[14] The production method according to [13] above, wherein the first physiologically active peptide portion constitutes a cyclic peptide and/or the second physiologically active peptide portion constitutes a cyclic peptide.
[15] A method for producing a peptide molecule, comprising the steps of:
   producing the peptide molecule by expressing a polynucleotide in a host cell containing the polynucleotide encoding the peptide molecule or by expressing a polynucleotide encoding the peptide molecule in a cell-free expression system, wherein
   the peptide molecule contains (I) a first physiologically active peptide portion constituting a cyclic peptide, (II) a linker portion composed of an amino acid residue, and (III) a second physiologically active peptide portion and/or an additional amino acid sequence composed of at least one amino acid residue, located on the opposite side of the linker portion from the first physiologically active peptide portion,
   the first physiologically active peptide portion and the second physiologically active peptide portion may be the same as or different from each other, and
   the additional amino acid sequence has a molecular weight of 10,000 or less and is composed of 50 or less amino acid residues.
[16] The production method according to [15] above, wherein the peptide molecule contains the second physiologically active peptide portion, and the second physiologically active peptide portion constitutes a cyclic peptide.
[17] The production method according to any one of [13] to [16] above, wherein the linker portion has a molecular weight of 10,000 or less and is composed of 50 or less amino acid residues.
[18] The production method according to any one of [13] to [16] above, wherein the linker portion is a linker portion having a molecular weight of 10,000 or less and composed of 49 or less amino acid residues, and at least 90% of the amino acid sequence thereof is composed of amino acid residues selected from alanine (A), proline (P), and serine (S).
[19] The production method according to any one of [13] to [18] above, wherein the linker portion repeatedly contains two or more amino acid sequences selected from the group consisting of AP, AAP, AS, ASP, and ASS.

According to an embodiment of the present invention, it is possible to produce a peptide molecule such as a dimer peptide easily and inexpensively as compared with the conventional chemical synthesis methods.

According to an embodiment of the present invention, it is possible to efficiently synthesize a dimer peptide such that the C-terminus of the first peptide binds to a linker and the N-terminus of the second peptide binds to a linker.

According to an embodiment of the present invention, it is possible to provide a dimer peptide having good activity.

### Brief Description of Drawings

Fig. 1 shows the results of SDS-polyacrylamide electrophoresis showing the secretory expression of the dimer peptide (FBP-PAS) in C. glutamicum. Lane 1 is a marker (XL-Ladder Broad, APRO science), lanes 2 to 5 are ones using pPK10_TorAss-FBP-PAS, and lane 6 is one using pPK10 (empty vector).
Fig. 2 shows measurement results for the dimer peptide (FBP-PAS) by MALDI-TOF-MS. The schematic molecular diagrams in the figure show the FBP-PAS dimer peptide, the central part (without color) represents PAS, and both side parts (colored) represent FBP.
Fig. 3 is a graph showing the activity evaluation of a monomer peptide (FBP) and a dimer peptide (FBP-PAS). The vertical axis represents relative receptor activity and the horizontal axis represents bFGF concentration. In the molecular schematic diagrams in the figure, the uncolored part represents PAS and the colored part represents FBP.
Fig. 4 shows the results of SDS-polyacrylamide electrophoresis showing the secretory expression of the dimer peptide (HGF-PAS) in C. glutamicum. Lane 1 is a marker (XL-Ladder Broad, APRO science), lanes 2 to 5 are ones using pPK10_TorAss-PAS-aMD4-PEG11, lanes 6 to 9 are ones using pPK10_TorAss-PAS-aMD4dY-PEG11, lanes 10 to 13 are ones using pPK10_TorAss-PAS-aMD4-PEG3, and lane 14 uses pPK10 (empty vector).
Fig. 5 shows the results of SDS-polyacrylamide electrophoresis showing the secretory expression of the dimer peptide (HGF-PAS) in C. glutamicum. Lane 1 is a marker (XL-Ladder Broad, APRO science), lanes 2 to 5 are ones using pPK4_CspBss-AET-PAS-aMD4-PEG11, lanes 6 to 9 are ones using pPK4_CspBss-AET-PAS-aMD4dY-PEGII, lanes 10 to 13 are ones using pPK4_CspBss-AET-PAS-aMD4-PEG3, and lane 14 uses pPK4 (empty vector).
Fig. 6 shows measurement results for the dimer peptide (HGF-PAS) by MALDI-TOF-MS.
Fig. 7 is a graph showing the activity evaluation of HGF and dimer peptide (HGF-PAS). The vertical axis represents relative receptor activity and the horizontal axis represents concentration.
Fig. 8 is a graph showing the activity evaluation of HGF and dimer peptide (HGF-PAS). The vertical axis represents relative receptor activity and the horizontal axis represents concentration.
Fig. 9 is a graph showing the activity evaluation of HGF and dimer peptide (HGF-PAS). The vertical axis represents relative receptor activity, and the horizontal axis represents concentration for HGF and, for the dimer peptide (HGF-PAS), the used culture supernatant containing a 10-fold diluted dimer peptide (HGF-PAS) with respect to the medium used for the activity evaluation.
Fig. 10 is a diagram evaluating the receptor tyrosine kinase (RTK) specificity of the dimer peptide (HGF-PAS) using Proteome Profiler Human Phospho-RTK Array Kit (R&D Systems). The dimer peptide (HGF-PAS) has been confirmed to exhibit Met (HGF receptor) specificity.
Fig. 11 is a diagram showing each array of Proteome Profiler Human Phospho-RTK Array Kit (R&D Systems).
Fig. 12 is a graph showing the evaluation of cell proliferation promoting activity of HGF and dimer peptide (HGF-PAS). The vertical axis represents the number of viable cells.
Fig. 13 is a micrograph in the evaluation of cell migration activity of HGF and dimer peptide (HGF-PAS).
Fig. 14 is a graph showing the evaluation of cell migration activity of HGF and dimer peptide (HGF-PAS). The vertical axis represents the relative movement efficiency.
Fig. 15 is a photographic diagram taken with a cell observation device in the evaluation of the wound healing effect of HGF and dimer peptide (HGF-PAS).
Fig. 16 is a graph showing the evaluation of the wound healing effect of HGF and dimer peptide (HGF-PAS). The vertical axis represents the wound healing rate.
Fig. 17 is a micrograph in the evaluation of the tubular structure forming effect of HGF and dimer peptide (HGF-PAS). The scale bar represents 500 µm.
Fig. 18 shows the results of SDS-polyacrylamide electrophoresis showing the secretory expression of the heterodimer peptide (HGF-PAS) in C. glutamicum. Lane 1 is a marker (XL-Ladder Broad, APRO science), lanes 2 to 5 are ones using pPK4_CspBss-AET-PAS-aMD4_aMD5-PEG11, and lane 6 is one using pPK4 (empty vector).
Fig. 19 shows measurement results for the heterodimer peptide (HGF-PAS) by MALDI-TOF-MS.

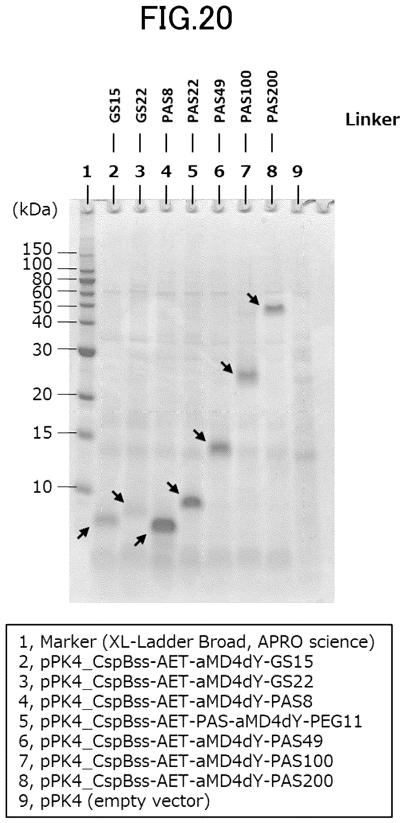

Fig. 20 shows the results of SDS-polyacrylamide electrophoresis showing the secretory expression of the dimer peptides (HGF-PAS and HGF-GS) in C. glutamicum. Lane 1 is a marker (XL-Ladder Broad, APRO science), lane 2 is one using pPK4_CspBss-AET-aMD4dY-GS15, lane 3 is one using pPK4_CspBss-AET-aMD4dY-GS22, lane 4 is one using pPK4_CspBss-AET-aMD4dY-PAS8, lane 5 is one using pPK4_CspBss-AET-PAS-aMD4dY-PEG11, lane 6 is one using pPK4_CspBss-AET-aMD4dY-PAS49, lane 7 is one using pPK4_CspBss-AET-aMD4dY-PAS100, lane 8 is one using pPK4_CspBss-AET-aMD4dY-PAS200, and lane 9 is one using pPK4 (empty vector).
Fig. 21 shows measurement results for the dimer peptide (HGF-GS) by LC-MS. Calc represents the theoretical value and Obs represents the observed value.
Fig. 22 shows measurement results for the dimer peptide (HGF-PAS) by LC-MS. Calc represents the theoretical value and Obs represents the observed value.
Fig. 23 shows measurement results for the dimer peptide (HGF-PAS) by LC-MS. Calc represents the theoretical value and Obs represents the observed value.
Fig. 24 shows measurement results for the dimer peptide (HGF-PAS) by LC-MS. Calc represents the theoretical value and Obs represents the observed value.
Fig. 25 shows measurement results for the dimer peptide (HGF-PAS) by LC-MS. Calc represents the theoretical value and Obs represents the observed value.
Fig. 26 shows measurement results for the dimer peptide (HGF-PAS) by LC-MS. Calc represents the theoretical value and Obs represents the observed value.

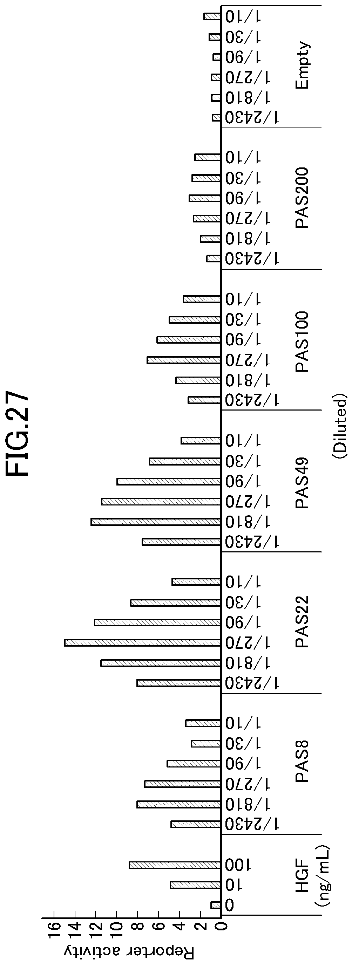

Fig. 27 is a graph showing the activity evaluation of HGF and dimer peptide (HGF-PAS). The vertical axis represents relative receptor activity, and the horizontal axis represents the concentration for HGF and, for dimer peptide (HGF-PAS), the dilution rate of the culture supernatant containing dimer peptide (HGF-PAS) with respect to the medium used for the activity evaluation. PAS8, PAS22, PAS49, PAS100, and PAS200 on the horizontal axis each represent AET-aMD4dY-PAS8, AET-aMD4dY-PAS22 (same as AET-PAS-aMD4dY-PEG11), AET-aMD4dY-PAS49, AET-aMD4dY-PAS100, and AET-aMD4dY-PAS200, respectively. Empty is the result of using an empty vector, and represents the dilution rate with respect to the medium used for the activity evaluation as in the case of using a culture supernatant containing the dimer peptide.

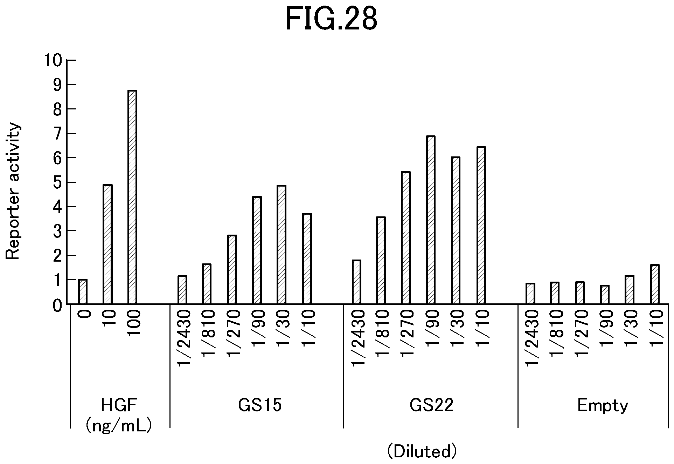

Fig. 28 is a graph showing the activity evaluation of HGF and dimer peptide (HGF-GS). The vertical axis represents relative receptor activity, and the horizontal axis represents the concentration for HGF and, for dimer peptide (HGF-GS), the dilution rate of the culture supernatant containing dimer peptide (HGF-PAS) with respect to the medium used for the activity evaluation. GS15 and GS22 on the horizontal axis represent AET-aMD4dY-GS 15 and AET-aMD4dY-GS22, respectively. Empty is the result of using an empty vector, and represents the dilution rate with respect to the medium used for the activity evaluation as in the case of using a culture supernatant containing the dimer peptide.
Fig. 29 shows the results of SDS-polyacrylamide electrophoresis showing the secretory expression of the dimer peptide (HGF-PAS) in C. glutamicum. Lane 1 is a marker (XL-Ladder Broad, APRO science), lanes 2 to 5 are ones using pPK4_CspBss-AET-aMD4dY-PAS22-PAS200, and lane 6 is one using pPK4 (empty vector).
Fig. 30 shows measurement results for the dimer peptide (HGF-PAS) by LC-MS. Calc represents the theoretical value and Obs represents the measured value.

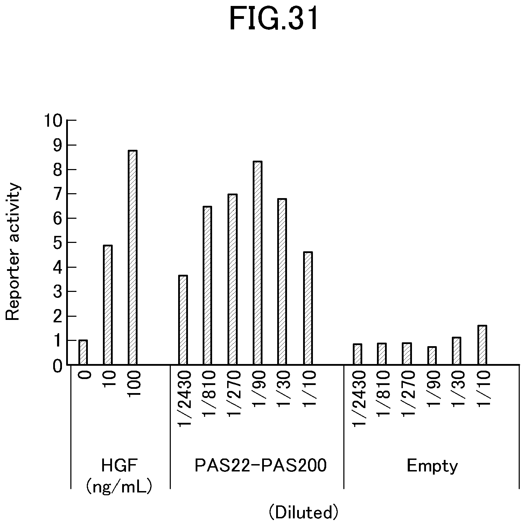

Fig. 31 is a graph showing the activity evaluation of HGF and dimer peptide (HGF-PAS). The vertical axis represents relative receptor activity, and the horizontal axis represents the concentration for HGF and, for dimer peptide (HGF-PAS), the dilution rate of the culture supernatant containing dimer peptide (HGF-PAS) with respect to the medium used for the activity evaluation. PAS22-PAS200 on the horizontal axis represents AET-aMD4dY-PAS22-PAS200. Empty is the result of using an empty vector, and represents the dilution rate with respect to the medium used for the activity evaluation as in the case of using a culture supernatant containing the dimer peptide.
Fig. 32 shows the results of SDS-polyacrylamide electrophoresis showing the secretory expression of the dimer peptide (EPO-PAS) in C. glutamicum. Lane 1 is a marker (XL-Ladder Broad, APRO science), lanes 2 to 5 are ones using pPK4_CspBss-EPO-PAS8, lanes 6 to 9 are ones using pPK4_CspBss-EPO-PAS22, lanes 10 to 13 are ones using pPK4_CspBss-AET-EPO-PAS8, lanes 14 to 17 are ones using pPK4_CspBss-AET-EPO-PAS22, and lane 18 is one using pPK4 (empty vector).
Fig. 33 shows measurement results of the dimer peptide (EPO-PAS) by LC-MS. Calc represents the theoretical value and Obs represents the measured value.
Fig. 34 shows measurement results of the dimer peptide (EPO-PAS) by LC-MS. Calc represents the theoretical value and Obs represents the measured value.
Fig. 35 is a graph showing the activity evaluation of rhEPO and dimer peptide (EPO-PAS). The vertical axis represents relative receptor activity and the horizontal axis represents concentration.
Fig. 36 is a graph showing the activity evaluation of the monomer peptide EMP35. The vertical axis represents relative receptor activity and the horizontal axis represents concentration.
Fig. 37 is the results of SDS-polyacrylamide electrophoresis showing the secretory expression of the dimer peptide (TPO-PAS) in C. glutamicum. Lane 1 is a marker (XL-Ladder Broad, APRO science), lanes 2 to 5 are ones using pPK4_CspBss-AET-TPO1-PAS8, lanes 6 to 9 are ones using pPK4_CspBss-TPO2-PAS8, lanes 10 to 13 are ones using pPK4_CspBss-AET-TPO2-PAS8, and lane 14 is one using pPK4 (empty vector).
Fig. 38 shows measurement results of the dimer peptide (TPO-PAS) by LC-MS. Calc represents the theoretical value and Obs represents the measured value.
Fig. 39 shows measurement results of the dimer peptide (TPO-PAS) by LC-MS. Calc represents the theoretical value and Obs represents the measured value.
Fig. 40 shows measurement results of the dimer peptide (TPO-PAS) by LC-MS. Calc represents the theoretical value and Obs represents the measured value.

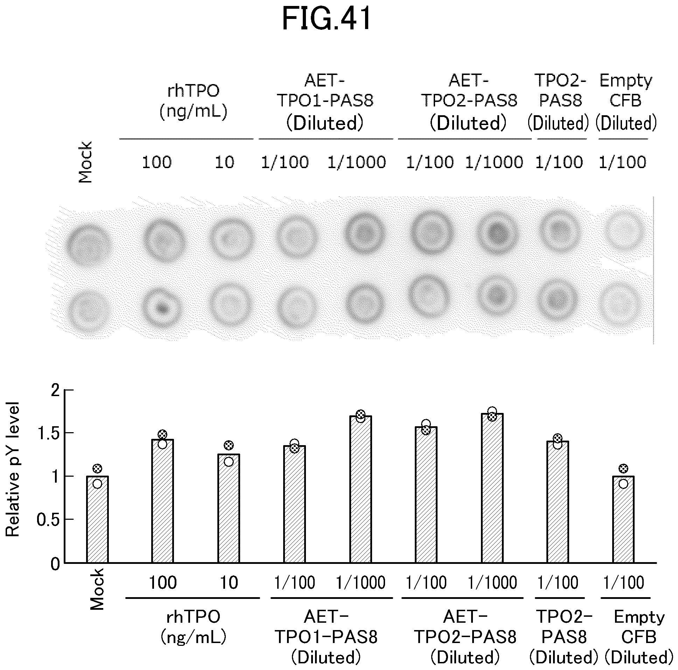

Fig. 41 is a graph showing the evaluation of tyrosine phosphorylation levels of rhTPO and dimer peptide (TPO-PAS). As shown in the upper figure, the detected dot luminescence intensity is quantified, and the quantification results are shown in the lower graph in terms of relative tyrosine phosphorylation level. The vertical axis represents the relative tyrosine phosphorylation level, and the horizontal axis represents concentration for rhTPO, and for the dimer peptide (TPO-PAS), the dilution rate of the culture supernatant containing the dimer peptide (TPO-PAS) with respect to the medium used for the activity evaluation. Empty CFB is the result for the supernatant when using an empty vector. Mock represents the PBS-added group.
Fig. 42 is a graph showing the evaluation of cell activation ability of rhTPO and dimer peptide (TPO-PAS). As shown in the upper figure, the detected dot luminescence intensity is quantified, and the quantification results are shown in the lower graph. The vertical axis represents the dot luminescence intensity, and the horizontal axis represents concentration for rhTPO, and for the dimer peptide (TPO-PAS), the dilution rate of the culture supernatant containing the dimer peptide (TPO-PAS) with respect to the medium used for the activity evaluation. Mock represents the PBS-added group.
Fig. 43 is a diagram evaluating the signal activation characteristics of rhTPO and dimer peptide (TPO-PAS) using Proteome Profiler Human Phospho-Kinase Array Kit (R&D Systems). For the dimer peptide (TPO-PAS), the dilution rate of the culture supernatant containing the dimer peptide (TPO-PAS) with respect to the medium used for the activity evaluation is shown. Mock represents the PBS-added group.

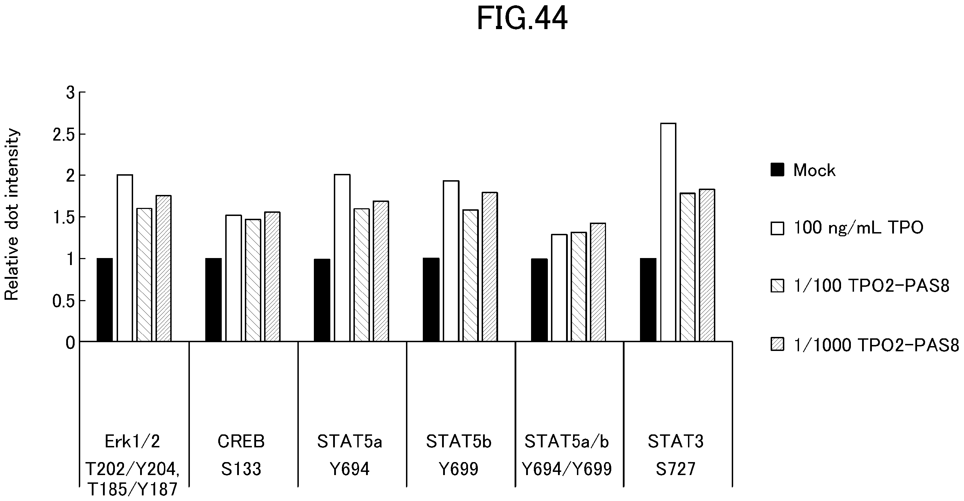

Fig. 44 is a graph showing quantitatively the phosphorylation ability of rhTPO and dimer peptide (TPO-PAS) measured using Proteome Profiler Human Phospho-Kinase Array Kit (R&D Systems). The vertical axis represents the relative dot luminescence intensity, and the horizontal axis represents the activated molecules. For the dimer peptide (TPO-PAS), the dilution rate of the culture supernatant containing the dimer peptide (TPO-PAS) with respect to the medium used for the activity evaluation is shown. Mock represents the PBS-added group.

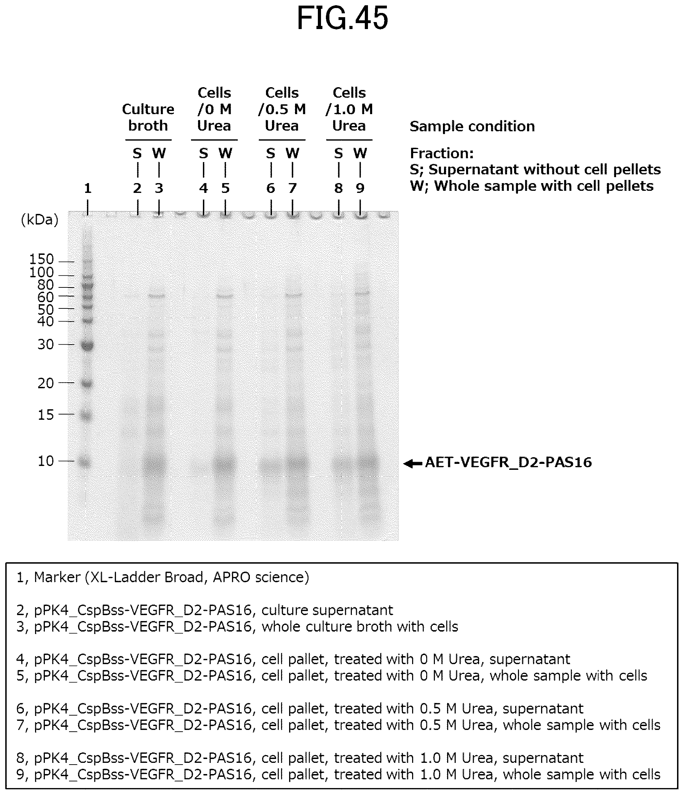

Fig. 45 shows the results of SDS-polyacrylamide electrophoresis showing the secretory expression of the heterodimer peptide (VEGFR_D2-PAS) in C. glutamicum. Lane 1 is a marker (XL-Ladder Broad, APRO science), lanes 2 and 3 are for the culture supernatant and for the entire culture solution containing cells, respectively, when pPK4_CspBss-VEGFR_D2-PAS16 is used, lanes 4 and 5 are for the supernatant fraction and for the entire mixture containing cells, respectively, after adding Tris-HCl to the precipitate fraction containing cells when pPK4_CspBss-VEGFR_D2-PAS16 is used, lanes 6 and 7 are for the supernatant fraction and for the entire mixture containing cells, respectively, after adding the urea treatment solution (0.5 M urea) to the precipitate fraction containing cells when pPK4_CspBss-VEGFR_D2-PAS16 is used, and lanes 8 and 9 are for the supernatant fraction and for the entire mixture containing cells, respectively, after adding the urea treatment solution (1.0 M urea) to the precipitate fraction containing cells when pPK4_CspBss-VEGFR_D2-PAS16 is used.
Fig. 46 shows measurement results of the heterodimer peptide (VEGFR D2-PAS) by LC-MS. Calc represents the theoretical value and Obs represents the measured value.
Fig. 47 is a graph showing the evaluation of the VEGF inhibitory ability of the heterodimer peptide (VEGFR_D2-PAS) and the monomeric peptides P3 and Pm4. The vertical axis represents relative VEGF activity (relative receptor activity), and the horizontal axis represents concentration.
Fig. 48 shows the results of SDS-polyacrylamide electrophoresis showing the secretory expression of the dimer peptides (EPO-PAS and EPO-GS) in C. glutamicum. Lane 1 is a marker (XL-Ladder Broad, APRO science), lanes 2 to 5 are ones using pPK4_CspBss-AET-EPO-PAS49, lanes 6 to 9 are ones using pPK4_CspBss-AET-EPO-PAS49-b, lanes 10 to 13 are ones using pPK4_CspBss-AET-EPO-PAS100, lanes 14 to 17 are ones using pPK4_CspBss-AET-EPO-PAS100-b, lanes 18 to 21 are ones using pPK4_CspBss-AET-EPO-GS8, lanes 22 to 25 are ones using pPK4_CspBss-AET-EPO-GS22, and lane 26 is one using pPK4 (empty vector).
Fig. 49 shows measurement results of the dimer peptide (EPO-GS) by LC-MS. Calc represents the theoretical value and Obs represents the measured value.
Fig. 50 shows measurement results of the dimer peptide (EPO-GS) by LC-MS. Calc represents the theoretical value and Obs represents the measured value.
Fig. 51 shows measurement results of the dimer peptide (EPO-PAS) by LC-MS. Calc represents the theoretical value and Obs represents the measured value.
Fig. 52 shows measurement results of the dimer peptide (EPO-PAS) by LC-MS. Calc represents the theoretical value and Obs represents the measured value.
Fig. 53 shows measurement results of the dimer peptide (EPO-PAS) by LC-MS. Calc represents the theoretical value and Obs represents the measured value.
Fig. 54 shows measurement results of the dimer peptide (EPO-PAS) by LC-MS. Calc represents the theoretical value and Obs represents the measured value.
Fig. 55 is a graph showing the activity evaluation of the dimer peptides (EPO-PAS and EPO-GS). The vertical axis represents relative receptor activity and the horizontal axis represents concentration.
Fig. 56 is a graph showing the activity evaluation of the dimer peptides (EPO-PAS and EPO-GS). The vertical axis represents relative receptor activity and the horizontal axis represents concentration.
Fig. 57 is a graph showing the activity evaluation of the dimer peptide (EPO-PAS). The vertical axis represents relative receptor activity and the horizontal axis represents concentration.
Fig. 58 shows the results of SDS-polyacrylamide electrophoresis showing the secretory expression of the dimer peptide (EPO-PAS) in C. glutamicum. Lane 1 is a marker (XL-Ladder Broad, APRO science), lanes 2 to 5 are ones pPK4_CspBss-AET-EPO-PAS22-PAS200, lanes 6 to 9 are ones pPK4_CspBss-AET-EPO-PAS22-PAS600, and lane 10 is one using pPK4 (empty vector).
Fig. 59 shows measurement results of the dimer peptide (EPO-PAS) by LC-MS. Calc represents the theoretical value and Obs represents the measured value.
Fig. 60 shows measurement results of the dimer peptide (EPO-PAS) by LC-MS. Calc represents the theoretical value and Obs represents the measured value.
Fig. 61 is a graph showing the activity evaluation of the dimer peptide (EPO-PAS). The vertical axis represents relative receptor activity and the horizontal axis represents concentration.
Fig. 62 shows measurement results for the dimer peptide (HGF-PAS) by MALDI-TOF-MS.
Fig. 63 shows measurement results for the dimer peptide (HGF-PAS) by MALDI-TOF-MS.
Fig. 64 shows measurement results for the dimer peptide (HGF-PAS) by MALDI-TOF-MS.
Fig. 65 shows measurement results of the dimer peptide (EPO-PAS) by MALDI-TOF-MS.
Fig. 66 shows measurement results of the dimer peptide (EPO-PAS) by MALDI-TOF-MS.
Fig. 67 shows measurement results of the dimer peptide (EPO-PAS) by MALDI-TOF-MS.
Fig. 68 shows measurement results of the dimer peptide (VEGFR_D2-PAS) by MALDI-TOF-MS.
Fig. 69 is a graph showing the activity evaluation of the dimer peptide (HGF-PAS). The vertical axis represents the relative receptor activity, and the horizontal axis represents the dilution factor of the reaction solution of the cell-free expression system containing the dimer peptide (HGF-PAS) with respect to the medium used for the activity evaluation. Blank is the result of using no expression DNA in cell-free expression.
Fig. 70 is a graph showing the activity evaluation of the dimer peptide (EPO-PAS). The vertical axis represents relative receptor activity and the horizontal axis represents concentration.
Fig. 71 is a graph showing the evaluation of the VEGF inhibitory ability of the heterodimer peptide (VEGFR D2-PAS). The vertical axis represents relative VEGF activity (relative receptor activity), and the horizontal axis represents concentration.
Fig. 72 is a graph showing the evaluation of VEGF inhibitory ability of the heterodimer peptides (VEGFR_D2-PAS and VEGFR_D2-GS). The vertical axis represents relative VEGF activity (relative receptor activity), and the horizontal axis represents concentration.
Fig. 73 shows the results of SDS-polyacrylamide electrophoresis showing the secretory expression of the dimer peptide and trimer peptide (HGF-PAS) in C. glutamicum. Lane 1 is a marker (XL-Ladder Broad, APRO science), lanes 2 to 5 are ones using pPK4_CspBss-AET-aMD4dY-PAS22-Trimer, lane 6 is one using pPK4_CspBss-AET-aMD4dY-PAS22, and lane 7 is one using pPK4 (empty vector).
Fig. 74 shows measurement results of the trimer peptide (HGF-PAS) by LC-MS. Calc represents the theoretical value and Obs represents the measured value.
Fig. 75 is a graph showing the activity evaluation of the dimer peptide and trimer peptide (HGF-PAS). The vertical axis represents relative receptor activity and the horizontal axis represents concentration.
Fig. 76 is a graph showing the activity evaluation of HGF, dimer peptide (HGF-PAS), and monomer peptide aMD4dY. The vertical axis represents relative receptor activity and the horizontal axis represents concentration.
Fig. 77 shows the results of SDS-polyacrylamide electrophoresis showing the secretory expression of the heterodimer peptides (VEGFR_D2-PAS and VEGFR_D2-GS) in C. glutamicum. Lane 1 is a marker (XL-Ladder Broad, APRO science), lanes 2 to 5 are ones using pPK4_CspBss-VEGFR_D2-PAS49, lanes 6 to 9 are ones using pPK4_CspBss-VEGFR_D2-PAS49-b, lanes 10 to 13 are ones using pPK4_CspBss-VEGFR_D2-PAS100, lanes 14 to 17 are ones using pPK4_CspBss-VEGFR_D2-PAS100-b, lanes 18 to 21 are ones using pPK4_CspBss-VEGFR_D2-GS16, and lane 22 is one using pPK4 (empty vector).
Fig. 78 shows measurement results of the heterodimer peptide (VEGFR_D2-GS) by LC-MS. Calc represents the theoretical value and Obs represents the measured value.
Fig. 79 shows measurement results of the heterodimer peptide (VEGFR_D2-PAS) by LC-MS. Calc represents the theoretical value and Obs represents the measured value.
Fig. 80 shows measurement results of the heterodimer peptide (VEGFR_D2-PAS) by LC-MS. Calc represents the theoretical value and Obs represents the measured value.
Fig. 81 shows measurement results of the heterodimer peptide (VEGFR_D2-PAS) by LC-MS. Calc represents the theoretical value and Obs represents the measured value.
Fig. 82 is a graph showing the evaluation of VEGF inhibitory ability of the heterodimer peptides (VEGFR_D2-PAS and VEGFR D2-GS). The vertical axis represents relative VEGF activity (relative receptor activity), and the horizontal axis represents concentration.
Fig. 83 is a graph showing the evaluation of VEGF inhibitory ability of the heterodimer peptide (VEGFR D2-PAS). The vertical axis represents relative VEGF activity (relative receptor activity), and the horizontal axis represents concentration.
Fig. 84 shows the results of SDS-polyacrylamide electrophoresis showing the secretory expression of the dimer peptides (TPO-PAS and TPO-GS) in C. glutamicum. Lane 1 is a marker (XL-Ladder Broad, APRO science), lanes 2 to 5 are ones using pPK4_CspBss-TPO2-PAS49, lanes 6 to 9 are ones using pPK4_CspBss-TPO2-PAS49-b, lanes 10 to 13 are ones using pPK4_CspBss-TPO2-PAS100, lanes 14 to 17 are ones using pPK4_CspBss-TPO2-PAS100-b, lanes 18 to 21 are ones using pPK4_CspBss-TPO2-GS8, and lane 22 is one using pPK4 (empty vector).
Fig. 85 shows measurement results of the dimer peptide (TPO-GS) by LC-MS. Calc represents the theoretical value and Obs represents the measured value.
Fig. 86 shows measurement results of the dimer peptide (TPO-PAS) by LC-MS. Calc represents the theoretical value and Obs represents the measured value.
Fig. 87 shows measurement results of the dimer peptide (TPO-PAS) by LC-MS. Calc represents the theoretical value and Obs represents the measured value.
Fig. 88 shows measurement results of the dimer peptide (TPO-PAS) by LC-MS. Calc represents the theoretical value and Obs represents the measured value.
Fig. 89 shows measurement results of the dimer peptide (TPO-PAS) by LC-MS. Calc represents the theoretical value and Obs represents the measured value.
Fig. 90 is a graph showing quantitatively the phosphorylation ability of rhTPO and dimer peptides (TPO-PAS and TPO-GS) measured using Phospho-CREB (Ser133) and Total CREB ELISA kit (RayBio). The vertical axis represents relative CREB phosphorylation levels and the horizontal axis represents activated molecules. Mock represents the PBS-added group.

### Description of Embodiments

An embodiment of the present invention relates to a peptide molecule. Such a peptide molecule is a peptide molecule comprising:
(I) a first physiologically active peptide portion;
(II) a linker portion composed of 49 or less amino acid residues; and
(III) a second physiologically active peptide portion located on the opposite side of the linker portion from the first physiologically active peptide portion, wherein
   the first physiologically active peptide portion and the second physiologically active peptide portion may be the same as or different from each other, and
   at least 90% of an amino acid sequence of the linker portion is composed of amino acid residues selected from alanine (A), proline (P), and serine (S).

In an embodiment of the peptide molecule of the present invention, it may contain a first physiologically active peptide portion on the N-terminus side of the linker portion and a second physiologically active peptide portion on the C-terminus side of the linker portion, or may contain a first physiologically active peptide portion on the C-terminus side of the linker portion and a second physiologically active peptide portion on the N-terminus side of the linker portion. Further, in an embodiment of the peptide molecule of the present invention, it may contain an arbitrary amino acid sequence as long as it does not inhibit the functions of the first physiologically active peptide and second physiologically active peptide on the N-terminus side and/or the C-terminus side of the first physiologically active peptide portion and/or second physiologically active peptide portion, or in the space with the linker portion. As such an arbitrary amino acid sequence, for example, a sequence useful for enhancing the expression or secretion of peptide molecules in a host cell, or a sequence for simply adjusting the length (such as 1, 2, 3, or 1 to 5 Gly) may be used.

In the peptide molecule of the present invention, each amino acid may be an L-amino acid or a D-amino acid, and may be a natural type or an unnatural type. Further, in the peptide molecule of the present invention, each amino acid is preferably an α-, β-, or γ-amino acid, and more preferably an α-amino acid.

In an embodiment of the peptide molecule of the present invention, the peptide molecule may be configured to have two or more linker portions, such as "first physiologically active peptide portion"-"linker portion"-"second physiologically active peptide portion"-"linker portion"-"arbitrary sequence" from the N-terminus side. Here, the arbitrary sequence may be a third physiologically active peptide portion (may be the same as or different from the first physiologically active peptide portion and/or second physiologically active peptide portion), or may be an additional amino acid sequence composed of at least one amino acid residue. Examples of the additional amino acid sequence include a sequence having a molecular weight of 10,000 or less and composed of 50 or less amino acid residues. Such additional amino acid sequences include enzyme identification sequences such as SortaseA identification signal and Butelase identification signal, tag sequences such as FLAG tag, PA tag, and histidine tag, reactive amino acids such as lysine, cysteine, glutamic acid, and aspartic acid, or sequences containing the reactive amino acids.

In an embodiment of the peptide molecule of the present invention,
the linker portion (II) is a first linker portion, and
the peptide molecule further comprising:
   a second linker portion on the opposite side of the second physiologically active peptide portion from the first linker portion; and
   a third physiologically active peptide portion located on the opposite side of the second linker portion from the second physiologically active peptide portion, wherein
the third physiologically active peptide portion may be the same as or different from the first physiologically active peptide portion and/or second physiologically active peptide portion, and
the second linker portion is composed of 49 or less amino acid residues, and at least 90% of the amino acid sequence thereof is composed of amino acid residues selected from alanine (A), proline (P), and serine (S), which may be the same as or different from the first linker portion.

As used in the present specification, the term "physiologically active peptide" means a peptide that can exhibit physiological activity in a living body such as a human or an animal. Here, the "physiological activity" is a property that acts on the physiological functions of a living body, and may include both a property that enhances physiological functions and a property that suppresses physiological functions.

In the present specification, the "physiologically active peptide" includes not only a peptide itself that can exhibit physiological activity in a living body such as a human or an animal, but also a peptide that exhibits physiological activity by modification in vivo (that is, a precursor substance), and the "physiologically active peptide" may be composed of a naturally occurring amino acid sequence or may be composed of an unnaturally occurring amino acid sequence. Examples of the "physiologically active peptide" include, but are not limited to, a target-binding peptide, a target-inhibiting peptide, a target-activating peptide, and an enzyme-recognizing peptide.

As used in the present specification, the term "target-binding peptide" means a physiologically active peptide that binds to a target (mainly a protein) such as a receptor, enzyme, ion channel, or transporter (including those that start exhibiting binding property to a target or physiological activity by modification in vivo). Examples of the target-binding peptide include, but are not limited to, an albumin-binding cyclic peptide and an organ delivery cyclic peptide.

As used in the present specification, the term "target-inhibiting peptide" means a physiologically active peptide that has an action of inhibiting the biological reactions caused by activation of a target (mainly a protein) such as a receptor, enzyme, ion channel, or transporter or the physiological functions in which the target is involved. The "target-inhibiting peptide" includes not only a physiologically active peptide that, by binding to a target itself, inhibits the biological reactions caused by activation of the target or the physiological functions in which the target is involved, but also a physiologically active peptide that inhibits the biological reactions caused by activation of the target or the physiological functions in which the target is involved based on binding to a factor that binds to the target to alter the properties or shape of that factor.

As used in the present specification, the term "target-activating peptide" means a physiologically active peptide that has an action of activating a target (mainly a protein) such as a receptor, enzyme, ion channel, or transporter to cause biological reactions or an action of enhancing the physiological functions in which the target is involved (including those that start exhibiting activating effects or enhancing effects by modification in vivo). The "target-activating peptide" includes not only a physiologically active peptide that, by binding to a target itself, activates the target to cause biological reactions or enhances the physiological functions in which the target is involved, but also a physiologically active peptide that activates the target to cause biological reactions or enhances the physiological functions in which the target is involved based on binding to a factor to alter the properties or shape of that factor and making the factor start binding to the target.

Targets in the "target-binding peptide", "target-inhibiting peptide", and "target-activating peptide" preferably include receptors that are activated by dimerization, such as proliferation factor receptors, growth factor receptors, and cytokine receptors.

As used in the present specification, the term "enzyme-recognizing peptide" means a physiologically active peptide that is recognized as a substrate by enzymes (including those that start being recognized as a substrate by enzymes or start exhibiting physiological activity by modification in vivo).

In an embodiment of the peptide molecule of the present invention, the molecular weights of the first physiologically active peptide portion and second physiologically active peptide portion are not particularly limited, but may be, for example, 250 or more, 500 or more, 800 or more, or 1,000 or more, and may be 10,000 or less, 9,000 or less, 8,000 or less, 7,000 or less, 6,000 or less, 5,000 or less, or 4,000 or less.

In an embodiment of the peptide molecule of the present invention, the lengths of the first physiologically active peptide portion and second physiologically active peptide portion are not particularly limited, but may be, for example, each independently one composed of 3 or more, 6 or more, 10 or more, or 12 or more amino acid residues, and may be one composed of 50 or less, 45 or less, 40 or less, 35 or less, 30 or less, 25 or less, or 20 or less amino acid residues.

In an embodiment of the peptide molecule of the present invention, the first physiologically active peptide portion and/or second physiologically active peptide portion may be a straight chain or a branched chain, and may constitute a cyclic peptide. The cyclic peptide in the first physiologically active peptide portion and/or second physiologically active peptide portion is not particularly limited in what kind of binding mode it has a cyclic structure, and it can form a cyclic structure, for example, by a disulfide bond between two cysteine residues, by a thioether bond (bond between a thiol group and a haloalkyl group), or by a bond of 1,2,3-triazole (bond between an azido group and an alkyne group). Note that the disulfide bond may be formed spontaneously inside or outside cells by culturing the cells, or by an enzymatic reaction by the cells.

In an embodiment of the peptide molecule of the present invention, the first physiologically active peptide portion may contain two cysteine residues, or at least two cysteine residues.

In an embodiment of the peptide molecule of the present invention, the second physiologically active peptide portion may contain two cysteine residues, or at least two cysteine residues.

In an embodiment of the peptide molecule of the present invention, preferably, the first physiologically active peptide portion constitutes a cyclic peptide.

In an embodiment of the peptide molecule of the present invention, preferably, the second physiologically active peptide portion constitutes a cyclic peptide.

When the peptide molecule of the present invention has a cyclic peptide in the first physiologically active peptide portion and/or second physiologically active peptide portion, examples of the cyclic peptide include, but are not particularly limited to, FGFR binding peptide, c-Met (HGFR) binding peptide, erythropoietin receptor binding peptide, thrombopoietin receptor binding peptide, albumin binding peptide, EGFR binding peptide, VEGFR binding peptide, PDGFR binding peptide, Axl binding peptide, PDGFR binding peptide, SCFR binding peptide, Flt-3 binding peptide, c-Ret binding peptide, ROR binding peptide, Tie binding peptide, NGFR binding peptide, Insulin receptor binding peptide, EphR binding peptide, Alk binding peptide, DDR binding peptide, TGFBR binding peptide, Activin receptor binding peptide, BMP receptor binding peptide, interleukin receptor binding peptide, T-cell receptor binding peptide, transferrin receptor binding peptide, lipoprotein receptor binding peptide, ubiquitin ligase binding peptide, antibody binding peptide, complement binding peptide, GPCR binding peptide, ion channel binding peptide, virus binding peptide, and the like.

In an embodiment of the peptide molecule of the present invention, as a specific example, the first physiologically active peptide portion and the second physiologically active peptide portion each constitute a cyclic peptide that binds to a c-Met (also referred to simply as Met) protein and may be the same as or different from each other. The cyclic peptide that binds to the c-Met protein includes, but is not limited to, a cyclic peptide having a cyclic structure formed by a disulfide bond, containing or composed of an amino acid sequence selected from the following (a) to (i):
(a) CYRQFNRRTHEVWNLDC (SEQ ID NO: 1);
(b) CRQFNRRTHEVWNLDC (SEQ ID NO: 2);
(c) CYWYYAWDQTYKAFPC (SEQ ID NO: 3);
(d) CWYYAWDQTYKAFPC (SEQ ID NO: 4);
(e) CYISWNEFNSPNWRFITC (SEQ ID NO: 5);
(f) CISWNEFNSPNWRFITC (SEQ ID NO: 6);
(g) a peptide in which one or two amino acid residues other than a cysteine residue are substituted, deleted, or added in any of the amino acid sequences (a) to (f), and which binds to a c-Met protein;
(h) a peptide which is composed of an amino acid sequence having 90% or more sequence identity with any of the amino acid sequences (a) to (f), but having cysteine residues at both ends, and which binds to a c-Met protein. For example, the sequence identity may be 92% or higher, 95% or higher, or 98% or higher. Note that in the present specification, the amino acid sequence homology may be calculated by using a known analysis tool, and may be calculated, for example, by using the National Center for Biotechnology Information (NCBI) homology algorithm BLAST (Basic local alignment search tool). In addition, the default (initial setting) parameters in the analysis tool may be used to calculate the amino acid sequence homology.
(i) A peptide in which at least one amino acid other than the cysteine residues at both ends in any of the amino acid sequences (a) to (h) is modified. Here, the amino acid modification is phosphorylation, methylation, acetylation, adenylylation, ADP-ribosylation, or glycosylation.

Note that it is known Met is an HGF receptor, a tyrosine kinase type receptor, and a cyclic peptide that binds to such Met (HGFR) functions as a Met (HGFR) agonist by dimerization.

As used in the present specification, in the expression "substituted, deleted, or added", substitution means substitution with a known amino acid, preferably a conservative amino acid substitution. A "conservative amino acid substitution" is a substitution in which an amino acid residue is substituted with another amino acid residue having a side chain R group with similar chemical properties (for example, charge or hydrophobicity). Examples of the amino acid groups having side chains with similar chemical properties include glycine, alanine, valine, leucine, and isoleucine having aliphatic side chains, serine and threonine having aliphatic hydroxyl side chains, asparagine and glutamine having amide-containing side chains, phenylalanine, tyrosine, and tryptophan having aromatic side chains, lysine, arginine, and histidine having basic side chains, and aspartic acid and glutamic acid having acidic side chains. Each amino acid introduced by substitution may be an L-amino acid or a D-amino acid, and may be a natural type amino acid or an unnatural type amino acid, and preferably an amino acid that constitutes a protein in humans and animals.

As used in the present specification, in the expression "substituted, deleted, or added", addition means that a known amino acid is added to the terminus of the amino acid sequence, or that a known amino acid is inserted between amino acid residues of the amino acid sequence. Each amino acid introduced by addition may be an L-amino acid or a D-amino acid, and may be a natural type amino acid or an unnatural type amino acid, and preferably an amino acid that constitutes a protein in humans and animals.

In an embodiment of the peptide molecule of the present invention, as a specific example, the first physiologically active peptide portion and the second physiologically active peptide portion each constitute a cyclic peptide that binds to an erythropoietin receptor and may be the same as or different from each other. The cyclic peptides that binds to an erythropoietin receptor includes, but is not limited to, a cyclic peptide having a cyclic structure formed by a disulfide bond, containing or composed of an amino acid sequence selected from the following (j) to (n):
(j) GGLYACHMGPMTWVCQPLRG (SEQ ID NO: 65);
(k) CISWNEFNSPNWRFITC (SEQ ID NO: 66);
(1) a peptide in which one or two amino acid residues other than a cysteine residue are substituted, deleted, or added in the amino acid sequence of (j) or (k), and which binds to an erythropoietin receptor;
(m) a peptide which is composed of an amino acid sequence having 90% or more sequence identity with either the amino acid sequence of (j) or (k), but having a cysteine residue at the same position in (j) or (k), and which binds to an erythropoietin receptor. For example, the sequence identity may be 92% or higher, 95% or higher, or 98% or higher.
(n) a peptide in which at least one amino acid other than the cysteine residue in any of the amino acid sequences (j) to (m) is modified. Here, the amino acid modification is phosphorylation, methylation, acetylation, adenylylation, ADP-ribosylation, or glycosylation.

In an embodiment of the peptide molecule of the present invention, as a specific example, the first physiologically active peptide portion and the second physiologically active peptide portion are each composed of a physiologically active peptide that binds to a thrombopoietin receptor and may be the same as or different from each other. The physiologically active peptide that binds to a thrombopoietin receptor includes, but is not limited to, a peptide containing or composed of an amino acid sequence selected from the following (o) to (u):
(o) IEGPTLRQWLAARA (SEQ ID NO: 67);
(p) GGCADGPTLREWISFCGG (SEQ ID NO: 68);
(q) GGCTLREWLHGGFCGG (SEQ ID NO: 69);
(r) LAIEGPTLRQWLHGNGRDT (SEQ ID NO: 70);
(s) a peptide in which one or two amino acid residues other than a cysteine residue are substituted, deleted, or added in any of the amino acid sequences (o) to (r), and which binds to a thrombopoietin receptor;
(t) a peptide which is composed of an amino acid sequence having 90% or more sequence identity with any of the amino acid sequences (o) to (r), but having a cysteine residue at the same position in (p) or (q) when based on (p) or (q), and which binds to a thrombopoietin receptor. For example, the sequence identity may be 92% or higher, 95% or higher, or 98% or higher.
(u) a peptide in which at least one amino acid other than the cysteine residue in any of the amino acid sequences (o) to (t) is modified. Here, the amino acid modification is phosphorylation, methylation, acetylation, adenylylation, ADP-ribosylation, or glycosylation.

The peptides set forth in SEQ ID NO: 68 and SEQ ID NO: 69 are each a cyclic peptide having a cyclic structure formed by a disulfide bond.

In an embodiment of the peptide molecule of the present invention, as a specific example, the first physiologically active peptide portion and the second physiologically active peptide portion each constitute a cyclic peptide that binds to a vascular endothelial growth factor receptor (VEGFR) and may be the same or different from each other. The cyclic peptide that binds to VEGFR includes, but is not limited to, a cyclic peptide having a cyclic structure formed by a disulfide bond, containing or composed of an amino acid sequence selected from the following (aa) to (ff):
(aa) AGPTWCEDDWYYCWLFGT (SEQ ID NO: 71);
(bb) VCWEDSWGGEVCWLFGT (SEQ ID NO: 72);
(cc) VCWEDSWGGEVCFRYDP (SEQ ID NO: 73);
(dd) a peptide in which one or two amino acid residues other than a cysteine residue are substituted, deleted, or added in any of the amino acid sequences (aa) to (cc), and which binds to VEGFR.
(ee) a peptide which is composed of an amino acid sequence having 90% or more sequence identity with any of the amino acid sequences of (aa) to (cc), but having a cysteine residue at the same position in (aa) or (cc), and which binds to VEGFR. For example, the sequence identity may be 92% or higher, 95% or higher, or 98% or higher.
(ff) a peptide in which at least one amino acid other than the cysteine residue in any of the amino acid sequences (aa) to (ee) is modified. Here, the amino acid modification is phosphorylation, methylation, acetylation, adenylylation, ADP-ribosylation, or glycosylation.

In an embodiment of the peptide molecule of the present invention, the linker portion may have a molecular weight of 10,000 or less.

In an embodiment of the peptide molecule of the present invention, for the linker portion, at least 90%, at least 95%, or 100% of the amino acid sequence thereof may be composed of amino acid residues selected from alanine (A), proline (P) and serine (S). The proline residues may constitute more than 4% and less than 40% in the amino acid sequence of the linker portion. For example, in the amino acid sequence of the linker portion, the proline residues may constitute more than about 4%, more than about 5%, more than about 6%, more than about 8%, more than about 10%, more than about 15%, or more than about 20%, and may constitute less than about 40% or less than about 35%. Further, the alanine residues and the serine residue may each independently constitute more than about 4%, more than about 10%, or more than about 20%, and may constitute less than about 50%.

As used in the present specification, the term "about X%" is not limited to the number X, but also includes values of additional 10% to 20% more residues, or 10% to 20% less residues. For example, the term "about 10%" also relates to 11% or 12% and 9% or 8%.

In the amino acid sequence of the linker portion, amino acid residues different from alanine, serine, and proline may be selected from, for example, the group consisting of Arg, Asn, Asp, Cys, Gln, Glu, Gly, His, Ile, Leu, Lys, Met, Phe, Thr, Trp, Tyr, and Val as well as ones obtained by modifying these amino acids and ones obtained by adding functional groups or protecting groups to these amino acids (such as chloroacetylated amino acids, homocysteine, mercapto norvaline, mercapto norleucine, 2-amino-7-mercaptoheptanoic acid, and 2-amino-8-mercaptooctanoic acid, as well as amino acids whose protecting groups have been deprotected after the SH groups of these amino acids have been protected once, propargylglycine, homopropargylglycine, 2-amino-6-heptynoic acid, 2-amino-7-octynoic acid, 2-amino-8-nonynoic acid, 4-pentynoylated and 5-hexynoylated amino acids, azidoalanine, 2-amino-4-azidobutanoic acid, azidoptonorvaline, azidonorleucine, 2-amino-7-azidoheptanoic acid, 2-amino-8-azidooctanoic acid, azidoacetylated and 3-azidopentanoylated amino acids, N-(4-aminomethyl-benzoyl)-phenylalanine (AMBF), 4-3-aminomethyltyrosine, 5-hydroxytryptophan (WOH), N-3-chloromethylbenzoyl-L-phenylalanine, N-3-chloromethylbenzoyl-L-tyrosine, N-3-chloromethylbenzoyl-L-tryptophane). Further, each amino acid may be an L-amino acid or a D-amino acid. Such amino acid residues different from alanine, serine, and proline are preferably ones without a hydrophobic side chain such as Val, Ile, Leu, Met, Phe, Tyr, or Trp, and/or ones without a charged side chain such as Lys, Arg, Asp, or Glu.

In an embodiment of the peptide molecule of the present invention, for the linker portion, at least 90%, at least 95%, or 100% of the amino acid sequence thereof may be composed of amino acid residues selected from alanine (A) and proline (P). In this case, proline residues may constitute more than 10% and less than 75% in the amino acid sequence of the linker portion. For example, in the amino acid sequence of the linker portion, the proline residues may constitute more than about 10%, more than about 12%, more than about 14%, more than about 18%, more than about 20%, more than about 22%, more than about 23%, more than about 24%, or more than about 25%, and may constitute less than about 75%, less than about 70%, less than about 65%, less than about 60%, less than about 55%, less than about 50%, less than about 48%, less than about 46%, less than about 44%, less than about 42%, less than about 41%, less than about 40%, less than about 39%, less than about 38%, less than about 37%, less than about 36%, or less than about 35%. Further, the alanine residues may constitute more than about 25%, more than about 30%, more than about 35%, more than about 40%, more than about 45%, more than about 50%, more than about 52%, more than about 54%, more than about 56%, more than about 58%, more than about 59%, more than about 60%, more than about 61%, more than about 62%, more than about 63%, more than about 64%, or more than about 65%, and may constitute less than about 90%, less than about 88%, less than about 86%, less than about 84%, less than about 82%, less than about 80%, less than about 79%, less than about 78%, less than about 77%, less than about 76%, or less than about 75%.

In the amino acid sequence of the linker portion, the amino acid residues different from alanine, serine and proline may be selected from the group consisting of Arg, Asn, Asp, Cys, Gln, Glu, Gly, His, Ile, Leu, Lys, Met, Phe, Thr, Trp, Tyr, and Val. Such amino acid residues different from alanine, serine, and proline are preferably ones without a hydrophobic side chain such as Val, Ile, Leu, Met, Phe, Tyr, or Trp, and/or ones without a charged side chain such as Lys, Arg, Asp, or Glu.

In an embodiment of the peptide molecule of the present invention, the linker portion contains multiple amino acid repeats, the amino acid repeats are composed of alanine (A), proline (P), and serine (S), and in the amino acid repeats, the number of consecutive identical amino acid residues may be 6 residues or less, 5 residues or less, 4 residues or less, or 3 residues or less.

In an embodiment of the peptide molecule of the present invention, the linker portion contains multiple amino acid repeats, the amino acid repeats are composed of alanine (A) and proline (P), and in the amino acid repeats, the number of consecutive identical amino acid residues may be 6 residues or less, 5 residues or less, 4 residues or less, or 3 residues or less.

In an embodiment of the peptide molecule of the present invention, the linker portion may repeatedly contain two or more amino acid sequences selected from the group consisting of AP, AAP, AS, ASP, and ASS.

In an embodiment of the peptide molecule of the present invention, the linker portion may be composed of, for example, 5 or more, 6 or more, 7 or more, or 8 or more amino acid residues.

In an embodiment of the peptide molecule of the present invention, specific examples of the linker portion include, but are not limited to, those containing the following amino acid sequences and those consisting of the following amino acid sequences. An amino acid sequence selected from AAPAAPAP (SEQ ID NO: 74), AAPAAPAPAAPAAPAP (SEQ ID NO: 75), AAPAAPAPAAPAAPAPAAPAAPAPAAPAAPAPAAPAAPAPAAPAAPAPA (SEQ ID NO: 76), SAPSPSSAPASASAPASPASASASASPASSPASASSASPAASSASPASS (SEQ ID NO: 77), AAPAAPAPAAPAAPAPAAPA (SEQ ID NO: 7), AAPAAPAPAAPAAPAPAAPAAP (SEQ ID NO: 8), AAPAAPAPAAPAAP (SEQ ID NO: 9), AAPAAAPAPAAPAAPAPAAP (SEQ ID NO 10), AAAPAAAPAAAPAAAPAAAP (SEQ ID NO 11), AAPAAPAAPAAPAAPAAPAAPAAP (SEQ ID NO 12), APAAAPAPAAAPAPAAAPAPAAAP (SEQ ID NO 13), AAAPAAPAAPPAAAAPAAPAAPPA (SEQ ID NO 14), and APAPAPAPAPAPAPAPAPAP (SEQ ID NO 15), ASPAAPAPASPAAPAPSAPA (SEQ ID NO 16), AAPASPAPAAPSAPAPAAPS (SEQ ID NO 17), APSSPSPSAPSSPSPASPSS (SEQ ID NO 18), SAPSSPSPSAPSSPSPASPS (SEQ ID NO 19), SSPSAPSPSSPASPSPSSPA (SEQ ID NO 20), AASPAAPSAPPAAASPAAPSAPPA (SEQ ID NO 21), and ASAAAPAAASAAASAPSAAA (SEQ ID NO 22), as well as circular permutation mutant sequences thereof.

Taking AAPAAPAPAAPAAPAPAAPA (SEQ ID NO: 7) as an example, a circular permutation mutant sequence may be easily prepared, for example, by removing the first alanine of the above sequence and adding another alanine to the terminus of the above sequence. Such a circular permutation mutant sequence for SEQ ID NO: 7 is APAAPAPAAPAAPAPAAPAA (SEQ ID NO: 23). Furthermore, non-limited examples of the circular permutation mutant sequence with circularly permutated amino acid residues for SEQ ID NO: 7 include the following. PAAPAPAAPAAPAPAAPAAA (SEQ ID NO: 24), AAPAPAAPAAPAPAAPAAAP (SEQ ID NO: 25), APAPAAPAAPAPAAPAAAPA (SEQ ID NO: 26), PAPAAPAAPAPAAPAAAPAA (SEQ ID NO: 27), APAAPAAPAPAAPAAAPAAP (SEQ ID NO: 28), PAAPAAPAPAAPAAAPAAPA (SEQ ID NO: 29), AAPAAPAPAAPAAAPAAPAP (SEQ ID NO: 30), APAAPAPAAPAAAPAAPAPA (SEQ ID NO: 31), and PAAPAPAAPAAAPAAPAPAA (SEQ ID NO: 32).

In an embodiment of the peptide molecule of the present invention, the peptide molecule may further contain a functional modification portion at a position not adjacent to the linker portion.

As used in the present specification, the term "functional modification portion" means a portion in a peptide molecule that imparts some function to the peptide molecule in addition to the functions of the physiologically active peptide, and is preferably composed of an amino acid sequence. Examples of the functional modification portion include, but are not limited to, a stabilized sequence, a water-solubility improved sequence, an enzyme identification sequence, a tag sequence, a target binding sequence, and the like.

Specific examples of the stabilized sequence or water-solubility improved sequence include, but are not limited to, a random coil polypeptide containing an amino acid sequence composed of at least 50 proline and alanine amino acid residues as described in Published Japanese Translation of PCT International Application No. 2013-531480 (cited herein by reference to the content of that document as constituting part of the present specification), a random coil polypeptide containing an amino acid sequence composed of proline, alanine, and serine amino acid residues as described in Published Japanese Translation of PCT International Application No. 2010-531139 (cited herein by reference to the content of that document as constituting part of the present specification), and the like.

Specific examples of the enzyme identification sequence include, but are not limited to, SortaseA identification signal, Butelase identification signal, Transglutaminase identification signal, and the like.

Specific examples of the tag sequence include, but are not limited to, FLAG tags, PA tags, histidine tags, and the like.

Specific examples of the target binding sequence include, but are not limited to, small molecule antibodies such as VHH and scFv.

In an embodiment of the peptide molecule of the present invention, the functional modification portion may be composed of, for example, 3 or more, 6 or more, 10 or more, or 12 or more amino acid residues, and may be composed of 1000 or less, 500 or less, 300 or less, or 150 or less amino acid residues.

An embodiment of the present invention relates to a method for producing a peptide molecule. The peptide molecule produced by the production method of the present invention contains
(I) a first physiologically active peptide portion,
(II) a linker portion composed of an amino acid residue, and
(III) a second physiologically active peptide portion or an additional amino acid sequence composed of at least one amino acid residue, located on the opposite side of the linker portion from the first physiologically active peptide portion.

In an embodiment of the production method of the present invention, the peptide molecule may have, but is not limited to, a structure "first physiologically active peptide portion"-"linker portion"-"second physiologically active peptide portion" from the N-terminus side or a structure "first physiologically active peptide portion"-"linker portion"-"additional amino acid sequence" from the N-terminus side, for example. In addition, the peptide molecule may be configured to have two or more linker portions, such as "first physiologically active peptide portion"-"linker portion"-"second physiologically active peptide portion"-"linker portion"-"arbitrary sequence" from the N-terminus side (so as to link three or more additional amino acid sequences in tandem, composed of the first physiologically active peptide portion as well as the second physiologically active peptide portion and/or at least one amino acid residue, via two or more linker portions). Here, the arbitrary sequence may be a physiologically active peptide portion (may be the same as the first physiologically active peptide portion and/or second physiologically active peptide portion), or may be an additional amino acid sequence composed of at least one amino acid residue.

A specific example of such a produced peptide molecule may be the above-mentioned peptide molecule comprising:
(I) a first physiologically active peptide portion;
(II) a linker portion composed of 49 or less amino acid residues; and
(III) a second physiologically active peptide portion located on the opposite side of the linker portion from the first physiologically active peptide portion, wherein
   the first physiologically active peptide portion and the second physiologically active peptide portion may be the same as or different from each other, and
   at least 90% of an amino acid sequence of the linker portion is composed of amino acid residues selected from alanine (A), proline (P), and serine (S).

In the peptide molecule produced by the production method of the present invention, the first physiologically active peptide portion and second physiologically active peptide portion each may have a molecular weight of 10,000 or less and may be composed of 50 or less amino acid residues, and may be the same as or different from each other. Examples of the first physiologically active peptide portion and/or second physiologically active peptide portion include, but are not limited to, FGFR binding peptide, c-Met (HGFR) binding peptide, erythropoietin receptor binding peptide, thrombopoietin receptor binding peptide, albumin binding peptide, EGFR binding peptide, VEGFR binding peptide, PDGFR binding peptide, Axl binding peptide, PDGFR binding peptide, SCFR binding peptide, Flt-3 binding peptide, c-Ret binding peptide, ROR binding peptide, Tie binding peptide, NGFR binding peptide, Insulin receptor binding peptide, EphR binding peptide, Alk binding peptide, DDR binding peptide, TGFBR binding peptide, Activin receptor binding peptide, BMP receptor binding peptide, interleukin receptor binding peptide, T-cell receptor binding peptide, transferrin receptor binding peptide, lipoprotein receptor binding peptide, ubiquitin ligase binding peptide, antibody binding peptide, complement binding peptide, GPCR binding peptide, ion channel binding peptide, virus binding peptide, and the like.

In an embodiment of the production method of the present invention, the molecular weights of the first physiologically active peptide portion and second physiologically active peptide portion may be, for example, 9,000 or less, 8,000 or less, 7,000 or less, 6,000 or less, 5,000 or less, or 4,000 or less, and 250 or more, 500 or more, 800 or more, or 1,000 or more.

In an embodiment of the production method of the present invention, the lengths of the first physiologically active peptide portion and second physiologically active peptide portion may be, for example, each independently composed of 45 or less, 40 or less, 35 or less, 30 or less, 25 or less, or 20 or less amino acid residues, and may be composed of 3 or more, 6 or more, 10 or more, or 12 or more amino acid residues.

In an embodiment of the production method of the present invention, the first physiologically active peptide portion and/or second physiologically active peptide portion may be a straight chain or a branched chain, and may constitute a cyclic peptide. The cyclic peptide in the first physiologically active peptide portion and/or second physiologically active peptide portion is not particularly limited in what kind of binding mode it has a cyclic structure, and it can form a cyclic structure, for example, by a disulfide bond between two cysteine residues, by a thioether bond (bond between a thiol group and a haloalkyl group), or by a bond of 1,2,3-triazole (bond between an azido group and an alkyne group). Note that the disulfide bond may be formed spontaneously inside or outside cells by culturing the cells, or by an enzymatic reaction by the cells.

In an embodiment of the production method of the present invention, the first physiologically active peptide portion may contain two cysteine residues, or at least two cysteine residues.

In an embodiment of the production method of the present invention, the second physiologically active peptide portion may contain two cysteine residues, or at least two cysteine residues.

It is considered that various cyclic peptides exist, but what kind of cyclic peptide is constituted in the first physiologically active peptide portion and/or second physiologically active peptide portion is not particularly limited.

In an embodiment of the production method of the present invention, when the peptide molecule is a dimer peptide, there may be one having higher physiological activity than a monomer peptide. For example, if the peptide molecule produced by the production method of the present invention is one obtained by binding two FGFR-binding cyclic peptides via a linker portion, it may have higher FGFR inhibitory activity than the case of one FGFR-binding cyclic peptide.

Specific examples of the cyclic peptide include cyclic peptides that bind to c-Met (also referred to simply as Met), but the cyclic peptide is not limited to this in the peptide molecule obtained by the production method of the present invention.
cyclic peptide having a cyclic structure formed by a disulfide bond, containing or composed of an amino acid sequence selected from the following (a) to (i):
(a) CYRQFNRRTHEVWNLDC (SEQ ID NO: 1);
(b) CRQFNRRTHEVWNLDC (SEQ ID NO: 2);
(c) CYWYYAWDQTYKAFPC (SEQ ID NO: 3);
(d) CWYYAWDQTYKAFPC (SEQ ID NO: 4);
(e) CYISWNEFNSPNWRFITC (SEQ ID NO: 5);
(f) CISWNEFNSPNWRFITC (SEQ ID NO: 6);
(g) a peptide in which one or two amino acid residues other than a cysteine residue are substituted, deleted, or added in any of the amino acid sequences (a) to (f), and which binds to a c-Met protein;
(h) a peptide which is composed of an amino acid sequence having 90% or more sequence identity with any of the amino acid sequences (a) to (f), but having cysteine residues at both ends, and which binds to a c-Met protein. For example, the sequence identity may be 92% or higher, 95% or higher, or 98% or higher. In the present specification, the amino acid sequence homology may be calculated by using a known analysis tool, and may be calculated, for example, by using the National Center for Biotechnology Information (NCBI) homology algorithm BLAST (Basic local alignment search tool). In addition, the default (initial setting) parameters in the analysis tool may be used to calculate the amino acid sequence homology.
(i) A peptide in which at least one amino acid other than the cysteine residues at both ends in any of the amino acid sequences (a) to (h) is modified. Here, the amino acid modification is phosphorylation, methylation, acetylation, adenylylation, ADP-ribosylation, or glycosylation.

Note that it is known Met is an HGF receptor, a tyrosine kinase type receptor, and a cyclic peptide that binds to such Met (HGFR) functions as a Met (HGFR) agonist by dimerization.

In an embodiment of the production method of the present invention, the first physiologically active peptide portion and/or second physiologically active peptide portion of the peptide molecule produced may constitute a cyclic peptide that binds to an erythropoietin receptor. If both the first physiologically active peptide portion and the second physiologically active peptide portion constitute a cyclic peptide that binds to an erythropoietin receptor, the cyclic peptides may be the same as or different from each other. The cyclic peptide that binds to an erythropoietin receptor includes, but is not limited to, a cyclic peptide having a cyclic structure formed by a disulfide bond, containing or composed of an amino acid sequence selected from the following (j) to (n).
(j) GGLYACHMGPMTWVCQPLRG (SEQ ID NO: 65);
(k) CISWNEFNSPNWRFITC (SEQ ID NO: 66);
(1) a peptide in which one or two amino acid residues other than a cysteine residue are substituted, deleted, or added in the amino acid sequence of (j) or (k), and which binds to an erythropoietin receptor;
(m) a peptide which is composed of an amino acid sequence having 90% or more sequence identity with either the amino acid sequence of (j) or (k), but having a cysteine residue at the same position in (j) or (k), and which binds to an erythropoietin receptor. For example, the sequence identity may be 92% or higher, 95% or higher, or 98% or higher.
(n) A peptide in which at least one amino acid other than the cysteine residue in any of the amino acid sequences (j) to (m) is modified. Here, the amino acid modification is phosphorylation, methylation, acetylation, adenylylation, ADP-ribosylation, or glycosylation.

In an embodiment of the production method of the present invention, the first physiologically active peptide portion and/or second physiologically active peptide portion of the peptide molecule produced may be composed of a physiologically active peptide that binds to a thrombopoietin receptor. If both the first physiologically active peptide portion and the second physiologically active peptide portion are composed of a physiologically active peptide that binds to a thrombopoietin receptor, the physiologically active peptides may be the same as or different from each other. The physiologically active peptide that binds to a thrombopoietin receptor includes, but is not limited to, a peptide containing or composed of an amino acid sequence selected from the following (o) to (u).
(o) IEGPTLRQWLAARA (SEQ ID NO: 67);
(p) GGCADGPTLREWISFCGG (SEQ ID NO: 68);
(q) GGCTLREWLHGGFCGG (SEQ ID NO: 69);
(r) LAIEGPTLRQWLHGNGRDT (SEQ ID NO: 70);
(s) a peptide in which one or two amino acid residues other than a cysteine residue are substituted, deleted, or added in any of the amino acid sequences (o) to (r), and which binds to a thrombopoietin receptor;
(t) a peptide which is composed of an amino acid sequence having 90% or more sequence identity with any of the amino acid sequences (o) to (r), but having a cysteine residue at the same position in (p) or (q) when based on (p) or (q), and which binds to a thrombopoietin receptor. For example, the sequence identity may be 92% or higher, 95% or higher, or 98% or higher.
(u) A peptide in which at least one amino acid other than the cysteine residue in any of the amino acid sequences (o) to (t) is modified. Here, the amino acid modification is phosphorylation, methylation, acetylation, adenylylation, ADP-ribosylation, or glycosylation.

The peptides set forth in SEQ ID NO: 68 and SEQ ID NO: 69 are each a cyclic peptide having a cyclic structure formed by a disulfide bond.

In an embodiment of the production method of the present invention, the first physiologically active peptide portion and/or second physiologically active peptide portion of the peptide molecule produced may constitute a cyclic peptide that binds to a vascular endothelial growth factor receptor (VEGFR). If both the first physiologically active peptide portion and the second physiologically active peptide portion constitute a cyclic peptide that binds to VEGFR, the cyclic peptides may be the same as or different from each other. The cyclic peptide that binds to VEGFR includes, but is not limited to, a cyclic peptide having a cyclic structure formed by a disulfide bond, containing or composed of an amino acid sequence selected from the following (aa) to (ff).
(aa) AGPTWCEDDWYYCWLFGT (SEQ ID NO: 71);
(bb) VCWEDSWGGEVCWLFGT (SEQ ID NO: 72);
(cc) VCWEDSWGGEVCFRYDP (SEQ ID NO: 73);
(dd) a peptide in which one or two amino acid residues other than a cysteine residue are substituted, deleted, or added in any of the amino acid sequences (aa) to (cc), and which binds to VEGFR;
(ee) a peptide which is composed of an amino acid sequence having 90% or more sequence identity with any of the amino acid sequences of (aa) to (cc), but having a cysteine residue at the same position in (aa) or (cc), and which binds to VEGFR. For example, the sequence identity may be 92% or higher, 95% or higher, or 98% or higher.
(ff) A peptide in which at least one amino acid other than the cysteine residue in any of the amino acid sequences (aa) to (ee) is modified. Here, the amino acid modification is phosphorylation, methylation, acetylation, adenylylation, ADP-ribosylation, or glycosylation.

In an embodiment of the production method of the present invention, the linker portion has a molecular weight of 10,000 or less and may be composed of 50 or less amino acid residues.

Further, in an embodiment of the production method of the present invention, the linker portion may be a linker portion having a molecular weight of 10,000 or less and composed of 50 or less or 49 or less amino acid residues, and at least 90%, at least 95%, or at least 100% of the amino acid sequence is composed of amino acid residues selected from alanine (A), proline (P), and serine (S). In this case, the proline residues may constitute more than 4% and less than 40% in the amino acid sequence of the linker portion. For example, in the amino acid sequence of the linker portion, the proline residues may constitute more than about 4%, more than about 5%, more than about 6%, more than about 8%, more than about 10%, more than about 15%, or more than about 20%, and may constitute less than about 40% or less than about 35%. Further, the alanine residues and the serine residue may each independently constitute more than about 4%, more than about 10%, or more than about 20%, and may constitute less than about 50%.

As used in the present specification, the term "about X%" is not limited to a concise number of percentages, but also includes values of additional 10% to 20% more residues, or 10% to 20% less residues. For example, the term "about 10%" also relates to 11% or 12% and 9% or 8%.

In the amino acid sequence of the linker portion, amino acid residues different from alanine, serine, and proline may be selected from the group consisting of Arg, Asn, Asp, Cys, Gln, Glu, Gly, His, Ile, Leu, Lys, Met, Phe, Thr, Trp, Tyr, and Val. Such amino acid residues different from alanine, serine, and proline are preferably ones without a hydrophobic side chain such as Val, Ile, Leu, Met, Phe, Tyr, or Trp, and/or ones without a charged side chain such as Lys, Arg, Asp, or Glu.

In an embodiment of the production method of the present invention, the linker portion may be a linker portion having a molecular weight of 10,000 or less and composed of 50 or less or 49 or less amino acid residues, and at least 90%, at least 95%, or at least 100% of the amino acid sequence is composed of amino acid residues selected from alanine (A) and proline (P). In this case, proline residues may constitute more than 10% and less than 75% in the amino acid sequence of the linker portion. For example, in the amino acid sequence of the linker portion, the proline residues may constitute more than about 10%, more than about 12%, more than about 14%, more than about 18%, more than about 20%, more than about 22%, more than about 23%, more than about 24%, or more than about 25%, and may constitute less than about 75%, less than about 70%, less than about 65%, less than about 60%, less than about 55%, less than about 50%, less than about 48%, less than about 46%, less than about 44%, less than about 42%, less than about 41%, less than about 40%, less than about 39%, less than about 38%, less than about 37%, less than about 36%, or less than about 35%. Further, the alanine residues may constitute more than about 25%, more than about 30%, more than about 35%, more than about 40%, more than about 45%, more than about 50%, more than about 52%, more than about 54%, more than about 56%, more than about 58%, more than about 59%, more than about 60%, more than about 61%, more than about 62%, more than about 63%, more than about 64%, or more than about 65%, and may constitute less than about 90%, less than about 88%, less than about 86%, less than about 84%, less than about 82%, less than about 80%, less than about 79%, less than about 78%, less than about 77%, less than about 76%, or less than about 75%.

In the amino acid sequence of the linker portion, the amino acid residues different from alanine, serine and proline may be selected from the group consisting of Arg, Asn, Asp, Cys, Gln, Glu, Gly, His, Ile, Leu, Lys, Met, Phe, Thr, Trp, Tyr, and Val. Such amino acid residues different from alanine, serine, and proline are preferably ones without a hydrophobic side chain such as Val, Ile, Leu, Met, Phe, Tyr, or Trp, and/or ones without a charged side chain such as Lys, Arg, Asp, or Glu.

In an embodiment of the production method of the present invention, the linker portion contains multiple amino acid repeats, the amino acid repeats are composed of alanine (A), proline (P), and serine (S), and in the amino acid repeats, the number of consecutive identical amino acid residues may be 6 residues or less, 5 residues or less, 4 residues or less, or 3 residues or less.

In an embodiment of the production method of the present invention, the linker portion contains multiple amino acid repeats, the amino acid repeats are composed of alanine (A) and proline (P), and in the amino acid repeats, the number of consecutive identical amino acid residues may be 6 residues or less, 5 residues or less, 4 residues or less, or 3 residues or less.

In an embodiment of the production method of the present invention, the linker portion may repeatedly contain two or more amino acid sequences selected from the group consisting of AP, AAP, AS, ASP, and ASS.

In an embodiment of the production method of the present invention, the linker portion may contain a sequence composed of glycine (G) and serine (S). In this case, the linker portion may repeatedly contain an amino acid sequence selected from the group consisting of GGGGS, GGGS, GGS, and GS.

In an embodiment of the production method of the present invention, the linker portion may repeatedly contain glycine or may be composed solely of glycine.

In an embodiment of the production method of the present invention, the linker portion may repeatedly contain the amino acid sequence represented by EAAAK

In an embodiment of the production method of the present invention, the amino acid sequence of the linker portion is preferably, but is not limited to, one composed of A, P, and S, one composed of A and P, or one composed of a repetition of the sequence represented by GGGGS.

In an embodiment of the production method of the present invention, the length of the linker portion may be composed of 50 or less amino acid residues, and for example, the linker portion may be composed of 49 or less, 40 or less, 35 or less, 30 or less, or 25 or less amino acid residues.

In an embodiment of the production method of the present invention, the linker portion may be composed of 1 or more amino acid residues, and for example, the linker portion may be composed of 4 or more, 8 or more, 12 or more, or 16 or more amino acid residues.

In an embodiment of the production method of the present invention, specific examples of the linker portion include, but are not limited to, those containing the following amino acid sequences and those consisting of the following amino acid sequences. An amino acid sequence selected from AAPAAPAP (SEQ ID NO: 74), AAPAAPAPAAPAAPAP (SEQ ID NO: 75), AAPAAPAPAAPAAPAPAAPAAPAPAAPAAPAPAAPAAPAPAAPAAPAPA (SEQ ID NO: 76), SAPSPSSAPASASAPASPASASASASPASSPASASSASPAASSASPASS (SEQ ID NO: 77), AAPAAPAPAAPAAPAPAAPAAPAPAAPAAPAPAAPAAPAPAAPAAPAPAAPAAPAP AAPAAPAPAAPAAPAPAAPAAPAPAAPAAPAPAAPAAPAPAAPA (SEQ ID NO: 78), SAPSPSSAPASASAPASPASASASASPASSPASASSASPAASSASPASSSASPSAPAA PSASAAPSASPASSSPAPASAPSPAAAASSPSPAPSSASSAA (SEQ ID NO: 79), AAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAP (SEQ ID NO: 80), AAPAAPAPAAPAAPAPAAPA (SEQ ID NO: 7), AAPAAPAPAAPAAPAPAAPAAP (SEQ ID NO: 8), AAPAAPAPAAPAAP (SEQ ID NO: 9), AAPAAAPAPAAPAAPAPAAP (SEQ ID NO 10), AAAPAAAPAAAPAAAPAAAP (SEQ ID NO 11), AAPAAPAAPAAPAAPAAPAAPAAP (SEQ ID NO 12), APAAAPAPAAAPAPAAAPAPAAAP (SEQ ID NO 13), AAAPAAPAAPPAAAAPAAPAAPPA (SEQ ID NO 14), and APAPAPAPAPAPAPAPAPAP (SEQ ID NO 15), ASPAAPAPASPAAPAPSAPA (SEQ ID NO 16), AAPASPAPAAPSAPAPAAPS (SEQ ID NO 17), APSSPSPSAPSSPSPASPSS (SEQ ID NO 18), SAPSSPSPSAPSSPSPASPS (SEQ ID NO 19), SSPSAPSPSSPASPSPSSPA (SEQ ID NO 20), AASPAAPSAPPAAASPAAPSAPPA (SEQ ID NO 21), and ASAAAPAAASAAASAPSAAA (SEQ ID NO 22), as well as circular permutation mutant sequences thereof.

Taking AAPAAPAPAAPAAPAPAAPA (SEQ ID NO: 7) as an example, a circular permutation mutant sequence may be easily prepared, for example, by removing the first alanine of the above sequence and adding another alanine to the terminus of the above sequence. Such a circular permutation mutant sequence for SEQ ID NO: 7 is APAAPAPAAPAAPAPAAPAA (SEQ ID NO: 23). Furthermore, non-limited examples of the circular permutation mutant sequence with circularly permutated amino acid residues for SEQ ID NO: 7 include the following. PAAPAPAAPAAPAPAAPAAA (SEQ ID NO: 24), AAPAPAAPAAPAPAAPAAAP (SEQ ID NO: 25), APAPAAPAAPAPAAPAAAPA (SEQ ID NO: 26), PAPAAPAAPAPAAPAAAPAA (SEQ ID NO: 27), APAAPAAPAPAAPAAAPAAP (SEQ ID NO: 28), PAAPAAPAPAAPAAAPAAPA (SEQ ID NO: 29), AAPAAPAPAAPAAAPAAPAP (SEQ ID NO: 30), APAAPAPAAPAAAPAAPAPA (SEQ ID NO: 31), and PAAPAPAAPAAAPAAPAPAA (SEQ ID NO: 32).

In an embodiment of the production method of the present invention, the peptide molecule produced by the production method of the present invention may contain an additional amino acid sequence composed of at least one amino acid residue on the opposite side of the linker portion from the first physiologically active peptide portion. This additional amino acid sequence has a molecular weight of 10,000 or less and is composed of 50 or less amino acid residues. Such additional amino acid sequences include enzyme identification sequences such as SortaseA identification signal and Butelase identification signal, tag sequences such as FLAG tag, PA tag, and histidine tag, reactive amino acids such as lysine, cysteine, glutamic acid, and aspartic acid, or sequences containing the reactive amino acids.

If the peptide molecule produced by the production method of the present invention contains an additional amino acid sequence composed of at least one amino acid residue on the opposite side of the linker portion from the first physiologically active peptide portion, and the additional amino acid sequence is a sequence composed of a reactive amino acid or containing a reactive amino acid, the peptide molecule may be used as a conjugate reagent for preparing a physiologically active peptide-drug conjugate or the like.

The peptide molecule produced by the production method of the present invention may contain a second physiologically active peptide portion and an additional amino acid sequence composed of at least one amino acid residue, on the opposite side of the linker portion from the first physiologically active peptide portion.

The peptide molecule produced by the production method of the present invention may contain a first physiologically active peptide portion on the N-terminus side of the linker portion and a second physiologically active peptide portion and/or an additional amino acid sequence on the C-terminus side of the linker portion. Further, the peptide molecule produced by the production method of the present invention may contain a first physiologically active peptide portion on the C-terminus side of the linker portion and a second physiologically active peptide portion and/or an additional amino acid sequence on the N-terminus side of the linker portion. Moreover, the peptide molecule produced by the production method of the present invention may contain an arbitrary amino acid sequence as long as it does not inhibit the functions of the first physiologically active peptide and second physiologically active peptide on the N-terminus side and/or the C-terminus side of the first physiologically active peptide portion and/or second physiologically active peptide portion, or in the space with the linker portion. As such an arbitrary amino acid sequence, for example, a sequence useful for enhancing the expression or secretion of peptide molecules in a host cell may be used.

In an embodiment of the production method of the present invention, the peptide molecule produced may further contain a functional modification portion at a position not adjacent to the linker portion. The functional modification portion is as described above.

The production method of the present invention includes the step of producing the peptide molecule by expressing a polynucleotide in a host cell containing the polynucleotide encoding the peptide molecule or by expressing a polynucleotide encoding the peptide molecule in a cell-free expression system. Host cells containing polynucleotides encoding the peptide molecule may be prepared by known techniques. For example, a host cell containing a polynucleotide encoding the peptide molecule may be prepared by, e.g., introducing a recombinant vector containing the polynucleotide into the host cell.

In an embodiment of the production method of the present invention, examples of host cells include, but are not limited to, prokaryotic cells (bacteria) such as genus Escherichia (such as Escherichia coli) and genus Corynebacterium (such as Corynebacterium glutamicum), fungal cells (fungi) such as yeast, plant cells, insect cells, and animal cells. As for the bacteria of the genus Corynebacterium as host cells, the ones described in WO2016/171224 can be used.

In an embodiment of the production method of the present invention, expression of the polynucleotide in a host cell may be transient or stable. The conditions for culturing the host cells may be appropriately selected by those skilled in the art based on common general technical knowledge according to the type of host cells used. For example, the temperature, humidity, carbon dioxide concentration, and other conditions of the culture environment may be such that polynucleotide expression is possible in the host cells, and the culture medium used is not limited to ones suitable for each host cell and may be selected from conventionally known ones.

In an embodiment of the production method of the present invention, the synthesis of the peptide molecule in the cell-free expression system may be carried out using a cell extract or using a commercially available cell-free expression reagent, and may be carried out based on a technique known to those skilled in the art. The components contained in the cell-free expression system include nucleic acids encoding the desired polypeptide as a template (DNA or mRNA), proteins required for transcription, translation, energy regeneration, and post-translational modification (such as RNA polymerase, ribosome, aminoacyl-tRNA synthetase, translation initiation factor, translation elongation factor, translation termination factor, ribosome recycling factor, nucleoside diphosphate kinase, adenosine kinase, creatine kinase, prolyl isomerase, disulfide-binding isomerase, and chaperone), tRNA, aminoacyl tRNA, natural amino acids, unnatural amino acids, NTP, buffer, and the like.

Examples of commercially available cell-free expression reagents include PUREfrex 2.0 (GeneFrontier Corporation) and PURExpress In Vitro Protein Synthesis Kit (New England BioLabs).

In producing a peptide molecule, the production method of the present invention may include the steps of introducing into a host cell a polynucleotide encoding a peptide molecule or a recombinant vector containing the polynucleotide, and expressing the polynucleotide in the host cell to generate a peptide molecule. The introduction means in the introduction step may be appropriately selected by those skilled in the art based on common general technical knowledge according to the type of host cells used. The vector usable may be one capable of functionally inserting a polynucleotide encoding a peptide molecule and carrying the fragments into the host cells to result in replication and/or expression of heterologous nucleic acid fragments in the host cells, and examples thereof include plasmids, phages, cosmids, and viruses. The vector can be appropriately selected by those skilled in the art according to the type of host cells used.

In an embodiment of the production method of the present invention, a polynucleotide encoding the peptide molecule may be appropriately synthesized by those skilled in the art using a conventional polynucleotide synthesis technique based on the amino acid sequence of that peptide molecule. Examples of the polynucleotide encoding the peptide molecule include one encoding a peptide molecule having a structure "first physiologically active peptide portion"-"linker portion"-"second physiologically active peptide portion" or "first physiologically active peptide portion"-"linker portion"-"additional amino acid sequence". Peptide molecules may be easily and inexpensively produced from such polynucleotides.

In an embodiment of the production method of the present invention, the polynucleotide encoding the peptide molecule may appropriately contain a promoter sequence or a signal sequence on the N-terminus side thereof. In addition, the polynucleotide may contain a restriction enzyme recognition sequence on the N-terminus side and/or the C-terminus side. Such promoter sequences, signal sequences, and restriction enzyme recognition sequences may be appropriately selected by those skilled in the art according to the type of host cells or vectors used.

In an embodiment of the production method of the present invention, the produced peptide molecule may be appropriately isolated from host cells, and may be purified and/or recovered based on the common general technical knowledge of those skilled in the art.

In an embodiment of the production method of the present invention, the produced peptide molecule may be appropriately isolated after being secreted from host cells, and may be purified and/or recovered based on the common general technical knowledge of those skilled in the art.

In an embodiment of the production method of the present invention, a desired peptide molecule such as a dimer peptide may be expressed from a single polynucleotide chain, and a desired peptide molecule such as a dimer peptide may be produced easily and inexpensively as compared with conventional chemical synthesis methods.

In an embodiment of the production method of the present invention, if the first physiologically active peptide portion and/or second physiologically active peptide portion constitutes a cyclic peptide, a straight chain peptide molecule is generated from a single polynucleotide chain, and it is possible to construct a cyclic peptide from the straight chain peptide molecule in the host cells or after secretion from the host cells. Therefore, regarding the synthesis of a peptide molecule containing a cyclic peptide, the conventional chemical synthesis methods require a large number of steps resulting in complexity, but according to the production method of the present invention, a peptide molecule containing a cyclic peptide may be produced easily and inexpensively. Regarding the composition of the cyclic peptide, a cyclic structure may be formed by a disulfide bond between two cysteine residues, but the present invention is not limited thereto.

An embodiment of the present invention relates to a polynucleotide for encoding a peptide molecule. The peptide molecule produced by the expression of this polynucleotide may contain (I) a first physiologically active peptide portion, (II) a linker portion composed of an amino acid residue, and (III) a second physiologically active peptide portion and/or an additional amino acid sequence composed of at least one amino acid residue, located on the opposite side of the linker portion from the first physiologically active peptide portion. Here, the first physiologically active peptide portion and second physiologically active peptide portion may be the same as or different from each other. Such polynucleotides may be appropriately synthesized by those skilled in the art using conventional polynucleotide synthesis techniques based on the amino acid sequence of the peptide molecule produced.

An embodiment of the polynucleotide of the present invention may be similar to that described in the production method of the present invention described above.

In an embodiment of the polynucleotide of the present invention, the polynucleotide may be for use in the production method of the invention described above.

In an embodiment of the polynucleotide of the present invention, the first physiologically active peptide portion, the linker portion composed of amino acid residues, the second physiologically active peptide portion, and the additional amino acid sequence composed of at least one amino acid residue in the peptide molecule produced by the expression of the polynucleotide may be similar to that described in the production method of the present invention described above.

An embodiment of the present invention relates not only to the above-mentioned peptide molecules but also to the following peptide molecules. This peptide molecule may contain (I) a first physiologically active peptide portion, (II) a linker portion composed of an amino acid residue, and (III) a second physiologically active peptide portion located on the opposite side of the linker portion from the first physiologically active peptide portion.

In an embodiment of the peptide molecule of the present invention, it may contain a first physiologically active peptide portion on the N-terminus side of the linker portion and a second physiologically active peptide portion on the C-terminus side of the linker portion, or may contain a first physiologically active peptide portion on the C-terminus side of the linker portion and a second physiologically active peptide portion on the N-terminus side of the linker portion. Further, in an embodiment of the peptide molecule of the present invention, it may contain an arbitrary amino acid sequence as long as it does not inhibit the functions of the first physiologically active peptide and second physiologically active peptide on the N-terminus side and/or the C-terminus side of the first physiologically active peptide portion and/or second physiologically active peptide portion, or in the space with the linker portion. As such an arbitrary amino acid sequence, for example, a sequence useful for enhancing the expression or secretion of peptide molecules in a host cell may be used.

In an embodiment of the peptide molecule of the present invention, the first physiologically active peptide portion and second physiologically active peptide portion each constitute a cyclic peptide that binds to a c-Met protein, and may be the same as or different from each other. Such cyclic peptide that binds to a c-Met (also referred to simply as Met) protein includes a cyclic peptide having a cyclic structure formed by a disulfide bond, containing an amino acid sequence selected from the following (a) to (i).
(a) CYRQFNRRTHEVWNLDC (SEQ ID NO: 1);
(b) CRQFNRRTHEVWNLDC (SEQ ID NO: 2);
(c) CYWYYAWDQTYKAFPC (SEQ ID NO: 3);
(d) CWYYAWDQTYKAFPC (SEQ ID NO: 4);
(e) CYISWNEFNSPNWRFITC (SEQ ID NO: 5);
(f) CISWNEFNSPNWRFITC (SEQ ID NO: 6);
(g) a peptide in which one or two amino acid residues other than a cysteine residue are substituted, deleted, or added in any of the amino acid sequences (a) to (f), and which binds to a c-Met protein;
(h) a peptide which is composed of an amino acid sequence having 90% or more sequence identity with any of the amino acid sequences (a) to (f), but having cysteine residues at both ends, and which binds to a c-Met protein. For example, the sequence identity may be 92% or higher, 95% or higher, or 98% or higher. In the present specification, the amino acid sequence homology may be calculated by using a known analysis tool, and may be calculated, for example, by using the National Center for Biotechnology Information (NCBI) homology algorithm BLAST (Basic local alignment search tool). In addition, the default (initial setting) parameters in the analysis tool may be used to calculate the amino acid sequence homology.
(i) A peptide in which at least one amino acid other than the cysteine residues at both ends in any of the amino acid sequences (a) to (h) is modified. Here, the amino acid modification is phosphorylation, methylation, acetylation, adenylylation, ADP-ribosylation, or glycosylation.

Note that it is known a cyclic peptide that binds to such Met (HGFR) functions as a Met (HGFR) agonist by dimerization.

In an embodiment of the peptide molecule of the present invention, the linker portion has a molecular weight of 10,000 or less and may be composed of 50 or less amino acid residues.

Further, in an embodiment of the peptide molecule of the present invention, the linker portion may be a linker portion having a molecular weight of 10,000 or less and composed of 50 or less or 49 or less amino acid residues, and at least 90%, at least 95%, or at least 100% of the amino acid sequence is composed of amino acid residues selected from alanine (A), proline (P), and serine (S). In this case, the proline residues may constitute more than 4% and less than 40% in the amino acid sequence of the linker portion. For example, in the amino acid sequence of the linker portion, the proline residues may constitute more than about 4%, more than about 5%, more than about 6%, more than about 8%, more than about 10%, more than about 15%, or more than about 20%, and may constitute less than about 40% or less than about 35%. Further, the alanine residues and the serine residue may each independently constitute more than about 4%, more than about 10%, or more than about 20%, and may constitute less than about 50%.

In the amino acid sequence of the linker portion, amino acid residues different from alanine, serine, and proline may be selected from the group consisting of Arg, Asn, Asp, Cys, Gln, Glu, Gly, His, Ile, Leu, Lys, Met, Phe, Thr, Trp, Tyr, and Val. Such amino acid residues different from alanine, serine, and proline are preferably ones without a hydrophobic side chain such as Val, Ile, Leu, Met, Phe, Tyr, or Trp, and/or ones without a charged side chain such as Lys, Arg, Asp, or Glu.

In an embodiment of the peptide molecule of the present invention, the linker portion may be a linker portion having a molecular weight of 10,000 or less and composed of 50 or less or 49 or less amino acid residues, and at least 90%, at least 95%, or at least 100% of the amino acid sequence is composed of amino acid residues selected from alanine (A) and proline (P). In this case, proline residues may constitute more than 10% and less than 75% in the amino acid sequence of the linker portion. For example, in the amino acid sequence of the linker portion, the proline residues may constitute more than about 10%, more than about 12%, more than about 14%, more than about 18%, more than about 20%, more than about 22%, more than about 23%, more than about 24%, or more than about 25%, and may constitute less than about 75%, less than about 70%, less than about 65%, less than about 60%, less than about 55%, less than about 50%, less than about 48%, less than about 46%, less than about 44%, less than about 42%, less than about 41%, less than about 40%, less than about 39%, less than about 38%, less than about 37%, less than about 36%, or less than about 35%. Further, the alanine residues may constitute more than about 25%, more than about 30%, more than about 35%, more than about 40%, more than about 45%, more than about 50%, more than about 52%, more than about 54%, more than about 56%, more than about 58%, more than about 59%, more than about 60%, more than about 61%, more than about 62%, more than about 63%, more than about 64%, or more than about 65%, and may constitute less than about 90%, less than about 88%, less than about 86%, less than about 84%, less than about 82%, less than about 80%, less than about 79%, less than about 78%, less than about 77%, less than about 76%, or less than about 75%.

In the amino acid sequence of the linker portion, the amino acid residues different from alanine, serine and proline may be selected from the group consisting of Arg, Asn, Asp, Cys, Gln, Glu, Gly, His, Ile, Leu, Lys, Met, Phe, Thr, Trp, Tyr, and Val. Such amino acid residues different from alanine, serine, and proline are preferably ones without a hydrophobic side chain such as Val, Ile, Leu, Met, Phe, Tyr, or Trp, and/or ones without a charged side chain such as Lys, Arg, Asp, or Glu.

In an embodiment of the peptide molecule of the present invention, the linker portion contains multiple amino acid repeats, the amino acid repeats are composed of alanine (A), proline (P), and serine (S), and in the amino acid repeats, the number of consecutive identical amino acid residues may be 6 residues or less, 5 residues or less, 4 residues or less, or 3 residues or less.

In an embodiment of the peptide molecule of the present invention, the linker portion contains multiple amino acid repeats, the amino acid repeats are composed of alanine (A) and proline (P), and in the amino acid repeats, the number of consecutive identical amino acid residues may be 6 residues or less, 5 residues or less, 4 residues or less, or 3 residues or less.

In an embodiment of the peptide molecule of the present invention, the linker portion may repeatedly contain two or more amino acid sequences selected from the group consisting of AP, AAP, AS, ASP, and ASS.

In an embodiment of the peptide molecule of the present invention, the linker portion may contain a sequence composed of glycine (G) and serine (S). In this case, the linker portion may repeatedly contain an amino acid sequence selected from the group consisting of GGGGS, GGGS, GGS, and GS.

In an embodiment of the production method of the present invention, the linker portion may repeatedly contain glycine or may be composed solely of glycine.

In an embodiment of the production method of the present invention, the linker portion may repeatedly contain the amino acid sequence represented by EAAAK

In an embodiment of the production method of the present invention, the amino acid sequence of the linker portion is preferably, but is not limited to, one composed of A, P, and S, one composed of A and P, or one composed of a repetition of the sequence represented by GGGGS.

In an embodiment of the peptide molecule of the present invention, the length of the linker portion may be composed of 50 or less amino acid residues, and for example, the linker portion may be composed of 49 or less, 40 or less, 35 or less, 30 or less, or 25 or less amino acid residues.

In an embodiment of the production method of the present invention, the linker portion may be composed of 1 or more amino acid residues, and for example, the linker portion may be composed of 4 or more, 8 or more, 12 or more, or 16 or more amino acid residues.

In an embodiment of the peptide molecule of the present invention, specific examples of the linker portion include, but are not limited to, those containing the following amino acid sequences and those consisting of the following amino acid sequences. An amino acid sequence selected from AAPAAPAP (SEQ ID NO: 74), AAPAAPAPAAPAAPAP (SEQ ID NO: 75), AAPAAPAPAAPAAPAPAAPAAPAPAAPAAPAPAAPAAPAPAAPAAPAPA (SEQ ID NO: 76), SAPSPSSAPASASAPASPASASASASPASSPASASSASPAASSASPASS (SEQ ID NO: 77), AAPAAPAPAAPAAPAPAAPAAPAPAAPAAPAPAAPAAPAPAAPAAPAPAAPAAPAP AAPAAPAPAAPAAPAPAAPAAPAPAAPAAPAPAAPAAPAPAAPA (SEQ ID NO: 78), SAPSPSSAPASASAPASPASASASASPASSPASASSASPAASSASPASSSASPSAPAA PSASAAPSASPASSSPAPASAPSPAAAASSPSPAPSSASSAA (SEQ ID NO: 79), AAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAPAAPAAPAP (SEQ ID NO: 80), AAPAAPAPAAPAAPAPAAPA (SEQ ID NO: 7), AAPAAPAPAAPAAPAPAAPAAP (SEQ ID NO: 8), AAPAAPAPAAPAAP (SEQ ID NO: 9), AAPAAAPAPAAPAAPAPAAP (SEQ ID NO 10), AAAPAAAPAAAPAAAPAAAP (SEQ ID NO 11), AAPAAPAAPAAPAAPAAPAAPAAP (SEQ ID NO 12), APAAAPAPAAAPAPAAAPAPAAAP (SEQ ID NO 13), AAAPAAPAAPPAAAAPAAPAAPPA (SEQ ID NO 14), and APAPAPAPAPAPAPAPAPAP (SEQ ID NO 15), ASPAAPAPASPAAPAPSAPA (SEQ ID NO 16), AAPASPAPAAPSAPAPAAPS (SEQ ID NO 17), APSSPSPSAPSSPSPASPSS (SEQ ID NO 18), SAPSSPSPSAPSSPSPASPS (SEQ ID NO 19), SSPSAPSPSSPASPSPSSPA (SEQ ID NO 20), AASPAAPSAPPAAASPAAPSAPPA (SEQ ID NO 21), and ASAAAPAAASAAASAPSAAA (SEQ ID NO 22), as well as circular permutation mutant sequences thereof. Taking AAPAAPAPAAPAAPAPAAPA (SEQ ID NO: 7) as an example, a circular permutation mutant sequence may be easily prepared, for example, by removing the first alanine of the above sequence and adding another alanine to the terminus of the above sequence. Such a circular permutation mutant sequence for SEQ ID NO: 7 is APAAPAPAAPAAPAPAAPAA (SEQ ID NO: 23). Furthermore, non-limited examples of the circular permutation mutant sequence with circularly permutated amino acid residues for SEQ ID NO: 7 include the following. PAAPAPAAPAAPAPAAPAAA (SEQ ID NO: 24), AAPAPAAPAAPAPAAPAAAP (SEQ ID NO: 25), APAPAAPAAPAPAAPAAAPA (SEQ ID NO: 26), PAPAAPAAPAPAAPAAAPAA (SEQ ID NO: 27), APAAPAAPAPAAPAAAPAAP (SEQ ID NO: 28), PAAPAAPAPAAPAAAPAAPA (SEQ ID NO: 29), AAPAAPAPAAPAAAPAAPAP (SEQ ID NO: 30), APAAPAPAAPAAAPAAPAPA (SEQ ID NO: 31), and PAAPAPAAPAAAPAAPAPAA (SEQ ID NO: 32).

### Examples

Hereinafter, the present invention will be described in more detail by way of examples, but the present invention is not limited thereto.

Note that the HGF used in the examples are all recombinant human HGF (rhHGF, R&D Systems), and the recombinant human HGF is simply referred to as HGF in the present specification and the drawings.

### Reference Example 1: Construction of a shortened vector (pPK10) of the tatABC gene amplification vector pPK6

The pPK6 vector described in WO2016/171224 is a vector for amplifying the tatABC gene encoding the Tat secretion system. By removing unnecessary sequences from pPK6, a shortened vector was constructed as follows.

First, pPK6 was used as a template to amplify the 5'-side of the removed region with the primers set forth in SEQ ID NO: 33 and SEQ ID NO: 34, and the 3'-side with the primers set forth in SEQ ID NO: 35 and SEQ ID NO: 36. The two amplified DNA fragments were introduced into the SalI-SalI site of pPK6 by an infusion reaction to obtain a plasmid with 548 bases removed from pPK6. This plasmid was named pPK6b.

Next, pPK6b was used as a template to amplify the 5'-side of the removed region with the primers set forth in SEQ ID NO: 37 and SEQ ID NO: 38, and the 3'-side with the primers set forth in SEQ ID NO: 39 and SEQ ID NO: 40. The two amplified DNA fragments were introduced into the ClaI-SpeI site of pPK6b by an infusion reaction to construct plasmids with 310 bases further removed from pPK6b. For the infusion reaction, In-Fusion (registered trademark) HD Cloning Kit (Takara Bio) was used, and the reaction conditions were according to the protocol recommended by the manufacturer.

In this way, an unnecessary sequence of 858 bases was removed from the pPK6 vector to construct a shortened plasmid. This vector was named pPK10.

**Table 1**

| Primer Sequence Number | Base Sequence |
|---|---|
| SEQ ID NO: 33 | tcagaggaatgtcgactgctcgcaaccggcccgt |
| SEQ ID NO: 34 | cctacattcagcgcaggttcccctagagcacgt |
| SEQ ID NO: 35 | tgcgctgaatgtagggaag |
| SEQ ID NO: 36 | atcgctgcaggtcgactctagaggatccccggaa |
| SEQ ID NO: 37 | cttcccatacaatcgatagattgtcgcacctgattg |
| SEQ ID NO: 38 | cctcaggctggtgttgggcgagctcgaattcgt |
| SEQ ID NO: 39 | aacaccagcctgaggcaa |
| SEQ ID NO: 40 | gatcttcgggactagttactttcaaatgctcagcatg |

### Example 1: Design of FBP-PAS dimer peptide and its secretory expression in C. glutamicum

### (1-1) Outline of FBP-PAS dimer peptide design

A peptide molecule with two FGFR1 binding cyclic peptides (FBPs) bound with a PAS linker is expressed as one polypeptide in C. glutamicum. Therefore, the peptide sequence was designed to be "first FBP"-"PAS linker"-"second FBP (same sequence as the above first FBP)". That is, the C-terminus of the first FBP and the N-terminus of the PAS linker are linked to the N-terminus of the second FBP and the C-terminus of the PAS linker by an amide bond, and may be expressed in C. glutamicum as a single polypeptide chain.

### (1-2) Preparation of FBP-PAS dimer peptide

The prepared FBP-PAS dimer peptide is as follows.

### (a) FBP-PAS

RPGCGPRKPRTPKKCGSHGAAPAAPAPAAPAAPAPAAPAGRPGCGPRKPRTPKK
CGSH (SEQ ID NO: 41)

### (1-3) Expression of FBP-PAS dimer peptide

Corynex (registered trademark) was used to examine the expression of FBP-PAS dimer peptide. Hereinafter, examples of expression examinations using Corynex (registered trademark) will be described.

### (1-4) Construction of secretory expression plasmid of FBP-PAS dimer peptide using TorA signal sequence

As the FBP-PAS dimer peptide, the amino acid sequence of the above (a) FBP-PAS (which may be hereinafter referred to as "FBP-PAS") was designed, and the base sequence encoding this protein was designed in consideration of the codon usage frequency of C. glutamicum. Furthermore, the following expression cassette was designed to enable secretion expression by C. glutamicum.

FBP-PAS was secretory-expressed as a fusion protein of FBP-PAS and a signal peptide 39 amino acid residue of TorA derived from E. coli (hereinafter referred to as "TorAss-FBP-PAS"). The base sequence and amino acid sequence encoding the designed TorAss-FBP-PAS are set forth in SEQ ID NOs: 42 and 43, respectively.
Base sequence encoding TorAss-FBP-PAS 
atgaacaataacgatctctttcaggcatcacgtcggcgttttctggcacaactcggcggcttaaccgtcgccgggatgctgggg
ccgtcattgttaacgccgcgacgtgcgactgcgcgtcctggttgtggtcctcgcaagccccgcacccctaagaaatgtggttct
cacggcgccgctcctgccgcacccgcacccgctgctccagcagccccagccccagctgcaccagcaggtcgcccaggct
gtggtccccgcaaaccccgcacccctaagaaatgtggctctcactaa (SEQ ID NO: 42)
Amino acid sequence of TorAss-FBP-PAS

MNNNDLFQASRRRFLAQLGGLTVAGMLGPSLLTPRRATARPGCGPRKPRTPKKC
GSHGAAPAAPAPAAPAAPAPAAPAGRPGCGPRKPRTPKKCGSH (SEQ ID NO: 43)

The promoter of the cspB gene derived from the C. glutamicum ATCC 13869 strain was linked upstream of the base sequence set forth in TorAss-FBP-PAS, and furthermore, an expression cassette of TorAss-FBP-PAS having a KpnI site on the 5'-side and an ApaI site on the 3'-side was designed and totally synthesized. The totally synthesized DNA fragment (TorAss-FBP-PAS expression cassette) was inserted into the KpnI-ApaI site of pPK10 described in Reference Example 1 to construct pPK10_TorAss-FBP-PAS, a secretory expression plasmid for the FBP-PAS dimer peptide using the TorA signal sequence. As a result of determining the base sequence of the inserted fragment, it was confirmed that the TorAss-FBP-PAS expression cassette as designed had been constructed. Base sequences were determined using BigDye (registered trademark) Terminator v3.1 Cycle Sequencing Kit (Applied Biosystems) and 3130 Genetic Analyzer (Applied Biosystems).

### (1-5) Secretory expression of FBP-PAS dimer peptide in C. glutamicum

The pPK10_TorAss-FBP-PAS constructed above was used to transform the C. glutamicum YDK0107 strain described in WO2016/171224 to obtain the YDK0107/pPK10_TorAss-FBP-PAS strain.

The obtained transformants were cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

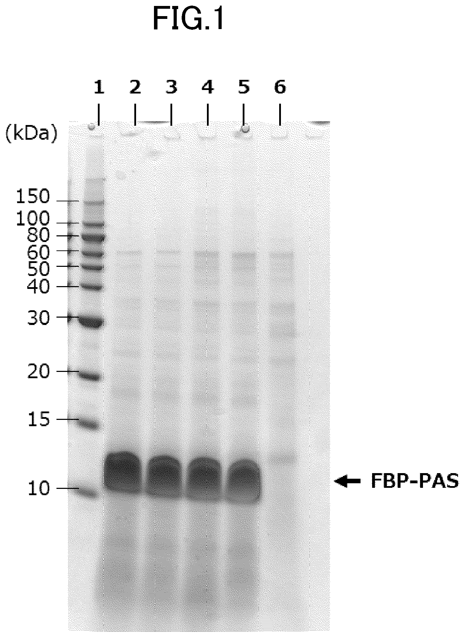

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be FBP-PAS was detected in the culture supernatant of the YDK0107/pPK10_TorAss-FBP-PAS strain (Fig. 1, lanes 2 to 5).

### (1-6) Purification of FBP-PAS dimer peptide

The culture supernatant containing FBP-PAS obtained above was purified by dialysis in PBS (Mg-/Ca-) buffer for 2 days using a dialysis membrane (MWCO=3.5 kDa, Spectrum).

### Example 2: Confirmation of molecular weight and disulfide cyclic structure of FBP-PAS dimer peptide

Disulfide bonds were reduced by adding 5 mM Tris(2-carboxyethyl)phosphine Hydrochloride (TCEP-HCl) to the FBP-PAS dimer peptide solution and incubating at room temperature for 48 hours. The FBP-PAS dimer peptides treated with FBP-PAS and TCEP-HCl were each mixed with α-Cyano-4-hydroxycinnamic Acid (CHCA), and the molecular weight was measured with MALDI-TOF-MS (Shimadzu, AXIMA-TOF 2).

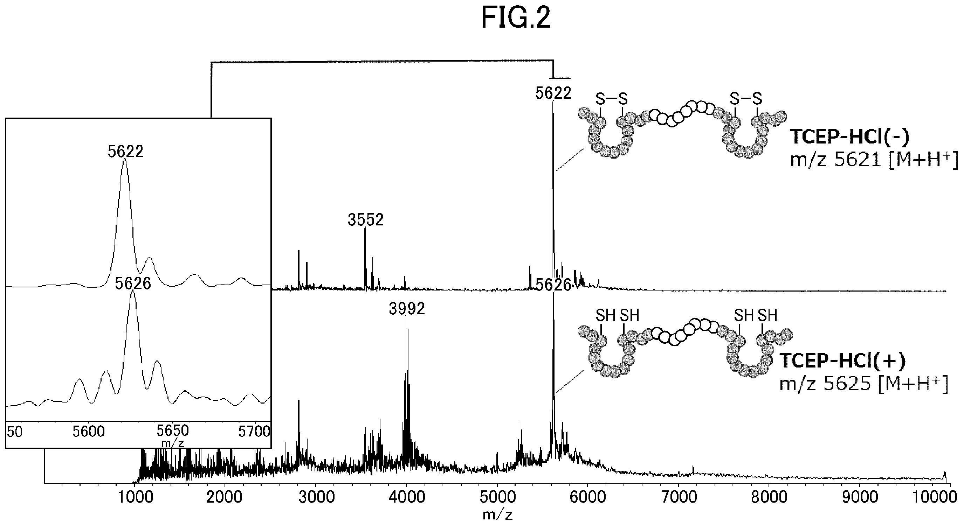

As shown in Fig. 2, a value close to the molecular weight (m/z 5621) of the FBP-PAS dimer peptide forming two disulfide bonds in the molecule was obtained before reduction with TCEP-HCl. In addition, after reduction with TCEP-HCl, a value close to the molecular weight (m/z 5625) with all disulfide bonds reduced was obtained.

From these results, it was confirmed that the FBP-PAS dimer peptide expressed by Corynex (registered trademark) had a peptide sequence as designed and had a structure containing two disulfide bonds in the molecule.

### Example 3: Evaluation of FGFR inhibitory activity of FBP-PAS dimer peptide

The inhibitory activity of the FBP monomer peptide chemically synthesized with the FBP-PAS dimer peptide on FGFR was evaluated by luciferase assay. To 25 µL of Opti-MEM medium (Thermo Fisher Scientific), 0.6 µL of Attractene Transfection Reagent (QIAGEN) was added, and the mixture was incubated at room temperature for 5 minutes. To the above mixed solution, a mixed solution of 25 µL of Opti-MEM and 1 µL of SRE reporter vector (QIAGEN) was added, and the mixture was incubated at room temperature for 20 minutes. This mixed solution was added to a 96-well plate, over which 40,000 cell/well HEK293E cells were seeded and incubated overnight in a 5% CO₂ incubator at 37°C. After transfection, all culture supernatant was removed, 100 µL of Opti-MEM medium (evaluation basal medium) containing 0.5% FBS (Thermo Fisher Scientific), 1% non-essential amino acid solution (Thermo Fisher Scientific), and penicillin-streptomycin (Nacalai Tesque) was added, and incubation was performed at 37°C for 4 hours, and thereby the cells were starved.

FBP-PAS dimer peptide or FBP monomer peptide at 10 µM was added to the evaluation basal medium containing 0 to 10 ng/mL bFGF, and 100 µL thereof was added to the cells. The cells were stimulated by culturing overnight in a 5% CO₂ incubator at 37°C.

The signal intensities were detected using Dual-Luciferase Reporter Assay System (Promega). The medium supernatant in an amount of 100 µL was removed, 50 µL of Glo reagent was added, and the cells were lysed for 10 minutes at room temperature. The luminescence of Firefly luciferase emitted from the cell lysate solution was detected with a plate reader to quantify the activation of the FGFR-Erk-SRE pathway. Subsequently, 50 µL of Glo & Stop reagent solution was added, and after 10 minutes, the luminescence of the internal standard Renilla luciferase was detected to quantify the number of cells. The signal activity of each sample was quantified as SRE activity=(Firefly luciferase luminescence intensity)/(Renilla luciferase luminescence intensity). The relative signal activity values for the samples without addition of the evaluation compound were determined and used as relative receptor activity.

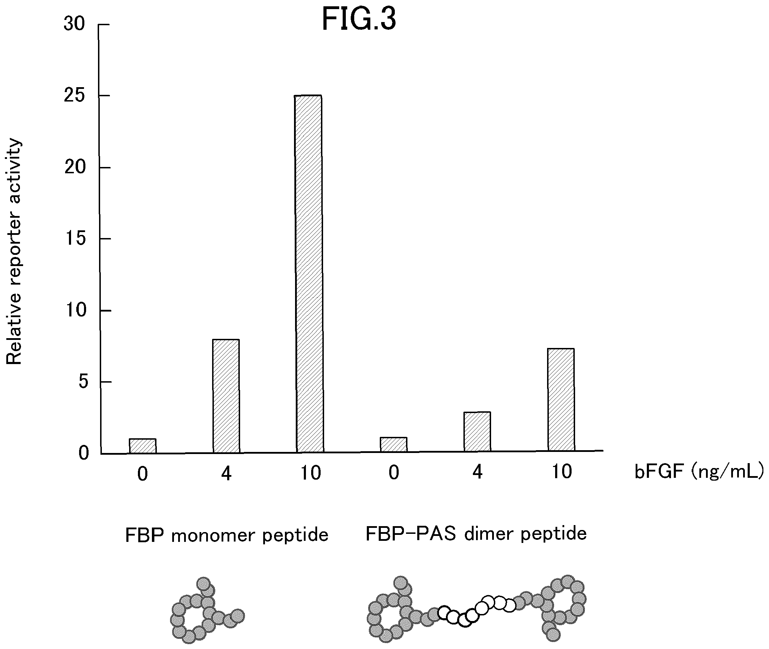

As shown in Fig. 3, it was revealed that the FBP-PAS dimer peptide strongly inhibited the FGFR signal by the same amount of bFGF as compared with the FBP monomer peptide. From these results, it was clarified that the FBP-PAS dimer peptide inhibited FGFR and had a higher inhibitory activity than the FBP monomer peptide.

### Example 4: Design of HGF-PAS dimer peptide and its secretory expression in C. glutamicum

### (4-1) Outline of HGF-PAS dimer peptide design

It is impossible to express peptide dimer molecules of Met-linked cyclic peptide aMD4 reported in K. Ito, et al. Nat. Commun. 6, 6373, 2015, dimerized by a PEG linker, as a single polypeptide chain in C. glutamicum for the following reasons.
(1) aMD4 is cyclized by an intramolecular thioether bond formed by the reaction of the chloroacetyl groups added to the N-terminus amino groups with the sulfhydryl groups of the cysteine-side chains in the sequence, but the thioether bond cannot express in C. glutamicum.
(2) Dimerization is achieved by binding the C-terminuses of the cyclic peptides to each other using a Bis-Maleimide-PEG linker, and cannot be expressed in C. glutamicum.

Therefore, the molecule was converted as follows to design an expressible form. Regarding (1) above, the thioether bond was converted into a disulfide bond by two cysteine-side chain sulfhydryl groups. The conversion to a disulfide bond lengthened the cyclized site by 2 atoms, but by deleting the residue next to cysteine on the N-terminus side (tyrosine in the case of aMD4), the cyclized site was designed to be shortened by one atom. The above (2) was addressed by using a PAS sequence instead of the PEG linker and further designing the peptide sequence to be "first disulfide conversion aMD4"-"PAS linker"-"second disulfide conversion aMD4" so that it could be expressed as a single polypeptide chain. That is, the C-terminus of the first disulfide conversion aMD4 and the N-terminus of the PAS linker, and the N-terminus of the second disulfide conversion aMD4 and the C-terminus of the PAS linker are bound by an amide bond, and can be expressed as a single polypeptide chain.

### (4-2) Preparation of HGF-PAS dimer peptides

The prepared HGF-PAS dimer peptides are as follows.
(b) PAS-aMD4-PEG11 
CYRQFNRRTHEVWNLDCGAAPAAPAPAAPAAPAPAAPAAPGCYRQFNRRTHEV
WNLDC (SEQ ID NO: 44)
(c) PAS-aMD4dY-PEG11 
CRQFNRRTHEVWNLDCGAAPAAPAPAAPAAPAPAAPAAPGCRQFNRRTHEVWN
LDC (SEQ ID NO: 45)
(d) PAS-aMD4-PEG3 CYRQFNRRTHEVWNLDCGAAPAAPAPAAPAAPGCYRQFNRRTHEVWNLDC (SEQ ID NO: 46)
(e)AET-PAS-aMD4-PEG11 
AETCYRQFNRRTHEVWNLDCGAAPAAPAPAAPAAPAPAAPAAPGCYRQFNRRT
HEVWNLDC (SEQ ID NO: 47)
(f) AET-PAS-aMD4dY-PEG11 
AETCRQFNRRTHEVWNLDCGAAPAAPAPAAPAAPAPAAPAAPGCRQFNRRTHE
VWNLDC (SEQ ID NO: 48)
(g) AET-PAS-aMD4-PEG3

AETCYRQFNRRTHEVWNLDCGAAPAAPAPAAPAAPGCYRQFNRRTHEVWNLD
C (SEQ ID NO: 49)

### (4-3) Expression of HGF-PAS dimer peptides

The expression of these HGF-PAS dimer peptides was examined using Corynex (registered trademark). Hereinafter, examples of expression examinations using Corynex (registered trademark) will be described.

### (4-4) Construction of each secretory expression plasmid of PAS-aMD4-PEG11, PAS-aMD4dY-PEG11, and PAS-aMD4-PEG3 using TorA signal sequence.

As the HGF-PAS dimer peptide, three types of amino acid sequences of the above (b) PAS-aMD4-PEG11 (which may be hereinafter referred to as "PAS-aMD4-PEG11"), (c) PAS-aMD4dY-PEG11 (which may be hereinafter referred to as "PAS-aMD4dY-PEG11"), and (d) PAS-aMD4-PEG3 (which may be hereinafter referred to as "PAS-aMD4-PEG3") were designed, and the base sequences encoding these proteins were designed in consideration of the codon usage frequency of C. glutamicum. Furthermore, the following expression cassettes were designed to enable secretory expression by C. glutamicum.

PAS-aMD4-PEG11 was secretory-expressed as a fusion protein of PAS-aMD4-PEG11 and a signal peptide 39 amino acid residue of TorA derived from E. coli (hereinafter referred to as "TorAss-PAS-aMD4-PEG11"). The base sequence and amino acid sequence encoding the designed TorAss-PAS-aMD4-PEG11 are set forth in SEQ ID NOs: 50 and 51, respectively.
Base sequence encoding TorAss-PAS-aMD4-PEG11 
atgaacaataacgatctctttcaggcatcacgtcggcgttttctggcacaactcggcggcttaaccgtcgccgggatgctgggg
ccgtcattgttaacgccgcgacgtgcgactgcgtgttaccgtcaattcaaccgtcgcacccacgaagtttggaacctggactgt
ggcgcagcacccgcagccccagcccccgcagcacctgcagccccagcaccagctgcaccagccgctccaggctgctacc
gtcagttcaaccgccgcacccacgaagtttggaatttggattgctaa (SEQ ID NO: 50)
Amino acid sequence of TorAss-PAS-aMD4-PEG11

MNNNDLFQASRRRFLAQLGGLTVAGMLGPSLLTPRRATACYRQFNRRTHEVWN
LDCGAAPAAPAPAAPAAPAPAAPAAPGCYRQFNRRTHEVWNLDC (SEQ ID NO:
51)

PAS-aMD4dY-PEG11 was secretory-expressed as a fusion protein of PAS-aMD4dY-PEG11 and a signal peptide 39 amino acid residue of TorA derived from E. coli (hereinafter referred to as "TorAss-PAS-aMD4dY-PEG11"). The base sequence and amino acid sequence encoding the designed TorAss-PAS-aMD4dY-PEG11 are set forth in SEQ ID NOs: 52 and 53, respectively.
Base sequence encoding TorAss-PAS-aMD4dY-PEG11 
atgaacaataacgatctctttcaggcatcacgtcggcgttttctggcacaactcggcggcttaaccgtcgccgggatgctgggg
ccgtcattgttaacgccgcgacgtgcgactgcgtgccgtcagttcaaccgtcgcacccacgaagtttggaatctggactgtgg
cgcagcccccgcagcacccgctcccgcagcacccgcagctccagcaccagcagccccagcagcaccaggctgccgcca
gttcaaccgtcgcacccacgaagtttggaatttggattgttag (SEQ ID NO: 52)
Amino acid sequence of TorAss-PAS-aMD4dY-PEG11

MNNNDLFQASRRRFLAQLGGLTVAGMLGPSLLTPRRATACRQFNRRTHEVWNL
DCGAAPAAPAPAAPAAPAPAAPAAPGCRQFNRRTHEVWNLDC (SEQ ID NO: 53)

PAS-aMD4-PEG3 was secretory-expressed as a fusion protein of PAS-aMD4-PEG3 and a signal peptide 39 amino acid residue of TorA derived from E. coli (hereinafter referred to as "TorAss-PAS-aMD4-PEG3"). The base sequence and amino acid sequence encoding the designed TorAss-PAS-aMD4-PEG3 are set forth in SEQ ID NOs: 54 and 55, respectively.
Base sequence encoding TorAss-PAS-aMD4-PEG3 
atgaacaataacgatctctttcaggcatcacgtcggcgttttctggcacaactcggcggcttaaccgtcgccgggatgctgggg
ccgtcattgttaacgccgcgacgtgcgactgcgtgttaccgtcagttcaaccgccgtacccacgaagtctggaacctggattgt
ggtgccgcccccgccgctcccgctcccgccgctccggcagcccctggctgctaccgtcagtttaaccgtcgcacccacgaa
gtctggaacctggattgctaa (SEQ ID NO: 54)
Amino acid sequence of TorAss-PAS-aMD4-PEG3

MNNNDLFQASRRRFLAQLGGLTVAGMLGPSLLTPRRATACYRQFNRRTHEVWN
LDCGAAPAAPAPAAPAAPGCYRQFNRRTHEVWNLDC (SEQ ID NO: 55)

The promoter of the cspB gene derived from the C. glutamicum ATCC 13869 strain was linked upstream of the base sequences set forth in TorAss-PAS-aMD4-PEG11, TorAss-PAS-aMD4dY-PEG11, and TorAss-PAS-aMD4-PEG3, and furthermore, an expression cassette of each HGF-PAS dimer peptide having a KpnI site on the 5'-side and an ApaI site on the 3'-side was designed and totally synthesized. The totally synthesized DNA fragment (HGF-PAS dimer peptide expression cassette) was inserted into the KpnI-ApaI site of pPK10 described in Reference Example 1 to construct pPK10_TorAss-PAS-aMD4-PEG11, pPK10_TorAss-PAS-aMD4dY-PEG11, and pPK10_TorAss-PAS-aMD4-PEG3, a secretory expression plasmid for each HGF-PAS dimer peptide using the TorA signal sequence. As a result of determining the base sequence of the inserted fragment, it was confirmed that each HGF-PAS dimer peptide expression cassette as designed had been constructed. Base sequences were determined using BigDye (registered trademark) Terminator v3.1 Cycle Sequencing Kit (Applied Biosystems) and 3130 Genetic Analyzer (Applied Biosystems).

### (4-5) Secretory expression of each HGF-PAS dimer peptide in C. glutamicum

The pPK10_TorAss-PAS-aMD4-PEG11, pPK10_TorAss-PAS-aMD4dY-PEG11, and pPK10_TorAss-PAS-aMD4-PEG3 constructed above were used to transform the C. glutamicum YDK0107 strain described in WO2016/171224 to obtain the YDK0107/pPK10_TorAss-PAS-aMD4-PEG11 strain, YDK0107/pPK10_TorAss-PAS-aMD4dY-PEG11 strain, and YDK0107/pPK10_TorAss-PAS-aMD4-PEG3 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

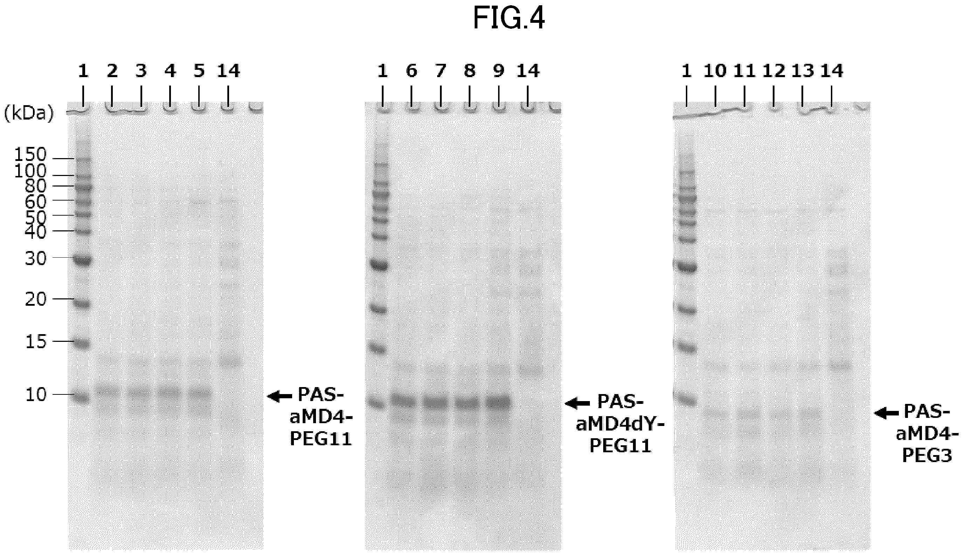

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be PAS-aMD4-PEG11 was detected in the culture supernatant of the YDK0107/pPK10_TorAss-PAS-aMD4-PEG11 strain (Fig. 4, lanes 2 to 5), a protein band presumed to be PAS-aMD4dY-PEG11 was detected in the culture supernatant of the YDK0107/pPK10_TorAss-PAS-aMD4dY-PEG11 strain (Fig. 4, lanes 6 to 9), and a protein band presumed to be PAS-aMD4-PEG3 was detected in the culture supernatant of the YDK0107/pPK10_TorAss-PAS-aMD4-PEG3 strain (Fig. 4, lanes 10 to 13).

### (4-6) Construction of each secretory expression plasmid of AET-PAS-aMD4-PEG11, AET-PAS-aMD4dY-PEG11, and AET-PAS-aMD4-PEG3 using CspB signal sequence

As the HGF-PAS dimer peptide, three types of amino acid sequences of the above (e) AET-PAS-aMD4-PEG11 (which may be hereinafter referred to as "AET-PAS-aMD4-PEG11"), (f) AET-PAS-aMD4dY-PEG11 (which may be hereinafter referred to as "AET-PAS-aMD4dY-PEG11"), and (g) AET-PAS-aMD4-PEG3 (which may be hereinafter referred to as "AET-PAS-aMD4-PEG3") were designed, and the base sequences encoding these proteins were designed in consideration of the codon usage frequency of C. glutamicum. Furthermore, the following expression cassettes were designed to enable secretory expression by C. glutamicum.

AET-PAS-aMD4-PEG11 was secretory-expressed as a fusion protein of AET-PAS-aMD4-PEG11 and a signal peptide 30 amino acid residue of CspB derived from C. glutamicum ATCC 13869 strain (hereinafter referred to as "CspBss-AET-PAS-aMD4-PEG11"). The base sequence and amino acid sequence encoding the designed CspBss-AET-PAS-aMD4-PEG11 are set forth in SEQ ID NOs: 56 and 57, respectively.
Base sequence encoding CspBss-AET-PAS-aMD4-PEG11 
atgtttaacaaccgtatccgcactgcagctctcgctggtgcaatcgcaatctccaccgcagcttccggcgtagctatcccagcat
tcgctgcagagacctgttaccgtcaattcaaccgtcgcacccacgaagtttggaacctggactgtggcgcagcacccgcagc
cccagcccccgcagcacctgcagccccagcaccagctgcaccagccgctccaggctgctaccgtcagttcaaccgccgca
cccacgaagtttggaatttggattgctaa (SEQ ID NO: 56)
Amino acid sequence of CspBss-AET-PAS-aMD4-PEG11

MFNNRIRTAALAGAIAISTAASGVAIPAFAAETCYRQFNRRTHEVWNLDCGAAP
AAPAPAAPAAPAPAAPAAPGCYRQFNRRTHEVWNLDC (SEQ ID NO: 57)

AET-PAS-aMD4dY-PEG11 was secretory-expressed as a fusion protein of AET-PAS-aMD4dY-PEG11 and a signal peptide 30 amino acid residue of CspB derived from C. glutamicum ATCC 13869 strain (hereinafter referred to as "CspBss-AET-PAS-aMD4dY-PEG11"). The base sequence and amino acid sequence encoding the designed CspBss-AET-PAS-aMD4dY-PEG11 are set forth in SEQ ID NOs: 58 and 59, respectively.
Base sequence encoding CspBss-AET-PAS-aMD4dY-PEG11 
atgtttaacaaccgtatccgcactgcagctctcgctggtgcaatcgcaatctccaccgcagcttccggcgtagctatcccagcat
tcgctgcagagacctgccgtcagttcaaccgtcgcacccacgaagtttggaatctggactgtggcgcagcccccgcagcacc
cgctcccgcagcacccgcagctccagcaccagcagccccagcagcaccaggctgccgccagttcaaccgtcgcacccac
gaagtttggaatttggattgttag (SEQ ID NO: 58)
Amino acid sequence of CspBss-AET-PAS-aMD4dY-PEG11

MFNNRIRTAALAGAIAISTAASGVAIPAFAAETCRQFNRRTHEVWNLDCGAAPA
APAPAAPAAPAPAAPAAPGCRQFNRRTHEVWNLDC (SEQ ID NO: 59)

AET-PAS-aMD4-PEG3 was secretory-expressed as a fusion protein of AET-PAS-aMD4-PEG3 and a signal peptide 30 amino acid residue of CspB derived from C. glutamicum ATCC 13869 strain (hereinafter referred to as "CspBss-AET-PAS-aMD4-PEG3"). The base sequence and amino acid sequence encoding the designed CspBss-AET-PAS-aMD4-PEG3 are set forth in SEQ ID NOs: 60 and 61, respectively.
Base sequence encoding CspBss-AET-PAS-aMD4-PEG3 
atgtttaacaaccgtatccgcactgcagctctcgctggtgcaatcgcaatctccaccgcagcttccggcgtagctatcccagcat
tcgctgcagagacctgttaccgtcagttcaaccgccgtacccacgaagtctggaacctggattgtggtgccgcccccgccgct
cccgctcccgccgctccggcagcccctggctgctaccgtcagtttaaccgtcgcacccacgaagtctggaacctggattgcta
a (SEQ ID NO: 60)
Amino acid sequence of CspBss-AET-PAS-aMD4-PEG3

MFNNRIRTAALAGAIAISTAASGVAIPAFAAETCYRQFNRRTHEVWNLDCGAAP
AAPAPAAPAAPGCYRQFNRRTHEVWNLDC (SEQ ID NO: 61)

The promoter of the cspB gene derived from the C. glutamicum ATCC 13869 strain was linked upstream of the base sequences set forth in AET-PAS-aMD4-PEG11, AET-PAS-aMD4dY-PEG11, and AET-PAS-aMD4-PEG3, and furthermore, an expression cassette of each HGF-PAS dimer peptide having a KpnI site on the 5'-side and a BamHI site on the 3'-side was designed and totally synthesized. The totally synthesized DNA fragment (HGF-PAS dimer peptide expression cassette) was inserted into the KpnI-BamHI site of pPK4 described in Japanese Patent Application Publication No. Hei 9-322774 to construct pPK4_CspBss-AET-PAS-aMD4-PEG11, pPK4_CspBss-AET-PAS-aMD4dY-PEG11, and pPK4_CspBss-AET-PAS-aMD4-PEG3, a secretory expression plasmid for each HGF-PAS dimer peptide using the CspB signal sequence. As a result of determining the base sequence of the inserted fragment, it was confirmed that each HGF-PAS dimer peptide expression cassette as designed had been constructed. Base sequences were determined using BigDye (registered trademark) Terminator v3.1 Cycle Sequencing Kit (Applied Biosystems) and 3130 Genetic Analyzer (Applied Biosystems).

### (4-7) Secretory expression of each HGF-PAS dimer peptide in C. glutamicum

The pPK4_CspBss-AET-PAS-aNM4-PEGI1, pPK4_CspBss-AET-PAS-aMD4dY-PEG11, and pPK4_CspBss-AET-PAS-aMD4-PEG3 constructed above were used to transform the C. glutamicum YDK010::phoS(W302C) strain described in WO2016/171224 to obtain the YDK010::phoS(W302C)/pPK4_CspBss-AET-PAS-aMD4-PEG11 strain, YDK010::phoS(W302C)/pPK4_CspBss-AET-PAS-aMD4dY-PEG11 strain, and YDK010::phoS(W302C)/pPK4_CspBss-AET-PAS-aMD4-PEG3 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

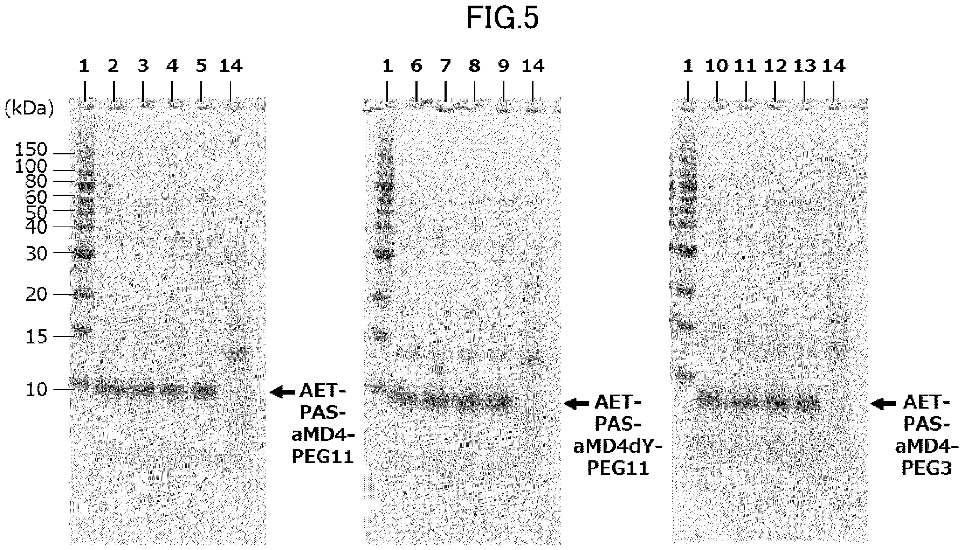

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be AET-PAS-aMD4-PEG11 was detected in the culture supernatant of the YDK010::phoS(W302C)/pPK4_CspBss-AET-PAS-aMD4-PEG11 strain (Fig. 5, lanes 2 to 5), a protein band presumed to be AET-PAS-aMD4dY-PEG11 was detected in the culture supernatant of the YDK010::phoS(W302C)/pPK4_CspBss-AET-PAS-aMD4dY-PEG11 strain (Fig. 5, lanes 6 to 9), and a protein band presumed to be AET-PAS-aMD4-PEG3 was detected in the culture supernatant of the YDK010::phoS(W302C)/pPK4_CspBss-AET-PAS-aMD4-PEG3 strain (Fig. 5, lanes 10 to 13).

### (4-8) Purification of HGF-PAS dimer peptide

The HGF-PAS dimer peptides obtained above were purified using AKTA explorer (GE Healthcare) and Superdex 75 10/300 GL columns (GE Healthcare). The culture supernatant in an amount of 1 mL was injected into the AKTA explorer, and PBS (10 mM phosphate buffer, 140 mM NaCl, pH 7.4) was allowed to flow under the condition of a flow rate of 0.5 mL/min to fractionate the protein, thereby obtaining the desired HGF-PAS dimer peptide fraction.

### Example 5: Molecular weight analysis of HGF-PAS dimer peptide

AET-PAS-aMD4dY-PEG11, AET-PAS-aMD4-PEG11, and AET-PAS-aMD4-PEG3 were each mixed with α-Cyano-4-hydroxycinnamic Acid (CHCA), and MALDI-TOF-MS (Shimadzu, AXIMA-TOF 2) was used to measure the molecular weights.

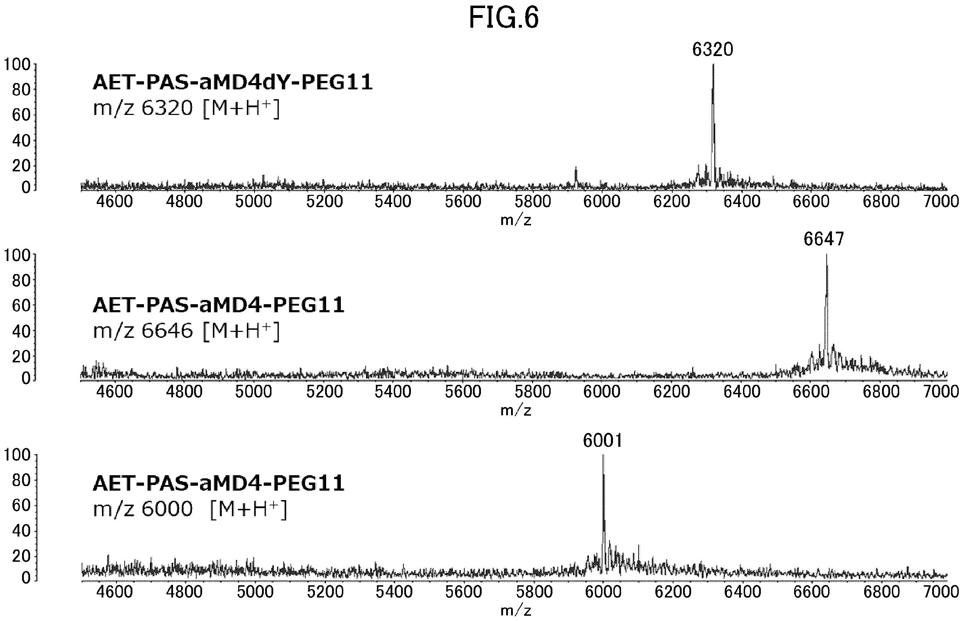

As shown in Fig. 6, a value substantially the same as the theoretical molecular weight value of each oxidized dimer peptide forming two disulfide bonds in the molecule (AET-PAS-aMD4dY-PEG11: m/z 6320, AET-PAS-aMD4-PEG11: m/z 6646, AET-PAS-aMD4-PEG3: m/z 6000) was obtained. It was confirmed that peptides having the correct amino acid sequence containing two disulfide bonds could be expressed.

### Example 6: Activity evaluation of HGF-PAS dimer peptide

The Met activation ability of the HGF-PAS dimer peptide was evaluated by luciferase assay. To 25 µL of Opti-MEM medium (Thermo Fisher Scientific), 0.6 µL of Attractene Transfection Reagent (QIAGEN) was added, and the mixture was incubated at room temperature for 5 minutes. To the above mixed solution, a mixed solution of 25 µL of Opti-MEM and 1 µL of SRE reporter vector (QIAGEN) was added, and the mixture was incubated at room temperature for 20 minutes. This mixed solution was added to a 96-well plate, over which 40,000 cell/well HEK293E cells were seeded and incubated overnight in a 5% CO₂ incubator at 37°C. After transfection, all culture supernatant was removed, 100 µL of Opti-MEM medium (evaluation basal medium) containing 0.5% FBS (Thermo Fisher Scientific), 1% non-essential amino acid solution (Thermo Fisher Scientific), and penicillin-streptomycin (Nacalai Tesque) was added, and incubation was performed at 37°C for 4 hours, and thereby the cells were starved.

To the evaluation basal medium, 0 to 200 ng/mL HGF was added, or 0 to 800 ng/mL AET-PAS-aMD4dY-PEG11, AET-PAS-aMD4-PEG11, or AET-PAS-aMD4-PEG3 was added, or a culture supernatant containing PAS-aMD4dY-PEG11, PAS-aNM4-PEG11, or PAS-aMD4-PEG3 was added so as to be diluted 10-fold with respect to the medium, and the evaluation basal medium was added in an amount of 100 µL to the cells. The cells were stimulated by culturing overnight in a 5% CO₂ incubator at 37°C.

The signal intensities were detected using Dual-Luciferase Reporter Assay System (Promega). The medium supernatant in an amount of 100 µL was removed, 50 µL of Glo reagent was added, and the cells were lysed for 10 minutes at room temperature. The luminescence of Firefly luciferase emitted from the cell lysate solution was detected with a plate reader to quantify the activation of the HGF-Erk-SRE pathway. Subsequently, 50 µL of Glo & Stop reagent solution was added, and after 10 minutes, the luminescence of the internal standard Renilla luciferase was detected to quantify the number of cells. The signal activity of each sample was quantified as SRE activity=(Firefly luciferase luminescence intensity)/(Renilla luciferase luminescence intensity). The relative signal activity values for the samples without addition of the evaluation compound were determined and used as relative receptor activity.

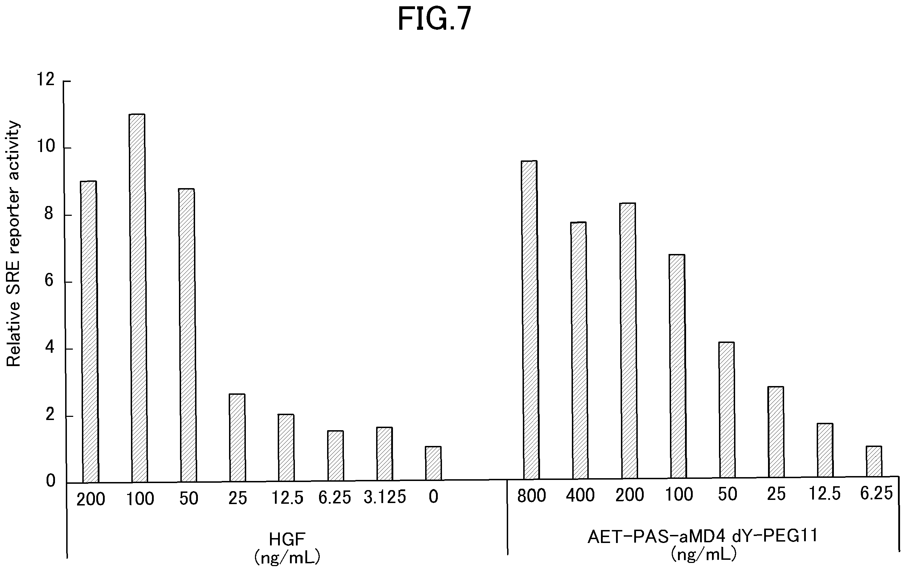

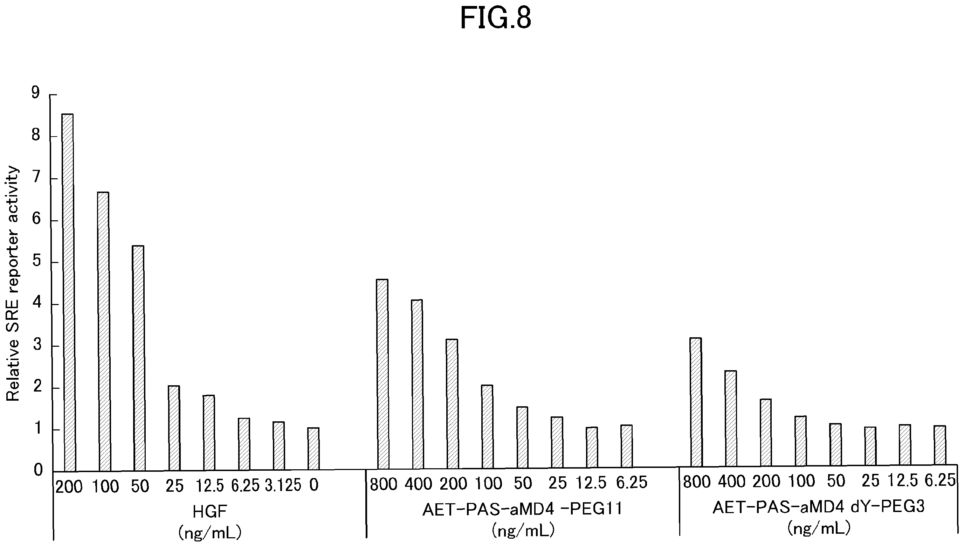

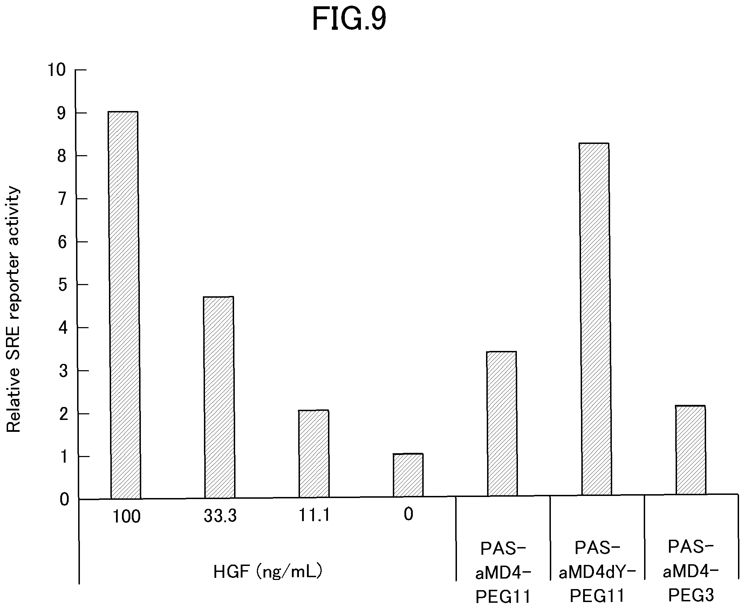

As shown in Figs. 7 to 9, it was clarified that all HGF-PAS dimer peptides had a signal activation ability. In particular, AET-PAS-aMD4dY-PEG11 and PAS-aMD4dY-PEG11 were found to have maximum activity comparable to 100 ng/mL HGF.

### Example 7: Specificity evaluation of HGF-PAS dimer peptide

The receptor tyrosine kinase (RTK) specificity of the HGF-PAS dimer peptide was evaluated using Proteome Profiler Human Phospho-RTK Array Kit (R&D Systems).

HEK293E cells confluent in a 10 cm cell culture dish were starved by culturing for 24 hours in D-MEM medium containing 0.5% FBS (Thermo Fisher Scientific) and penicillin-streptomycin (Nacalai Tesque). The starved HEK293E cells were stimulated with 200 ng/mL AET-PAS-aMD4dY-PEG11 for 10 minutes. As a positive control, 100 ng/mL HGF was similarly stimulated for 10 minutes. As a negative control, PBS was added and stimulated for 10 minutes (Mock group). After removing the medium and washing the cells once with PBS, the cells were lysed by adding 1 mL of Lysis buffer 17 containing a Protease inhibitor cocktail (Nacalai Tesque). The lysate solution was centrifuged at 14,000 G for 5 minutes to obtain the supernatant as a lysate.

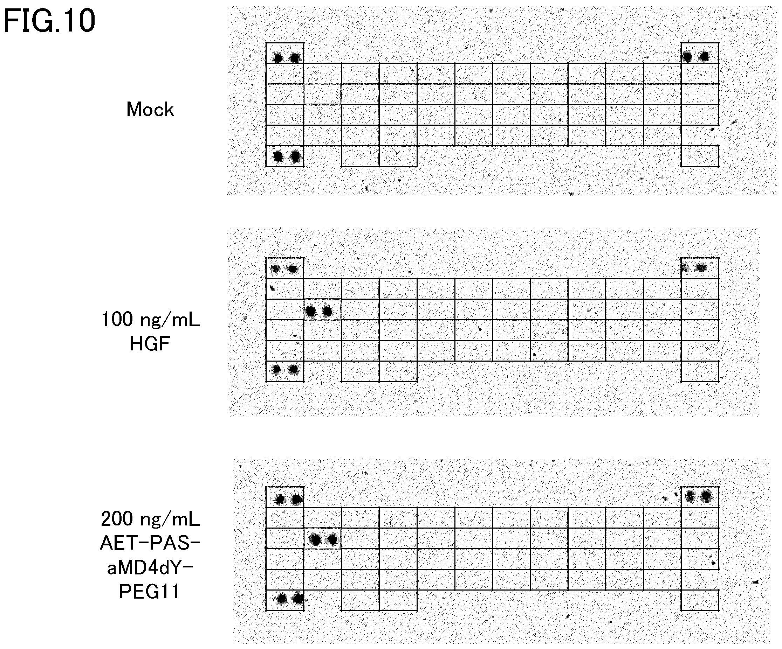

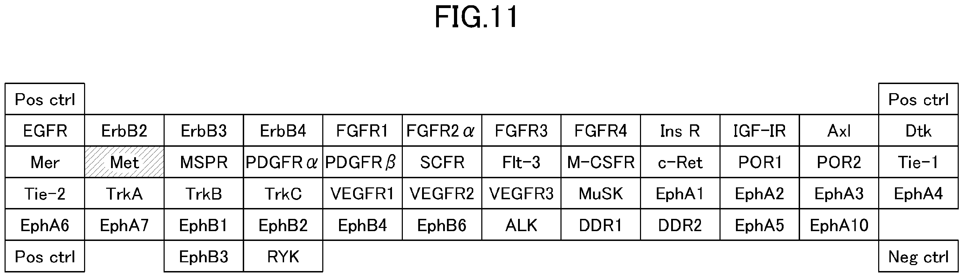

The lysate was analyzed using Proteome Profiler Human Phospho-RTK Array Kit. Molecular Imager ChemiDoc XRS System (Bio-Rad) was used to detect chemiluminescence. As shown in Fig. 10, it was confirmed that all RTK of the cells stimulated with PBS (Mock group) were in a dephosphorylated state. It was also confirmed that when stimulated with HGF, the HGF receptor Met was specifically phosphorylated. When stimulated with AET-PAS-aMD4dY-PEG11, Met was specifically phosphorylated as in the case of HGF. From this result, it was clarified that AET-PAS-aMD4dY-PEG11 performed Met-specific activation. Note that Fig. 11 shows each array of Proteome Profiler Human Phospho-RTK Array Kit (R&D Systems).

### Example 8: Evaluation of cell proliferation promoting activity of HGF-PAS dimer peptide

HGF has been reported to promote cell proliferation by activating Met (K. Ito, et al. Nat. Commun. 6, 6373, 2015). Therefore, HuCCT1 cell culture was used to evaluate whether the HGF-PAS dimer peptide had the same activity of promoting cell proliferation as HGF.

RPMI 1640 medium (Nacalai Tesque) containing 5% FBS was added to a 24-well cell culture plate, and HuCCT1 cells were seeded at 5,000 cell/well. HGF at 35 ng/mL or AET-PAS-aMD4dY-PEG11 at 40 ng/mL was added, and the cells were cultured at 37°C and 5% CO₂ for 5 days. The cells were detached with 0.25% Trypsin/EDTA (Thermo Fisher Scientific) and the number of cells was quantified with Countess Automated Cell Counter (Thermo Fisher Scientific) (N=3).

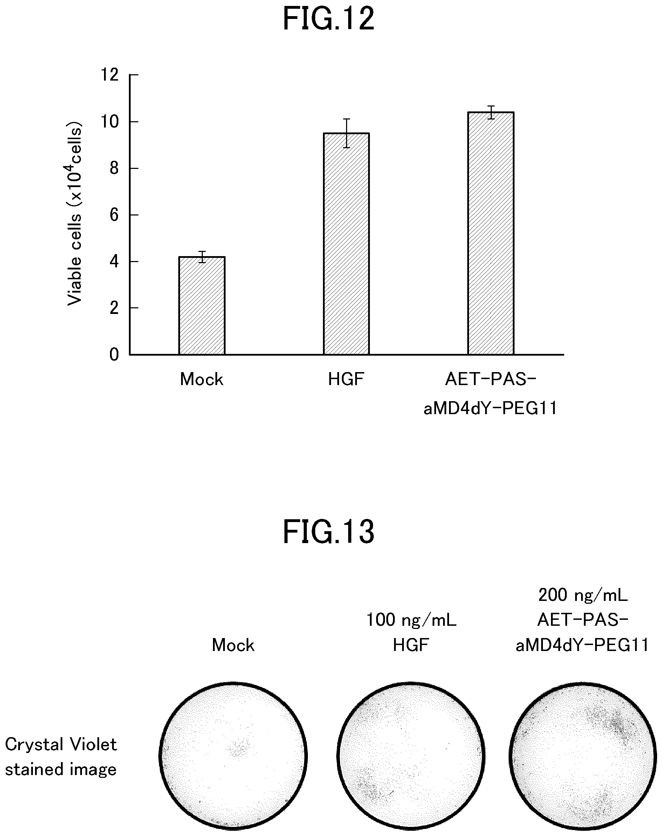

As shown in Fig. 12, all the cells cultured with the addition of HGF or AET-PAS-aMD4dY-PEG11 showed a proliferation ability about 2.5 times higher than that of the non-added group (Mock). From this, it was clarified that AET-PAS-aMD4dY-PEG11 promoted cell proliferation as in the case of HGF.

### Example 9: Evaluation of migration activity of HGF-PAS dimer peptide

HGF has been reported to promote cell migration ability by activating Met (K. Ito, et al. Nat. Commun. 6, 6373, 2015). Therefore, HuCCT1 cell culture was used to evaluate whether the HGF-PAS dimer peptide had the same activity of promoting cell migration as HGF.

To Transwell inserts for 24-well (pore size 3.0 µm, Corning), 20,000 HuCCT1 cells suspended in low serum medium (RPMI 1640 medium with 0.5% FBS) were added. A low serum medium containing 100 ng/mL HGF or 200 ng/mL AET-PAS-aMD4dY-PEG11 was added to the lower layer. After culturing for 24 hours under the conditions of 37°C and 5% CO₂, the Transwell inserts were washed 3 times with PBS and cell-fixed and stained with methanol containing 0.5% Crystal Violet (Nacalai Tesque) for 10 minutes. Immobilized and stained Transwell inserts were washed with distilled water. The cells inside the inserts were removed with a scraper. The cells migrated to the outside of the inserts and stained were photographed in the bright field observation mode of a BZ-X microscope (Keyence). Fig. 13 shows a bright field image of the lower part of the Transwell inserts stained with Crystal Violet and a quantitative value of migration ability.

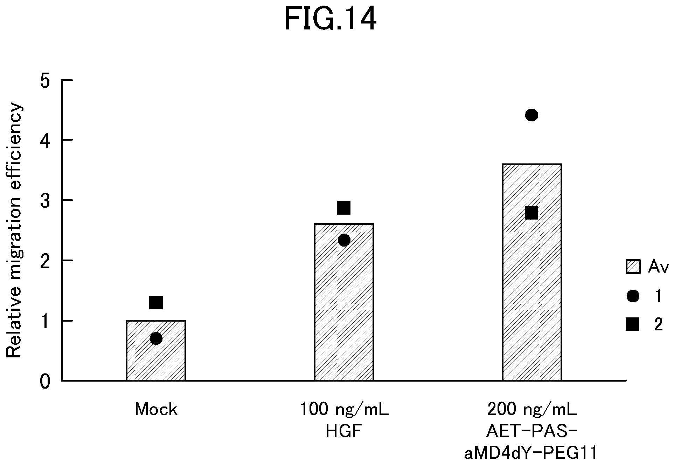

The migration ability was calculated as (area of stained region)/(insert area) and as a relative value (Relative migration efficiency) to the value of the non-added group (Mock) (N=2). Fig. 14 shows the results.

When HGF or AET-PAS-aMD4dY-PEG11 was added and cultured, the proportion of the cells migrating to the lower part of the Transwell inserts increased, and therefore it was clarified that AET-PAS-aMD4dY-PEG11 promoted cell migration as in the case of HGF.

### Example 10: Evaluation of wound healing effect of HGF-PAS dimer peptide

HGF has been reported to promote cell wound healing ability by activating Met (K. Ito, et al. Nat. Commun. 6, 6373, 2015). Therefore, HuCCT1 cell culture was used to evaluate whether the HGF-PAS dimer peptide would promote wound healing as HGF.

To 96-well cell culture plates, 50,000 HuCCT1 cells suspended in RPMI 1640 medium with 10% FBS were added per well and incubated overnight under the conditions of 37°C and 5% CO₂. Cells in the center of the culture wells were scraped using a 1000 µL Thermo Scientific ART chip (Thermo Fisher Scientific) and washed with low serum medium (RPMI 1640 medium with 0.5% FBS). Low serum medium containing 33 ng/mL HGF or 100 ng/mL AET-PAS-aMD4dY-PEG11 was added, and the cells were cultured under the conditions of 37°C and 5% CO₂. BioStudio-T (Nikon Corporation) was used for cell observation, and cells were automatically photographed every hour. The rate of wound healing (µm/h) was calculated as ((wound width 1 hour after wound creation)-(wound width 7 hours after wound creation))/6 (N=4).

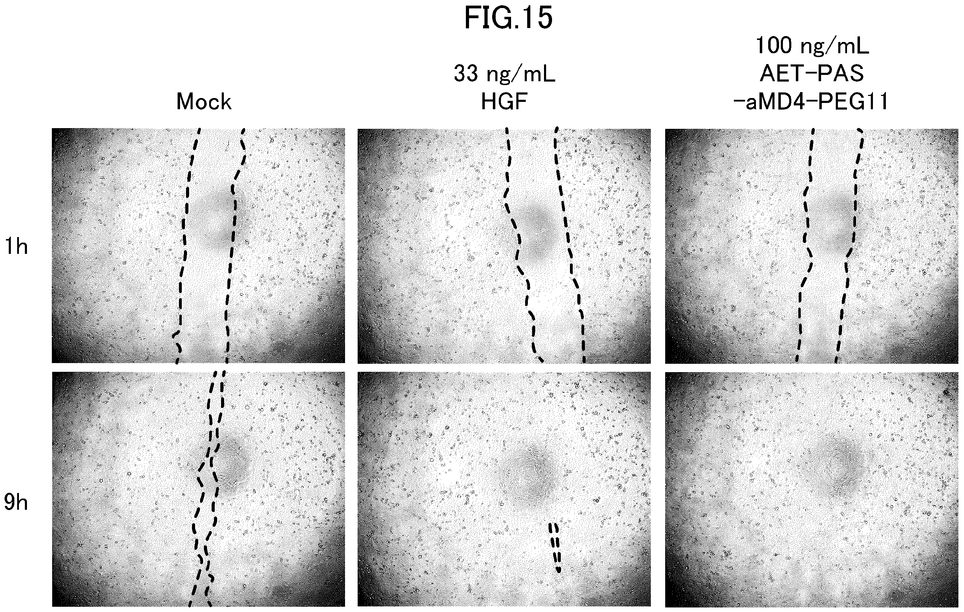

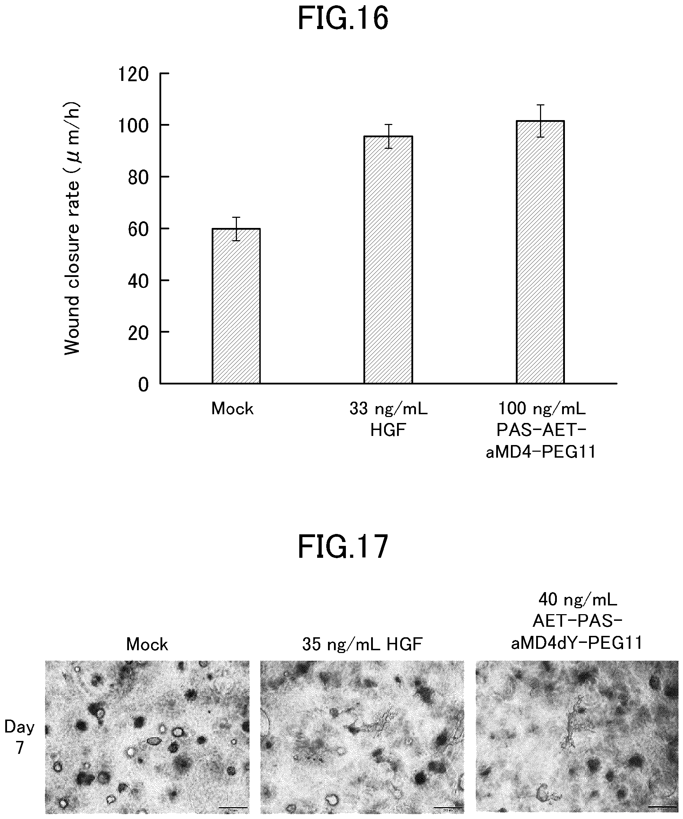

Fig. 15 shows phase difference images of cells 1 hour and 9 hours after wound creation, and Fig. 16 shows the rate of wound healing. Cells supplemented with HGF or AET-PAS-aMD4dY-PEG11 improved in the rate of wound healing by about 1.6 times, and therefore it was clarified that AET-PAS-aMD4dY-PEG11 improve the wound healing ability of cells as well as HGF.

### Example 11: Evaluation of tubular structure forming effect of HGF-PAS dimer peptide

It is known that HGF is allowed to act on HUVEC cells and HuCCT1 cells contained in collagen gel to form a tubular structure (K. Ito, et al. Nat. Commun. 6, 6373, 2015). It has also been reported that a Met full agonist having a function similar to that of HGF can form a tubular structure in the above cells, whereas a partial agonist having only a partial function cannot promote the formation of a tubular structure (W. Miao, et al. Int. J. Mol. Sci. 19, 3141, 2018). Therefore, it was decided to confirm whether the HGF-PAS dimer peptide was a full agonist or a partial agonist by using a tubular structure formation experiment using HuCCT1 cells.

To 8 equivalents of CellMatrix type IA (Nitta Gelatin), 1 equivalent of 10-fold concentrated D-MEM/F12 (Thermo Fisher Scientific) and 1 equivalent of reconstitution buffer (22 g/L sodium bicarbonate, 47.7 g/L HEPES, 0.05N sodium hydroxide aqueous solution) were mixed in order on ice. HuCCT1 cells were added at 25,000 cells per mL of the mixture and mixed on ice. To a 48-well cell culture plate, 400 µL of the above mixture was added, and the mixture was allowed to stand for 4 hours under the conditions of 37°C and 5% CO₂ for gelation. Onto the formed intracellular encapsulated collagen gel, 500 µL of RPMI 1640 medium containing 10% FBS was added. The cell-stimulating group was cultured by adding 35 ng/mL HGF or 40 ng/mL AET-PAS-aMD4dY-PEG11. The whole medium was replaced at a frequency not exceeding 3 days. On day 7 of culture, the morphology of the cells in the collagen gel was observed with an inverted microscope.

Fig. 17 shows the morphology of HuCCT1 cells on day 7 of culture (scale bar: 500 µm). The cells supplemented with HGF or AET-PAS-aMD4dY-PEG11 formed a tubular structure of about 500 to 1000 µm, whereas the cells not supplemented showed a spherical morphology. From these results, it was clarified that AET-PAS-aMD4dY-PEG11 could promote the tubular structure formation of HuCCT1 cells as in the case of rhHGF and was a full agonist for Met.

### Example 12: Design of HGF-PAS heterodimer peptide and its secretory expression in C. glutamicum

### (12-1) Outline of HGF-PAS heterodimer peptide design

The Met-linked cyclic peptides aMD4 and aMD5 reported in K. Ito, et al. Nat. Commun. 6, 6373, 2015 were expressed as heterodimeric molecules. In the dimer peptide synthesis method in K. Ito, et al. Nat. Commun. 6, 6373, 2015, it is difficult to selectively synthesize a dimer peptide having "aMD4"-"linker"-"aMD5". That is, when two types of peptides are bound via a PEG molecule, four types of dimer peptides of "aMD4"-"linker"-"aMD4", "aMD4"-"linker"-"aMD5", "aMD5"-"linker"-"aMD4", and "aMD5"-"linker"-"aMD5" are produced. Therefore, in this example, the following design enables the heterodimer peptide dimerized by the PAS linker to be expressed in C. glutamicum as one polypeptide chain.
(1) aMD4 and aMD5 are cyclized by an intramolecular thioether bond formed by the reaction of the chloroacetyl groups added to the N-terminus amino groups with the sulfhydryl groups of the cysteine-side chains in the sequence, and therefore cyclized by a disulfide bond due to the sulfhydryl groups of the two cysteine side chains.
(2) Dimerization is achieved by binding the C-terminuses of the cyclic peptides to each other using a Bis-Maleimide-PEG linker, and cannot be expressed in C. glutamicum, so that the C-terminus of aMD4 and the N-terminus of aMD5 are bound with a PAS linker peptide.

With the above design, the "aMD4"-"PAS linker"-"aMD5" peptide is selectively expressed.

### (12-2) Preparation of HGF-PAS heterodimer peptide

The prepared HGF-PAS heterodimer peptide is as follows.
(h) AET-PAS-aMD4_aMD5-PEG11

AETCYRQFNRRTHEVWNLDCGAAPAAPAPAAPAAPAPAAPAAPGCYWYYAWD
QTYKAFPC (SEQ ID NO: 62)

### (12-3) Expression of HGF-PAS heterodimer peptide

Corynex (registered trademark) was used to examine the expression of HGF-PAS heterodimer peptide. Hereinafter, examples of expression examinations using Corynex (registered trademark) will be described.

### (12-4) Construction of secretory expression plasmid of AET-PAS-aMD4_aMD5-PEG11 using CspB signal sequence

As the HGF-PAS heterodimer peptide, the amino acid sequence of the above (h) AET-PAS-aMD4_aMD5-PEG11 (which may be hereinafter referred to as "AET-PAS-aMD4_aMDS-PEG11") was designed, and the base sequence encoding this protein was designed in consideration of the codon usage frequency of C. glutamicum. Furthermore, the following expression cassette was designed to enable secretion expression by C. glutamicum.

AET-PAS-aMD4_aMD5-PEG11 was secretory-expressed as a fusion protein of AET-PAS-aMD4_aMD5-PEG11 and a signal peptide 30 amino acid residue of CspB derived from C. glutamicum ATCC 13869 strain (hereinafter referred to as "CspBss-AET-PAS-aMD4_aMDS-PEG11"). The base sequence and amino acid sequence encoding the designed CspBss-AET-PAS-aMD4_aMD5-PEG11 are set forth in SEQ ID NOs: 63 and 64, respectively.
Base sequence encoding CspBss-AET-PAS-aMD4_aMD5-PEG11 
atgtttaacaaccgtatccgcactgcagctctcgctggtgcaatcgcaatctccaccgcagcttccggcgtagctatcccagcat
tcgctgcagagacctgttaccgtcagttcaaccgtcgcacccacgaagtttggaacctggattgtggcgcagcccccgcagcc
cccgcacccgcagcccctgcagccccagccccagctgcaccagcagcacctggctgttactggtattatgcgtgggatcaga
cctacaaagcgtttccgtgttag (SEQ ID NO: 63)
Amino acid sequence of CspBss-AET-PAS-aMD4_aMD5-PEG11

MFNNRIRTAALAGAIAISTAASGVAIPAFAAETCYRQFNRRTHEVWNLDCGAAP
AAPAPAAPAAPAPAAPAAPGCYWYYAWDQTYKAFPC (SEQ ID NO: 64)

The promoter of the cspB gene derived from the C. glutamicum ATCC 13869 strain was linked upstream of the base sequence set forth in AET-PAS-aMD4_aMD5-PEG11, and furthermore, an expression cassette of HGF-PAS heterodimer peptide having a KpnI site on the 5'-side and an BamHI site on the 3'-side was designed and totally synthesized. The totally synthesized DNA fragment (HGF-PAS heterodimer peptide expression cassette) was inserted into the KpnI-BamHI site of pPK4 described in Japanese Patent Application Publication No. Hei 9-322774 to construct pPK4_CspBss-AET-PAS-aMD4_aMD5-PEG1, a secretory expression plasmid for the HGF-PAS heterodimer peptide using the CspB signal sequence. As a result of determining the base sequence of the inserted fragment, it was confirmed that the HGF-PAS heterodimer peptide expression cassette as designed had been constructed. Base sequences were determined using BigDye (registered trademark) Terminator v3.1 Cycle Sequencing Kit (Applied Biosystems) and 3130 Genetic Analyzer (Applied Biosystems).

### (12-5) Secretory expression of HGF-PAS heterodimer peptide in C. glutamicum

The pPK4_CspBss-AET-PAS-aMD4_aMD5-PEG11 constructed above was used to transform the C. glutamicum YDK010::phoS(W302C) strain described in WO2016/171224 to obtain the YDK010::phoS(W302C)/pPK4_CspBss-AET-PAS-aMD4_aMD5-PEG11 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

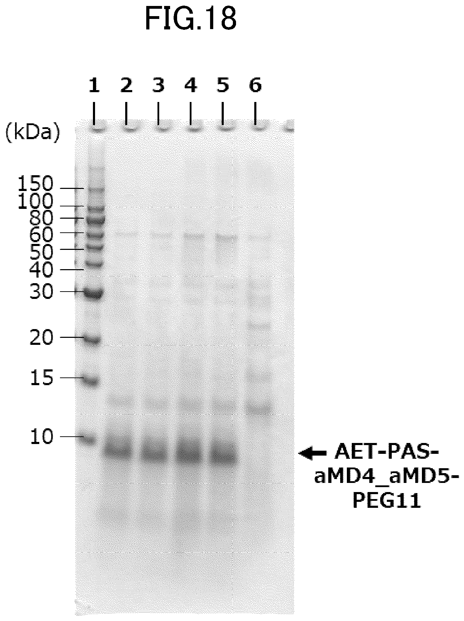

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be AET-PAS-aMD4_aMD5-PEG11 was detected in the culture supernatant of the YDK010::phoS(W302C)/pPK4_CspBss-AET-PAS-aMD4_aMD5-PEG11 strain (Fig. 18, lanes 2 to 5).

### Example 13: Molecular weight analysis of HGF-PAS heterodimer peptide

AET-PAS-aMD4_aMD5-PEG11 peptide expression supernatant was mixed with α-Cyano-4-hydroxycinnamic Acid (CHCA), and MALDI-TOF-MS (Shimadzu, AXIMA-TOF 2) was used to measure the molecular weight.

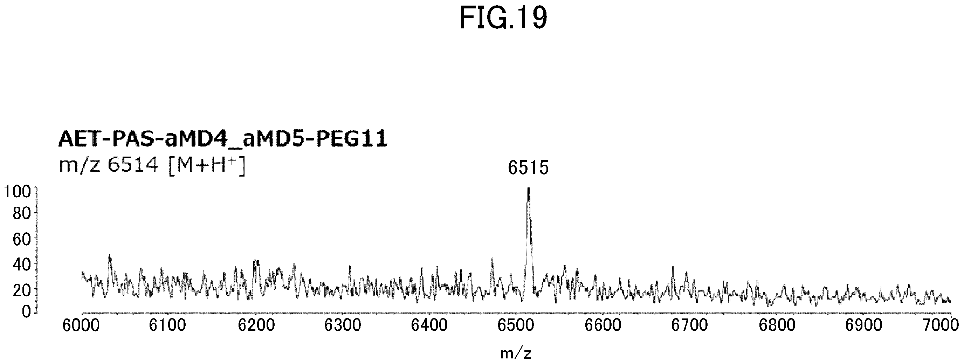

As shown in Fig. 19, a value substantially the same as the theoretical molecular weight value of the oxidized HGF-PAS heterodimer peptide forming two disulfide bonds in the molecule (AET-PAS-aMD4-aMD5-PEG11: m/z 6514) was obtained. It was confirmed that peptides having the correct amino acid sequence containing two disulfide bonds could be expressed.

### Example 14: Design of HGF-PAS dimer peptide linker variant and its secretory expression in C. glutamicum

### (14-1) Outline of HGF-PAS dimer peptide linker variant design

AET-PAS-aMD4dY-PEG11 with HGF-like activity has a structure in which a PAS linker composed of 22 amino acids is linked between the first aMD4dY cyclic peptide (set forth in SEQ ID NO: 2) and the second aMD4dY cyclic peptide. By changing the length of this PAS linker, it is expected that the HGF-like activity will change. Therefore, a linker variant was designed in which the PAS linker portion was changed to a PAS repeat sequence from 8 amino acids to 200 amino acids.

In addition to the PAS linker, a GS linker, which is a repeating sequence of glycine and serin, is also known as a flexible linker. Therefore, the peptide sequence was designed to be a linker variant in which aMD4dY was bound to the N-terminus and C-terminus of the GS linker composed of a repeating sequence of 4 glycine residues and 1 serine residue (GGGGS), that is, a "first aMD4dY cyclic peptide"-"GS linker"-"second aMD4dY cyclic peptide".

### (14-2) Preparation of HGF-PAS dimer peptide linker variants

The prepared HGF-PAS dimer peptide linker variants are as follows.
(i) AET-aMD4dY-PAS8 AETCRQFNRRTHEVWNLDCGAAPAAPAPGCRQFNRRTHEVWNLDC (SEQ ID NO: 81)
(j) AET-aMD4dY-PAS49 
AETCRQFNRRTHEVWNLDCGAAPAAPAPAAPAAPAPAAPAAPAPAAPAAPAPAAPAAPAPAA
PAAPAPAAPAAPAPAGCRQFNRRTHEVWNLDC (SEQ ID NO: 82)
(k) AET-aMD4dY-PAS100 
AETCRQFNRRTHEVWNLDCGAAPAAPAPAAPAAPAPAAPAAPAPAAPAAPAPAA
PAAPAPAAPAAPAPAAPAAPAPAAPAAPAPAAPAAPAPAAPAAPAPAAPAAPAPAA
PAAPAPAAPAGCRQFNRRTHEVWNLDC (SEQ ID NO: 83)
(1) AET-aMD4dY-PAS200 
AETCRQFNRRTHEVWNLDCGAAPAAPAPAAPAAPAPAAPAAPAPAAPAAPAPAA
PAAPAPAAPAAPAPAAPAAPAPAAPAAPAPAAPAAPAPAAPAAPAPAAPAAPAPAA
PAAPAPAAPAAPAPAAPAAPAPAAPAAPAPAAPAAPAPAAPAAPAPAAPAAPAPAA
PAAPAPAAPAAPAPAAPAAPAPAAPAAPAPAAPAAPAPAAPAAPAPAAPAAPAPGC
RQFNRRTHEVWNLDC (SEQ ID NO: 84)
(m) AET-aMD4dY-GS15 
AETCRQFNRRTHEVWNLDCGGGGGSGGGGSGGGGSGCRQFNRRTHEVWNLD
C (SEQ ID NO: 85)
(n) AET-aMD4dY-GS22

AETCRQFNRRTHEVWNLDCGGGGGSGGGGSGGGGSGGGGSGGGCRQFNRRT
HEVWNLDC (SEQ ID NO: 86)

### (14-3) Expression of HGF-PAS dimer peptide linker variants

The expression of these HGF-PAS dimer peptide linker variants was examined using Corynex (registered trademark). Hereinafter, examples of expression examinations using Corynex (registered trademark) will be described.

### (14-4) Construction of each secretory expression plasmid of AET-aMD4dY-PAS8, AET-aMD4dY-PAS49, AET-aMD4dY-PAS100, AET-aMD4dY-PAS200, AET-aMD4dY-GS15, and AET-aMD4dY-GS22 using CspB signal sequence

As the HGF-PAS dimer peptide linker variant, six types of amino acid sequences of the above (i) AET-aMD4dY-PAS8 (which may be hereinafter referred to as "AET-aMD4dY-PAS8"), (j) AET-aMD4dY-PAS49 (which may be hereinafter referred to as "AET-aMD4dY-PAS49"), (k) AET-aMD4dY-PAS100 (which may be hereinafter referred to as "AET-aMD4dY-PAS100"), (1) AET-aMD4dY-PAS200 (which may be hereinafter referred to as "AET-aMD4dY-PAS200"), (m) AET-aMD4dY-GS15 (which may be hereinafter referred to as "AET-aMD4dY-GS15"), and (n) AET-aMD4dY-GS22 (which may be hereinafter referred to as "AET-aMD4dY-GS22") were designed, and base sequences encoding these proteins were designed in consideration of the codon usage frequency of C. glutamicum. Furthermore, the following expression cassettes were designed to enable secretory expression by C. glutamicum.

AET-aMD4dY-PAS8 was secretory-expressed as a fusion protein of AET-aMD4dY-PAS8 and a signal peptide 30 amino acid residue of CspB derived from C. glutamicum ATCC 13869 strain (hereinafter referred to as "CspBss-AET-aMD4dY-PAS8"). The base sequence and amino acid sequence encoding the designed CspBss-AET-aMD4dY-PAS8 are set forth in SEQ ID NOs: 87 and 88, respectively.
Base sequence encoding CspBss-AET-aMD4dY-PAS8 
atgtttaacaaccgtatccgcactgcagctctcgctggtgcaatcgcaatctccaccgcagcttccggcgtagctatcccagcat

tcgctgcagagacctgccgccagttcaaccgccgcacccacgaagtgtggaacctggattgcggcgctgcaccagccgctc
 cagcaccaggctgccgccagttcaaccgccgcacccacgaagtgtggaacctggattgctaa (SEQ ID NO: 87)
Amino acid sequence of CspBss-AET-aMD4dY-PAS8

MFNNRIRTAALAGAIAISTAASGVAIPAFAAETCRQFNRRTHEVWNLDCGAAPA
APAPGCRQFNRRTHEVWNLDC (SEQ ID NO: 88)

AET-aMD4dY-PAS49 was secretory-expressed as a fusion protein of AET-aMD4dY-PAS49 and a signal peptide 30 amino acid residue of CspB derived from C. glutamicum ATCC 13869 strain (hereinafter referred to as "CspBss-AET-aMD4dY-PAS49"). The base sequence and amino acid sequence encoding the designed CspBss-AET-aMD4dY-PAS49 are set forth in SEQ ID NOs: 89 and 90, respectively.
Base sequence encoding CspBss-AET-aMD4dY-PAS49 
atgtttaacaaccgtatccgcactgcagctctcgctggtgcaatcgcaatctccaccgcagcttccggcgtagctatcccagcat
tcgctgcagagacctgtcgtcagttcaaccgtcgcacccacgaagtttggaatttggactgtggcgcagcaccagcggcacc
agcaccagcagccccagcggcaccagccccagctgcaccagccgctccagcaccagcagcacccgcagcccccgctcc
cgcagcccccgccgcaccagcacccgcagcaccagcagcacccgctccagcaggttgtcgccaattcaaccgccgcacc
cacgaagtgtggaacctggattgctaa (SEQ ID NO: 89)
Amino acid sequence of CspBss-AET-aMD4dY-PAS49

MFNNRIRTAALAGAIAISTAASGVAIPAFAAETCRQFNRRTHEVWNLDCGAAPA
APAPAAPAAPAPAAPAAPAPAAPAAPAPAAPAAPAPAAPAAPAPAGCRQFNRRTHE
VWNLDC (SEQ ID NO: 90)

AET-aMD4dY-PAS100 was secretory-expressed as a fusion protein of AET-aMD4dY-PAS100 and a signal peptide 30 amino acid residue of CspB derived from C. glutamicum ATCC 13869 strain (hereinafter referred to as "CspBss-AET-aMD4dY-PAS100"). The base sequence and amino acid sequence encoding the designed CspBss-AET-aMD4dY-PAS100 are set forth in SEQ ID NOs: 91 and 92, respectively.
Base sequence encoding CspBss-AET-aMD4dY-PAS100 
atgtttaacaaccgtatccgcactgcagctctcgctggtgcaatcgcaatctccaccgcagcttccggcgtagctatcccagcat
tcgctgcagagacctgtcgtcagttcaaccgtcgcacccacgaagtttggaatttggactgtggcgcagcaccagcggcacc
agcaccagcagccccagcggcaccagccccagctgcaccagccgctccagcaccagcagccccagcagcccccgctcc
 
agcagcacccgcagcaccagctcccgcagccccagcagccccagccccagcagcaccagcagccccagctccagcagc
cccagctgcaccagctccagccgcaccagcagctccagccccagccgctcccgccgccccagcccccgccgcaccagcc
gcccctgctcccgccgccccagccgctcccgctccagccgctcctgccggttgccgccagttcaaccgccgcacccacgaa
gtgtggaacctggattgctaa (SEQ ID NO: 91)
Amino acid sequence of CspBss-AET-aMD4dY-PAS100

MFNNRIRTAALAGAIAISTAASGVAIPAFAAETCRQFNRRTHEVWNLDCGAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAGCRQFNRRTHEVWNL
DC (SEQ ID NO: 92)

AET-aMD4dY-PAS200 was secretory-expressed as a fusion protein of AET-aMD4dY-PAS200 and a signal peptide 30 amino acid residue of CspB derived from C. glutamicum ATCC 13869 strain (hereinafter referred to as "CspBss-AET-aMD4dY-PAS200"). The base sequence and amino acid sequence encoding the designed CspBss-AET-aMD4dY-PAS200 are set forth in SEQ ID NOs: 93 and 93, respectively.
Base sequence encoding CspBss-AET-aMD4dY-PAS200 
atgtttaacaaccgtatccgcactgcagctctcgctggtgcaatcgcaatctccaccgcagcttccggcgtagctatcccagcat
tcgctgcagagacctgtcgtcagttcaaccgtcgcacccacgaagtttggaatttggactgtggcgcagcaccagcggcacc
agcaccagcagccccagcggcaccagccccagctgcaccagccgctccagcaccagccgcccccgccgcccccgcccc
tgctgctcctgctgctcccgctcccgctgctcccgctgcccctgctcctgctgctcccgctgcgccagccccagcagcaccag
ctgccccagcaccagctgcaccagccgcaccagctccagcagccccagctgctccagcaccagcagcaccagcagcacc
agctccagcggcaccagcagcaccagcacctgcggcaccagcggctccagccccagcagcaccagcagcgccagcacc
agcagctccagcagcaccagcaccagcagcaccagcggcaccagccccagctgcaccagccgctccagcaccagctgcc
ccagcagctccagccccagccgcacctgccgcacctgcccctgccgctcctgctgcccccgctcctgccgctcctgctgccc
ctgcccctgctgctcctgctgctccagctccagccgcaccagccgctccagcaccagctgcaccagcagctccagccccag
ctgccccagcagctccagcccctggttgccgccaatttaatcgccgcacccacgaggtctggaatcttgactgctaa (SEQ
ID NO: 93)
Amino acid sequence of CspBss-AET-aMD4dY-PAS200

MFNNRIRTAALAGAIAISTAASGVAIPAFAAETCRQFNRRTHEVWNLDCGAAPA
APAPAAPAAPAPAAPAAPAPAAPAAPAPAAPAAPAPAAPAAPAPAAPAAPAPAAPA

APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPGCRQFNRRTHEVWNLDC (SEQ ID NO:
94)

AET-aMD4dY-GS15 was secretory-expressed as a fusion protein of AET-aMD4dY-GS15 and a signal peptide 30 amino acid residue of CspB derived from C. glutamicum ATCC 13869 strain (hereinafter referred to as "CspBss-AET-aMD4dY-GS15"). The base sequence and amino acid sequence encoding the designed CspBss-AET-aMD4dY-GS15 are set forth in SEQ ID NOs: 95 and 96, respectively.
Base sequence encoding CspBss-AET-aMD4dY-GS15 
atgtttaacaaccgtatccgcactgcagctctcgctggtgcaatcgcaatctccaccgcagcttccggcgtagctatcccagcat
tcgctgcagagacctgccgccagttcaaccgccgcacccacgaagtgtggaacctggattgcggcggcggcggcggctcc
ggcggcggcggctccggcggcggcggctccggctgccgccagttcaaccgccgcacccacgaagtgtggaacctggatt
gctaa (SEQ ID NO: 95)
Amino acid sequence of CspBss-AET-aMD4dY-GS15

MFNNRIRTAALAGAIAISTAASGVAIPAFAAETCRQFNRRTHEVWNLDCGGGGG
SGGGGSGGGGSGCRQFNRRTHEVWNLDC (SEQ ID NO: 96)

AET-aMD4dY-GS22 was secretory-expressed as a fusion protein of AET-aMD4dY-GS22 and a signal peptide 30 amino acid residue of CspB derived from C. glutamicum ATCC 13869 strain (hereinafter referred to as "CspBss-AET-aMD4dY-GS22"). The base sequence and amino acid sequence encoding the designed CspBss-AET-aMD4dY-GS22 are set forth in SEQ ID NOs: 97 and 98, respectively.
Base sequence encoding CspBss-AET-aMD4dY-GS22 
atgtttaacaaccgtatccgcactgcagctctcgctggtgcaatcgcaatctccaccgcagcttccggcgtagctatcccagcat
tcgctgcagagacctgccgccagttcaaccgccgcacccacgaagtgtggaacctggattgcggcggcggcggcggctcc
ggcggcggcggttctggcggtggcggttctggcggcggcggttctggcggcggttgtcgtcagttcaatcgtcgcacccacg
aagtctggaatctcgactgctaa (SEQ ID NO: 97)
Amino acid sequence of CspBss-AET-aMD4dY-GS22

MFNNRIRTAALAGAIAISTAASGVAIPAFAAETCRQFNRRTHEVWNLDCGGGGG
SGGGGSGGGGSGGGGSGGGCRQFNRRTHEVWNLDC (SEQ ID NO: 98)

The promoter of the cspB gene derived from the C. glutamicum ATCC 13869 strain was linked upstream of the base sequences set forth in CspBss-AET-aMD4dY-PAS8, CspBss-AET-aMD4dY-PAS49, CspB ss-AET-aMD4dY-PAS100, CspBss-AET-aMD4dY-PAS200, CspBss-AET-aMD4dY-GS15, and CspBss-AET-aMD4dY-GS22, and furthermore, an expression cassette of each HGF-PAS dimer peptide linker variant having a KpnI site on the 5'-side and an BamHI site on the 3'-side was designed and totally synthesized. The totally synthesized DNA fragment (HGF-PAS dimer peptide linker variant expression cassette) was inserted into the KpnI-BamHI site of pPK4 described in Japanese Patent Application Publication No. Hei 9-322774 to construct pPK4_CspBss-AET-aMD4dY-PAS8, pPK4_CspBss-AET-aMD4dY-PAS49, pPK4_CspBss-AET-aMD4dY-PAS100, pPK4_CspBss-AET-aMD4dY-PAS200, pPK4_CspBss-AET-aMD4dY-GS15, and pPK4_CspBss-AET-aMD4dY-GS22, a secretory expression plasmid for each HGF-PAS dimer peptide linker variant using the CspB signal sequence. As a result of determining the base sequence of the inserted fragment, it was confirmed that each HGF-PAS dimer peptide linker variant expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

In addition, pPK4_CspBss-AET-PAS-aMD4dY-PEG11 was constructed in the same manner as above, and its construction was confirmed. AET-PAS-aMD4dY-PEG11 is from Example 4 and has a PAS linker composed of 22 amino acids.

### (14-5) Secretory expression of each HGF-PAS dimer peptide in C. glutamicum

The pPK4_CspBss-AET-aMD4dY-PAS8, pPK4_CspBss-AET-aMD4dY-PAS49, pPK4_CspBss-AET-aMD4dY-PAS100, pPK4_CspBss-AET-aMD4dY-PAS200, pPK4_C spBss-AET-aMD4dY-GS15, and pPK4_CspBss-AET-aMD4dY-GS22 constructed above were used to transform the C. glutamicum YDK010::phoS(W302C) strain described in WO2016/171224 to obtain the YDK010::phoS(W302C)/pPK4_CspBss-AET-aMD4dY-PAS8 strain, YDK010::phoS(W302C)/pPK4_CspBss-AET-aMD4dY-PAS49 strain, YDK010::phoS(W302C)/pPK4_CspBss-AET-aMD4dY-PAS100 strain, YDK010::phoS(W302C)/pPK4_CspBss-AET-aMD4dY-PAS200 strain, YDK010::phoS(W302C)/pPK4_CspBss-AET-aMD4dY-GS15 strain, and YDK010::phoS(W302C)/pPK4_CspBss-AET-aMD4dY-GS22 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be AET-aMD4dY-PAS8 was detected in the culture supernatant of the YDK010::phoS(W302C)/pPK4_CspBss-AET-aMD4dY-PAS8 strain (Fig. 20, lane 4), a protein band presumed to be AET-aMD4dY-PAS49 was detected in the culture supernatant of the YDK010::phoS(W302C)/pPK4_CspBss-AET-aMD4dY-PAS49 strain (Fig. 20, lane 6), a protein band presumed to be AET-aMD4dY-PAS100 was detected in the culture supernatant of the YDK010::phoS(W302C)/pPK4_CspBss-AET-aMD4dY-PAS100 strain (Fig. 20, lane 7), a protein band presumed to be AET-aMD4dY-PAS200 was detected in the culture supernatant of the YDK010::phoS(W302C)/pPK4_CspBss-AET-aMD4dY-PAS200 strain (Fig. 20, lane 8), a protein band presumed to be AET-aMD4dY-GS15 was detected in the culture supernatant of the YDK010::phoS(W302C)/pPK4_CspBss-AET-aMD4dY-GS15 strain (Fig. 20, lane 2), and a protein band presumed to be AET-aMD4dY-GS22 was detected in the culture supernatant of the YDK010::phoS(W302C)/pPK4_CspBss-AET-aMD4dY-GS22 strain (Fig. 20, lane 3).

In addition, the YDK010::phoS(W302C)/pPK4_CspBss-AET-PAS-aMD4dY-PEG11 strain obtained by the same method as above was cultured in the same manner as above, and a protein band presumed to be AET-PAS-aMD4dY-PEG11 (hereinafter also referred to as "AET-aMD4dY-PAS22") was detected in the culture supernatant (Fig. 20, lane 5).

When comparing 22 residues with the same linker length, AET-aMD4dY-PAS22 (Fig. 20, lane 5: the same molecule as AET-PAS-aMD4dY-PEG11) showed higher secretion than AET-aMD4dY-GS22 (Fig. 20, lane 3), that is, the use of the PAS linker showed than that of the GS linker.

### Example 15: Molecular weight analysis of HGF-PAS dimer peptide

The cell filtrate of each HGF-PAS dimer peptide after culturing in Example 14 was subjected to molecular weight measurement using LC-MS (Waters, ACQUITY UPLC H-Class/SQD2). The analysis conditions are shown in Table 2.

**Table 2**

| **LC set up** | | | | | |
|---|---|---|---|---|---|
| **System** | | **ACQUITYUPLC H-Class /SQD2** | | | |
| **Column** | | **TMC-Triart CB 50 × 2.0 mmI.D. 1.9 um, 12nm(TO12SP9-0502PT)** | | | |
| **Solvent** | **A** | **0.1% TFA** | | | |
| | **B** | **0.1% TFA, 80% Acetonitrile** | | | |
| **Flow rate** | | **1.0 mL/min** | | | |
| **Column temp.** | | **40 °C** | | | |
| **Detection** | | **220 nm** | | | |
| **Sample Vol.** | | **10 uL** | | | |
| **Gradient program** | | **Time (min)** | **%A** | **%B** | |
| | | **0** | **100** | **0** | |
| | | **50** | **50** | **50** | |
| | | **53** | **0** | **100** | |
| | | **53.1** | **100** | **0** | |
| | | **56** | **100** | **0** | |
| | | **56.1** | **0** | **100** | |
| | | **59** | **0** | **100** | |
| | | **59.1** | **100** | **0** | |
| | | **65** | **100** | **0** | |

| **MS spectrometer set up** | | | | | |
|---|---|---|---|---|---|
| **Ion mode** | | **ESI positive** | | | |
| **Range** | | **800-2500 m/z** | | | |
| **Scan time** | | **0.4 amu/sec** | | | |
| **Capillary Voltage** | | **2.0kV** | | | |
| **Cone voltage** | | **10V, 20V, 40V** | | | |
| **Desolvation temp** | | **500 °C** | | | |
| **Desolvation Gas Flow** | | **1000 L/hr** | | | |
| **Cone Gas Flow** | | **50 L/hr** | | | |
| **Software** | | **Deconvolution is performed using "MassLynx"** | | | |

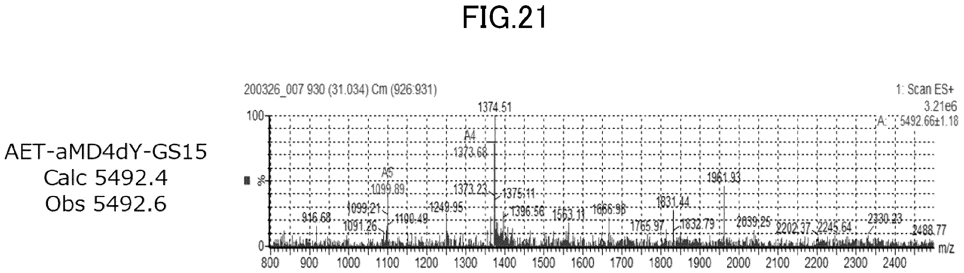

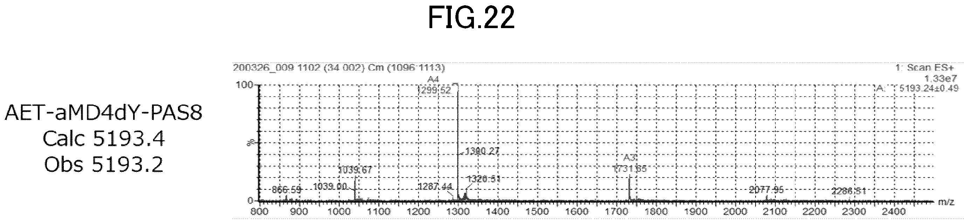

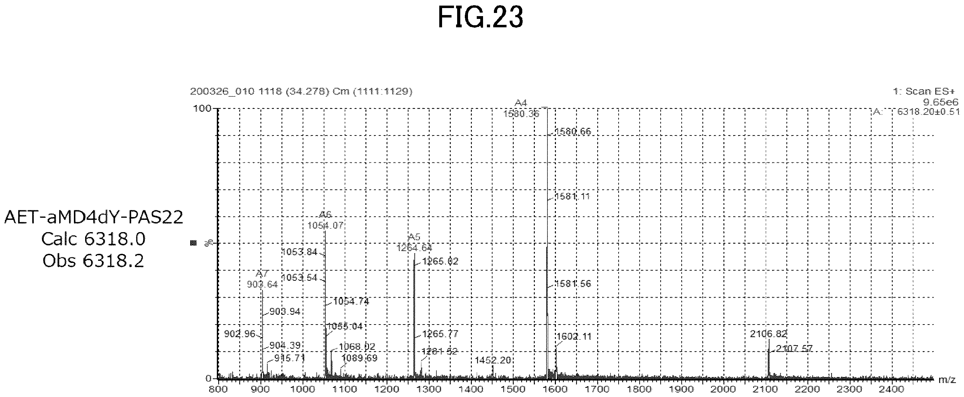

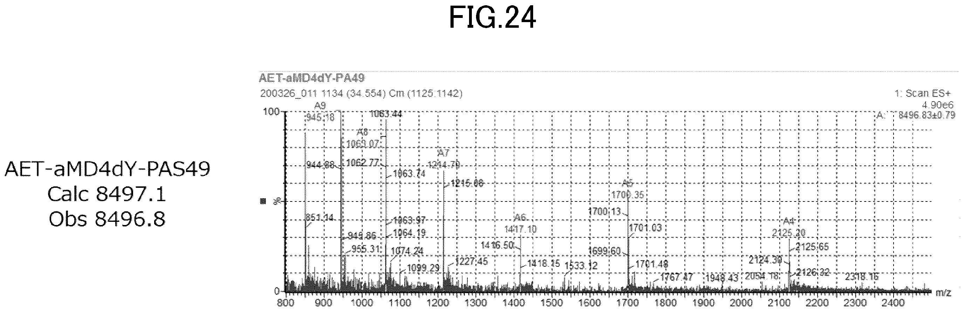

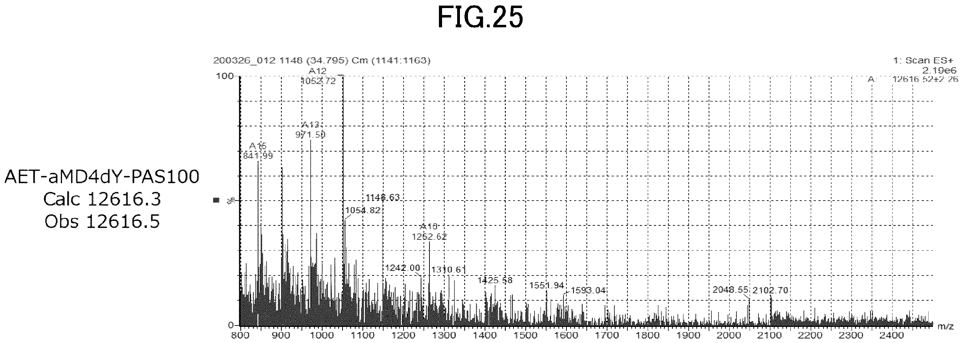

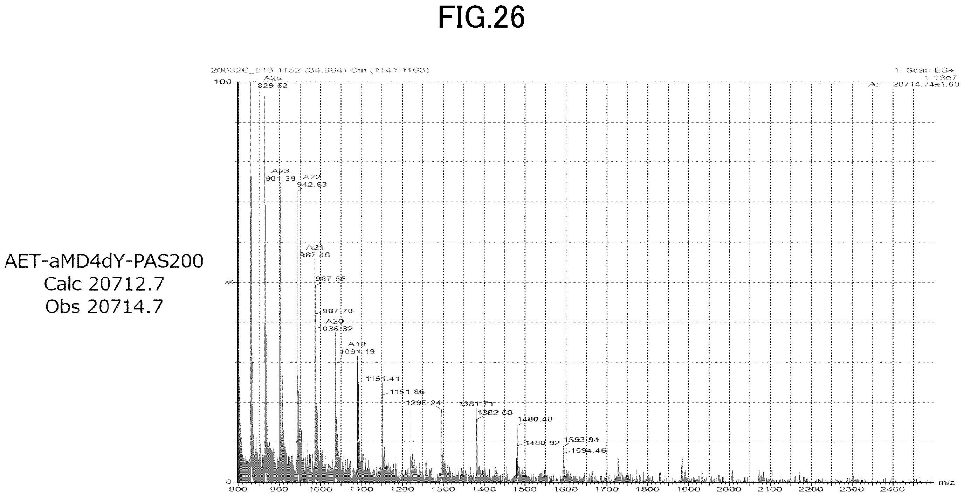

As shown in Figs. 21 to 26, almost the same value as the theoretical value of the molecular weight of each oxidized dimer peptide forming two disulfide bonds in the molecule was obtained. From this, it was confirmed that a peptide having the correct amino acid sequence containing two disulfide bonds could be expressed.

### Example 16: Activity evaluation of HGF-PAS dimer peptide linker variant

The Met activation ability of the HGF-PAS dimer peptide linker variant was evaluated by luciferase assay. To 25 µL of Opti-MEM medium (Thermo Fisher Scientific), 0.6 µL of Attractene Transfection Reagent (QIAGEN) was added, and the mixture was incubated at room temperature for 5 minutes. To the above mixed solution, a mixed solution of 25 µL of Opti-MEM and 1 µL of SRE reporter vector (QIAGEN) was added, and the mixture was incubated at room temperature for 20 minutes. This mixed solution was added to a 96-well plate, over which 40,000 cell/well HEK293E cells were seeded and incubated overnight in a 5% CO₂ incubator at 37°C. After transfection, all culture supernatant was removed, 100 µL of Opti-MEM medium (evaluation basal medium) containing 0.5% FBS (Thermo Fisher Scientific), 1% non-essential amino acid solution (Thermo Fisher Scientific), and penicillin-streptomycin (Nacalai Tesque) was added, and incubation was performed at 37°C for 4 hours, and thereby the cells were starved.

To the evaluation basal medium, 0 to 100 ng/mL HGF was added, or a culture supernatant containing AET-aMD4dY-PAS8, AET-aMD4dY-PAS22 (the same molecule as AET-PAS-aMD4dY-PEG11), AET-aMD4dY-PAS49, AET-aMD4dY-PAS100, AET-aMD4dY-PAS200, AET-aMD4dY-GS15, or AET-aMD4dY-GS22 was added to the medium so as to be diluted at a predetermined ratio, and the evaluation basal medium was added in an amount of 100 µL to the cells. The cells were stimulated by culturing overnight in a 5% CO₂ incubator at 37°C.

The signal intensities were detected using Dual-Luciferase Reporter Assay System (Promega). The medium supernatant in an amount of 100 µL was removed, 50 µL of Glo reagent was added, and the cells were lysed for 10 minutes at room temperature. The luminescence of Firefly luciferase emitted from the cell lysate solution was detected with a plate reader to quantify the activation of the HGF-Erk-SRE pathway. Subsequently, 50 µL of Glo & Stop reagent solution was added, and after 10 minutes, the luminescence of the internal standard Renilla luciferase was detected to quantify the number of cells. The signal activity of each sample was quantified as SRE activity=(Firefly luciferase luminescence intensity)/(Renilla luciferase luminescence intensity). The relative signal activity values for the samples without addition of the evaluation compound were determined and used as relative receptor activity.

As shown in Fig. 27, it was clarified that all HGF-PAS dimer peptides having different PAS linker lengths activated HGF signals. It was clarified that the highest efficiency was exhibited when the length of the PAS linker was 22 amino acids, and the maximum activity (EC50) was halved when the length exceeded 100 amino acids as shown in Table 3 below. In addition, as shown in Fig. 28, activity was also obtained by dimerization with 15 amino acid and 22 amino acid GS linkers, and their maximum activity (EC50) was less than half that of the PAS linker, as shown in Table 3 below. Thus, it was clarified that a dimer peptide having high activity could be produced by using a PAS linker.

**Table 3**

| Peptide | Eₘₐₓ (%) |
|---|---|
| AET-aMD4dY-PAS22 | 100.0 |
| AET-aMD4dY-PAS8 | 56.4 |
| AET-aMD4dY-PAS49 | 82.8 |
| AET-aMD4dY-PAS100 | 47.2 |
| AET-aMD4dY-PAS200 | 20.4 |
| AET-aMD4dY-GS15 | 45.8 |
| AET-aMD4dY-GS22 | 32.3 |

### Example 17: Design of stabilized HGF-PAS dimer peptide and its secretory expression in C. glutamicum

### (17-1) Outline of design

The HGF-PAS peptide with enhanced stability was designed as follows. As a method for enhancing the stability of peptides and proteins, a method for adding a long-chain PAS sequence has been reported (Published Japanese Translation of PCT International Application No. 2013-531480 and Published Japanese Translation of PCT International Application No. 2010-531139). As an HGF-PAS peptide with enhanced stability, a peptide was designed in which a PAS sequence composed of 200 amino acids (PAS200) was bound to the C-terminus of AET-aMD4dY-PAS22 (the same molecule as AET-PAS-aMD4dY-PEG11). That is, the peptide sequence was designed to be "first aMD4dY cyclic peptide"-"PAS linker"-"second aMD4dY cyclic peptide"-"long-chain PAS sequence (PAS200)".

### (17-2) Preparation of stabilized HGF-PAS dimer peptide

The prepared stabilized HGF-PAS dimer peptide is as follows.
(o) AET-aMD4dY-PAS22-PAS200

AETCRQFNRRTHEVWNLDCGAAPAAPAPAAPAAPAPAAPAAPGCRQFNRRTHE
VWNLDCAAPAAPAPAAPAAPAPAAPAAPAPAAPAAPAPAAPAAPAPAAPAAPAPA
APAAPAPAAPAAPAPAAPAAPAPAAPAAPAPAAPAAPAPAAPAAPAPAAPAAPAPA
APAAPAPAAPAAPAPAAPAAPAPAAPAAPAPAAPAAPAPAAPAAPAPAAPAAPAPA
APAAPAPAAPAAPAPAAPAAPAPAAPAAPAPAAPAAPAP (SEQ ID NO: 99)

### (17-3) Expression of stabilized HGF-PAS dimer peptide

Corynex (registered trademark) was used to examine the expression of stabilized HGF-PAS dimer peptide. Hereinafter, examples of expression examinations using Corynex (registered trademark) will be described.

### (17-4) Construction of secretory expression plasmid of AET-aMD4dY-PAS22-PAS200 using CspB signal sequence

As the stabilized HGF-PAS dimer peptide, the amino acid sequence of the above (a) AET-aMD4dY-PAS22-PAS200 (which may be hereinafter referred to as "AET-aMD4dY-PAS22-PAS200") was designed, and the base sequence encoding this protein was designed in consideration of the codon usage frequency of C. glutamicum. Furthermore, the following expression cassette was designed to enable secretion expression by C. glutamicum.

AET-aMD4dY-PAS22-PAS200 was secretory-expressed as a fusion protein of AET-aMD4dY-PAS22-PAS200 and a signal peptide 30 amino acid residue of CspB derived from C. glutamicum ATCC 13869 strain (hereinafter referred to as "CspBss-AET-aMD4dY-PAS22-PAS200"). The base sequence and amino acid sequence encoding the designed CspBss-AET-aMD4dY-PAS22-PAS200 are set forth in SEQ ID NOs: 100 and 101, respectively.
Base sequence encoding CspBss-AET-aMD4dY-PAS22-PAS200 
atgtttaacaaccgtatccgcactgcagctctcgctggtgcaatcgcaatctccaccgcagcttccggcgtagctatcccagcat
tcgctgcagagacctgccgtcagttcaaccgccgtacccacgaagtttggaatctcgactgtggtgccgcccctgccgctcct
gcccctgccgccccagccgctccagcaccagccgctccagcagccccaggctgccgccagttcaaccgccgcacccacg
aagtgtggaacctggattgcgcagctccagcagcccctgcccccgccgccccagccgcccctgcccccgccgcccccgcc
gcacccgcccctgccgcccccgccgcccccgcccctgctgctcctgctgctcccgctcccgctgctcccgctgcccctgctc
ctgctgctcccgctgcgccagccccagcagcaccagctgccccagcaccagctgcaccagccgcaccagctccagcagcc
ccagctgctccagcaccagcagcaccagcagcaccagctccagcggcaccagcagcaccagcacctgcggcaccagcg
gctccagccccagcagcaccagcagcgccagcaccagcagctccagcagcaccagcaccagcagcaccagcggcacca
gccccagctgcaccagccgctccagcaccagctgccccagcagctccagccccagctgcccctgctgcccccgctcctgcc
gctcccgctgcccctgcccctgctgctcctgctgctcccgctcctgccgctcccgctgcgccagctccagcagccccagctgc
accagccccagccgcaccagccgccccagcccccgcagcacccgcagcaccagccccataa (SEQ ID NO: 100)
Amino acid sequence of CspBss-AET-aMD4dY-PAS22-PAS200

MFNNRIRTAALAGAIAISTAASGVAIPAFAAETCRQFNRRTHEVWNLDCGAAPA
APAPAAPAAPAPAAPAAPGCRQFNRRTHEVWNLDCAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAP (SEQ ID NO: 101)

The promoter of the cspB gene derived from the C. glutamicum ATCC 13869 strain was linked upstream of the base sequence set forth in CspBss-AET-aMD4dY-PAS22-PAS200, and furthermore, an expression cassette of stabilized HGF-PAS dimer peptide having a KpnI site on the 5'-side and an BamHI site on the 3'-side was designed and totally synthesized. The totally synthesized DNA fragment (stabilized HGF-PAS dimer peptide expression cassette) was inserted into the KpnI-BamHI site of pPK4 described in Japanese Patent Application Publication No. Hei 9-322774 to construct pPK4_CspBss-AET-aMD4dY-PAS22-PAS200, a secretory expression plasmid for the stabilized HGF-PAS dimer peptide using the CspB signal sequence. As a result of determining the base sequence of the inserted fragment, it was confirmed that the stabilized HGF-PAS dimer peptide expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (17-5) Secretory expression of stabilized HGF-PAS dimer peptide in C. glutamicum

The pPK4_CspBss-AET-aMD4dY-PAS22-PAS200 constructed above was used to transform the C. glutamicum YDK010::phoS(W302C) strain described in WO2016/171224 to obtain the YDK010: :phoS(W302C)/pPK4_CspBss-AET-aMD4dY-PAS22-PAS200 strain.

The obtained transformants were cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

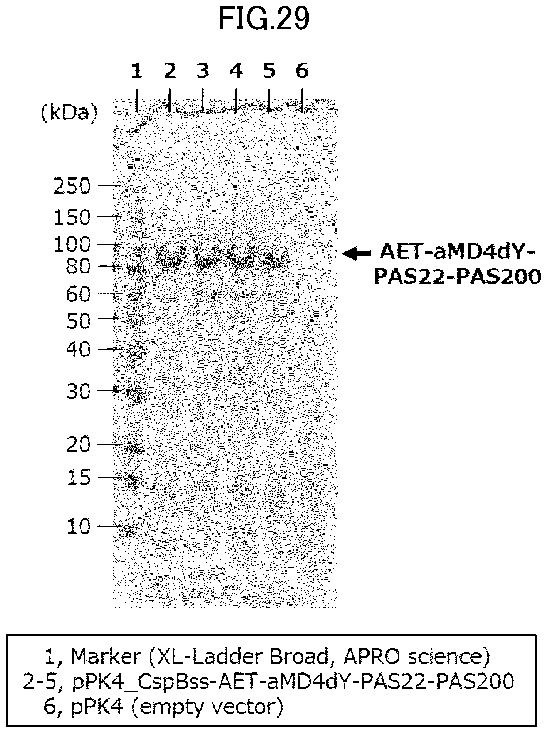

After completion of the culture, 6.5 µL of the culture supernatant obtained by centrifuging each culture solution was subjected to reduced SDS-PAGE using NuPAGE (registered trademark) 4 to 12% Bis-Tirs Gel (Thermo Fisher Scientific), and then stained with Quick-CBB (Wako). As a result, a protein band presumed to be AET-aMD4dY-PAS22-PAS200 was detected in the culture supernatant of the YDK010::phoS(W302C)/pPK4_CspBss-AET-aMD4dY-PAS22-PAS200 strain (Fig. 29, lanes 2 to 5).

### Example 18: Molecular weight analysis of stabilized HGF-PAS dimer peptide

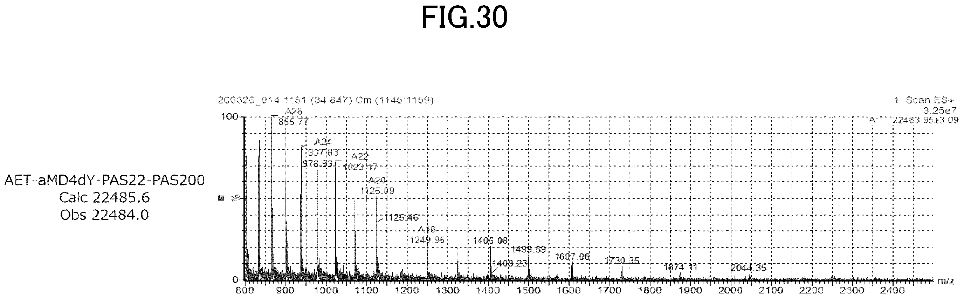

The cell filtrate of the stabilized HGF-PAS dimer peptide after culturing in Example 17 was subjected to molecular weight measurement using LC-MS (Waters, ACQUITY UPLC H-Class/SQD2). The analysis conditions are shown in Table 2. As shown in Fig. 30, almost the same value as the theoretical value of the molecular weight of each oxidized dimer peptide forming two disulfide bonds in the molecule was obtained. From this, it was confirmed that a peptide having the correct amino acid sequence containing two disulfide bonds could be expressed.

### Example 19: Activity evaluation of stabilized HGF-PAS dimer peptide

The Met activation ability of the stabilized HGF-PAS dimer peptide was evaluated by luciferase assay. To 25 µL of Opti-MEM medium (Thermo Fisher Scientific), 0.6 µL of Attractene Transfection Reagent (QIAGEN) was added, and the mixture was incubated at room temperature for 5 minutes. To the above mixed solution, a mixed solution of 25 µL of Opti-MEM and 1 µL of SRE reporter vector (QIAGEN) was added, and the mixture was incubated at room temperature for 20 minutes. This mixed solution was added to a 96-well plate, over which 40,000 cell/well HEK293E cells were seeded and incubated overnight in a 5% CO₂ incubator at 37°C. After transfection, all culture supernatant was removed, 100 µL of Opti-MEM medium (evaluation basal medium) containing 0.5% FBS (Thermo Fisher Scientific), 1% non-essential amino acid solution (Thermo Fisher Scientific), and penicillin-streptomycin (Nacalai Tesque) was added, and incubation was performed at 37°C for 4 hours, and thereby the cells were starved.

To the evaluation basal medium, 0 to 100 ng/mL HGF was added, or a culture supernatant containing AET-aMD4dY-PAS22-PAS200 was added to the medium so as to be diluted at a predetermined ratio, and the evaluation basal medium was added in an amount of 100 µL to the cells. The cells were stimulated by culturing overnight in a 5% CO₂ incubator at 37°C.

The signal intensities were detected using Dual-Luciferase Reporter Assay System (Promega). The medium supernatant in an amount of 100 µL was removed, 50 µL of Glo reagent was added, and the cells were lysed for 10 minutes at room temperature. The luminescence of Firefly luciferase emitted from the cell lysate solution was detected with a plate reader to quantify the activation of the HGF-Erk-SRE pathway. Subsequently, 50 µL of Glo & Stop reagent solution was added, and after 10 minutes, the luminescence of the internal standard Renilla luciferase was detected to quantify the number of cells. The signal activity of each sample was quantified as SRE activity=(Firefly luciferase luminescence intensity)/(Renilla luciferase luminescence intensity). The relative signal activity values for the samples without addition of the evaluation compound were determined and used as relative receptor activity.

As shown in Fig. 31, it was clarified that the stabilized HGF-PAS dimer peptide had a signal activation ability and the maximum activity was about the same as that of 100 ng/mL HGF.

### Example 20: Design of EPO-PAS dimer peptide and its secretory expression in C. glutamicum

### (20-1) Outline of design

Peginesatide has been reported as an erythropoietin mimetic peptide. Pedinesatide has a structure in which a lysine is added to the C-terminus of an erythropoietin receptor-binding peptide and ε-amino groups are bound to each other with a dicarboxylic acid linker linked by a long-chain PEG. However, expression of Peginesatide in C. glutamicum as a single polypeptide chain cannot be performed for the following reasons.
(1) Peginesatide contains unnatural amino acids, and peptides containing unnatural amino acids cannot be expressed in C. glutamicum.
(2) Peptides containing a dicarboxylic acid linker cannot be expressed in C. glutamicum.
(3) Dimerization is achieved by binding the C-terminuses of two cyclic peptide molecules with the ε-amino group of lysine, but a structure with C-terminuses bound to each other and a structure with the ε-amino group of lysine and another amino acid forming an amide bond cannot be expressed in C. glutamicum.

Therefore, the molecule was converted as follows to design an expressible form. The above (1) can be expressed by using an erythropoietin receptor-binding cyclic peptide (US5773569A) that does not contain an unnatural amino acid. The above (2) can be expressed by using a peptidic linker. Regarding (3) above, the N-terminus of one cyclic peptide and the C-terminus of the other peptide were bound to each other using a PAS sequence instead of the C-terminuses of the cyclic peptides. That is, the obstacles were dealt with by designing the peptide sequence to be "first erythropoietin receptor-binding cyclic peptide"-"PAS linker"-"second erythropoietin receptor-binding cyclic peptide". That is, the C-terminus of the first erythropoietin receptor-binding cyclic peptide and the N-terminus of the PAS linker are linked by an amide bond to the N-terminus of the second erythropoietin receptor-binding cyclic peptide and the C-terminus of the PAS linker, respectively, and the peptide chain can be expressed as a single polypeptide chain.

### (20-2) Preparation of EPO-PAS dimer peptides

The prepared EPO-PAS dimer peptides are as follows.
(p) EPO-PAS8 GGLYACHMGPMTWVCQPLRGAAPAAPAPGGLYACHMGPMTWVCQPLRG (SEQ ID NO: 102)
(q) EPO-PAS22 
GGLYACHMGPMTWVCQPLRGAAPAAPAPAAPAAPAPAAPAAPGGLYACHMGP
MTWVCQPLRG (SEQ ID NO: 103)
(r) AET-EPO-PAS8 
AETGGLYACHMGPMTWVCQPLRGAAPAAPAPGGLYACHMGPMTWVCQPLRG
(SEQ ID NO: 104)
(s) AET-EPO-PAS22

AETGGLYACHMGPMTWVCQPLRGAAPAAPAPAAPAAPAPAAPAAPGGLYACHM
GPMTWVCQPLRG (SEQ ID NO: 105)

### (20-3) Expression of EPO-PAS dimer peptides

The expression of these EPO-PAS dimer peptides was examined using Corynex (registered trademark). Hereinafter, examples of expression examinations using Corynex (registered trademark) will be described.

### (20-4) Construction of EPO-PAS8 and EPO-PAS22 secretory expression plasmids using CspA signal sequence

As the EPO-PAS dimer peptide, two types of amino acid sequences of the above (p) EPO-PAS8 (which may be hereinafter referred to as "EPO-PAS8") and (q) EPO-PAS22 (which may be hereinafter referred to as "EPO-PAS22") were designed, and the base sequences encoding these proteins were designed in consideration of the codon usage frequency of C. glutamicum. Furthermore, the following expression cassettes were designed to enable secretory expression by C. glutamicum.

EPO-PAS8 was secretory-expressed as a fusion protein of EPO-PAS8 and a signal peptide 25 amino acid residue of CspA derived from C. ammoniagenes ATCC 6872 strain (hereinafter referred to as "CspAss-EPO-PAS8"). The base sequence and amino acid sequence encoding the designed CspAss-EPO-PAS8 are set forth in SEQ ID NOs: 106 and 107, respectively.
Base sequence encoding CspAss-EPO-PAS8 
atgaaacgcatgaaatcgctggctgcggcgctcaccgtcgctggggccatgctggccgcacctgtggcaacggcaggtggc

ctttacgcttgtcacatgggtccaatgacctgggtttgtcagcccctccgtggtgccgctcctgccgcccccgctcctggtggcc

tgtacgcatgccatatgggtccaatgacttgggtgtgccagccactccgcggctaa (SEQ ID NO: 106)
Amino acid sequence of CspAss-EPO-PAS8

MKRMKSLAAALTVAGAMLAAPVATAGGLYACHMGPMTWVCQPLRGAAPAAP
APGGLYACHMGPMTWVCQPLRG (SEQ ID NO: 107)

EPO-PAS22 was secretory-expressed as a fusion protein of EPO-PAS22 and a signal peptide 25 amino acid residue of CspA derived from C. ammoniagenes ATCC 6872 strain (hereinafter referred to as "CspAss-EPO-PAS22"). The base sequence and amino acid sequence encoding the designed CspAss-EPO-PAS22 are set forth in SEQ ID NOs: 108 and 109, respectively.
Base sequence encoding CspAss-EPO-PAS22 
atgaaacgcatgaaatcgctggctgcggcgctcaccgtcgctggggccatgctggccgcacctgtggcaacggcaggcgg
cctttacgcttgtcacatgggtccaatgacctgggtctgtcaacctctccgtggtgcagccccggcagcccccgctccagccg
caccagcagccccagccccagctgcaccagccgctccaggcggcctctacgcctgccacatgggtcctatgacctgggtgt
gccaaccacttcgcggctaa (SEQ ID NO: 108)
Amino acid sequence of CspAss-EPO-PAS22

MKRMKSLAAALTVAGAMLAAPVATAGGLYACHMGPMTWVCQPLRGAAPAAP
APAAPAAPAPAAPAAPGGLYACHMGPMTWVCQPLRG (SEQ ID NO: 109)

The promoter of the cspB gene derived from the C. glutamicum ATCC 13869 strain was linked upstream of the base sequences set forth in CspAss-EPO-PAS8 and CspAss-EPO-PAS22, and furthermore, an expression cassette of each EPO-PAS dimer peptide having a KpnI site on the 5'-side and an BamHI site on the 3'-side was designed and totally synthesized. The totally synthesized DNA fragment (EPO-PAS dimer peptide expression cassette) was inserted into the KpnI-BamHI site of pPK4 described in Japanese Patent Application Publication No. Hei 9-322774 to construct pPK4_CspAss-EPO-PAS8 and pPK4_CspAss-EPO-PAS22, a secretory expression plasmid for each EPO-PAS dimer peptide using the CspA signal sequence. As a result of determining the base sequence of the inserted fragment, it was confirmed that each EPO-PAS dimer peptide expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (20-5) Construction of each secretory expression plasmid of AET-EPO-PAS8 and AET-EPO-PAS22 using CspB signal sequence

As the EPO-PAS dimer peptide, two types of amino acid sequences of the above (r) AET-EPO-PAS8 (which may be hereinafter referred to as "AET-EPO-PAS8") and (s) AET-EPO-PAS22 (which may be hereinafter referred to as "AET-EPO-PAS22") were designed, and the base sequences encoding these proteins were designed in consideration of the codon usage frequency of C. glutamicum. Furthermore, the following expression cassettes were designed to enable secretory expression by C. glutamicum.

AET-EPO-PAS8 was secretory-expressed as a fusion protein of AET-EPO-PAS8 and a signal peptide 30 amino acid residue of CspB derived from C. glutamicum ATCC 13869 strain (hereinafter referred to as "CspBss-AET-EPO-PAS8"). The base sequence and amino acid sequence encoding the designed CspBss-AET-EPO-PAS8 are set forth in SEQ ID NOs: 110 and 111, respectively.
Base sequence encoding CspBss-AET-EPO-PAS8 
atgtttaacaaccgtatccgcactgcagctctcgctggtgcaatcgcaatctccaccgcagcttccggcgtagctatcccagcat
tcgctgcagagaccggtggcctttacgcttgtcacatgggtccaatgacctgggtttgtcagcccctccgtggtgccgctcctg
ccgcccccgctcctggtggcctgtacgcatgccatatgggtccaatgacttgggtgtgccagccactccgcggctaa (SEQ
ID NO: 110)
Amino acid sequence of CspBss-AET-EPO-PAS8

MFNNRIRTAALAGAIAISTAASGVAIPAFAAETGGLYACHMGPMTWVCQPLRGA
APAAPAPGGLYACHMGPMTWVCQPLRG (SEQ ID NO: 111)

AET-EPO-PAS22 was secretory-expressed as a fusion protein of AET-EPO-PAS22 and a signal peptide 30 amino acid residue of CspB derived from C. glutamicum ATCC 13869 strain (hereinafter referred to as "CspBss-AET-EPO-PAS22"). The base sequence and amino acid sequence encoding the designed CspBss-AET-EPO-PAS22 are set forth in SEQ ID NOs: 112 and 113, respectively.
Base sequence encoding CspBss-AET-EPO-PAS22 
atgtttaacaaccgtatccgcactgcagctctcgctggtgcaatcgcaatctccaccgcagcttccggcgtagctatcccagcat
tcgctgcagagaccggcggcctttacgcttgtcacatgggtccaatgacctgggtctgtcaacctctccgtggtgcagccccg
gcagcccccgctccagccgcaccagcagccccagccccagctgcaccagccgctccaggcggcctctacgcctgccacat
gggtcctatgacctgggtgtgccaaccacttcgcggctaa (SEQ ID NO: 112)
Amino acid sequence of CspBss-AET-EPO-PAS22

MFNNRIRTAALAGAIAISTAASGVAIPAFAAETGGLYACHMGPMTWVCQPLRGA
APAAPAPAAPAAPAPAAPAAPGGLYACHMGPMTWVCQPLRG (SEQ ID NO: 113)

The promoter of the cspB gene derived from the C. glutamicum ATCC 13869 strain was linked upstream of the base sequences set forth in CspBss-AET-EPO-PAS8 and CspBss-AET-EPO-PAS22, and furthermore, an expression cassette of each EPO-PAS dimer peptide having a KpnI site on the 5'-side and an BamHI site on the 3'-side was designed and totally synthesized. The totally synthesized DNA fragment (EPO-PAS dimer peptide expression cassette) was inserted into the KpnI-BamHI site of pPK4 described in Japanese Patent Application Publication No. Hei 9-322774 to construct pPK4_CspBss-AET-EPO-PAS8 and pPK4_CspBss-AET-EPO-PAS22, a secretory expression plasmid for each EPO-PAS dimer peptide using the CspB signal sequence. As a result of determining the base sequence of the inserted fragment, it was confirmed that each EPO-PAS dimer peptide expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (20-6) Secretory expression of each EPO-PAS dimer peptide in C. glutamicum

The pPK4_CspAss-EPO-PAS8, pPK4_CspAss-EPO-PAS22, pPK4_CspBss-AET-EPO-PAS8, and pPK4_CspBss-AET-EPO-PAS22 constructed above were used to transform the C. glutamicum YDK0107 strain described in WO2016/171224 to obtain the YDK0107/pPK4_CspAss-EPO-PAS8 strain, YDK0107/pPK4_CspAss-EPO-PAS22 strain, YDK0107/pPK4_CspBss-AET-EPO-PAS8 strain, and YDK0107/pPK4_CspBss-AET-EPO-PAS22 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

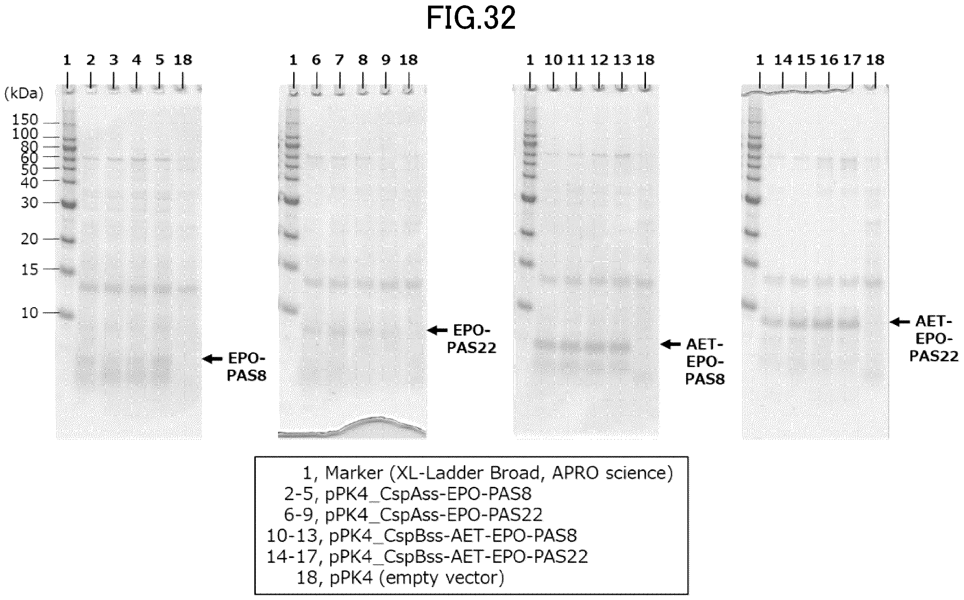

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be EPO-PAS8 was detected in the culture supernatant of the YDK0107/pPK4_CspAss-EPO-PAS8 strain (Fig. 32, lanes 2 to 5), a protein band presumed to be EPO-PAS22 was detected in the culture supernatant of the YDK0107/pPK4_CspAss-EPO-PAS22 strain (Fig. 32, lanes 6 to 9), a protein band presumed to be AET-EPO-PAS8 was detected in the culture supernatant of the YDK0107/pPK4_CspBss-AET-EPO-PAS8 strain (Fig. 32, lanes 10 to 13), and a protein band presumed to be AET-EPO-PAS22 was detected in the culture supernatant of the YDK0107/pPK4_CspBss-AET-EPO-PAS22 strain (Fig. 32, lanes 14 to 17).

### Example 21: Molecular weight analysis of EPO-PAS dimer peptide

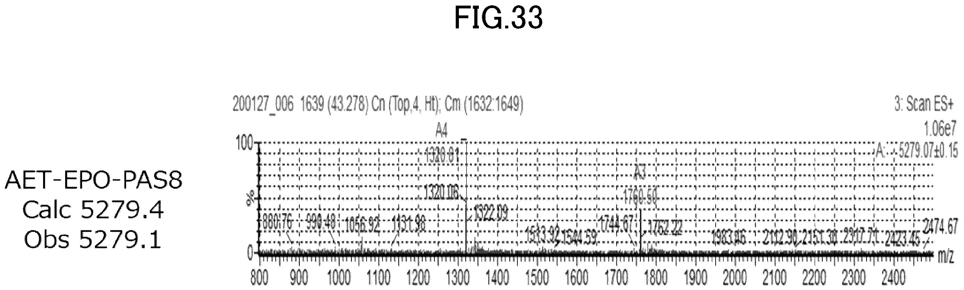

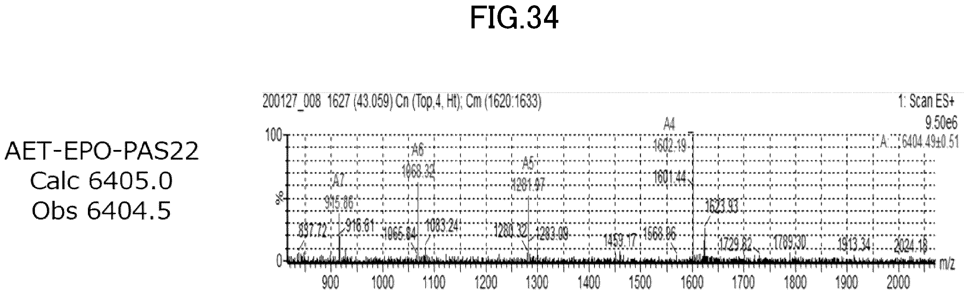

The cell filtrate of AET-EPO-PAS8 and AET-EPO-PAS22 after culturing in Example 20 was subjected to molecular weight measurement using LC-MS (Waters, ACQUITY UPLC/SQD2). The analysis conditions are shown in Table 2. As shown in Figs. 33 and 34, almost the same value as the theoretical value of the molecular weight of each oxidized dimer peptide forming two disulfide bonds in the molecule was obtained. From this, it was confirmed that a peptide having the correct amino acid sequence containing two disulfide bonds could be expressed.

### Example 22: Activity evaluation of EPO-PAS dimer peptide

The EpoR activation ability of the EPO-PAS dimer peptide was evaluated using PathHunter (registered trademark) eXpress EpoR-JAK2 Functional Assay Kit (DiscoverX).

Cells for EPO activity evaluation included in PathHunter (registered trademark) eXpress EpoR-JAK2 Functional Assay Kit were seeded on a 96-well plate and cultured for 24 hours at 37°C under 5% CO₂. To the cells, 860 pg/L to 900 µg/L recombinant human erythropoietin (rhEPO, Peprotech), or a culture supernatant containing AET-EPO-PAS8 or AET-EPO-PAS22 diluted to a predetermined concentration (cultured as described in Example 20), or the monomer peptide EMP35 (GGLYACHMGPMTWVCQPLRG, synthesized by GenScript Japan) was added. As a negative control, the culture supernatant (Fig. 32, lane 18) obtained by culturing as described in Example 20 except for using a vector containing no gene for expressing the EPO-PAS dimer peptide, diluted at the same ratio as the culture supernatant containing AET-EPO-PAS8 or AET-EPO-PAS22, was added. The prepared Substrate Reagent was added to the cells stimulated at room temperature for 3 hours, and the cells were incubated for 60 minutes. The chemiluminescence intensity was quantified with a Nivo plate reader (Perkin Elmer).

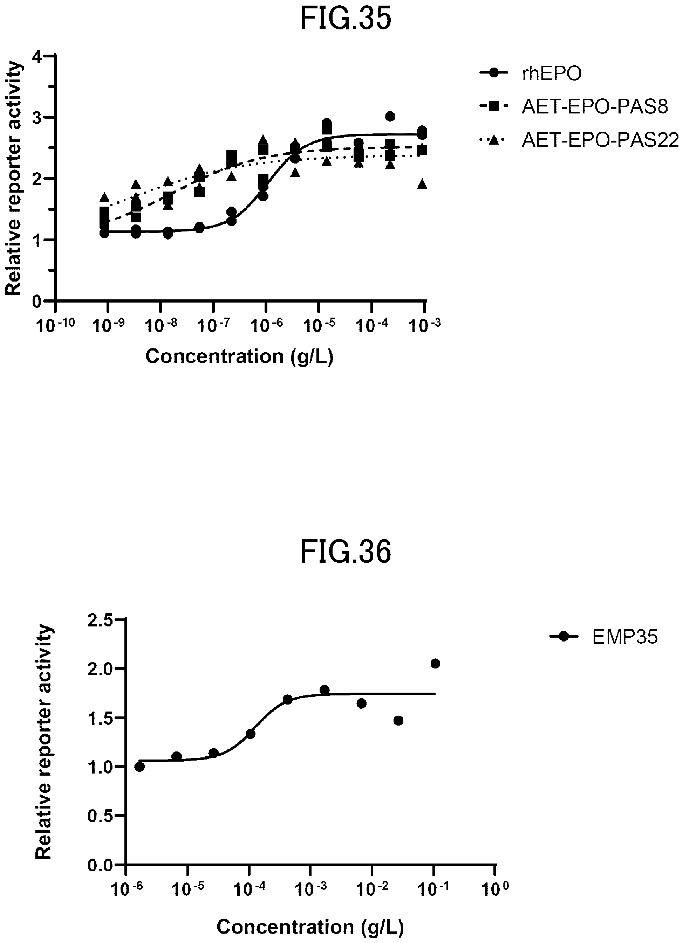

As shown in Fig. 35, it was clarified that AET-EPO-PAS8 and AET-EPO-PAS22 activated EpoR in a concentration-dependent manner. The maximum activation ability was almost the same as that of rhEPO. In addition, as shown in Table 4 below, as for the EC50 values, rhEPO was found to be 1.1 × 10⁻⁶ g/L, while AET-EPO-PAS8 was found to be 1.9 × 10⁻⁷ g/L and AET-EPO-PAS22 was found to be 3.4 × 10⁻⁷ g/L, with high activity. In addition, the monomer peptide EMP35 showed an activity of 1.1 × 10⁻⁴ g/L, which was about 1000 times lower than that of the EPO-PAS dimer peptide (Fig. 36, Table 4), and it was clarified that higher agonist activity was obtained by dimerization.

**Table 4**

| Peptide | EC50 (g/L) |
|---|---|
| rhEPO | 1.1 × 10⁻⁶ |
| AET-EPO-PAS8 | 1.9 × 10⁻⁷ |
| AET-EPO-PAS22 | 3.4 × 10⁻⁷ |
| EMP35 | 1.1 × 10⁻⁴ |

### Example 23: Design of TPO-PAS dimer peptide and its secretory expression in C. glutamicum

### (23-1) Outline of design

Peptide dimers that bind to the thrombopoietin receptor c-Mpl cannot be expressed in C. glutamicum for the following reasons. Specifically, a peptide dimer molecule (AF13948) obtained by dimerizing the straight chain c-Mpl-binding peptide AF12505 reported in S. E. Cwirla, et al. Science, 276, 1696-1699, 1997 with the α- and ε-amino groups of lysine cannot be expressed in C. glutamicum as a single polypeptide chain for the following reasons.
(1) Dimerization is achieved by binding the C-terminuses of the straight chain peptides AF12505 to each other via the α- and ε-amino groups of lysine, and cannot be expressed in C. glutamicum. (2) The non-protein amino acid β-alanine is bound to the C-terminus of AF12505 bound to the α-amino group of lysine, and peptides containing non-protein amino acids cannot be expressed in C. glutamicum. Therefore, the molecule was converted as follows to design an expressible form. The obstacles were dealt with by using PAS sequences instead of lysine and β-alanine and further designing the peptide sequence to be "first AF12505"-"PAS linker"-"second AF12505" so that it could be expressed as a single polypeptide chain. Specifically, the C-terminal of the first AF12505 and the N-terminal of the PAS linker are linked by an amide bond, and the N-terminal of the second AF12505 and the C-terminal of the PAS linker are linked by an amide bond, so that they can be expressed as a single polypeptide chain.

In addition, the peptide reported in WO9640750, a cyclic c-Mpl binding peptide AF12285 with biotin added to the C-terminus and multimerized with streptavidin, cannot be expressed in C. glutamicum as a single polypeptide chain for the following reasons.
(1) Biotin is modified at the C-terminal of the cyclic peptide AF12285 via the ε-amino group of lysine. Peptides containing biotin cannot be expressed at C. glutamicum.
(2) The biotinylated cyclic peptide is multimerized with streptavidin. It is impossible to express streptavidin at the same time as a peptide in C. glutamicum. Therefore, the molecule was converted as follows to design an expressible form. The obstacles were dealt with by using PAS sequences instead of biotin-streptavidin multimerization and further designing the peptide sequence to be "first AF12285"-"PAS linker"-"second AF12285" so that it could be expressed as a single polypeptide chain. Specifically, the C-terminal of the first AF12505 and the N-terminal of the PAS linker are linked by an amide bond, and the N-terminal of the second AF12505 and the C-terminal of the PAS linker are linked by an amide bond, so that they can be expressed as a single polypeptide chain.

### (23-2) Preparation of TPO-PAS dimer peptides

The prepared TPO-PAS dimer peptides are as follows.
(t) TPO2-PAS8 GGCADGPTLREWISFCGGAAPAAPAPGGCADGPTLREWISFCGG (SEQ ID NO: 114)
(u) AET-TPO1-PAS8 AETIEGPTLRQWLAARAAAPAAPAPIEGPTLRQWLAARA (SEQ ID NO: 115)
(v) AET-TPO2-PAS8 AETGGCADGPTLREWISFCGGAAPAAPAPGGCADGPTLREWISFCGG (SEQ ID NO: 116)

### (23-3) Expression of TPO-PAS dimer peptides

The expression of these TPO-PAS dimer peptides was examined using Corynex (registered trademark). Hereinafter, examples of expression examinations using Corynex (registered trademark) will be described.

### (23-4) Construction of secretory expression plasmid of TPO2-PAS8 using CspA signal sequence

As the TPO-PAS dimer peptide, the amino acid sequence of the above (t) TPO2-PAS8 (which may be hereinafter referred to as "TPO2-PAS8") was designed, and the base sequence encoding this protein was designed in consideration of the codon usage frequency of C. glutamicum. Furthermore, the following expression cassette was designed to enable secretion expression by C. glutamicum.

TPO2-PAS8 was secretory-expressed as a fusion protein of TPO2-PAS8 and a signal peptide 25 amino acid residue of CspA derived from C. ammoniagenes ATCC 6872 strain (hereinafter referred to as "CspAss-TPO2-PAS8"). The base sequence and amino acid sequence encoding the designed CspAss-TPO2-PAS8 are set forth in SEQ ID NOs: 117 and 118, respectively.
Base sequence encoding CspAss-TPO2-PAS8 
atgaaacgcatgaaatcgctggctgcggcgctcaccgtcgctggggccatgctggccgcacctgtggcaacggcaggtggt

tgtgctgatggtcctaccttgcgtgaatggatttccttctgcggcggcgctgctccggctgcccctgcccctggcggttgcgca

gatggcccaaccctgcgcgaatggatctccttctgcggcggctaa (SEQ ID NO: 117)
Amino acid sequence of CspAss-TPO2-PAS8

MKRMKSLAAALTVAGAMLAAPVATAGGCADGPTLREWISFCGGAAPAAPAPG
GCADGPTLREWISFCGG (SEQ ID NO: 118)

The promoter of the cspB gene derived from the C. glutamicum ATCC 13869 strain was linked upstream of the base sequence set forth in CspAss-TPO2-PAS8, and furthermore, an expression cassette of TPO-PAS dimer peptide having a KpnI site on the 5'-side and an BamHI site on the 3'-side was designed and totally synthesized. The totally synthesized DNA fragment (TPO-PAS dimer peptide expression cassette) was inserted into the KpnI-BamHI site of pPK4 described in Japanese Patent Application Publication No. Hei 9-322774 to construct pPK4_CspAss-TPO2-PAS8, a secretory expression plasmid for the TPO-PAS dimer peptide using the CspA signal sequence. As a result of determining the base sequence of the inserted fragment, it was confirmed that the TPO-PAS dimer peptide expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (23-5) Construction of each secretory expression plasmid of AET-TPO1-PAS8 and AET-TPO2-PAS8 using CspB signal sequence

As the TPO-PAS dimer peptide, two types of amino acid sequences of the above (u) AET-TPO1-PAS8 (which may be hereinafter referred to as "AET-TPO1-PAS8") and (v) AET-TPO2-PAS8 (which may be hereinafter referred to as "AET-EPO2-PAS8") were designed, and the base sequences encoding these proteins were designed in consideration of the codon usage frequency of C. glutamicum. Furthermore, the following expression cassettes were designed to enable secretory expression by C. glutamicum.

AET-TPO1-PAS8 was secretory-expressed as a fusion protein of AET-TPO1-PAS8 and a signal peptide 30 amino acid residue of CspB derived from C. glutamicum ATCC 13869 strain (hereinafter referred to as "CspBss-AET-TPO1-PAS8"). The base sequence and amino acid sequence encoding the designed CspBss-AET-TPO1-PAS8 are set forth in SEQ ID NOs: 119 and 120, respectively.
Base sequence encoding CspBss-AET-TPO1-PAS8 
atgtttaacaaccgtatccgcactgcagctctcgctggtgcaatcgcaatctccaccgcagcttccggcgtagctatcccagcat
tcgctgcagagaccatcgaaggtcctaccctccgtcaatggctggctgctcgtgccgccgctcccgccgctcctgctccaatc
gaaggtcccaccctccgtcagtggctggcagcccgcgcataa (SEQ ID NO: 119)
Amino acid sequence of CspBss-AET-TPO1-PAS8

MFNNRIRTAALAGAIAISTAASGVAIPAFAAETIEGPTLRQWLAARAAAPAAPAPI
EGPTLRQWLAARA (SEQ ID NO: 120)

AET-TPO2-PAS8 was secretory-expressed as a fusion protein of AET-TPO2-PAS8 and a signal peptide 30 amino acid residue of CspB derived from C. glutamicum ATCC 13869 strain (hereinafter referred to as "CspBss-AET-TPO2-PAS8"). The base sequence and amino acid sequence encoding the designed CspBss-AET-TPO2-PAS8 are set forth in SEQ ID NOs: 121 and 122, respectively.
Base sequence encoding CspBss-AET-TPO2-PAS8 
atgtttaacaaccgtatccgcactgcagctctcgctggtgcaatcgcaatctccaccgcagcttccggcgtagctatcccagcat
tcgctgcagagaccggtggttgtgctgatggtcctaccttgcgtgaatggatttccttctgcggcggcgctgctccggctgccc
ctgcccctggcggttgcgcagatggcccaaccctgcgcgaatggatctccttctgcggcggctaa (SEQ ID NO:
121)
Amino acid sequence of CspBss-AET-TPO2-PAS8

MFNNRIRTAALAGAIAISTAASGVAIPAFAAETGGCADGPTLREWISFCGGAAPA
APAPGGCADGPTLREWISFCGG (SEQ ID NO: 122)

The promoter of the cspB gene derived from the C. glutamicum ATCC 13869 strain was linked upstream of the base sequences set forth in CspBss-AET-TPO1-PAS8 and CspBss-AET-TPO2-PAS8, and furthermore, an expression cassette of each TPO-PAS dimer peptide having a KpnI site on the 5'-side and an BamHI site on the 3'-side was designed and totally synthesized. The totally synthesized DNA fragment (TPO-PAS dimer peptide expression cassette) was inserted into the KpnI-BamHI site of pPK4 described in Japanese Patent Application Publication No. Hei 9-322774 to construct pPK4_CspBss-AET-TPO1-PAS8 and pPK4_CspBss-AET-TPO2-PAS8, a secretory expression plasmid for each TPO-PAS dimer peptide using the CspB signal sequence. As a result of determining the base sequence of the inserted fragment, it was confirmed that each TPO-PAS dimer peptide expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (23-6) Secretory expression of each TPO-PAS dimer peptide in C. glutamicum

The pPK4_CspAss-TPO2-PAS8, pPK4_CspBss-AET-TPO1-PAS8, and pPK4_CspBss-AET-TPO2-PAS8 constructed above were used to transform the C. glutamicum YDK0107 strain described in WO2016/171224 to obtain the YDK0107/pPK4_CspAss-TPO2-PAS8 strain, YDK0107/pPK4_CspBss-AET-TPO1-PAS8 strain, and YDK0107/pPK4_CspBss-AET-TPO2-PAS8 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

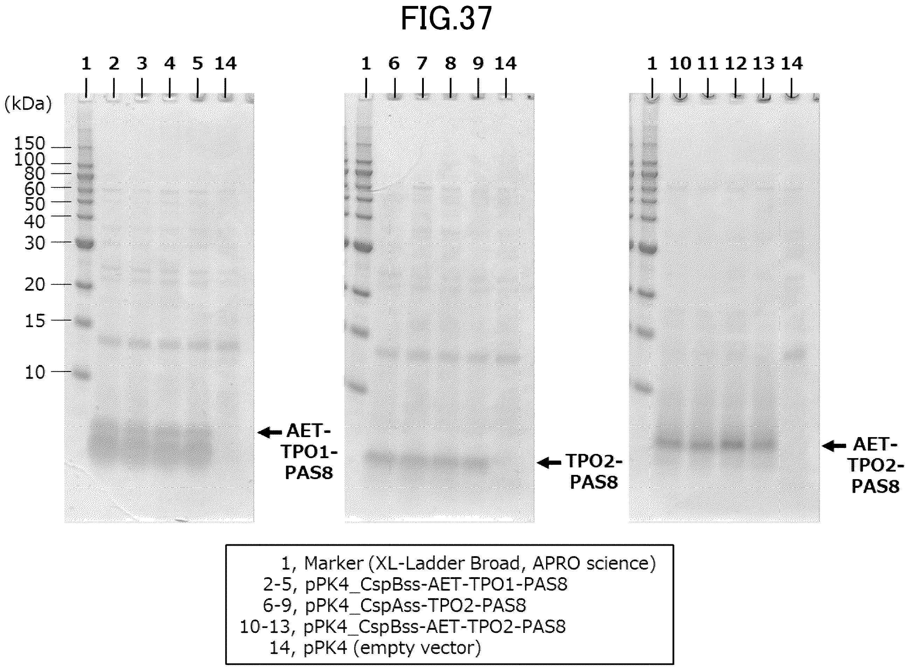

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be AET-TPO1-PAS8 was detected in the culture supernatant of the YDK0107/pPK4_CspBss-AET-TPO1-PAS8 strain (Fig. 37, lanes 2 to 5), a protein band presumed to be TPO2-PAS8 was detected in the culture supernatant of the YDK0107/pPK4_CspAss-TPO2-PAS8 strain (Fig. 37, lanes 6 to 9), and a protein band presumed to be AET-TPO2-PAS8 was detected in the culture supernatant of the YDK0107/pPK4_CspBss-AET-TPO2-PAS8 strain (Fig. 37, lanes 10 to 13).

### Example 24: Molecular weight analysis of TPO-PAS dimer peptide

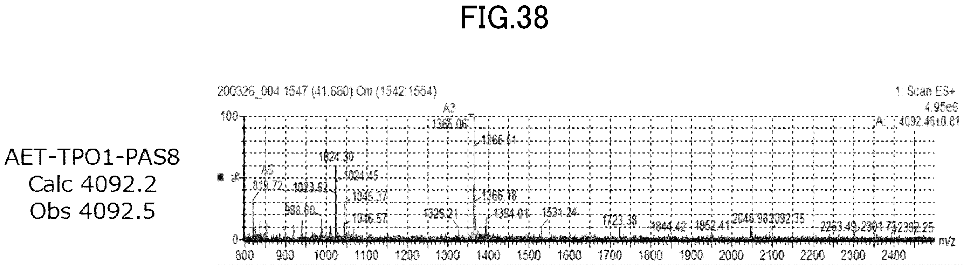

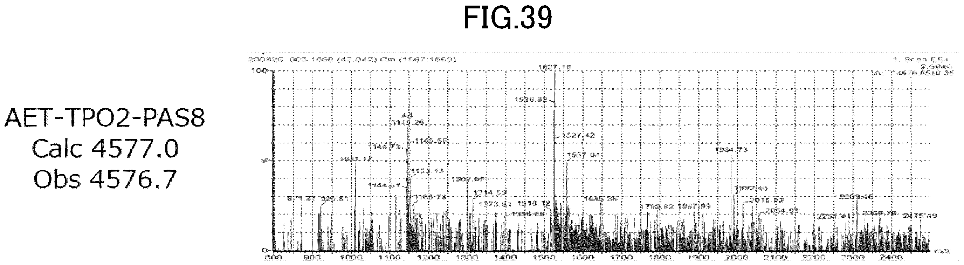

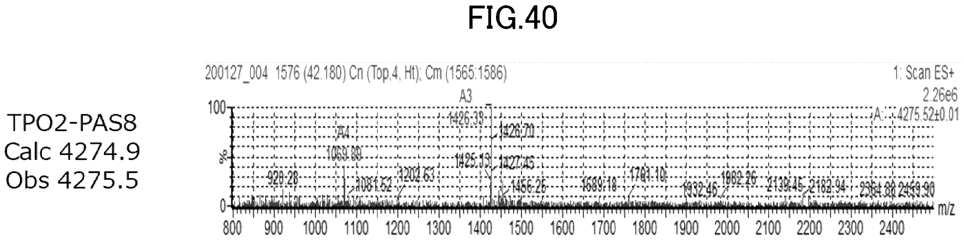

The cell filtrate of the TPO-PAS dimer peptide after culturing in Example 23 was subjected to molecular weight measurement using LC-MS (Waters, ACQUITY UPLC/SQD2). The analysis conditions are shown in Table 2. As shown in Figs. 38 to 40, almost the same value as the theoretical value of the molecular weight of each oxidized dimer peptide forming two disulfide bonds in the molecule was obtained. From this, it was confirmed that a peptide having the correct amino acid sequence containing two disulfide bonds could be expressed.

### Example 25: Activity evaluation of TPO-PAS dimer peptide

The thrombopoietin-like activity of the TPO-PAS dimer peptide was evaluated by quantification of tyrosine phosphorylation levels in HEL cells, a human leukemia-derived blood cell line. 200,000 HEL cells were cultured in RPMI 1640 medium without FBS (Thermo Fisher Scientific) for 24 hours and starved. The cells were stimulated for 20 minutes in RPMI 1640 medium supplemented with 10 to 100 ng/mL recombinant human TPO (rhTPO, Peprotech) or a culture supernatant containing AET-TPO1-PAS8, TPO2-PAS8, or AET-TPO2-PAS8 at a predetermined dilution factor. As a negative control, PBS (Mock group) or a supernatant expressing no peptide (Empty group) by using an empty vector was added, and stimulation was performed for 20 minutes. After removing the medium and washing the cells once with PBS, the cells were lysed by adding 100 µL of Lysis buffer 17 (R&D Systems) supplemented with Protease inhibitor cocktail (Nacalai Tesque). The lysate solution was centrifuged at 14,000 G for 5 minutes to obtain the supernatant as lysate.

A PVDF membrane (Immobilon-P membrane 0.45 µm, Merck) was treated with methanol for 1 minute and then with ultrapure water for 5 minutes for hydrophilic treatment. The PVDF membrane was dried, and each cell lysate solution was spotted with a 1 µL pipette and dried. The PVDF membrane was blocked with PVDF Blocking Reagent for Can Get Signal (NOF) at room temperature for 1 hour, and then Can Get Signal Solution 2 (NOF), supplemented with anti-pY antibody-HGF conjugate (Cell Signaling Technology) at 1:5000, was added, followed by an antibody reaction at 4°C for 24 hours. The membrane was washed 3 times with TBST buffer, Luminata Crescendo Western HRP Substrate (Merck) was added, and a chemiluminescence reaction was carried out. Chemiluminescence was detected using Amersham Imager 600 (GE Healthcare). The luminescence intensity of the detected dots was quantified by ImageJ.

As shown in Fig. 41, AET-TPO1-PAS8, AET-TPO2-PAS8, and TPO2-PAS8 were all observed to increase in tyrosine phosphorylation levels equal to or higher than that of rhTPO. From this result, it was clarified that AET-TPO1-PAS8, AET-TPO2-PAS8, and TPO2-PAS8 activated HEL cells in the same manner as TPO.

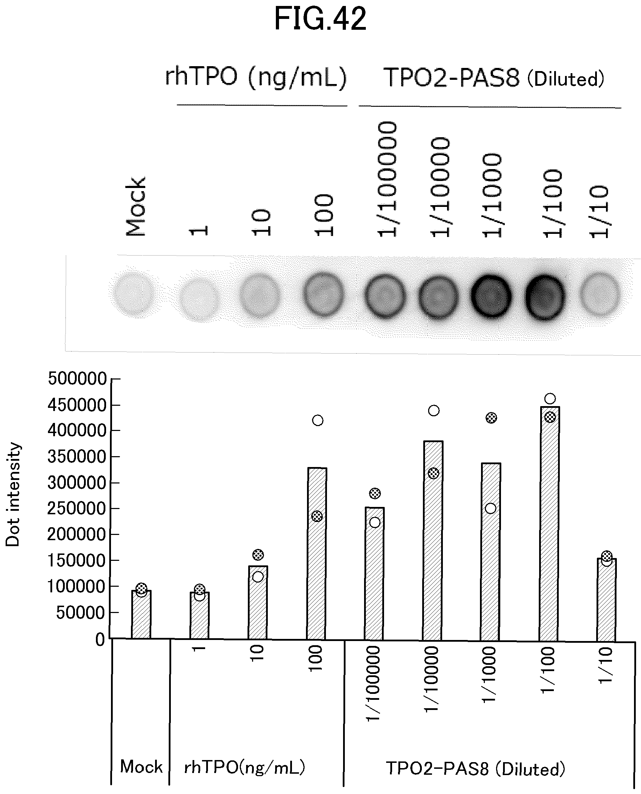

Fig. 42 shows the concentration-dependent ability of TPO2-PAS8 to activate HEL cells. Within the 1/100 to 1/10000 dilution range, the TPO2-PAS8 stimulation group showed luminescence intensity equal to or higher than that of the 100 ng/mL rhTPO stimulation group, and from this, it was clarified that the HEL cell activation ability was equal to or higher than that of rhTPO in this concentration range.

### Example 26: Evaluation of signal characteristics of TPO-PAS dimer peptide

The signal characteristics of the TPO-PAS dimer peptide were evaluated using Proteome Profiler Human Phospho-Kinase Array Kit (R&D Systems).

1,000,000 HEL cells were cultured in RPMI 1640 medium without FBS (Thermo Fisher Scientific) for 24 hours and starved. The cells were stimulated for 20 minutes in RPMI 1640 medium supplemented with 100 ng/mL recombinant human TPO (rhTPO, Peprotech) or a culture supernatant containing TPO2-PAS8 at a dilution of 1/100 to 1/1000. As a negative control, PBS was added, and stimulation was performed for 20 minutes (Mock group). After removing the medium and washing the cells once with PBS, the cells were lysed by adding 200 µL of Lysis buffer 17 (R&D Systems) supplemented with Protease inhibitor cocktail (Nacalai Tesque). The lysate solution was centrifuged at 14,000 G for 5 minutes to obtain the supernatant as lysate.

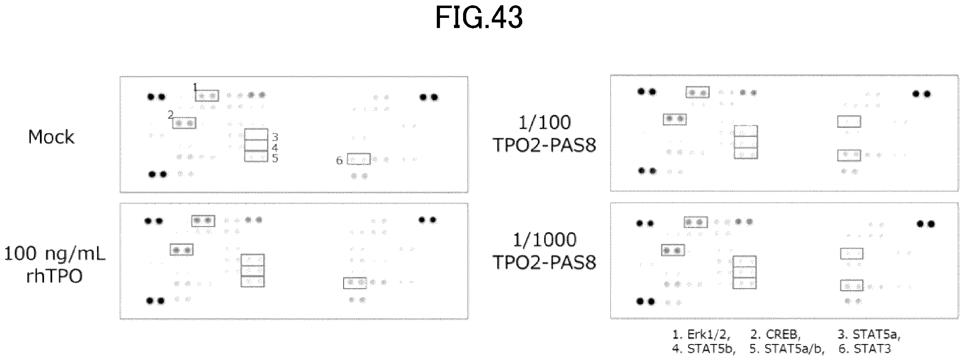

The lysate was analyzed using Proteome Profiler Human Phospho-Kinase Array Kit. Amersham Imager 600 (GE Healthcare) was used to detect chemiluminescence. The chemiluminescence intensity of the dots was quantified using ImageJ. As shown in Fig. 43, it was confirmed that compared to the cells stimulated with PBS (Mock group), the cells stimulated with rhTPO or TPO2-PAS8 increased in phosphorylation levels of Erk, CREB, Stat3, Stat5a, and Stat5b, and their phosphorylation patterns were similar. The signal pathway stimulated by rhTPO is mainly the JAK2/Stat3,5 pathway, and all of the proteins with increased phosphorylation levels are included in the pathway. From this result, it has been confirmed that TPO2-PAS8 activates a signal similar to rhTPO.

### Example 27: Design of VEGFR_D2-PAS heterodimer peptide and its secretory expression in C. glutamicum

### (27-1) Outline of design

It is impossible to express the VEGFR-binding cyclic peptides P-3 and P-4 reported in A. Shrivastava, et al. Protein Eng. Des. Select. 18, 417, 2005, dimerized by a glutarate linker, as a single polypeptide chain in C. glutamicum for the following reasons. Dimerization is achieved by binding the ε-amino groups of the lysine residues located at the C-terminus of each of the cyclic peptides P-3 and P-4 via glutaric acid, and cannot be expressed in C. glutamicum. Therefore, the molecule was converted as follows to design an expressible form. The obstacles were dealt with by using a PAS sequence instead of a glutarate linker and further designing the peptide sequence to be "first VEGFR-binding sequence (P-3)"-"PAS linker"-"second VEGFR-binding sequence (P-4)" so that it could be expressed as a single polypeptide chain. Specifically, the C-terminal of P-3 and the N-terminal of the PAS linker are linked by an amide bond, and the N-terminal of P-4 and the C-terminal of the PAS linker are linked by an amide bond, so that they can be expressed as a single polypeptide chain.

### (27-2) Preparation of VEGFR_D2-PAS heterodimer peptide

The prepared VEGFR_D2-PAS heterodimer peptide is as follows.
(w) AET-VEGFR_D2-PAS16

AETAGPTWCEDDWYYCWLFGTAAPAAPAPAAPAAPAPVCWEDSWGGEVCWLF
GT (SEQ ID NO: 123)

### (27-3) Expression of VEGFR_D2-PAS heterodimer peptide

Corynex (registered trademark) was used to examine the expression of VEGFR_D2-PAS heterodimer peptide. Hereinafter, examples of expression examinations using Corynex (registered trademark) will be described.

### (27-4) Construction of AET-VEGFR_D2-PAS16 secretory expression plasmid using CspB signal sequence

As the VEGFR_D2-PAS heterodimer peptide, the amino acid sequence of the above (w) AET-VEGFR_D2-PAS16 (which may be hereinafter referred to as "AET-VEGFR D2-PAS16", and may be simply referred to as "VEGFR D2-PAS16") was designed, and the base sequence encoding this protein was designed in consideration of the codon usage frequency of C. glutamicum. Furthermore, the following expression cassette was designed to enable secretion expression by C. glutamicum.

AET-VEGFR_D2-PAS16 was secretory-expressed as a fusion protein of AET-VEGFR_D2-PAS16 and a signal peptide 30 amino acid residue of CspB derived from C. glutamicum ATCC 13869 strain (hereinafter referred to as "CspBss-VEGFR_D2-PAS16"). The base sequence and amino acid sequence encoding the designed CspBss-AET-VEGFR_D2-PAS16 are set forth in SEQ ID NOs: 124 and 125, respectively.
Base sequence encoding CspBss-AET-VEGFR_D2-PAS16 
atgtttaacaaccgtatccgcactgcagctctcgctggtgcaatcgcaatctccaccgcagcttccggcgtagctatcccagcat
tcgctgcagagaccgctggtcctacctggtgtgaagacgactggtactactgctggctgtttggcaccgctgctcccgctgccc
ctgctcctgccgctccggcagccccagctcctgtgtgttgggaagattcttggggcggcgaagtttgctggctgtttggcacct
aa (SEQ ID NO: 124)
Amino acid sequence of CspBss-AET-VEGFR_D2-PAS16

MFNNRIRTAALAGAIAISTAASGVAIPAFAAETAGPTWCEDDWYYCWLFGTAAP
AAPAPAAPAAPAPVCWEDSWGGEVCWLFGT (SEQ ID NO: 125)

The promoter of the cspB gene derived from the C. glutamicum ATCC 13869 strain was linked upstream of the base sequence set forth in CspBss-AET-VEGFR_D2-PAS16, and furthermore, an expression cassette of VEGFR_D2-PAS heterodimer peptide having a KpnI site on the 5'-side and an BamHI site on the 3'-side was designed and totally synthesized. The totally synthesized DNA fragment (VEGFR_D2-PAS heterodimer peptide expression cassette) was inserted into the KpnI-BamHI site of pPK4 described in Japanese Patent Application Publication No. Hei 9-322774 to construct pPK4_CspBss-AET-VEGFR_D2-PAS16, a secretory expression plasmid for the VEGFR_D2-PAS heterodimer peptide using the CspB signal sequence. As a result of determining the base sequence of the inserted fragment, it was confirmed that the VEGFR_D2-PAS heterodimer peptide expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (27-5) Secretory expression of VEGFR_D2-PAS heterodimer peptide in C. glutamicum

The pPK4_CspBss-AET-VEGFR_D2-PAS16 constructed above was used to transform the C. glutamicum YDK0107 strain described in WO2016/171224 to obtain the YDK0107/pPK4_CspBss-AET-VEGFR_D2-PAS16 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

After completion of the culture, 6.5 µL of the culture supernatant obtained by centrifuging the culture solution or the culture solution containing the cells and the culture supernatant uniformly without centrifugation was each subjected to reduced SDS-PAGE using NuPAGE (registered trademark) 12% Bis-Tirs Gel (Thermo Fisher Scientific), and then stained with Quick-CBB (Wako). As a result, a very small amount of a protein band presumed to be AET-VEGFR_D2-PAS16 was detected in the culture supernatant of the YDK0107/pPK4_CspBss-AET-VEGFR_D2-PAS16 strain (Fig. 45, lane 2), whereas a protein band presumed to be AET-VEGFR_D2-PAS16 was significantly detected in the culture solution sample containing the cells (Fig. 45, lane 3).

From the above results, it was considered that after secretion, most of AET-VEGFR_D2-PAS16 was aggregated in the culture supernatant or adsorbed on the cell surface of the cultured cells.

### (27-6) Solubilization of VEGFR_D2-PAS heterodimer peptide by urea treatment

To the precipitate fraction containing the cells obtained by centrifuging the culture solution of the YDK0107/pPK4_CspBss-AET-VEGFR_D2-PAS16 strain obtained above, an equal volume of 0.5 M Urea (50 mM Tris-HCl, pH 8.0) or 1.0 M Urea (50 mM Tris-HCl, pH 8.0) was added to the removed culture supernatant and mixed. 6.5 µL of the supernatant fraction obtained by centrifuging each Urea treatment solution or the mixture containing the cells and the supernatant uniformly without centrifugation was each subjected to reduced SDS-PAGE using NuPAGE (registered trademark) 12% Bis-Tirs Gel (Thermo Fisher Scientific), and then stained with Quick-CBB (Wako). As a result, in the 0.5 M Urea and 1.0 M Urea treatment samples, a protein band presumed to be AET-VEGFR_D2-PAS16 was extracted into the supernatant and obtained as a soluble fraction (Fig. 45, lanes 6 and 8).

### Example 28: Molecular weight analysis of VEGFR_D2-PAS heterodimer

The solution obtained by washing the cells expressing the VEGFR_D2-PAS heterodimer after culturing in Example 27 with 8 M Urea was subjected to cell filtration and then molecular weight measurement using LC-MS (Waters, ACQUITY UPLC/SQD2). The analysis conditions are shown in Table 5.

**Table 5**

| **LC set up** | | | | | |
|---|---|---|---|---|---|
| **System** | | **ACQUITYUPLC H-Class /SQD2** | | | |
| **Column** | | **TMC-Triart C8 50 × 2.0 mmI.D. 1.9 um, 12nm(TO12SP9-0502PT)** | | | |
| **Solvent** | **A** | **0.1% TFA** | | | |
| | **B** | **0.1% TFA, 80% Acetonitrile** | | | |
| **Flow rate** | | **1.0 mL/min** | | | |
| **Column temp.** | | **40 °C** | | | |
| **Detection** | | **220 nm** | | | |
| **Sample Vol.** | | **10 uL** | | | |
| **Gradient program** | | **Time (min)** | **%A** | **%B** | |
| | | **0** | **100** | **0** | |
| | | **50** | **100** | **100** | |
| | | **53** | **0** | **100** | |
| | | **53.1** | **100** | **0** | |
| | | **56** | **100** | **0** | |
| | | **56.1** | **0** | **100** | |
| | | **59** | **0** | **100** | |
| | | **59.1** | **100** | **0** | |
| | | **65** | **100** | **0** | |

| **MS spectrometer set up** | | | | | |
|---|---|---|---|---|---|
| **Ion mode** | | **ESI positive** | | | |
| **Range** | | **800-2500 m/z** | | | |
| **Scan time** | | **0.4 amu/sec** | | | |
| **Capillary Voltage** | | **2.0kV** | | | |
| **Cone voltage** | | **10V, 20V, 40V** | | | |
| **Desolvation temp** | | **500 °C** | | | |
| **Desolvation Gas Flow** | | **1000 L/hr** | | | |
| **Cone Gas Flow** | | **50 L/hr** | | | |
| **Software** | | **Deconvolution is performed using "MassLynx"** | | | |

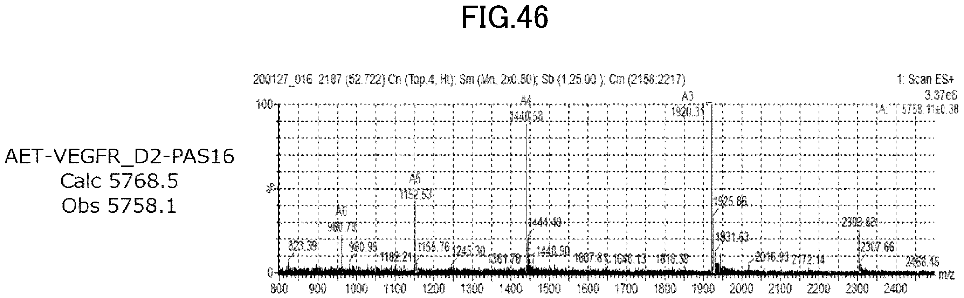

As shown in Fig. 46, almost the same value as the theoretical value of the molecular weight of the oxidized dimer peptide forming two disulfide bonds in the molecule was obtained. From this, it was confirmed that a peptide having the correct amino acid sequence containing two disulfide bonds could be expressed.

### Example 29: Activity evaluation of VEGFR_D2-PAS heterodimer

The VEGF inhibitory ability of the VEGFR_D2-PAS heterodimer peptide was evaluated by luciferase assay. 40,000 cell/well VEGFR/NFAT Reporter HEK293 cells (BPS Bioscience) were seeded and incubated overnight in a 5% CO₂ incubator at 37°C. The cells were incubated overnight in Assay medium (MEM medium (Nacalai Tesque), 1 × MEM Non-Essential Amino Acids Solution (Thermo Fisher Scientific), 1 mM Sodium Pyruvate (Nacalai Tesque), 1% Penicillin-Streptomycin (Sigma Aldrich), and 0.3% FBS (Thermo Fisher Scientific)) to starvation. To the Assay medium, 10 ng/mL human recombinant VEGF (R&D Systems) was added, and further, VEGFR_D2-PAS16 purified by HPLC, or a monomer peptide P3 (AGPTWCEDDWYYCWLFGT (SEQ ID NO: 71), synthesized by GenScript Japan) or Pm4 (VCWEDSWGGEVCWLFGT (SEQ ID NO: 72), synthesized by GenScript Japan) was added at a predetermined dilution factor, and 100 µL of the Assay medium was added to the cells. The cells were stimulated by culturing in a 5% CO₂ incubator at 37°C for 4 hours.

One-Glo Luciferase Assay System (Promega) was used to detect the signal intensity. Medium supernatant in an amount of 50 µL was removed, 50 µL of One-Glo reagent was added, and the cells were lysed for 10 minutes at room temperature. The luminescence emitted from the cell lysate solution was detected with a Nivo plate reader (Perkin Elmer) to quantify the activation of the VEGFR-NFAT pathway, and the relative signal activity value for the sample without addition of evaluation compound was determined and used as relative receptor activity.

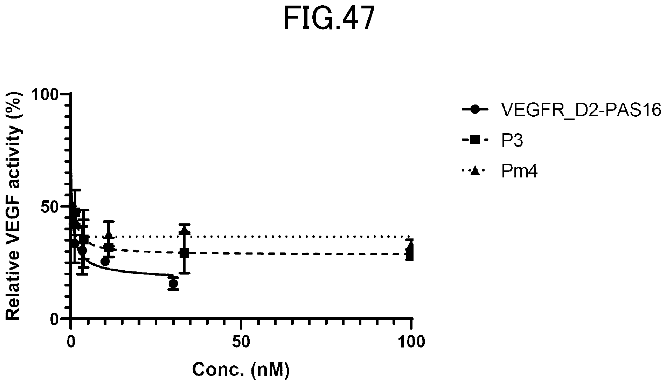

As shown in Fig. 47, VEGFR_D2-PAS16 showed higher inhibitory activity than the monomer peptides P3 and Pm4, so that it was clarified that high inhibitory activity could be obtained by heterodimerization of cyclic peptides.

### Example 30: Design of EPO-PAS dimer peptide linker variant and its secretory expression in C. glutamicum

### (30-1) Outline of linker variant design

The erythropoietin mimetic peptides AET-EPO-PAS8 and AET-EPO-PAS22 have a structure in which a PAS linker composed of 8 amino acids or 22 amino acids is linked between the first erythropoietin receptor-binding cyclic peptide and the second erythropoietin receptor-binding cyclic peptide. By changing the length of this PAS linker, it is expected that the erythropoietin receptor binding activity will change. Therefore, a linker variant was designed in which the PAS linker portion was changed to a PAS repeat sequence of 49 amino acids or 100 amino acids.

In addition to the PAS linker, a GS linker, which is a repeating sequence of glycine and serin, is also known as a flexible linker. Therefore, the peptide sequence was designed to be a linker variant in which an EPO cyclic peptide was bound to the N-terminus and C-terminus of the GS linker composed of a repeating sequence of 4 glycine residues and 1 serine residue (GGGGS), that is, a "first erythropoietin receptor-binding cyclic peptide"-"GS linker"-"second erythropoietin receptor-binding cyclic peptide".

### (30-2) Preparation of EPO-PAS dimer peptide linker variants

The prepared EPO-PAS dimer peptide linker variants are as follows.
(x) AET-EPO-PAS49 
AETGGLYACHMGPMTWVCQPLRGAAPAAPAPAAPAAPAPAAPAAPAPAAPAAPAPAAPAAP
APAAPAAPAPAAPAAPAPAGGLYACHMGPMTWVCQPLRG (SEQ ID NO: 126)
(y) AET-EPO-PAS49-b 
AETGGLYACHMGPMTWVCQPLRGSAPSPSSAPASASAPASPASASASASPASSPA
SASSASPAASSASPASSGGLYACHMGPMTWVCQPLRG (SEQ ID NO: 127)
(z) AET-EPO-PAS100 
AETGGLYACHMGPMTWVCQPLRGAAPAAPAPAAPAAPAPAAPAAPAPAAPAAP
APAAPAAPAPAAPAAPAPAAPAAPAPAAPAAPAPAAPAAPAPAAPAAPAPAAPAAP
APAAPAAPAPAAPAGGLYACHMGPMTWVCQPLRG ((SEQ ID NO: 128)
(A) AET-EPO-PAS100-b AETGGLYACHMGPMTWVCQPLRGSAPSPSSAPASASAPASPASASASASPASSPA 
SASSASPAASSASPASSSASPSAPAAPSASAAPSASPASSSPAPASAPSPAAAASSPS
PAPSSASSAAGGLYACHMGPMTWVCQPLRG (SEQ ID NO: 129)
(B) AET-EPO-GS8 
AETGGLYACHMGPMTWVCQPLRGGGGGSGGGGLYACHMGPMTWVCQPLR
G (SEQ ID NO: 130)
(C) AET-EPO-GS22

AETGGLYACHMGPMTWVCQPLRGGGGGSGGGGSGGGGSGGGGSGGGGLYAC
HMGPMTWVCQPLRG (SEQ ID NO: 131)

### (30-3) Expression of EPO-PAS dimer peptide linker variant

The expression of these EPO-PAS dimer peptide linker variants was examined using Corynex (registered trademark). Hereinafter, examples of expression examinations using Corynex (registered trademark) will be described.

### (30-4) Construction of each secretory expression plasmid of AET-EPO-PAS49, AET-EPO-PAS49-b, AET-EPO-PAS100, AET-EPO-PAS100-b, AET-EPO-GS8, and AET-EPO-GS22 using CspB signal sequence

As the EPO-PAS dimer peptide linker variant, six types of amino acid sequences of the above (x) AET-EPO-PAS49 (which may be hereinafter referred to as "AET-EPO-PAS49"), (y) AET-EPO-PAS49-b (which may be hereinafter referred to as "AET-EPO-PAS49-b"), (z) AET-EPO-PAS100 (which may be hereinafter referred to as "AET-EPO-PAS100"), (A) AET-EPO-PAS100-b (which may be hereinafter referred to as "AET-EPO-PAS100-b"), (B) AET-EPO-GS8 (which may be hereinafter referred to as "AET-EPO-GS8"), and (C) AET-EPO-GS22 (which may be hereinafter referred to as "AET-EPO-GS22") were designed, and base sequences encoding these proteins were designed in consideration of the codon usage frequency of C. glutamicum. Furthermore, the following expression cassettes were designed to enable secretory expression by C. glutamicum.

AET-EPO-PAS49 was secretory-expressed as a fusion protein of AET-EPO-PAS49 and a signal peptide 30 amino acid residue of CspB derived from C. glutamicum ATCC 13869 strain (hereinafter referred to as "CspBss-AET-EPO-PAS49"). The base sequence and amino acid sequence encoding the designed CspBss-AET-EPO-PAS49 are set forth in SEQ ID NOs: 132 and 133, respectively.
Base sequence encoding CspBss-AET-EPO-PAS49 
atgtttaacaaccgtatccgcactgcagctctcgctggtgcaatcgcaatctccaccgcagcttccggcgtagctatcccagcat
tcgctgcagagaccggtggtctctacgcctgtcacatgggtcctatgacctgggtgtgccaacctctccgtggtgccgccccc
gccgcccccgcccccgccgccccagctgcaccagctccagcagccccagccgcaccagccccagcagctccagctgccc
cagcaccagcagcaccagccgctccagcaccagctgctccagcagctccagccccagccggcggcctttacgcctgccac
atgggtcctatgacctgggtttgccagcctcttcgcggttag (SEQ ID NO: 132)
Amino acid sequence of CspBss-AET-EPO-PAS49

MFNNRIRTAALAGAIAISTAASGVAIPAFAAETGGLYACHMGPMTWVCQPLRGA
APAAPAPAAPAAPAPAAPAAPAPAAPAAPAPAAPAAPAPAAPAAPAPAGGLYACH
MGPMTWVCQPLRG (SEQ ID NO: 133)

AET-EPO-PAS49-b was secretory-expressed as a fusion protein of AET-EPO-PAS49-b and a signal peptide 30 amino acid residue of CspB derived from C. glutamicum ATCC 13869 strain (hereinafter referred to as "CspBss-AET-EPO-PAS49-b"). The base sequence and amino acid sequence encoding the designed CspBss-AET-EPO-PAS49-b are set forth in SEQ ID NOs: 134 and 135, respectively.
Base sequence encoding CspBss-AET-EPO-PAS49-b 
atgtttaacaaccgtatccgcactgcagctctcgctggtgcaatcgcaatctccaccgcagcttccggcgtagctatcccagcat
tcgctgcagagaccggcggactgtacgcctgccatatggggccaatgacctgggtgccaaccgttgcgtggatcagcgc
catcgccctcttccgctccagcctctgcgtctgctcccgcatccccagcgtcagcttccgcatccgcttcgcctgcaagctcccc
tgctagcgcgtcctctgcctcccctgcagcatcctcggcttccccggcaagctcaggtggcctctatgcctgccacatgggtcc
gatgacttgggtctgtcagccacttcgcggctaa (SEQ ID NO: 134)
Amino acid sequence of CspBss-AET-EPO-PAS49-b

MFNNRIRTAALAGAIAISTAASGVAIPAFAAETGGLYACHMGPMTWVCQPLRGS
APSPSSAPASASAPASPASASASASPASSPASASSASPAASSASPASSGGLYACHMG
PMTWVCQPLRG (SEQ ID NO: 135)

AET-EPO-PAS100 was secretory-expressed as a fusion protein of AET-EPO-PAS100 and a signal peptide 30 amino acid residue of CspB derived from C. glutamicum ATCC 13869 strain (hereinafter referred to as "CspBss-AET-EPO-PAS100"). The base sequence and amino acid sequence encoding the designed CspBss-AET-EPO-PAS100 are set forth in SEQ ID NOs: 136 and 137, respectively.
Base sequence encoding CspBss-AET-EPO-PAS100 
atgtttaacaaccgtatccgcactgcagctctcgctggtgcaatcgcaatctccaccgcagcttccggcgtagctatcccagcat
tcgctgcagagaccggtggtttgtacgcttgccacatgggtcctatgacctgggtttgtcagcctcttcgtggtgccgcccccgc
agcccccgctcccgctgcaccagccgcaccagcaccagcagctccagcagctccagcaccagctgcaccagccgcccca
gcaccagcagccccagccgctccagcaccagctgccccagctgctccagctccagcagcaccagctgccccagccccag
cagcaccagccgctccagccccagctgccccagcagctccagctccagctgctccagcagccccagcaccagccgcccca
gcagccccagctccagctgcaccagctgcaccagccccagccgcaccagccggcggtttgtacgcttgccacatgggtccg
atgacctgggtttgtcagccccttcgcggttag (SEQ ID NO: 136)
Amino acid sequence of CspBss-AET-EPO-PAS100

MFNNRIRTAALAGAIAISTAASGVAIPAFAAETGGLYACHMGPMTWVCQPLRGA
APAAPAPAAPAAPAPAAPAAPAPAAPAAPAPAAPAAPAPAAPAAPAPAAPAAPAPA
APAAPAPAAPAAPAPAAPAAPAPAAPAAPAPAAPAAPAPAAPAGGLYACHMGPMT
WVCQPLRG (SEQ ID NO: 137)

AET-EPO-PAS100-b was secretory-expressed as a fusion protein of AET-EPO-PAS100-b and a signal peptide 30 amino acid residue of CspB derived from C. glutamicum ATCC 13869 strain (hereinafter referred to as "CspBss-AET-EPO-PAS100-b"). The base sequence and amino acid sequence encoding the designed CspBss-AET-EPO-PAS100-b are set forth in SEQ ID NOs: 138 and 139, respectively.
Base sequence encoding CspBss-AET-EPO-PAS100-b 
atgtttaacaaccgtatccgcactgcagctctcgctggtgcaatcgcaatctccaccgcagcttccggcgtagctatcccagcat
tcgctgcagagaccggcggcttgtacgcctgccatatggggcctatgacctgggtctgccaaccactgcgtggaagcgcacc
ctcaccatcctccgcaccagctagcgcctccgctcctgcaagcccagcgtccgcatccgcttcggcctcaccagcgtcctctc
cagcttccgcttcgagcgctagcccagcggcatcctctgcctccccagcctcctcgtctgcatccccgtccgctccagccgca
ccttctgcatctgcggctccgtccgcctccccggcatcttcctcgcctgctcccgcgtcagcgccttccccggctgcagcggcc
tcctctccgtcacccgctccttcaagcgcgtcgtctgcagcaggaggcctctatgcctgccacatgggtccgatgacttgggtg
tgtcagccccttcgcggttaa (SEQ ID NO: 138)
Amino acid sequence of CspBss-AET-EPO-PAS100-b

MFNNRIRTAALAGAIAISTAASGVAIPAFAAETGGLYACHMGPMTWVCQPLRGS
APSPSSAPASASAPASPASASASASPASSPASASSASPAASSASPASSSASPSAPAAP
SASAAPSASPASSSPAPASAPSPAAAASSPSPAPSSASSAAGGLYACHMGPMTWV
CQPLRG (SEQ ID NO: 139)

AET-EPO-GS8 was secretory-expressed as a fusion protein of AET-EPO-GS8 and a signal peptide 30 amino acid residue of CspB derived from C. glutamicum ATCC 13869 strain (hereinafter referred to as "CspBss-AET-EPO-GS8"). The base sequence and amino acid sequence encoding the designed CspBss-AET-EPO-GS8 are set forth in SEQ ID NOs: 140 and 141, respectively.
Base sequence encoding CspBss-AET-EPO-GS8 
atgtttaacaaccgtatccgcactgcagctctcgctggtgcaatcgcaatctccaccgcagcttccggcgtagctatcccagcat
tcgctgcagagaccggcggcctctatgcatgccatatggggccaatgacctgggtctgccaaccgttgcgtggcggaggtgg
cggttccggtggaggtggtggcctgtacgcttgccacatgggacccatgacttgggtgtgtcagcctcttcgcggctaa
(SEQ ID NO: 140)
Amino acid sequence of CspBss-AET-EPO-GS8

MFNNRIRTAALAGAIAISTAASGVAIPAFAAETGGLYACHMGPMTWVCQPLRGG
GGGSGGGGGLYACHMGPMTWVCQPLRG (SEQ ID NO: 141)

AET-EPO-GS22 was secretory-expressed as a fusion protein of AET-EPO-GS22 and a signal peptide 30 amino acid residue of CspB derived from C. glutamicum ATCC 13869 strain (hereinafter referred to as "CspBss-AET-EPO-GS22"). The base sequence and amino acid sequence encoding the designed CspBss-AET-EPO-GS22 are set forth in SEQ ID NOs: 142 and 143, respectively.
Base sequence encoding CspBss-AET-EPO-GS22 
atgtttaacaaccgtatccgcactgcagctctcgctggtgcaatcgcaatctccaccgcagcttccggcgtagctatcccagcat
tcgctgcagagaccggtgggctgtatgcttgccatatggggcctatgacttgggtgtgccaacccttgcgtggtggaggcgga
ggctccggcggtggtgggagcggaggaggcggttccggtggcggtggctctggcggaggcggcctctacgcatgccaca
tgggtccgatgacctgggtctgtcagccacttcgcggctaa (SEQ ID NO: 142)
Amino acid sequence of CspBss-AET-EPO-GS22

MFNNRIRTAALAGAIAISTAASGVAIPAFAAETGGLYACHMGPMTWVCQPLRGG

GGGSGGGGSGGGGSGGGGSGGGGLYACHMGPMTWVCQPLRG (SEQ ID NO:
143)

The promoter of the cspB gene derived from the C. glutamicum ATCC 13869 strain was linked upstream of the base sequences set forth in CspBss-AET-EPO-PAS49, CspBss-AET-EPO-PAS49-b, CspBss-AET-EPO-PAS100, CspBss-AET-EPO-PAS100-b, CspBss-AET-EPO-GS8, and CspBss-AET-EPO-GS22, and furthermore, an expression cassette of each EPO-PAS dimer peptide linker variant having a KpnI site on the 5'-side and an BamHI site on the 3'-side was designed and totally synthesized. The totally synthesized DNA fragment (EPO-PAS dimer peptide linker variant expression cassette) was inserted into the KpnI-BamHI site of pPK4 described in Japanese Patent Application Publication No. Hei 9-322774 to construct pPK4_CspBss-AET-EPO-PAS49, pPK4_CspBss-AET-EPO-PAS49-b, pPK4_CspBss-AET-EPO-PAS100, pPK4_CspBss-AET-EPO-PAS100-b, pPK4_CspBss-AET-EPO-GS8, and pPK4_CspBss-AET-EPO-GS22, a secretory expression plasmid for each EPO-PAS dimer peptide linker variant using the CspB signal sequence. As a result of determining the base sequence of the inserted fragment, it was confirmed that each EPO-PAS dimer peptide linker variant expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (30-5) Secretory expression of each EPO-PAS dimer peptide in C. glutamicum

The pPK4_CspBss-AET-EPO-PAS49, pPK4_CspBss-AET-EPO-PAS49-b, pPK4_CspBss-AET-EPO-PAS100, pPK4_CspBss-AET-EPO-PAS100-b, pPK4_CspBss-AET-EPO-GS8, and pPK4_CspBss-AET-EPO-GS22 constructed above were used to transform the C. glutamicum YDK0107 strain described in WO2016/171224 to obtain the YDK0107/pPK4_CspBss-AET-EPO-PAS49 strain, YDK0107/pPK4_CspBss-AET-EPO-PAS49-b strain, YDK0107/pPK4_CspBss-AET-EPO-PAS100 strain, YDK0107/pPK4_CspBss-AET-EPO-PAS100-b strain, YDK0107/pPK4_CspBss-AET-EPO-GS8 strain, and YDK0107/pPK4_CspBss-AET-EPO-GS22 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

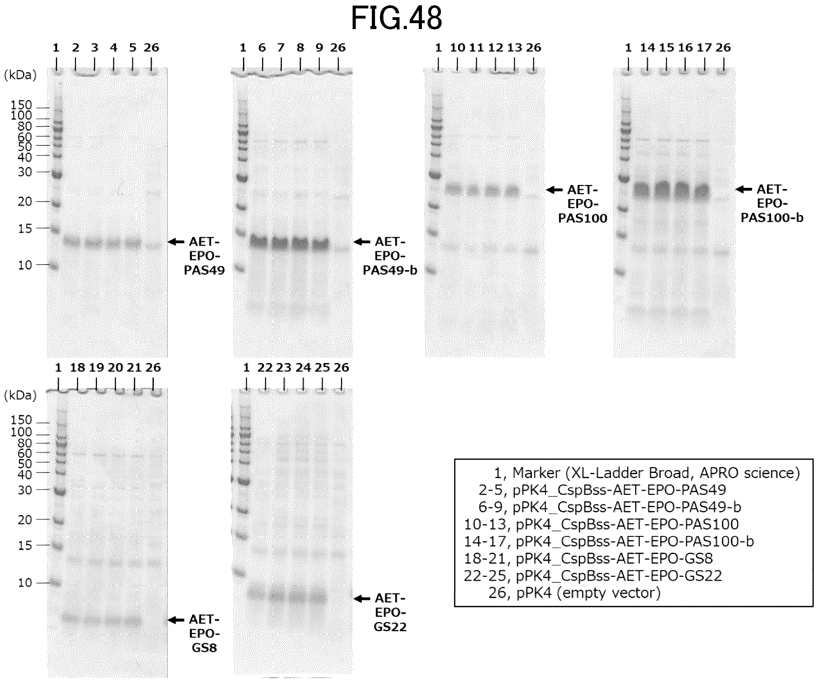

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be AET-EPO-PAS49 was detected in the culture supernatant of the YDK0107/pPK4_CspBss-AET-EPO-PAS49 strain (Fig. 48, lanes 2 to 5), a protein band presumed to be AET-EPO-PAS49-b was detected in the culture supernatant of the YDK0107/pPK4_CspBss-AET-EPO-PAS49-b strain (Fig. 48, lanes 6 to 9), a protein band presumed to be AET-EPO-PAS100 was detected in the culture supernatant of the YDK0107/pPK4_CspBss-AET-EPO-PAS100 strain (Fig. 48, lanes 10 to 13), a protein band presumed to be AET-EPO-PAS100-b was detected in the culture supernatant of the YDK0107/pPK4_CspBss-AET-EPO-PAS100-b strain (Fig. 48, lanes 14 to 17), a protein band presumed to be AET-EPO-GS8 was detected in the culture supernatant of the YDK0107/pPK4_CspBss-AET-EPO-GS8 strain (Fig. 48, lanes 18 to 21), and a protein band presumed to be AET-EPO-GS22 was detected in the culture supernatant of the YDK0107/pPK4_CspBss-AET-EPO-GS22 strain (Fig. 48, lanes 22 to 25).

### Example 31: Molecular weight analysis of EPO-PAS dimer peptide linker variant

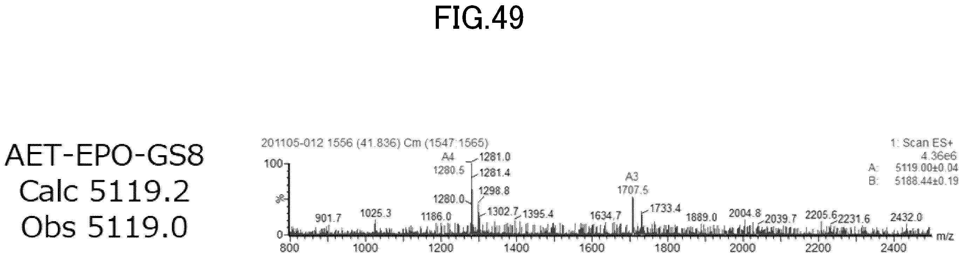

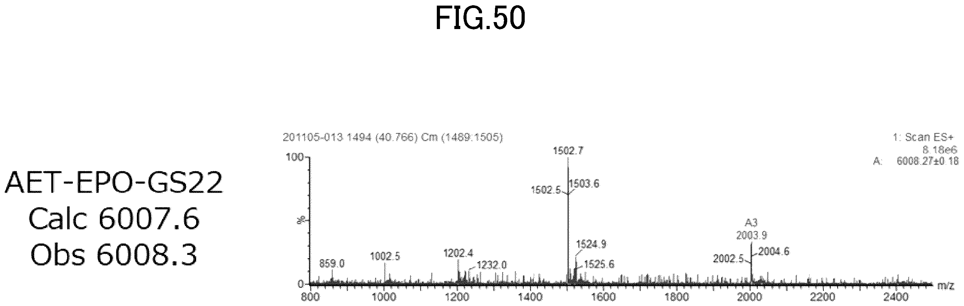

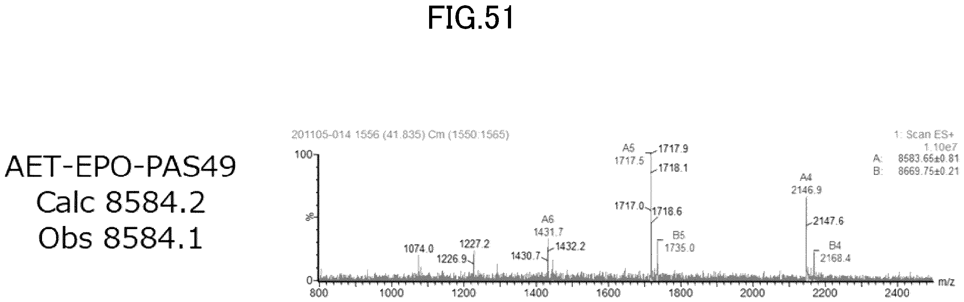

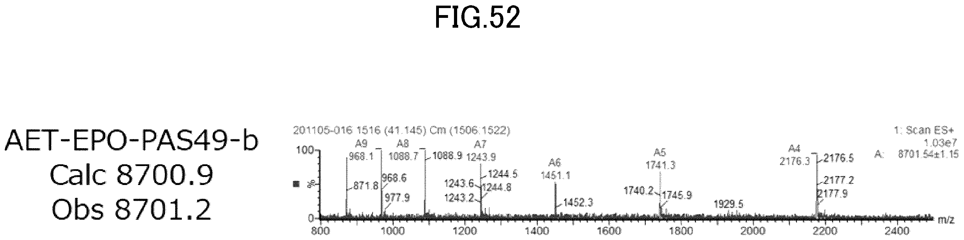

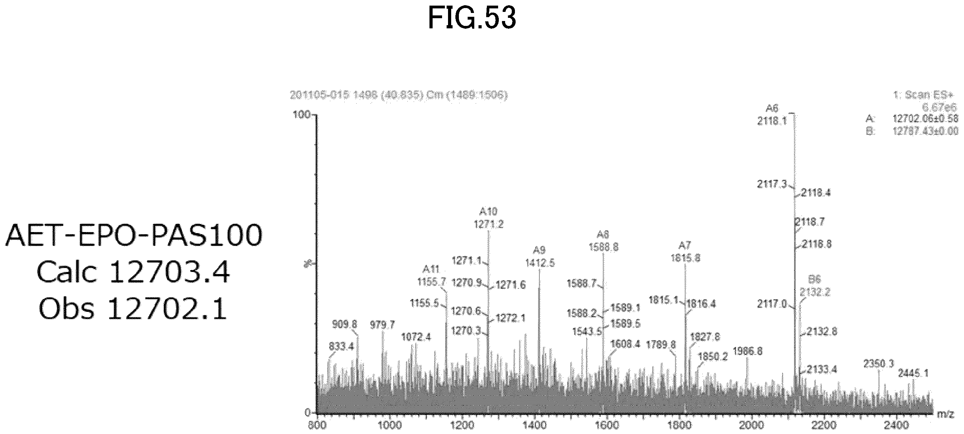

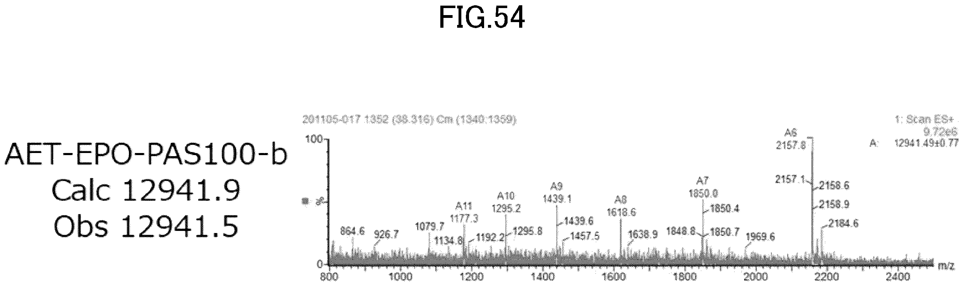

The cell filtrate of the EPO-PAS dimer peptide linker variant after culturing in Example 30 was subjected to molecular weight measurement using LC-MS (Waters, ACQUITY UPLC/SQD2). The analysis conditions are shown in Table 2. As shown in Figs. 49 to 54, almost the same value as the theoretical value of the molecular weight of each oxidized dimer peptide forming two disulfide bonds in the molecule was obtained. From this, it was confirmed that a peptide having the correct amino acid sequence containing two disulfide bonds could be expressed.

### Example 32: Activity evaluation of EPO-PAS dimer peptide linker variant

The EpoR activation ability of the EPO-PAS dimer peptide linker variant was evaluated using PathHunter (registered trademark) eXpress EpoR-JAK2 Functional Assay Kit (DiscoverX).

Cells for EPO activity evaluation included in PathHunter (registered trademark) eXpress EpoR-JAK2 Functional Assay Kit were seeded on a 96-well plate and cultured for 24 hours at 37°C under 5% CO₂. To the cells, a culture supernatant containing AET-EPO-PAS8, AET-EPO-GS8, AET-EPO-PAS22, or AET-EPO-GS22 diluted to a predetermined concentration (cultured as described in Example 20 or 30), or the monomer peptide EMP35 (GGLYACHMGPMTWVCQPLRG, synthesized by GenScript Japan) was added. As a negative control, the culture supernatant (Fig. 48, lane 26) obtained by culturing as described in Example 30 except for using a vector containing no gene for expressing the EPO-PAS dimer peptide, diluted at the same ratio as the culture supernatant containing AET-EPO-PAS8 or AET-EPO-PAS22, was added. The prepared Substrate Reagent was added to the cells stimulated at room temperature for 3 hours, and the cells were incubated for 60 minutes. The chemiluminescence intensity was quantified with a Nivo plate reader (Perkin Elmer).

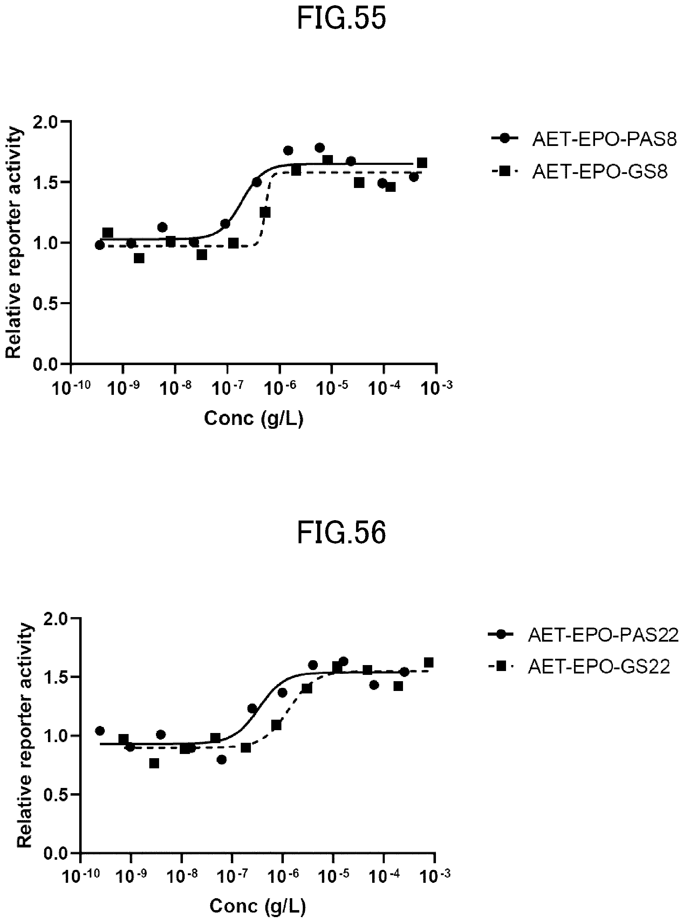

As shown in Figs. 55 to 57, it was clarified that all dimer peptides activated EpoR in a concentration-dependent manner. The EC50 value for each construct is shown in Table 6 below, and it was clarified that AET-EPO-PAS8 showed higher activity than AET-EPO-GS8, and AET-EPO-PAS22 had higher activity than AET-EPO-GS22. Note that the EC50 values of rhEPO, AET-EPO-PAS8, and AET-EPO-PAS22 are those in Example 22. It was also clarified that AET-EPO-PAS100 had lower activity than AET-EPO-PAS49, AET-EPO-PAS22, and AET-EPO-PAS8. From this, it has been clarified that a dimer peptide can be developed that has higher activity than a GS linker by using a PAS linker and that has high agonist activity by using a PAS linker of 49 amino acids or less.

**Table 6**

| Peptide | EC50 (g/L) |
|---|---|
| rhEPO | 1.1 × 10⁻⁶ |
| AET-EPO-PAS8 | 1.9 × 10⁻⁷ |
| AET-EPO-GS8 | 5.3 × 10⁻⁷ |
| AET-EPO-PAS22 | 3.4 × 10⁻⁷ |
| AET-EPO-GS22 | 1.3 × 10⁻⁶ |
| AET-EPO-PAS49 | 4.3 × 10⁻⁶ |
| AET-EPO-PAS100 | 6.6 × 10⁻⁶ |

### Example 33: Design of stabilized EPO-PAS dimer peptide and its secreted expression in C. glutamicum

### (33-1) Outline of design

The EPO-PAS peptide with enhanced stability was designed as follows. As a method for enhancing the stability of peptides and proteins, a method for adding a long-chain PAS sequence has been reported (Published Japanese Translation of PCT International Application No. 2013-531480 and Published Japanese Translation of PCT International Application No. 2010-531139). As an EPO-PAS peptide with enhanced stability, a peptide was designed in which a PAS sequence composed of 200 amino acids (PAS200) or a PAS sequence composed of 600 amino acids (PAS600) was bound to the C-terminus of AET-EPO-PAS22. That is, the peptide sequence was designed to be "first erythropoietin receptor-binding cyclic peptide"-"PAS linker"-"second erythropoietin receptor-binding cyclic peptide"-"long-chain PAS sequence (PAS200 or PAS600)" .

### (33-2) Preparation of stabilized EPO-PAS dimer peptides

The prepared stabilized EPO-PAS dimer peptides are as follows.
(D) AET-EPO-PAS22-PAS200 
AETGGLYACHMGPMTWVCQPLRGAAPAAPAPAAPAAPAPAAPAAPGGLYACHM
GPMTWVCQPLRGAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
 
AAPAPAAPAAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAP (SEQ ID NO:
144)
(E) AET-EPO-PAS22-PAS600

AETGGLYACHMGPMTWVCQPLRGAAPAAPAPAAPAAPAPAAPAAPGGLYACHM
GPMTWVCQPLRGAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAAPAPAAPAAPAP
(SEQ ID NO: 145)

### (33-3) Expression of stabilized EPO-PAS dimer peptides

Corynex (registered trademark) was used to examine the expression of stabilized EPO-PAS dimer peptide. Hereinafter, examples of expression examinations using Corynex (registered trademark) will be described.

### (33-4) Construction of AET-EPO-PAS22-PAS200 secretory expression plasmid using CspB signal sequence

As the stabilized EPO-PAS dimer peptide, the amino acid sequences of the above (D) AET-EPO-PAS22-PAS200 (which may be hereinafter referred to as "AET-EPO-PAS22-PAS200") and (E) AET-EPO-PAS22-PAS600 (which may be hereinafter referred to as "AET-EPO-PAS22-PAS600") were designed, and the base sequences encoding these proteins were designed in consideration of the codon usage frequency of C. glutamicum. Furthermore, the following expression cassette was designed to enable secretion expression by C. glutamicum.

AET-EPO-PAS22-PAS200 was secretory-expressed as a fusion protein of AET-EPO-PAS22-PAS200 and a signal peptide 30 amino acid residue of CspB derived from C. glutamicum ATCC 13869 strain (hereinafter referred to as "CspBss-AET-EPO-PAS22-PAS200"). The base sequence and amino acid sequence encoding the designed CspBss-AET-EPO-PAS22-PAS200 are set forth in SEQ ID NOs: 146 and 147, respectively.
Base sequence encoding CspBss-AET-EPO-PAS22-PAS200 
atgttcaacaaccgtattcgtaccgctgccctggccggcgccattgccatctccaccgctgcgtccggcgtcgccattcctgcg
ttcgcagccgaaaccggcggcctgtacgcatgccacatgggtccaatgacctgggtgtgccagccactgcgcggcgctgca
ccagctgctccagcaccagcagccccagctgcaccagcaccagctgctccagctgcaccaggcggcctgtacgcttgtcat
atgggtccaatgacttgggtctgccagccactccgcggcgctgcaccagcagctcctgctccagcagcacctgcagcacca
gcaccagcagctccagcagcacctgccccagcagctcctgcagctcctgccccagcagcacctgccgctcctgctcctgca
gctccagcagcacccgccccagcagctcccgcagctcctgcgccagcagcacctgccgcccctgctcccgcagctccagc
agcaccggcacccgcagctccagcagctcctgcacctgcagcacctgccgctcccgctccggcagctccagcagcaccag
cccctgcagctcctgctgcacctgctccagcagcacccgcggctcctgctccggctgctccagcagcccctgctccagcagc
tcccgctgcacctgcaccagcagcacctgccgcgcctgctcctgctgcccctgctgctcctgctcctgcagcccctgctgcac
ccgctccagcagcacctgcggctcctgctccggccgcccctgctgctccagcaccagctgcccctgcagcccctgcccctg
cagccccagctgcaccagcaccagcagcaccagcagcacccgcaccagcagccccagcagccccagccccttag
(SEQ ID NO: 146)
Amino acid sequence of CspBss-AET-EPO-PAS22-PAS200

MFNNRIRTAALAGAIAISTAASGVAIPAFAAETGGLYACHMGPMTWVCQPLRGA
APAAPAPAAPAAPAPAAPAAPGGLYACHMGPMTWVCQPLRGAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAP (SEQ ID NO: 147)

AET-EPO-PAS22-PAS600 was secretory-expressed as a fusion protein of AET-EPO-PAS22-PAS600 and a signal peptide 30 amino acid residue of CspB derived from C. glutamicum ATCC 13869 strain (hereinafter referred to as "CspBss-AET-EPO-PAS22-PAS600"). The base sequence and amino acid sequence encoding the designed CspBss-AET-EPO-PAS22-PAS600 are set forth in SEQ ID NOs: 148 and 149, respectively.
Base sequence encoding CspBss-AET-EPO-PAS22-PAS600 
atgtttaacaaccgtattcgtaccgctgccctcgctggtgccattgccatctctaccgctgcgtccggcgtcgctattcctgccttt
gcagccgaaaccggcggcctgtacgcatgccacatgggtccaatgacctgggtgtgccagccactccgcggcgctgcacc
agcagctccagcaccagcagccccagctgcaccagctccagctgctccagctgcaccaggcggcctgtacgcttgtcatatg
ggtccaatgacttgggtctgtcaacctctgcgcggcgctgctcctgcagctccagctccagcagcaccagctgcaccagccc
ctgctgcaccagcagcaccagcaccagcagcaccagctgcccctgcccctgctgccccagctgcaccagcccccgctgcc
cctgccgctcctgctcctgcagcaccagctgcccccgcccctgctgctcctgcagcccctgctcccgctgcccctgccgccc
ccgcccctgcagcaccagctgcgcctgcccccgctgcgccagctgcaccagccccggctgcccctgccgctcccgccccc
gcagcaccagctgccccggccccggctgcgcctgctgcaccagctcctgctgcccctgccgctccggccccggcagcacc
agctgccccagcacctgctgcaccagctgcaccagcgcctgctgcccctgccgccccggcccccgcagctcctgctgcccc
cgcacctgccgcaccagctgcaccagcacctgctgctcccgccgctcctgctcccgcagcaccagctgcgcccgccccgg
ctgcgcccgcagcccctgctcccgccgcacctgctgctcccgcgcctgcagcaccagctgctcctgcacctgctgctcctgc
cgctccagctcctgctgccccggccgctcctgctccggcagcaccagctgcgccggcccccgctgcccccgcagcccctg
ctccggctgcccctgccgcccctgctccagcagctcctgctgccccagcacctgccgctcctgcagctcctgctccagctgcc
cctgccgctccagccccagcagcaccagctgcgccagcacctgccgctcccgcagctcctgctccagccgcacctgctgct
ccggcgcccgcagcaccagctgctcccgcacctgcggctccagcagcccctgcccctgctgcgcctgccgctcctgctcca
gcagctcccgctgcacctgccccggccgctccagctgcaccagccccagctgctccagccgctcccgctccggcagctcct
gctgctcccgctccggctgctcctgccgcaccagctcctgcagctccagcagcccccgctccggcagctcccgctgctcccg
ctccagccgctcctgcagccccagcaccagctgctccagccgcccccgctccggcagcccctgctgctcccgctcccgctg
ctcctgcggcaccagctcccgcagctccagcagccccggctccggcagctccggctgctcccgctcccgccgctcccgca
gcccctgctccagctgctccagccgcgcccgctccagcagctccagctgcacctgctccggccgcccctgccgctccagct
ccggcagctccagcagccccagcgcctgcagctcctgctgctccggctccagccgctcccgcagcccccgctccagctgct
ccagcggctcccgctccagcagcccccgccgcacctgctccagccgctccggcagccccagctccagccgctccagccgc
tccagcgcctgcagctccagcggctcccgccccagccgctcctgcagcgccagctccagcggctcctgccgctcctgcccc
tgcagctccagcagcgcccgctccagccgccccggcagctcctgctcctgctgctccagccgctccagcacctgcagctcc
agcagctcctgccccagccgctcccgcagcgccagctccagcagcgccggctgcaccggcccctgcagcaccagccgct
cccgctcccgctgctcccgccgcccccgcacccgccgcccccgctgctcccgctccctaa (SEQ ID NO: 148)
Amino acid sequence of CspBss-AET-EPO-PAS22-PAS600

MFNNRIRTAALAGAIAISTAASGVAIPAFAAETGGLYACHMGPMTWVCQPLRGA
APAAPAPAAPAAPAPAAPAAPGGLYACHMGPMTWVCQPLRGAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAPAAPAAPAPAAPAAPAPAAPAAPAPAAPA
APAPAAPAAPAPAAPAAPAPAAPAAPAP (SEQ ID NO: 149)

The promoter of the cspB gene derived from the C. glutamicum ATCC 13869 strain was linked upstream of the base sequences set forth in CspBss-AET-EPO-PAS22-PAS200 and CspBss-AET-EPO-PAS22-PAS600, and furthermore, an expression cassette of each stabilized EPO-PAS dimer peptide having a KpnI site on the 5'-side and an BamHI site on the 3'-side was designed and totally synthesized. The totally synthesized DNA fragment (stabilized EPO-PAS dimer peptide expression cassette) was inserted into the KpnI-BamHI site of pPK4 described in Japanese Patent Application Publication No. Hei 9-322774 to construct pPK4_CspBss-AET-EPO-PAS22-PAS200 and pPK4_CspBss-AET-EPO-PAS22-PAS600, a secretory expression plasmid for each stabilized EPO-PAS dimer peptide using the CspB signal sequence. As a result of determining the base sequence of the inserted fragment, it was confirmed that the stabilized EPO-PAS dimer peptide expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (33-5) Secretory expression of stabilized EPO-PAS dimer peptide in C. glutamicum

The pPK4_CspBss-AET-EPO-PAS22-PAS200 and pPK4_CspBss-AET-EPO-PAS22-PAS600 constructed above were used to transform the C. glutamicum YDK0107 strain described in WO2016/171224 to obtain the YDK0107/pPK4_CspBss-AET-EPO-PAS22-PAS200 strain and YDK0107/pPK4_CspBss-AET-EPO-PAS22-PAS600 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

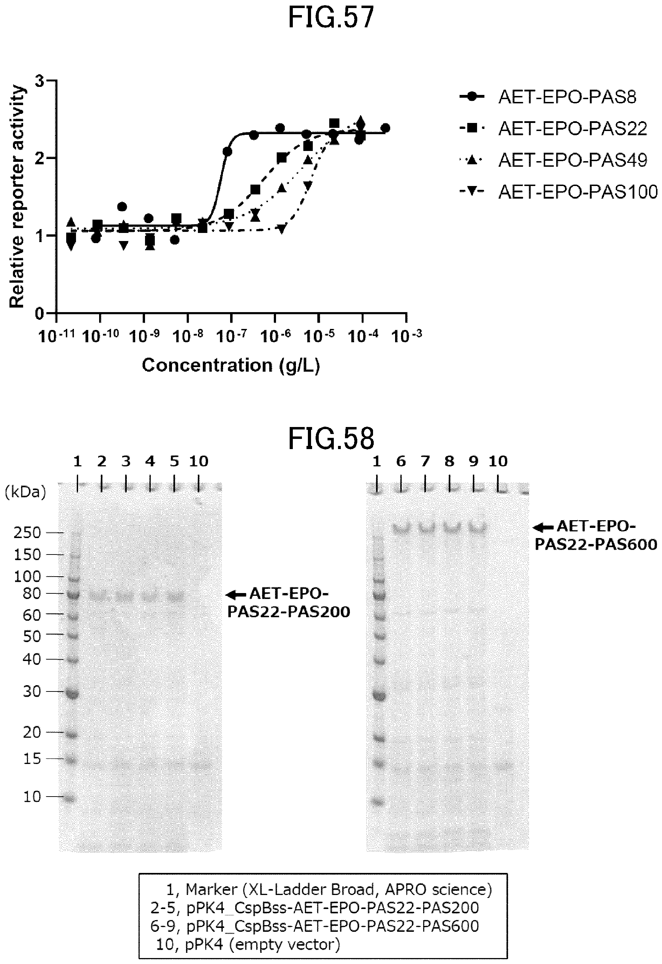

After completion of the culture, 6.5 µL of the culture supernatant obtained by centrifuging each culture solution was subjected to reduced SDS-PAGE using NuPAGE (registered trademark) 4 to 12% Bis-Tirs Gel (Thermo Fisher Scientific), and then stained with Quick-CBB (Wako). As a result, a protein band presumed to be AET-EPO-PAS22-PAS200 was detected in the culture supernatant of the YDK0107/pPK4_CspBss-AET-EPO-PAS22-PAS200 strain (Fig. 58, lanes 2 to 5) and a protein band presumed to be AET-EPO-PAS22-PAS600 was detected in the culture supernatant of the YDK0107/pPK4_CspBss-AET-EPO-PAS22-PAS600 strain (Fig. 58, lanes 6 to 9).

### Example 34: Molecular weight analysis of stabilized EPO-PAS dimer peptide

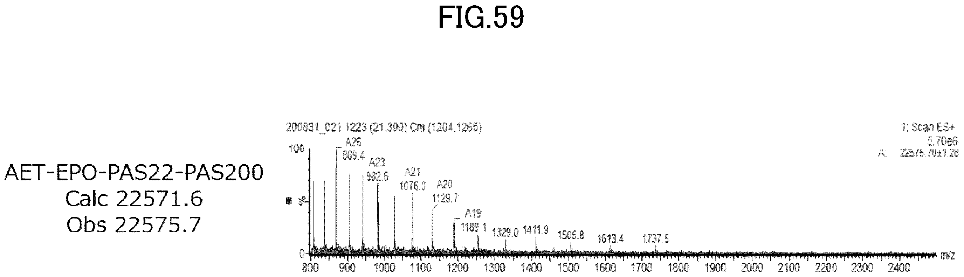

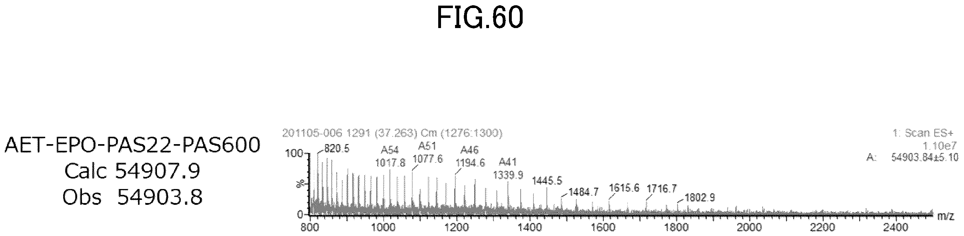

The cell filtrate of each stabilized EPO-PAS dimer peptide after culturing in Example 33 was subjected to molecular weight measurement using LC-MS (Waters, ACQUITY UPLC/SQD2). The analysis conditions are shown in Table 2. As shown in Figs. 59 and 60, almost the same value as the theoretical value of the molecular weight of each oxidized dimer peptide forming two disulfide bonds in the molecule was obtained. From this, it was confirmed that a peptide having the correct amino acid sequence containing two disulfide bonds could be expressed.

### Example 35: Activity evaluation of stabilized EPO-PAS dimer peptide

The EpoR activation ability of the stabilized EPO-PAS dimer peptide was evaluated using PathHunter (registered trademark) eXpress EpoR-JAK2 Functional Assay Kit (DiscoverX).

Cells for EPO activity evaluation included in PathHunter (registered trademark) eXpress EpoR-JAK2 Functional Assay Kit were seeded on a 96-well plate and cultured for 24 hours at 37°C under 5% CO₂. To the cells, a culture supernatant containing 14.2 µg/L to 220 pg/L AET-EPO-PAS22, AET-EPO-PAS22-PAS200, or AET-EPO-PAS22-PAS600 (cultured as described in Example 20 or 33) was added. As a negative control, the culture supernatant (Fig. 58, lane 20) obtained by culturing as described in Example 33 except for using a vector containing no gene for expressing the EPO-PAS dimer peptide, diluted at the same ratio as the culture supernatant containing AET-EPO-PAS22, AET-EPO-PAS22-PAS200, or AET-EPO-PAS22-PAS600, was added. The prepared Substrate Reagent was added to the cells stimulated at room temperature for 3 hours, and the cells were incubated for 60 minutes. The chemiluminescence intensity was quantified with a Nivo plate reader (Perkin Elmer).

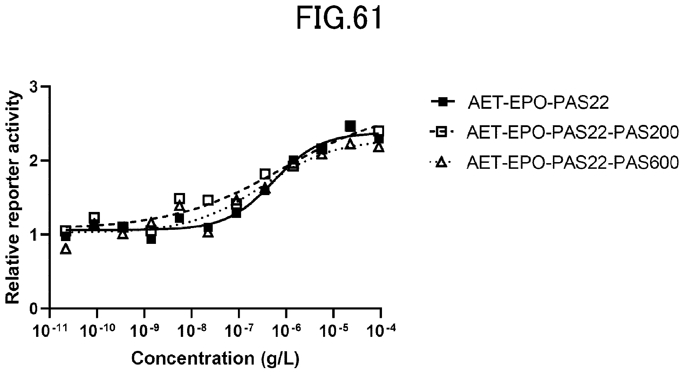

As shown in Fig. 61, it was clarified that AET-EPO-PAS22, AET-EPO-PAS22-PAS200, or AET-EPO-PAS22-PAS600 activated EpoR in a concentration-dependent manner. In addition, the EC50 values were calculated as shown in Table 7 below, and it was clarified that the agonistic ability of the EPO-PAS dimer peptide did not decrease even if the C-terminus was stabilized and modified. Note that the EC50 value of AET-EPO-PAS22 is that of Example 22.

**Table 7**

| Peptide | EC50 (g/L) |
|---|---|
| AET-EPO-PAS22 | 3.4 × 10⁻⁷ |
| AET-EPO-PAS22-PAS200 | 8.5 × 10⁻⁷ |
| AET-EPO-PAS22-PAS600 | 3.2 × 10⁻⁷ |

### Example 36: Cell-free expression of dimer peptides

### (36-1) Construction of gene construct of cell-free expression dimer peptides

The following were designed as cell-free expression dimer peptides.
(F) MAET-aMD4dY-PAS22 
MAETCRQFNRRTHEVWNLDCGAAPAAPAPAAPAAPAPAAPAAPGCRQFNRRTH
EVWNLDC (SEQ ID NO: 150)
(G) MAET-aMD4dY-PAS8 MAETCRQFNRRTHEVWNLDCGAAPAAPAPGCRQFNRRTHEVWNLDC (SEQ ID NO: 151)
(H) MAET-aMD4-PAS22 
MAETCYRQFNRRTHEVWNLDCGAAPAAPAPAAPAAPAPAAPAAPGCRQFNRRT
HEVWNLDC (SEQ ID NO: 152)
(I) MAET-EPO-PAS8 
MAETGGLYACHMGPMTWVCQPLRGAAPAAPAPGGLYACHMGPMTWVCQPLR
G (SEQ ID NO: 153)
(J) MAET-EPO-PAS49-b 
MAETGGLYACHMGPMTWVCQPLRGSAPSPSSAPASASAPASPASASASASPASSP
ASASSASPAASSASPASSGGLYACHMGPMTWVCQPLRG (SEQ ID NO: 154)
(K) MAET-EPO-PAS100-b 
MAETGGLYACHMGPMTWVCQPLRGSAPSPSSAPASASAPASPASASASASPASSP
ASASSASPAASSASPASSSASPSAPAAPSASAAPSASPASSSPAPASAPSPAAAASSP
SPAPSSASSAAGGLYACHMGPMTWVCQPLRG (SEQ ID NO: 155)
(L) MAET-VEGFR_D2-PAS16 
MAETAGPTWCEDDWYYCWLFGTAAPAAPAPAAPAAPAPVCWEDSWGGEVCW
LFGT (SEQ ID NO: 156)
(M) MAET-VEGFR_D2-PAS49-b 
MAETAGPTWCEDDWYYCWLFGTSAPSPSSAPASASAPASPASASASASPASSPAS
ASSASPAASSASPASSVCWEDSWGGEVCWLFGT (SEQ ID NO: 157)
(N) MAET-VEGFR_D2-PAS100-b 
MAETAGPTWCEDDWYYCWLFGTSAPSPSSAPASASAPASPASASASASPASSPAS
ASSASPAASSASPASSSASPSAPAAPSASAAPSASPASSSPAPASAPSPAAAASSPSP
APSSASSAAVCWEDSWGGEVCWLFGT (SEQ ID NO: 158)
(O) MAET-VEGFR_D2-GS16

MAETAGPTWCEDDWYYCWLFGTGGGGSGGGGSGGGGSGVCWEDSWGGEVC
WLFGT (SEQ ID NO: 159)

Base sequence encoding the amino acid sequences of the above dimer peptides were designed. Furthermore, 5'-sides were added with T7 promoter sequences and ribosome binding sequences required for cell-free expression, and their 3'-sides were added with stop codons and untranslated regions. The corresponding cell-free expression base sequences are shown below. These DNA sequences were totally synthesized and cloned into the pEX-K4J2 vector by Eurofins Genomics.
Base sequence encoding MAET-aMD4dY-PAS22 
gaaattaatacgactcactatagggagaccacaacggtttccctctagaaataattttgtttaactttaagaaggagatataccaat
gcagagacctgtcgtcagtttaaccgtcgtacgcacgaagtttggaatctggactgtggcgcagcccccgcagcaccagctc
cagcagcacccgcagctccagcaccagcagcccctgcagccccaggctgccgccaattcaatcgccgcacccatgaagtg
tggaacttagattgctagtgaataactaatcc(SEQ ID NO: 160)
Base sequence encoding MAET-aMD4dY-PAS8
gaaattaatacgactcactatagggagaccacaacggtttccctctagaaataattttgtttaactttaagaaggagatataccaat
ggcagagacctgtcgtcagtttaaccgtcgtacgcacgaagtttggaatctggactgtggcgcagcacccgcagctccagca
ccaggctgccgccaattcaatcgccgcacccatgaagtgtggaacttagattgctagtgaataactaatcc(SEQ ID NO:
161)
Base sequence encoding MAET-aMD4-PAS22 
gaaattaatacgactcactatagggagaccacaacggtttccctctagaaataattttgtttaactttaagaaggagatataccaat
ggcagagacctgttatcgtcagtttaaccgtcgtacgcacgaagtttggaatctggactgtggcgcagcccccgcagcaccag
ctccagcagcacccgcagctccagcaccagcagcccctgcagccccaggctgctatcgccaattcaatcgccgcacccatg
aagtgtggaacttagattgctagtgaataactaatcc(SEQ ID NO: 162)
Base sequence encoding MAET-EPO-PAS8
gaaattaatacgactcactatagggagaccacaacggtttccctctagaaataattttgtttaactttaagaaggagatataccaat
ggcagaaactggtggtttatacgcctgtcacatgggcccgatgacttgggtttgccagccattgcgtggcgcagcacccgcag
ctccagcaccaggtggcctgtatgcgtgccatatggggccaatgacctgggtgtgtcagccgttacgcggctagtgaataact
aatcc(SEQ ID NO: 163)
Base sequence encoding MAET-EPO-PAS49-b 
gaaattaatacgactcactatagggagaccacaacggtttccctctagaaataattttgtttaactttaagaaggagatataccaat
ggcagaaactggtggtttatacgcctgtcacatgggcccgatgacttgggtttgccagccattgcgtggctctgctccatcacct
tcttccgcaccagcatcagcttctgcaccagcgtcgcctgcttctgcatctgcgtctgcttcaccagcatcctcacctgcatcagc
atcgtctgcttctccagcagcttcctcagcttcacctgcttcgtctggtggcctgtatgcgtgccatatggggccaatgacctggg
tgtgtcagccgttacgcggctagtgaataactaatcc(SEQ ID NO: 164)
Base sequence encoding MAET-EPO-PAS100-b 
gaaattaatacgactcactatagggagaccacaacggtttccctctagaaataattttgtttaactttaagaaggagatataccaat
ggcagaaactggtggtttatacgcctgtcacatgggcccgatgacttgggtttgccagccattgcgtggctctgctccatcacct
tcttccgcaccagcatcagcttctgcaccagcgtcgcctgcttctgcatctgcgtctgcttcaccagcatcctcacctgcatcagc
atcgtctgcttctccagcagcttcctcagcttcacctgcttcgtcttccgcatccccatctgctccagcagcaccttcagcttctgc
agctccatctgcttcacctgcctcatcgtctccagcacctgcgtcagctccatcccctgcagcagcggcttcatcgccatctcca
gcaccttcttcagcgtcatccgctgcaggtggcctgtatgcgtgccatatggggccaatgacctgggtgtgtcagccgttacgc
ggctagtgaataactaatcc(SEQ ID NO: 165)
Base sequence encoding MAET-VEGFR_D2-PAS16 
gaaattaatacgactcactatagggagaccacaacggtttccctctagaaataattttgtttaactttaagaaggagatataccaat
ggcggaaaccgcgggtcccacgtggtgtgaggatgactggtactattgctggttattcgggactgcggcccctgctgccccg
gctccggcagcaccggccgcaccagcgccggtttgctgggaagatagctggggtggcgaagtgtgttggctgtttggcacct
agtgaataactaatcc (SEQ ID NO: 166)
Base sequence encoding MAET-VEGFR_D2-PAS49-b 
gaaattaatacgactcactatagggagaccacaacggtttccctctagaaataattttgtttaactttaagaaggagatataccaat
ggcggaaaccgcgggtcccacgtggtgtgaggatgactggtactattgctggttattcgggacttctgctccatcaccttcttcc
gcaccagcatcagcttctgcaccagcgtcgcctgcttctgcatctgcgtctgcttcaccagcatcctcacctgcatcagcatcgt
ctgcttctccagcagcttcctcagcttcacctgcttcgtctgtttgctgggaagatagctggggtggcgaagtgtgttggctgtttg
gcacctagtgaataactaatcc (SEQ ID NO: 167)
Base sequence encoding MAET-VEGFR_D2-PAS100-b 
gaaattaatacgactcactatagggagaccacaacggtttccctctagaaataattttgtttaactttaagaaggagatataccaat
ggcagaaactggtggtttatacgcctgtcacatgggcccgatgacttgggtttgccagccattgcgtggctctgctccatcacct
tcttccgcaccagcatcagcttctgcaccagcgtcgcctgcttctgcatctgcgtctgcttcaccagcatcctcacctgcatcagc
atcgtctgcttctccagcagcttcctcagcttcacctgcttcgtcttccgcatccccatctgctccagcagcaccttcagcttctgc
agctccatctgcttcacctgcctcatcgtctccagcacctgcgtcagctccatcccctgcagcagcggcttcatcgccatctcca
 
gcaccttcttcagcgtcatccgctgcaggtggcctgtatgcgtgccatatggggccaatgacctgggtgtgtcagccgttacgc
ggctagtgaataactaatcc (SEQ ID NO: 168)
Base sequence encoding MAET-VEGFR_D2-GS16

gaaattaatacgactcactatagggagaccacaacggtttccctctagaaataattttgtttaactttaagaaggagatataccaat
ggcggaaaccgcgggtcccacgtggtgtgaggatgactggtactattgctggttattcgggactggtagcggtagcggcagc
ggcagcggtagtggtagtggcagcggcagcgtttgctgggaagatagctggggtggcgaagtgtgttggctgtttggcacct
agtgaataactaatcc (SEQ ID NO: 169)

### (36-2) Cell-free expression of dimer peptides

The genes of the dimer peptides constructed above were amplified by PCR. PCR was performed using the primers of the sequences shown below and KOD -Plus-Ver. 2 (Toyobo) as the PCR enzyme.
Cell-free primer Fwd:
   gaaattaatacgactcactataggg (SEQ ID NO: 170)
Cell-free primer Rev:
   cactgactggattagttattcac (SEQ ID NO: 171)

Next, the peptide dimers were expressed by a cell-free expression system based on the gene fragments amplified by PCR. PUREfrex 2.0 (GeneFrontier) was used for cell-free expression.

For cell-free expression with PUREfrex 2.0, to the gene fragments amplified by 2 nM PCR, 10 µL of Solution I, 1 µL of Solution II, 2 µL of Solution III, and 7 µL of water were added, and the mixture was incubated at 37°C for 6 hours to carry out a translation reaction.

### (36-3) Molecular weight analysis of cell-free expression dimer peptides

A solution, obtained by expressing HGF-PAS dimer peptides (MAET-aMD4-PAS22, MAET-aMD4dY-PAS8, MAET-aMD4dY-PAS22), EPO-PAS dimer peptides (MAET-EPO-PAS8, MAET-EPO-PAS49-b, MAET-EPO-PAS100-b), and a VEGFR_D2-PAS dimer peptide (MAET-VEGFR_D2-PAS16) in a cell-free expression system, was mixed with α-Cyano-4-hydroxycinnamic Acid (CHCA), and subjected to molecular weight measurement with MALDI-TOF-MS (Shimadzu, AXIMA TOF 2).

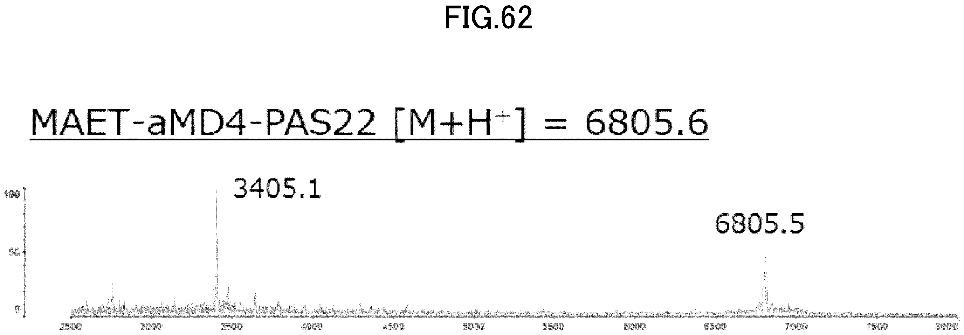

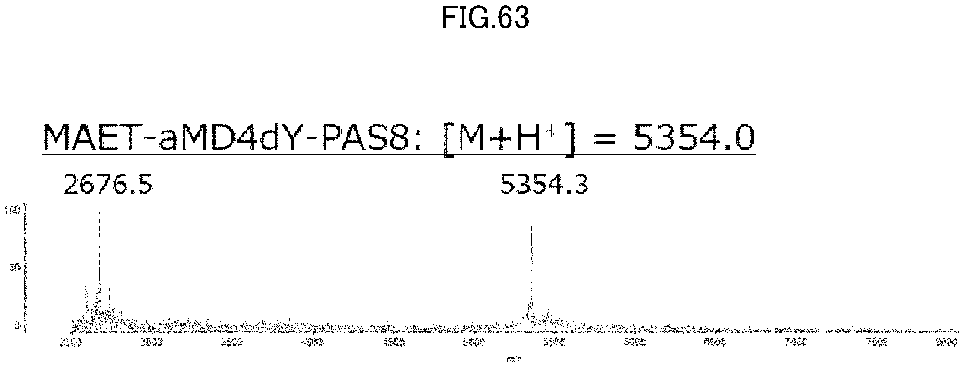

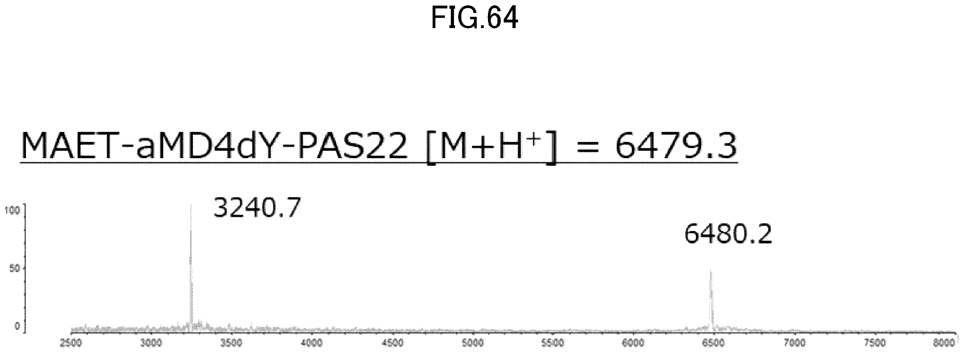

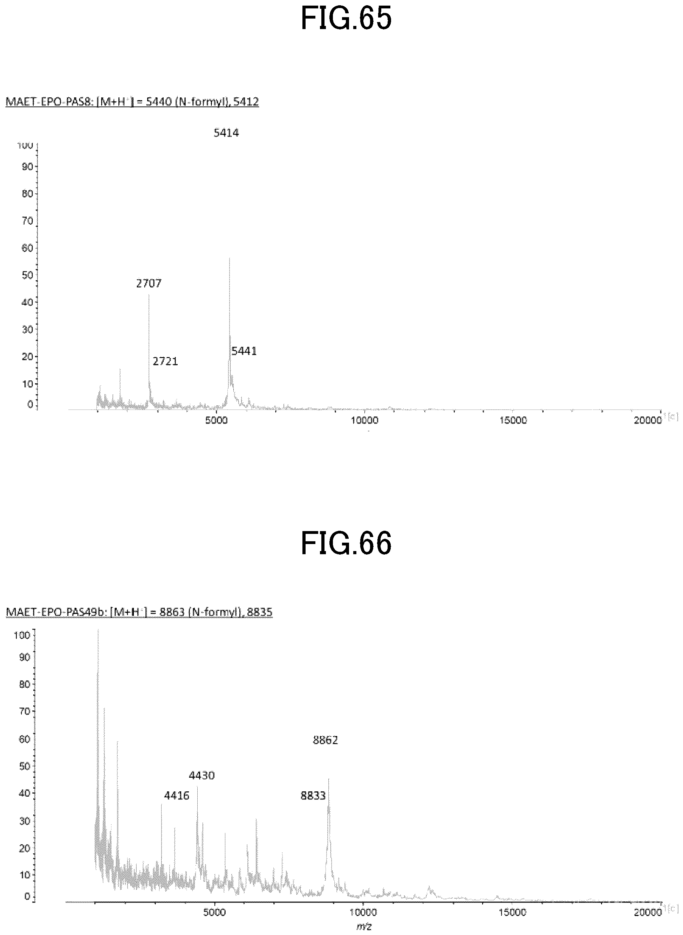

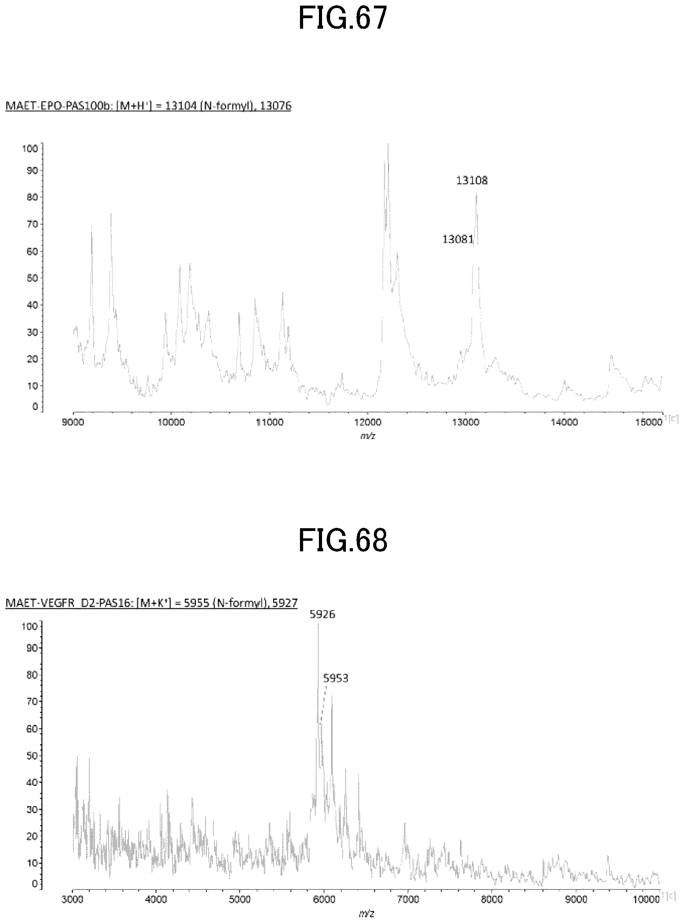

As shown in Figs. 62 to 68, for the HGF-PAS dimer peptides, values were obtained close to the molecular weight with two formed disulfide bonds and a formylated N-terminus, and for the EPO-PAS dimer peptides and VEGFR_D2-PAS dimer peptide, both the molecular weight with two formed disulfide bonds and a formylated N-terminus and the molecular weight without deformylated N-terminus were obtained, each demonstrating that dimer peptides containing two disulfide bonds could be expressed.

### Example 37: Activity evaluation of cell-free expression HGF-PAS dimer peptides

The Met activation ability of the cell-free expression HGF-PAS dimer peptides was evaluated by luciferase assay. To 25 µL of Opti-MEM medium (Thermo Fisher Scientific), 0.6 µL of Attractene Transfection Reagent (QIAGEN) was added, and the mixture was incubated at room temperature for 5 minutes. To the above mixed solution, a mixed solution of 25 µL of Opti-MEM and 1 µL of SRE reporter vector (QIAGEN) was added, and the mixture was incubated at room temperature for 20 minutes. This mixed solution was added to a 96-well plate, over which 40,000 cell/well HEK293E cells were seeded and incubated overnight in a 5% CO₂ incubator at 37°C. After transfection, all culture supernatant was removed, 100 µL of Opti-MEM medium (evaluation basal medium) containing 0.5% FBS (Thermo Fisher Scientific), 1% non-essential amino acid solution (Thermo Fisher Scientific), and penicillin-streptomycin (Nacalai Tesque) was added, and incubation was performed at 37°C for 4 hours, and thereby the cells were starved.

To the evaluation basal medium, 0 to 100 ng/mL HGF was added, or a cell-free translation solution with translated AET-aMD4dY-PAS22 was added to the medium so as to be diluted at a predetermined ratio, or a cell-free translation solution with untranslated peptide was added to dilute at the same ratio, and the evaluation basal medium was added in an amount of 100 µL to the cells. The cells were stimulated by culturing overnight in a 5% CO₂ incubator at 37°C.

The signal intensities were detected using Dual-Luciferase Reporter Assay System (Promega). The medium supernatant in an amount of 100 µL was removed, 50 µL of Glo reagent was added, and the cells were lysed for 10 minutes at room temperature. The luminescence of Firefly luciferase emitted from the cell lysate solution was detected with a plate reader to quantify the activation of the HGF-Erk-SRE pathway. Subsequently, 50 µL of Glo & Stop reagent solution was added, and after 10 minutes, the luminescence of the internal standard Renilla luciferase was detected to quantify the number of cells. The signal activity of each sample was quantified as SRE activity=(Firefly luciferase luminescence intensity)/(Renilla luciferase luminescence intensity). The relative signal activity values for the samples without addition of the evaluation compound were determined and used as relative receptor activity.

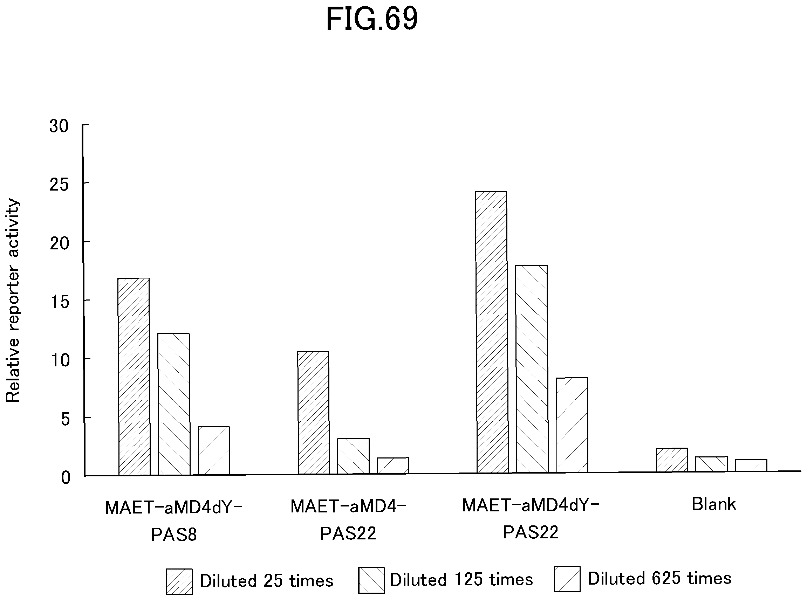

As shown in Fig. 69, it was clarified that the cell-free translations MAET-aMD4dY-PAS22, MAET-aMD4dY-PAS8, and MAET-aMD4-PAS22 had Met activation ability.

### Example 38: Activity evaluation of cell-free expression EPO-PAS dimer peptide

The EpoR activation ability of the EPO-PAS dimer peptide linker variant was evaluated using PathHunter (registered trademark) eXpress EpoR-JAK2 Functional Assay Kit (DiscoverX).

Cells for EPO activity evaluation included in PathHunter (registered trademark) eXpress EpoR-JAK2 Functional Assay Kit were seeded on a 96-well plate and cultured for 24 hours at 37°C under 5% CO₂. To the cells, a cell-free translation solution with translated AET-EPO-PAS8, AET-EPO-PAS49-b, or AET-EPO-PAS100-b diluted to a predetermined concentration was added. As a negative control, a cell-free translation solution with untranslated peptide, diluted at the same ratio as the cell-free translation solution with translated AET-EPO-PAS8, AET-EPO-PAS49, or AET-EPO-PAS100, was added. The prepared Substrate Reagent was added to the cells stimulated at room temperature for 3 hours, and the cells were incubated for 60 minutes. The chemiluminescence intensity was quantified with a Nivo plate reader (Perkin Elmer).

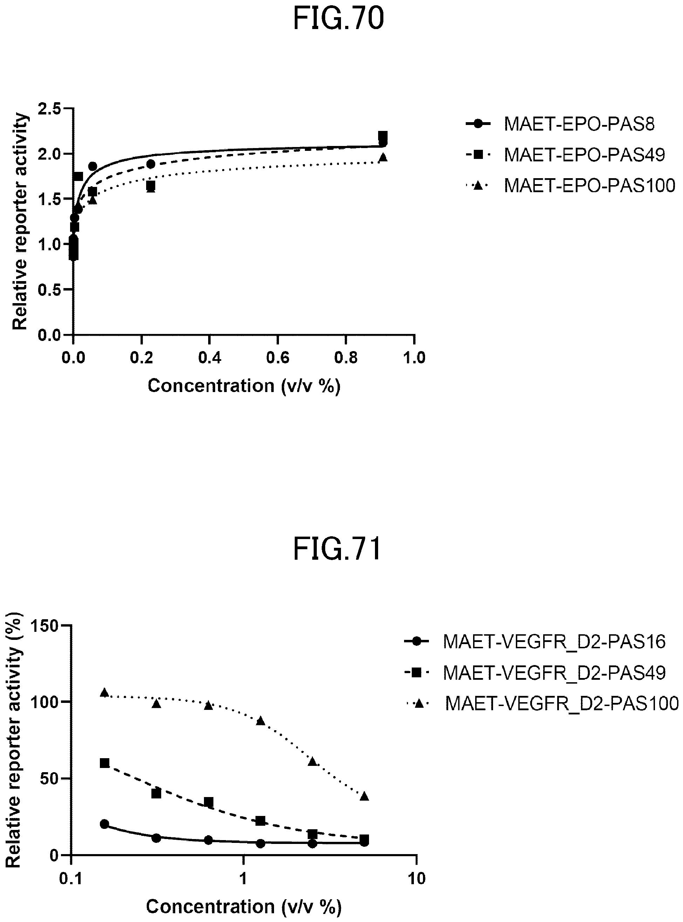

As shown in Fig. 70, it was clarified that all dimer peptides activated EpoR in a concentration-dependent manner. In addition, MAET-EPO-PAS8 and MAET-EPO-PAS49-b showed higher activity than MAET-EPO-PAS100-b. From this, it was been clarified that a dimer peptide having high activity could be constructed by using a short-chain PAS linker of 49 amino acids or less.

### Example 39: Activity evaluation of cell-free expression VEGFR_D2-PAS heterodimer

The VEGF inhibitory ability of the cell-free expressed VEGFR_D2-PAS heterodimer peptide was evaluated by luciferase assay. 40,000 cell/well VEGFR/NFAT Reporter HEK293 cells (BPS Bioscience) were seeded and incubated overnight in a 5% CO₂ incubator at 37°C. The cells were incubated overnight in Assay medium (MEM medium (Nacalai Tesque), 1 × MEM Non-Essential Amino Acids Solution (Thermo Fisher Scientific), 1 mM Sodium Pyruvate (Nacalai Tesque), 1% Penicillin-Streptomycin (Sigma Aldrich), and 0.3% FBS (Thermo Fisher Scientific)) to starvation. To the Assay medium, 100 µL of 10 ng/mL human recombinant VEGF (R&D Systems) was added, and further, MAET-VEGFRD2-PAS16, MAET-VEGFR_D2-PAS49-b, MAET-VEGFR_D2-PAS100-b, or a cell-free expression solution containing MAET-VEGFR_D2-GS16, or a cell-free expression solution with unexpressed peptide was added at a predetermined dilution factor, and 100 µL of the Assay medium was added to the cells. The cells were stimulated by culturing in a 5% CO₂ incubator at 37°C for 4 hours.

One-Glo Luciferase Assay System (Promega) was used to detect the signal intensity. Medium supernatant in an amount of 50 µL was removed, 50 µL of One-Glo reagent was added, and the cells were lysed for 10 minutes at room temperature. The luminescence emitted from the cell lysate solution was detected with a Nivo plate reader (Perkin Elmer) to quantify the activation of the VEGFR-NFAT pathway, and the relative signal activity value for the sample without addition of evaluation compound was determined and used as relative receptor activity.

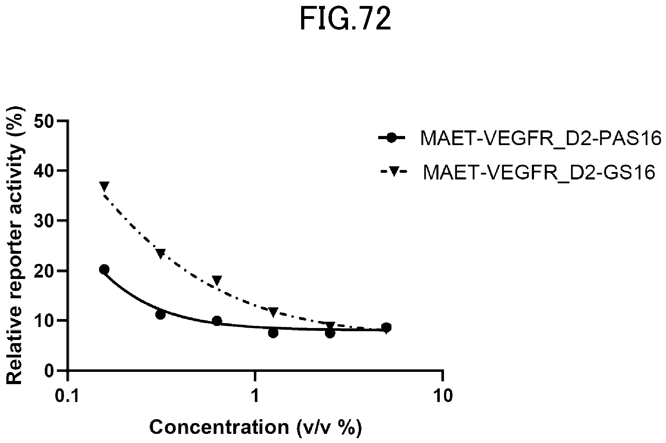

As shown in Figs. 71 and 72, MAET-VEGFR_D2-PAS16 and MAET-VEGFR_D2-PAS49-b showed higher inhibitory activity than MAET-VEGFR_D2-PAS100-b, so that it was clarified that a dimer peptide having high activity could be constructed by using a short-chain PAS linker of 49 amino acids or less. In addition, MAET-VEGFR_D2-PAS16 showed higher inhibitory activity than MAET-VEGFR_D2-GS 16, so that it was clarified that a dimer peptide having higher activity than a GS linker can be constructed by using a PAS linker.

### Example 40: Design of HGF-PAS trimer peptide and its secretory expression in C. glutamicum

### (40-1) Outline of design

The HGF-PAS trimer peptide was designed as follows. A peptide was designed in which an aMD4dY sequence was further bound to the C-terminus of AET-aMD4dY-PAS22 mentioned above via a PAS linker. That is, the peptide sequence was designed to be "first aMD4dY cyclic peptide"-"PAS linker"-"second aMD4dY cyclic peptide"-"PAS linker"-"third aMD4dY cyclic peptide".

### (40-2) Preparation of HGF-PAS trimer peptide

The prepared HGF-PAS trimer peptide is as follows.
(P) AET-aMD4dY-PAS22-Trimer

AETCRQFNRRTHEVWNLDCGAAPAAPAPAAPAAPAPAAPAAPGCRQFNRRTHE
VWNLDCGAAPAAPAPAAPAAPAPAAPAAPGCRQFNRRTHEVWNLDC (SEQ ID
NO: 172)

### (40-3) Expression of HGF-PAS trimer peptide

Corynex (registered trademark) was used to examine the expression of stabilized HGF-PAS trimer peptide. Hereinafter, examples of expression examinations using Corynex (registered trademark) will be described.

### (40-4) Construction of AET-aMD4dY-PAS22-Trimer secretory expression plasmid using CspB signal sequence

As the stabilized HGF-PAS dimer peptide, the amino acid sequence of the above (P) AET-aMD4dY-PAS22-Trimer was designed, and the base sequence encoding this protein was designed in consideration of the codon usage frequency of C. glutamicum. Furthermore, the following expression cassette was designed to enable secretion expression by C. glutamicum.

AET-aMD4dY-PAS22-Trimer was secretory-expressed as a fusion protein of AET-aMD4dY-PAS22-Trimer and a signal peptide 30 amino acid residue of CspB derived from C. glutamicum ATCC 13869 strain (hereinafter referred to as "CspBss-AET-aMD4dY-PAS22-Trimer"). The base sequence and amino acid sequence encoding the designed CspBss-AET-aMD4dY-PAS22-Trimer are set forth in SEQ ID NOs: 173 and 174, respectively.
Base sequence encoding CspBss-AET-aMD4dY-PAS22-Trimer 
atgtttaacaaccgtatccgcactgcagctctcgctggtgcaatcgcaatctccaccgcagcttccggcgtagctatcccagcat
tcgctgcagagacctgccgccagttcaatcgccggacccacgaagtctggaatctggattgcggcgcagctcctgccgcccc
agcaccggctgcgccagctgctccagcgccggctgcccctgcggcaccgggttgccgtcagtttaaccgccgtactcacga
ggtttggaaccttgactgtggagcggctcccgcagcaccagccccagccgcccctgctgcacccgcaccagcggcacccg
ccgctcctggctgtcgccaattcaaccgacgcacccatgaagtgtggaacctcgattgctaa (SEQ ID NO: 173)
Amino acid sequence of CspBss-AET-aMD4dY-PAS22-Trimer

MFNNRIRTAALAGAIAISTAASGVAIPAFAAETCRQFNRRTHEVWNLDCGAAPA
APAPAAPAAPAPAAPAAPGCRQFNRRTHEVWNLDCGAAPAAPAPAAPAAPAPAA
PAAPGCRQFNRRTHEVWNLDC (SEQ ID NO: 174)

The promoter of the cspB gene derived from the C. glutamicum ATCC 13869 strain was linked upstream of the base sequence set forth in CspBss-AET-aMD4dY-PAS22-Trimer, and furthermore, an expression cassette of HGF-PAS trimer peptide having a KpnI site on the 5'-side and an BamHI site on the 3'-side was designed and totally synthesized. The totally synthesized DNA fragment (HGF-PAS trimer peptide expression cassette) was inserted into the KpnI-BamHI site of pPK4 described in Japanese Patent Application Publication No. Hei 9-322774 to construct pPK4_CspBss-AET-aMD4dY-PAS22-Trimer, a secretory expression plasmid for the HGF-PAS trimer peptide using the CspB signal sequence. As a result of determining the base sequence of the inserted fragment, it was confirmed that the HGF-PAS trimer peptide expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (40-5) Secretory expression of HGF-PAS trimer peptide in C. glutamicum

The pPK4_CspBss-AET-aMD4dY-PAS22-Trimer constructed above was used to transform the C. glutamicum YDK010::phoS(W302C) strain described in WO2016/171224 to obtain the YDK010: :phoS(W302C)/pPK4_CspBss-AET-aMD4dY-PAS22-Trimer strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

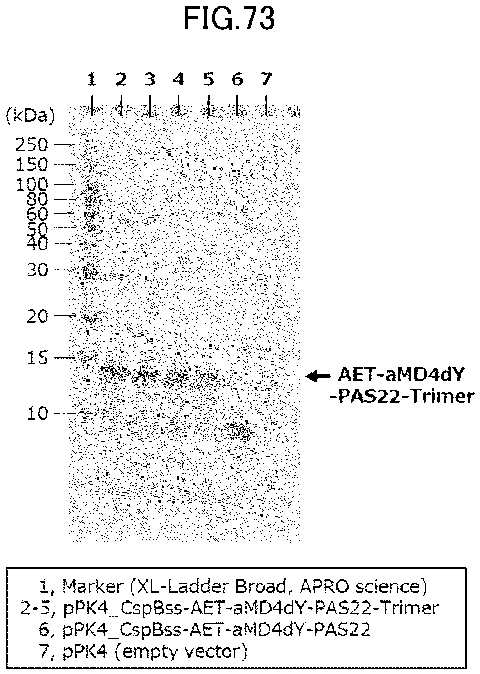

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be AET-aMD4dY-PAS22-Trimer was detected in the culture supernatant of the YDK010::phoS(W302C)/pPK4_CspBss-AET-aMD4dY-PAS22-Trimer strain (Fig. 73, lanes 2 to 5).

In addition, the YDK010::phoS(W302C)/pPK4_CspBss-AET-aMD4dY-PAS22 strain obtained by the same method as above was cultured in the same manner as above, and a protein band presumed to be AET-aMD4dY-PAS22 was detected in the culture supernatant (Fig. 73, lane 6).

### Example 41: Molecular weight analysis of HGF-PAS trimer peptide

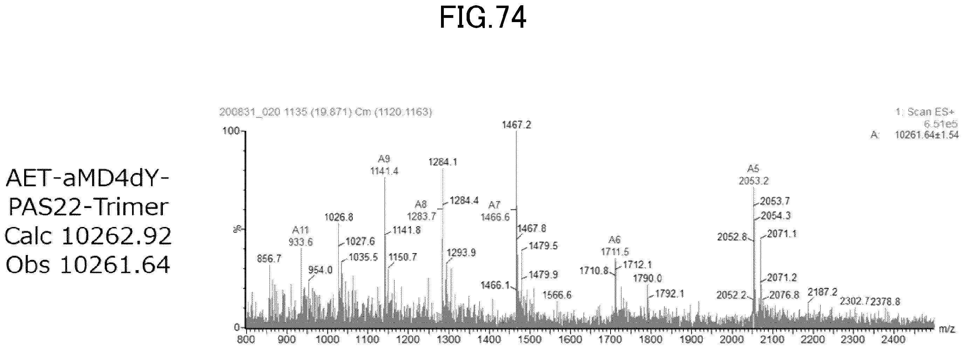

The cell filtrate of AET-aMD4dY-PAS22-Trimer after culturing in Example 40 was subjected to molecular weight measurement using LC-MS (Waters, ACQUITY UPLC/SQD2). The analysis conditions are shown in Table 2. As shown in Fig. 74, almost the same value as the theoretical value of the molecular weight of the oxidized dimer peptide forming three disulfide bonds in the molecule was obtained. From this, it was confirmed that a peptide having the correct amino acid sequence containing three disulfide bonds could be expressed.

### Example 42: Activity evaluation of HGF-PAS trimer peptide

The Met activation ability of the HGF-PAS trimer peptide was evaluated by luciferase assay. To 25 µL of Opti-MEM medium (Thermo Fisher Scientific), 0.6 µL of Attractene Transfection Reagent (QIAGEN) was added, and the mixture was incubated at room temperature for 5 minutes. To the above mixed solution, a mixed solution of 25 µL of Opti-MEM and 1 µL of SRE reporter vector (QIAGEN) was added, and the mixture was incubated at room temperature for 20 minutes. This mixed solution was added to a 96-well plate, over which 40,000 cell/well HEK293E cells were seeded and incubated overnight in a 5% CO₂ incubator at 37°C. After transfection, all culture supernatant was removed, 100 µL of Opti-MEM medium (evaluation basal medium) containing 0.5% FBS (Thermo Fisher Scientific), 1% non-essential amino acid solution (Thermo Fisher Scientific), and penicillin-streptomycin (Nacalai Tesque) was added, and incubation was performed at 37°C for 4 hours, and thereby the cells were starved.

To the evaluation basal medium, 0 to 100 ng/mL HGF was added, or a culture supernatant containing AET-aMD4dY-PAS22 or AET-aMD4dY-PAS22-Trimer was added to the medium so as to be diluted at a predetermined ratio, and the evaluation basal medium was added in an amount of 100 µL to the cells. The cells were stimulated by culturing overnight in a 5% CO₂ incubator at 37°C.

The signal intensities were detected using Dual-Luciferase Reporter Assay System (Promega). The medium supernatant in an amount of 100 µL was removed, 50 µL of Glo reagent was added, and the cells were lysed for 10 minutes at room temperature. The luminescence of Firefly luciferase emitted from the cell lysate solution was detected with a plate reader to quantify the activation of the HGF-Erk-SRE pathway. Subsequently, 50 µL of Glo & Stop reagent solution was added, and after 10 minutes, the luminescence of the internal standard Renilla luciferase was detected to quantify the number of cells. The signal activity of each sample was quantified as SRE activity = (Firefly luciferase luminescence intensity)/(Renilla luciferase luminescence intensity). The relative signal activity values for the samples without addition of the evaluation compound were determined and used as relative receptor activity.

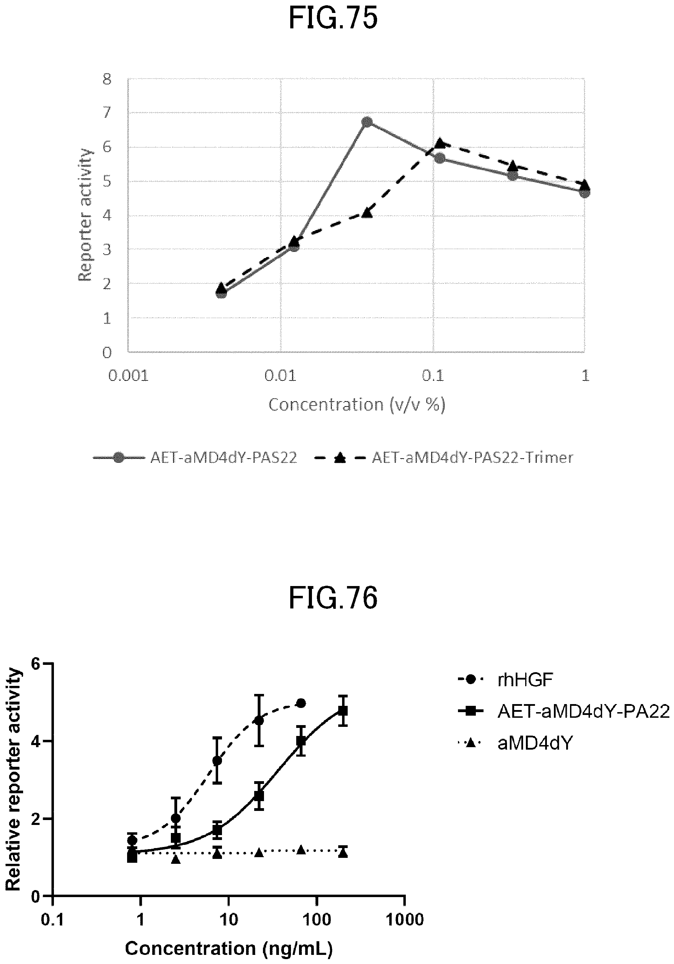

As shown in Fig. 75, it was clarified that the HGF-PAS trimer peptide (AET-aMD4dY-PAS22-Trimer) had a signal activation ability similar to the dimer peptide (AET-aMD4dY-PAS22).

### Example 43: Comparison of activity between HGF-PAS dimer peptide and monomer peptide

The Met activation ability of HGF-PAS dimer peptide and monomer peptide was compared by luciferase assay. To 25 µL of Opti-MEM medium (Thermo Fisher Scientific), 0.6 µL of Attractene Transfection Reagent (QIAGEN) was added, and the mixture was incubated at room temperature for 5 minutes. To the above mixed solution, a mixed solution of 25 µL of Opti-MEM and 1 µL of SRE reporter vector (QIAGEN) was added, and the mixture was incubated at room temperature for 20 minutes. This mixed solution was added to a 96-well plate, over which 40,000 cell/well HEK293E cells were seeded and incubated overnight in a 5% CO₂ incubator at 37°C. After transfection, all culture supernatant was removed, 100 µL of Opti-MEM medium (evaluation basal medium) containing 0.5% FBS (Thermo Fisher Scientific), 1% non-essential amino acid solution (Thermo Fisher Scientific), and penicillin-streptomycin (Nacalai Tesque) was added, and incubation was performed at 37°C for 4 hours, and thereby the cells were starved.

To the evaluation basal medium, 0 to 100 ng/mL HGF was added, or a AET-aMD4dY-PAS22 or aMD4dY monomer peptide (CRQFNRRTHEVWNLDC (SEQ ID NO: 2), synthesized by GenScript Japan) was added at a predetermined concentration, and added in an amount of 100 µL to the cells. The cells were stimulated by culturing overnight in a 5% CO₂ incubator at 37°C.

The signal intensities were detected using Dual-Luciferase Reporter Assay System (Promega). The medium supernatant in an amount of 100 µL was removed, 50 µL of Glo reagent was added, and the cells were lysed for 10 minutes at room temperature. The luminescence of Firefly luciferase emitted from the cell lysate solution was detected with a plate reader to quantify the activation of the HGF-Erk-SRE pathway. Subsequently, 50 µL of Glo & Stop reagent solution was added, and after 10 minutes, the luminescence of the internal standard Renilla luciferase was detected to quantify the number of cells. The signal activity of each sample was quantified as SRE activity=(Firefly luciferase luminescence intensity)/(Renilla luciferase luminescence intensity). The relative signal activity values for the samples without addition of the evaluation compound were determined and used as relative receptor activity.

As shown in Fig. 76, HGF and the dimer peptide (AET-aMD4dY-PAS22) showed signal activation ability, whereas the monomer peptide (aMD4dY) showed no signal activation ability. From this result, it was clarified that the peptide acquired the signal activation ability by dimerization.

### Example 44: Design of VEGFR_D2-PAS heterodimer peptide linker variant and its secretory expression in C. glutamicum

### (44-1) Outline of linker variant design

AET-VEGFR_D2-PAS16 has a structure in which a PAS linker composed of 16 amino acids is linked between the first VEGFR-binding sequence (P-3) and the second VEGFR-binding sequence (P-4). By changing the length of this PAS linker, it is expected that the VEGFR-binding activity will change. Therefore, a linker variant was designed in which the PAS linker portion was changed to a PAS repeat sequence of 49 amino acids or 100 amino acids.

In addition to the PAS linker, a GS linker, which is a repeating sequence of glycine and serin, is also known as a flexible linker. Therefore, the peptide sequence was designed to be a linker variant in which a VEGFR-binding cyclic peptide was bound to the N-terminus and C-terminus of the GS linker composed of a repeating sequence of 4 glycine residues and 1 serine residue (GGGGS), that is, a "first VEGFR-binding sequence (P-3)"-"GS linker"-"second VEGFR-binding sequence (P-4)". That is, the C-terminus of P-3 and the N-terminus of the PAS linker are linked by an amide bond to the N-terminus of P-4 and the C-terminus of the PAS linker, respectively, and the peptide chain can be expressed as a single polypeptide chain.

### (44-2) Preparation of VEGFR_D2-PAS heterodimer peptide linker variants

The prepared VEGFR_D2-PAS heterodimer peptide linker variants are as follows.
(Q) AET-VEGFR_D2-PAS49 AETAGPTWCEDDWYYCWLFGTAAPAAPAPAAPAAPAPAAPAAPAPAAPAAPAPA APAAPAPAAPAAPAPAVCWEDSWGGEVCWLFGT (SEQ ID NO: 175)
(R) AET-VEGFR_D2-PAS49-b 
AETAGPTWCEDDWYYCWLFGTSAPSPSSAPASASAPASPASASASASPASSPASA
SSASPAASSASPASSVCWEDSWGGEVCWLFGT (SEQ ID NO: 176)
(S) AET-VEGFR_D2-PAS100 
AETAGPTWCEDDWYYCWLFGTAAPAAPAPAAPAAPAPAAPAAPAPAAPAAPAPAAPAAPAPA
APAAPAPAAPAAPAPAAPAAPAPAAPAAPAPAAPAAPAPAAPAAPAPAAPAAPAPAAPAAPAPA
APAAPAPAAPAVCWEDSWGGEVCWLFGT (SEQ ID NO: 177)
(T) AET-VEGFR_D2-PAS 100-b 
AETAGPTWCEDDWYYCWLFGTSAPSPSSAPASASAPASPASASASASPASSPASA
SSASPAASSASPASSSASPSAPAAPSASAAPSASPASSSPAPASAPSPAAAASSPSPA
PSSASSAAVCWEDSWGGEVCWLFGT (SEQ ID NO: 178)
(U) AET-VEGFR D2-GS16

AETAGPTWCEDDWYYCWLFGTGGGGSGGGGSGGGGSGVCWEDSWGGEVCW
LFGT (SEQ ID NO: 179)

### (44-3) Expression of VEGFR_D2-PAS heterodimer peptide linker variants

Corynex (registered trademark) was used to examine the expression of VEGFR_D2-PAS heterodimer peptide linker variants. Hereinafter, examples of expression examinations using Corynex (registered trademark) will be described.

### (44-4) Construction of each secretory expression plasmid of AET-VEGFR_D2-PAS49, AET-VEGFR _D2-PAS49-b, AET-VEGFR_D2-PAS 100, AET-VEGFR_D2-PAS 100-b, and AET-VEGFR _D2-GS 16 using CspB signal sequence

As the VEGFR_D2-PAS heterodimer peptide linker variant, five types of amino acid sequences of the above (Q) AET-VEGFR_D2-PAS49 (which may be hereinafter referred to as "AET-VEGFR D2-PAS49", and may be simply referred to as "VEGFR D2-PAS49"), (R) AET-VEGFR_D2-PAS49-b (which may be hereinafter referred to as "AET-VEGFR_D2-PAS49-b", and may be simply referred to as "VEGFR_D2-PAS49-b"), (S) AET-VEGFR_D2-PAS100 (which may be hereinafter referred to as "AET-VEGFR D2-PAS100", and may be simply referred to as "VEGFR_D2-PAS100"), (T) AET-VEGFR_D2-PAS100-b (which may be hereinafter referred to as "AET-VEGFR_D2-PAS100-b", and may be simply referred to as "VEGFRD2-PAS100-b"), and (U) AET-VEGFR_D2-GS16 (which may be hereinafter referred to as "AET-VEGFR_D2-GS16", and may be simply referred to as "VEGFR D2-GS16") were designed, and the base sequences encoding these proteins were designed in consideration of the codon usage frequency of C. glutamicum. Furthermore, the following expression cassettes were designed to enable secretory expression by C. glutamicum.

VEGFR_D2-PAS49 was secretory-expressed as a fusion protein of VEGFR_D2-PAS49 and a signal peptide 30 amino acid residue of CspB derived from C. glutamicum ATCC 13869 strain (hereinafter referred to as "CspBss-VEGFR_D2-PAS49"). The base sequence and amino acid sequence encoding the designed CspBss-VEGFR_D2-PAS49 are set forth in SEQ ID NOs: 180 and 181, respectively.
Base sequence encoding CspBss-VEGFR_D2-PAS49 
atgtttaacaaccgtatccgcactgcagctctcgctggtgcaatcgcaatctccaccgcagcttccggcgtagctatcccagcat
tcgctgcagagaccgctggccctacctggtgtgaggacgattggtactactgttggctctttggcaccgccgcccctgctgctc
ccgcccccgccgcccctgccgcaccagctccagcagccccagctgcaccagcaccagccgctccagcagctccagcacc
agctgctccagcagcaccagccccagcagctccagccgcaccagctcctgcggtttgctgggaggattcttggggcggcga
agtttgctggctctttggcacctaa (SEQ ID NO: 180)
Amino acid sequence of CspBss-VEGFR_D2-PAS49

MFNNRIRTAALAGAIAISTAASGVAIPAFAAETAGPTWCEDDWYYCWLFGTAAP
AAPAPAAPAAPAPAAPAAPAPAAPAAPAPAAPAAPAPAAPAAPAPAVCWEDSWGG
EVCWLFGT (SEQ ID NO: 181)

VEGFR_D2-PAS49-b was secretory-expressed as a fusion protein of VEGFR_D2-PAS49-b and a signal peptide 30 amino acid residue of CspB derived from C. glutamicum ATCC 13869 strain (hereinafter referred to as "CspBss-VEGFR_D2-PAS49-b"). The base sequence and amino acid sequence encoding the designed CspBss-VEGFR_D2-PAS49-b are set forth in SEQ ID NOs: 182 and 183, respectively.
Base sequence encoding CspBss-VEGFR_D2-PAS49-b 
atgtttaacaaccgtatccgcactgcagctctcgctggtgcaatcgcaatctccaccgcagcttccggcgtagctatcccagcat
tcgctgcagagaccgcaggacccacttggtgcgaagatgactggtactattgctggctctttggcacctcagcacccagccca
tcctccgctccagcgtctgcgagcgctcctgcatccccagcctctgcctccgcatcggcatcaccggcttccagcccagcgtc
ggcatcgtcagcctctccggctgcctcctccgcttctcctgcgtcctccgtgtgttgggaggattcctggggtggtgaagtctgc
tggctgttcggcacctaa (SEQ ID NO: 182)
Amino acid sequence of CspBss-VEGFR_D2-PAS49-b

MFNNRIRTAALAGAIAISTAASGVAIPAFAAETAGPTWCEDDWYYCWLFGTSAP
SPSSAPASASAPASPASASASASPASSPASASSASPAASSASPASSVCWEDSWGGE
VCWLFGT (SEQ ID NO: 183)

VEGFR_D2-PAS100 was secretory-expressed as a fusion protein of VEGFR_D2-PAS100 and a signal peptide 30 amino acid residue of CspB derived from C. glutamicum ATCC 13869 strain (hereinafter referred to as "CspBss-VEGFR_D2-PAS100"). The base sequence and amino acid sequence encoding the designed CspBss-VEGFR_D2-PAS100 are set forth in SEQ ID NOs: 184 and 185, respectively.
Base sequence encoding CspBss-VEGFR_D2-PAS100 
atgtttaacaaccgtatccgcactgcagctctcgctggtgcaatcgcaatctccaccgcagcttccggcgtagctatcccagcat
tcgctgcagagaccgccggtccaacctggtgtgaggatgactggtactactgctggctctttggcaccgctgctcccgctgctc
ccgcccctgctgctccagctgccccagccccagctgccccagctgctccagcaccagccgctccagctgccccagcaccag
ctgcaccagcagcaccagcaccagcagctccagccgctccagccccagccgctccagcagcaccagccccagcagctcc
agctgcaccagctccagccgccccagcagcaccagctccagcagccccagcagctccagctccagccgcaccagctgctc
cagccccagccgcaccagcagccccagctccagctgctcctgctgtctgttgggaggactcttggggcggcgaagtttgttg
gttgtttggcacctaa (SEQ ID NO: 184)
Amino acid sequence of CspBss-VEGFR_D2-PAS100

MFNNRIRTAALAGAIAISTAASGVAIPAFAAETAGPTWCEDDWYYCWLFGTAAP
AAPAPAAPAAPAPAAPAAPAPAAPAAPAPAAPAAPAPAAPAAPAPAAPAAPAPAAP
AAPAPAAPAAPAPAAPAAPAPAAPAAPAPAAPAAPAPAAPAVCWEDSWGGEVCW
LFGT (SEQ ID NO: 185)

VEGFR_D2-PAS100-b was secretory-expressed as a fusion protein of VEGFR_D2-PAS100-b and a signal peptide 30 amino acid residue of CspB derived from C. glutamicum ATCC 13869 strain (hereinafter referred to as "CspBss-VEGFR_D2-PAS100-b"). The base sequence and amino acid sequence encoding the designed CspBss-VEGFR_D2-PAS100-b are set forth in SEQ ID NOs: 186 and 187, respectively.
Base sequence encoding CspBss-VEGFR_D2-PAS100-b 
atgtttaacaaccgtatccgcactgcagctctcgctggtgcaatcgcaatctccaccgcagcttccggcgtagctatcccagcat
tcgctgcagagaccgccggacctacctggtgtgaggatgactggtactattgctggctcttcggcacttccgccccttccccttc
cagcgcaccagcgagcgcttcggctccggcctccccagcgtccgcatctgcgtctgcatcccctgcgtcgtcacccgcttca
gcctcctcagcctcccctgcggcttcatccgcctcgcccgcatccagctccgcatcaccctctgcgccagcagctccatccgc
ttctgctgccccatcggcctctccggcttccagctctcccgcaccggcatccgctccgagcccagcagcagctgcatcctccc
catctccggcaccatcctcggcctctagcgcggctgtgtgctgggaagattcctggggtggtgaagtctgctggctgtttggca
cctaa (SEQ ID NO: 186)
Amino acid sequence of CspBss-VEGFR_D2-PAS100-b

MFNNRIRTAALAGAIAISTAASGVAIPAFAAETAGPTWCEDDWYYCWLFGTSAP
SPSSAPASASAPASPASASASASPASSPASASSASPAASSASPASSSASPSAPAAPSA
SAAPSASPASSSPAPASAPSPAAAASSPSPAPSSASSAAVCWEDSWGGEVCWLFG
T (SEQ ID NO: 187)

VEGFR_D2-GS16 was secretory-expressed as a fusion protein of VEGFR_D2-GS16 and a signal peptide 30 amino acid residue of CspB derived from C. glutamicum ATCC 13869 strain (hereinafter referred to as "CspBss-VEGFR_D2-GS16"). The base sequence and amino acid sequence encoding the designed CspBss-VEGFR_D2-GS16 are set forth in SEQ ID NOs: 188 and 189, respectively.
Base sequence encoding CspBss-VEGFR_D2-GS16 
atgtttaacaaccgtatccgcactgcagctctcgctggtgcaatcgcaatctccaccgcagcttccggcgtagctatcccagcat
tcgctgcagagaccgcgggtccgacctggtgtgaggatgactggtactactgttggttgttcggcaccggcggcggcggctc
cggcggcggcggctctggcggcggcggctctggcgtttgttgggaggactcttggggcggcgaagtttgctggctgtttggc
acctaa (SEQ ID NO: 188)
Amino acid sequence of CspBss-VEGFR_D2-GS16

MFNNRIRTAALAGAIAISTAASGVAIPAFAAETAGPTWCEDDWYYCWLFGTGGG

GSGGGGSGGGGSGVCWEDSWGGEVCWLFGT (SEQ ID NO: 189)

The promoter of the cspB gene derived from the C. glutamicum ATCC 13869 strain was linked upstream of the base sequences set forth in CspBss-VEGFR_D2-PAS49, CspBss-VEGFR_D2-PAS49-b, CspBss-VEGFR_D2-PAS100, CspBss-VEGFR_D2-PAS100-b, and CspBss-VEGFR_D2-GS16, and furthermore, an expression cassette of each VEGFR_D2-PAS heterodimer peptide linker variant having a KpnI site on the 5'-side and an BamHI site on the 3'-side was designed and totally synthesized. The totally synthesized DNA fragment (VEGFR_D2-PAS heterodimer peptide linker variant expression cassette) was inserted into the KpnI-BamHI site of pPK4 described in Japanese Patent Application Publication No. Hei 9-322774 to construct pPK4_CspBss-VEGFR_D2-PAS49, pPK4_CspBss-VEGFR_D2-PAS49-b, pPK4_CspBss-VEGFR D2-PAS100, pPK4_CspBss-VEGFR D2-PAS100-b, and pPK4_CspBss-VEGFR_D2-GS16, a secretory expression plasmid for each VEGFR_D2-PAS heterodimer peptide linker variant using the CspB signal sequence. As a result of determining the base sequence of the inserted fragment, it was confirmed that each VEGFR_D2-PAS heterodimer peptide linker variant expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (44-5) Secretory expression of each VEGFR_D2-PAS heterodimer peptide linker variant in C. glutamicum

The pPK4_CspBss-VEGFR_D2-PAS49, pPK4_CspBss-VEGFR_D2-PAS49-b, pPK4_CspBss-VEGFR D2-PAS100, pPK4_CspBss-VEGFR _D2-PAS100-b, and pPK4_CspBss-VEGFR_D2-GS16 constructed above were used to transform the C. glutamicum YDK0107 strain described in WO2016/171224 to obtain the YDK0107/pPK4_CspBss-VEGFR_D2-PAS49 strain, YDK0107/pPK4_CspBss-VEGFR_D2-PAS49-b strain, YDK0107/pPK4_CspB ss-VEGFR_D2-PAS 100 strain, YDK0107/pPK4_CspBss-VEGFR_D2-PAS100-b strain, and YDK0107/pPK4_CspBss-VEGFR_D2-GS16 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

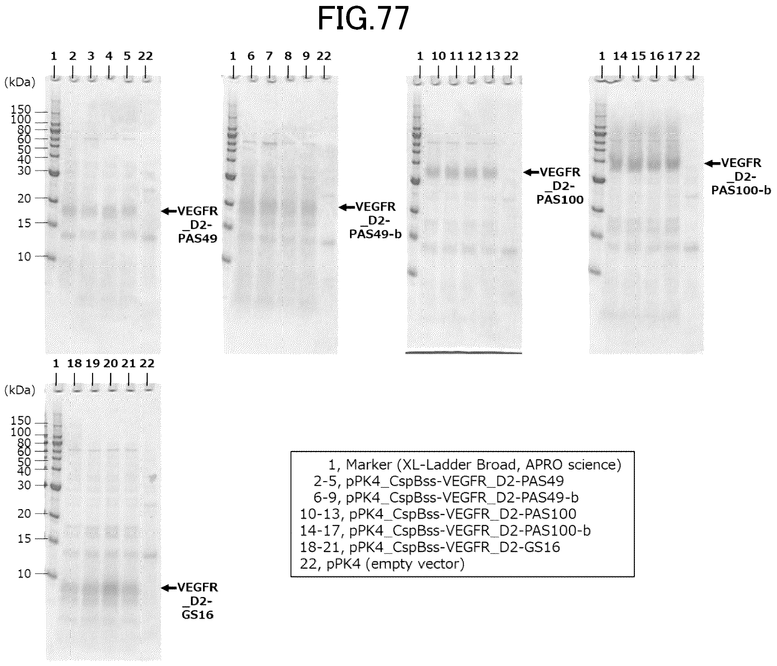

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be VEGFR_D2-PAS49 was detected in the culture supernatant of the YDK0107/pPK4_CspBss-VEGFR_D2-PAS49 strain (Fig. 77, lanes 2 to 5), a protein band presumed to be VEGFR_D2-PAS49-b was detected in the culture supernatant of the YDK0107/pPK4_CspBss-VEGFR_D2-PAS49-b strain (Fig. 77, lanes 6 to 9), a protein band presumed to be VEGFR_D2-PAS100 was detected in the culture supernatant of the YDK0107/pPK4_CspBss-VEGFR_D2-PAS100 strain (Fig. 77, lanes 10 to 13), a protein band presumed to be VEGFR_D2-PAS100-b was detected in the culture supernatant of the YDK0107/pPK4_CspBss-VEGFR_D2-PAS100-b strain (Fig. 77, lanes 14 to 17), and a protein band presumed to be VEGFR_D2-GS16 was detected in the culture supernatant of the YDK0107/pPK4_CspBss-VEGFR_D2-GS16 strain (Fig. 77, lanes 18 to 21).

### Example 45: Molecular weight analysis of VEGFR _D2-PAS heterodimer peptide linker variant

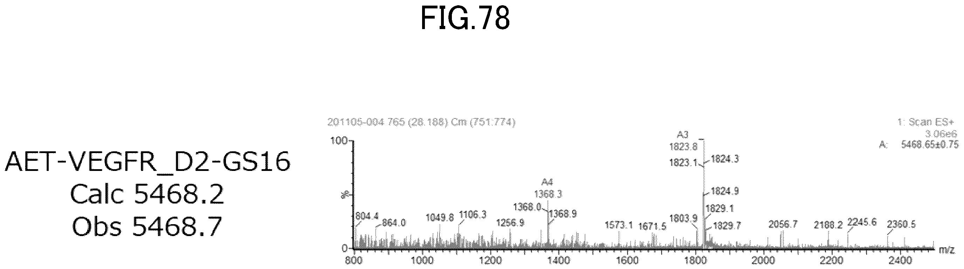

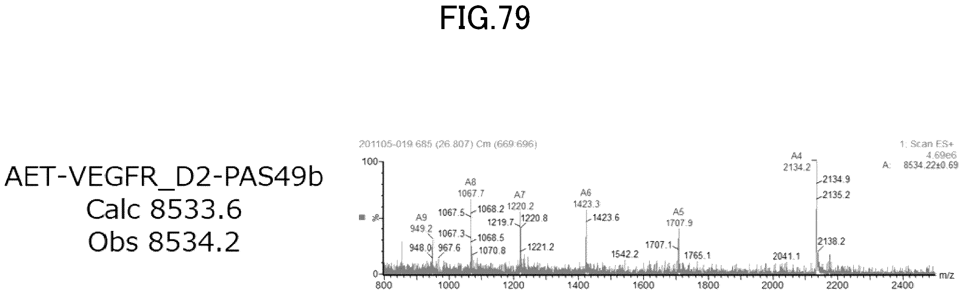

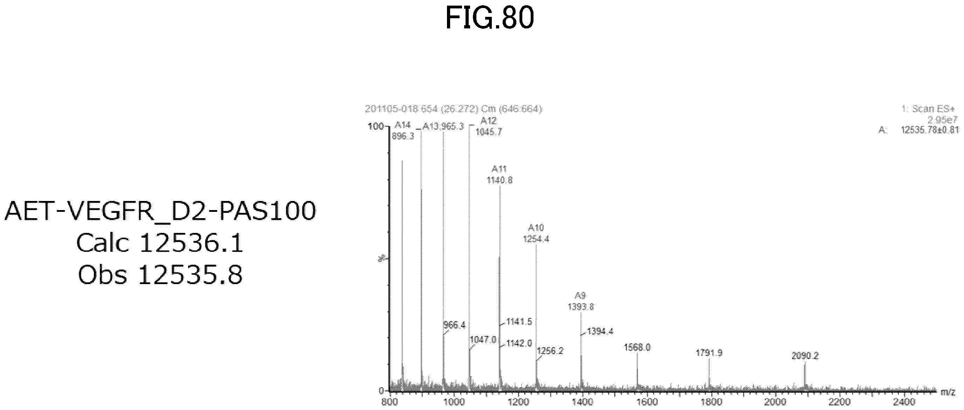

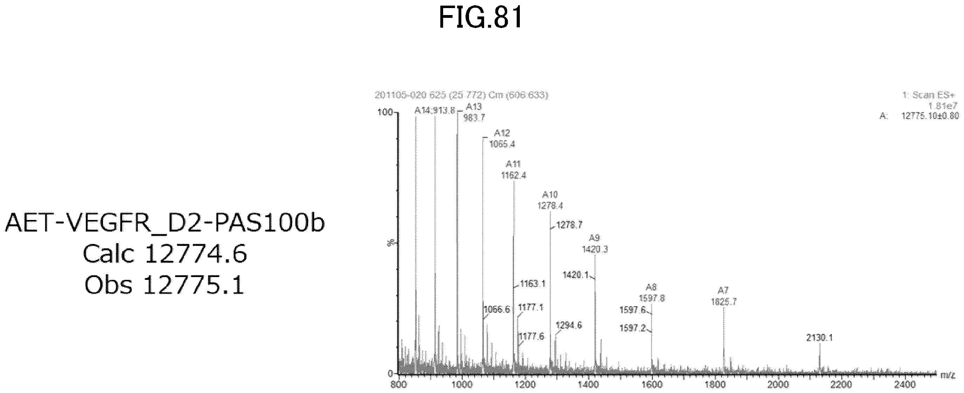

The cell filtrate with expressed VEGFR_D2-PAS heterodimer variant after culturing in Example 44 was subjected to cell filtration and then molecular weight measurement using LC-MS (Waters, ACQUITY UPLC/SQD2). The analysis conditions are shown in Table 5. As shown in Figs. 78 to 81, almost the same value as the theoretical value of the molecular weight of the oxidized dimer peptide forming two disulfide bonds in the molecule was obtained. From this, it was confirmed that a peptide having the correct amino acid sequence containing two disulfide bonds could be expressed.

### Example 46: Activity evaluation of VEGFR_D2-PAS heterodimer peptide linker variant

The VEGF inhibitory ability of the VEGFR_D2-PAS heterodimer peptide linker variant was evaluated by luciferase assay. 40,000 cell/well VEGFR/NFAT Reporter HEK293 cells (BPS Bioscience) were seeded and incubated overnight in a 5% CO₂ incubator at 37°C. The cells were incubated overnight in Assay medium (MEM medium (Nacalai Tesque), 1 × MEM Non-Essential Amino Acids Solution (Thermo Fisher Scientific), 1 mM Sodium Pyruvate (Nacalai Tesque), 1% Penicillin-Streptomycin (Sigma Aldrich), and 0.3% FBS (Thermo Fisher Scientific)) to starvation. To the Assay medium, 10 ng/mL human recombinant VEGF (R&D Systems) was added, and further, a culture supernatant containing VEGFR_D2-PAS16, VEGFR_D2-PAS49, or VEGFR_D2-PAS100 at a predetermined concentration was added, and 100 µL of the Assay medium was added to the cells. The cells were stimulated by culturing in a 5% CO₂ incubator at 37°C for 4 hours. Note that VEGFR_D2-PAS16 was obtained according to the method described in Example 27.

One-Glo Luciferase Assay System (Promega) was used to detect the signal intensity. Medium supernatant in an amount of 50 µL was removed, 50 µL of One-Glo reagent was added, and the cells were lysed for 10 minutes at room temperature. The luminescence emitted from the cell lysate solution was detected with a Nivo plate reader (Perkin Elmer) to quantify the activation of the VEGFR-NFAT pathway, and the relative signal activity value for the sample without addition of evaluation compound was determined and used as relative receptor activity.

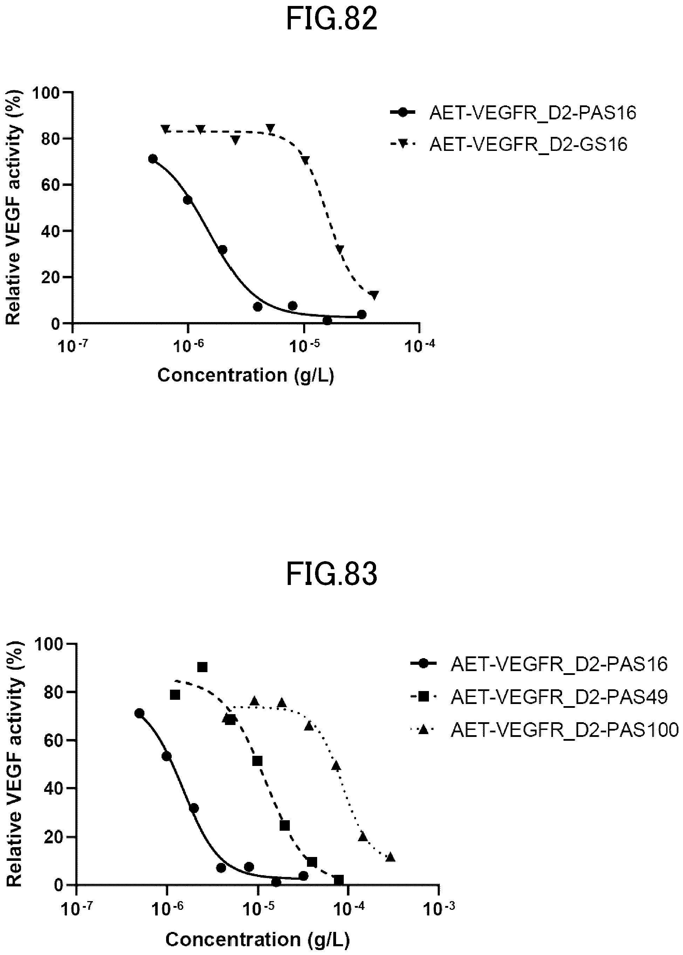

The evaluation results are shown in Figs. 82 and 83, and the results of calculating the IC50 value of each peptide dimer are shown in Table 8 below. VEGFR_D2-PAS16 showed higher inhibitory activity than VEGFR_D2-GS16. In addition, VEGFR_D2-PAS16 and VEGFR_D2-PAS49 showed higher inhibitory activity than VEGFR_D2-PAS100, so that it has been clarified that a dimer peptide can be developed that has higher inhibitory activity than a GS linker by using a PAS linker and that has high inhibitory activity by using a PAS linker of 49 amino acids or less.

**Table 8**

| Peptide | IC50 (g/L) |
|---|---|
| AET-VEGFR D2-PAS 16 | 1.5 × 10⁻⁶ |
| AET-VEGFR_D2-GS 16 | 1.6 × 10⁻⁵ |
| AET-VEGFR D2-PAS49 | 1.2 × 10⁻⁵ |
| AET-VEGFR D2-PAS 100 | 8.6 × 10⁻⁵ |

### Example 47: Design of TPO-PAS dimer peptide linker variant and its secretory expression in C. glutamicum

### (47-1) Outline of linker variant design

TPO2-PAS8, which has thrombopoietin-like activity, has a structure in which a PAS linker composed of 8 amino acids is linked between the first AF12285 peptide and the second AF12285 peptide. By changing the length of this PAS linker, it is expected that the thrombopoietin-like activity will change. Therefore, a linker variant was designed in which the PAS linker portion was changed to a PAS repeat sequence of 49 amino acids or 100 amino acids.

In addition to the PAS linker, a GS linker, which is a repeating sequence of glycine and serin, is also known as a flexible linker. Therefore, the peptide sequence was designed to be a linker variant in which EPO cyclic peptide was bound to the N-terminus and C-terminus of the GS linker composed of a repeating sequence of 4 glycine residues and 1 serine residue (GGGGS), that is, a "first AF12285 peptide"-"GS linker"-"first AF 12285 peptide".

### (47-2) Preparation of TPO-PAS dimer peptide linker variant

The prepared TPO-PAS dimer peptide linker variants are as follows.
(V) TPO2-PAS49 
GGCADGPTLREWISFCGGAAPAAPAPAAPAAPAPAAPAAPAPAAPAAPAPAAPAA
PAPAAPAAPAPAGGCADGPTLREWISFCGG (SEQ ID NO: 190)
(W)TPO2-PAS49-b 
GGCADGPTLREWISFCGGSAPSPSSAPASASAPASPASASASASPASSPASASSASP
AASSASPASSGGCADGPTLREWISFCGG (SEQ ID NO: 191)
(X)TPO2-PAS100 
GGCADGPTLREWISFCGGAAPAAPAPAAPAAPAPAAPAAPAPAAPAAPAPAAPAA
PAPAAPAAPAPAAPAAPAPAAPAAPAPAAPAAPAPAAPAAPAPAAPAAPAPAAPAA
PAPAAPAGGCADGPTLREWISFCGG (SEQ ID NO: 192)
(Y)TPO2-PAS100-b 
GGCADGPTLREWISFCGGSAPSPSSAPASASAPASPASASASASPASSPASASSASP
AASSASPASSSASPSAPAAPSASAAPSASPASSSPAPASAPSPAAAASSPSPAPSSAS
SAAGGCADGPTLREWISFCGG (SEQ ID NO: 193)
(Z)TP02-GS8 GGCADGPTLREWISFCGGGGGGSGGGGGCADGPTLREWISFCGG (SEQ ID NO: 194)

### (47-3) Expression of TPO-PAS dimer peptide linker variants

Corynex (registered trademark) was used to examine the expression of these TPO-PAS dimer peptide linker variants. Hereinafter, examples of expression examinations using Corynex (registered trademark) will be described.

### (47-4) Construction of each secretory expression plasmid of TPO2-PAS49, TPO2-PAS49-b, TPO2-PAS100, TPO2-PAS100-b, and TPO2-GS8 using CspA signal sequence.

As the TPO-PAS dimer peptide linker variant, five types of amino acid sequences of the above (V) TPO2-PAS49 (which may be hereinafter referred to as "TPO2-PAS49"), (W) TPO2-PAS49-b (which may be hereinafter referred to as "TPO2-PAS49-b"), (X) TPO2-PAS100 (which may be hereinafter referred to as "TPO2-PAS100"), (Y) TPO2-PAS100-b (which may be hereinafter referred to as "TPO2-PAS100-b"), and (Z) TPO2-GS8 (which may be hereinafter referred to as "TPO2-GS8") were designed, and the base sequences encoding these proteins were designed in consideration of the codon usage frequency of C. glutamicum. Furthermore, the following expression cassettes were designed to enable secretory expression by C. glutamicum.

TPO2-PAS49 was secretory-expressed as a fusion protein of TPO2-PAS49 and a signal peptide 25 amino acid residue of CspA derived from C. ammoniagenes ATCC 6872 strain (hereinafter referred to as "CspAss-TPO2-PAS49"). The base sequence and amino acid sequence encoding the designed CspAss-TPO2-PAS49 are set forth in SEQ ID NOs: 195 and 196, respectively.
Base sequence encoding CspAss-TPO2-PAS49 
atgaaacgcatgaaatcgctggctgcggcgctcaccgtcgctggggccatgctggccgcacctgtggcaacggcaggtggc
tgtgctgacggtcctacccttcgtgagtggatttctttttgcggcggtgctgctcctgctgcccctgctcctgctgctcctgcagca
ccagccccagcagctccagccgccccagctccagccgcaccagctgccccagcaccagccgctccagcagcaccagctc
cagcagccccagctgcaccagccccagcgggcggttgtgctgacggtcctacccttcgtgagtggatttccttctgtggtggct
aa (SEQ ID NO: 195)
Amino acid sequence of CspAss-TPO2-PAS49

MKRMKSLAAALTVAGAMLAAPVATAGGCADGPTLREWISFCGGAAPAAPAPAA
PAAPAPAAPAAPAPAAPAAPAPAAPAAPAPAAPAAPAPAGGCADGPTLREWISFCG
G (SEQ ID NO: 196)

TPO2-PAS49-b was secretory-expressed as a fusion protein of TPO2-PAS49-b and a signal peptide 25 amino acid residue of CspA derived from C. ammoniagenes ATCC 6872 strain (hereinafter referred to as "CspAss-TPO2-PAS49-b"). The base sequence and amino acid sequence encoding the designed CspAss-TPO2-PAS49-b are set forth in SEQ ID NOs: 197 and 198, respectively.
Base sequence encoding CspAss-TPO2-PAS49-b 
atgaaacgcatgaaatcgctggctgcggcgctcaccgtcgctggggccatgctggccgcacctgtggcaacggcaggcgg
gtgtgcagacggacccactctgcgcgaatggatctccttttgcggcggttccgctccgtcgccatcctctgctccggcatccgc
gtctgcgccagcatccccagccagcgcttcagcctcagcgtcccctgcctcctctccagcatccgccagcagcgcatctcctg
cggctagctcggcatcacccgcttcgtccggaggttgcgctgatggccctaccctccgtgagtggatttccttctgcggtggct
aa (SEQ ID NO: 197)
Amino acid sequence of CspAss-TPO2-PAS49-b

MKRMKSLAAALTVAGAMLAAPVATAGGCADGPTLREWISFCGGSAPSPSSAPAS
ASAPASPASASASASPASSPASASSASPAASSASPASSGGCADGPTLREWISFCGG

(SEQ ID NO: 198)

TPO2-PAS100 was secretory-expressed as a fusion protein of TPO2-PAS100 and a signal peptide 25 amino acid residue of CspA derived from C. ammoniagenes ATCC 6872 strain (hereinafter referred to as "CspAss-TPO2-PAS100"). The base sequence and amino acid sequence encoding the designed CspAss-TPO2-PAS100 are set forth in SEQ ID NOs: 199 and 200, respectively.
Base sequence encoding CspAss-TPO2-PAS100 
atgaaacgcatgaaatcgctggctgcggcgctcaccgtcgctggggccatgctggccgcacctgtggcaacggcaggcggt
tgtgctgacggtcctaccttgcgtgaatggatttctttctgtggtggtgcggctccggctgctccggctcctgcggctccggctg
caccagcaccagccgcaccagctgcaccagccccagctgcaccagccgctccagcaccagctgccccagctgcaccagct
ccagccgcaccagcagccccagctccagcagccccagcagctccagcaccagccgctccagctgccccagcaccagctg
ctccagcagctccagcaccagcagctccagctgctccagccccagccgccccagccgctccagccccagcagcaccagcc
gccccagctccagccgccccagcaggtggctgtgctgatggtcctacccttcgtgaatggatttccttttgtggcggttag
(SEQ ID NO: 199)
Amino acid sequence of CspAss-TPO2-PAS100

MKRMKSLAAALTVAGAMLAAPVATAGGCADGPTLREWISFCGGAAPAAPAPAA
PAAPAPAAPAAPAPAAPAAPAPAAPAAPAPAAPAAPAPAAPAAPAPAAPAAPAPAA
PAAPAPAAPAAPAPAAPAAPAPAAPAAPAPAAPAGGCADGPTLREWISFCGG
(SEQ ID NO: 200)

TPO2-PAS100-b was secretory-expressed as a fusion protein of TPO2-PAS100-b and a signal peptide 25 amino acid residue of CspA derived from C. ammoniagenes ATCC 6872 strain (hereinafter referred to as "CspAss-TPO2-PAS100-b"). The base sequence and amino acid sequence encoding the designed CspAss-TPO2-PAS100-b are set forth in SEQ ID NOs: 201 and 202, respectively.
Base sequence encoding CspAss-TPO2-PAS100-b 
atgaaacgcatgaaatcgctggctgcggcgctcaccgtcgctggggccatgctggccgcacctgtggcaacggcaggcgg
gtgtgcagacggtcccactctccgcgaatggatctcctttgcggcggaagcgcgccctccccgtcatctgcccctgcatctgc
gtccgcaccagcatccccagcctctgcgtctgcttctgcatcaccggcctcgtccccagcaagcgcatcgtccgcgtcaccag
ctgcgtcctcagcctcccctgcttcctcgtccgcttcgccttcggctcctgccgctccctctgcatccgctgccccatccgcctca
 
ccagccagctcctctccggcaccagcgtccgctccttcccctgcagctgcggcgagctctccaagcccggctccctcctccg
catccagcgctgccggtggatgcgcagatggcccgaccctgcgtgagtggattagcttctgcggcggttaa (SEQ ID
NO: 201)
Amino acid sequence of CspAss-TPO2-PAS100-b

MKRMKSLAAALTVAGAMLAAPVATAGGCADGPTLREWISFCGGSAPSPSSAPAS
ASAPASPASASASASPASSPASASSASPAASSASPASSSASPSAPAAPSASAAPSASP
ASSSPAPASAPSPAAAASSPSPAPSSASSAAGGCADGPTLREWISFCGG (SEQ ID
NO: 202)

TPO2-GS8 was secretory-expressed as a fusion protein of TPO2-GS8 and a signal peptide 25 amino acid residue of CspA derived from C. ammoniagenes ATCC 6872 strain (hereinafter referred to as "CspAss-TPO2-GS8"). The base sequence and amino acid sequence encoding the designed CspAss-TPO2-GS8 are set forth in SEQ ID NOs: 203 and 204, respectively.
Base sequence encoding CspAss-TPO2-GS8 
atgaaacgcatgaaatcgctggctgcggcgctcaccgtcgctggggccatgctggccgcacctgtggcaacggcaggtggc
tgtgcggatggtcctaccttgcgtgagtggatttctttctgtggcggtggtggtggttctggtggtggtggttgcgcaga
tggcccaaccctgcgcgaatggatctccttctgcggcggctaa (SEQ ID NO: 203)
Amino acid sequence of CspAss-TPO2-GS8

MKRMKSLAAALTVAGAMLAAPVATAGGCADGPTLREWISFCGGGGGGSGGGG
GCADGPTLREWISFCGG (SEQ ID NO: 204)

The promoter of the cspB gene derived from the C. glutamicum ATCC 13869 strain was linked upstream of the base sequences set forth in CspAss-TPO2-PAS49, CspAss-TPO2-PAS49-b, CspAss-TPO2-PAS100, CspAss-TPO2-PAS 100-b, and CspAss-TPO2-GS8, and furthermore, an expression cassette of each TPO-PAS dimer peptide linker variant having a KpnI site on the 5'-side and an BamHI site on the 3'-side was designed and totally synthesized. The totally synthesized DNA fragment (TPO-PAS dimer peptide linker variant expression cassette) was inserted into the KpnI-BamHI site of pPK4 described in Japanese Patent Application Publication No. Hei 9-322774 to construct pPK4_CspAss-TPO2-PAS49, pPK4_CspAss-TPO2-PAS49-b, pPK4_CspAss-TPO2-PAS100, pPK4_CspAss-TPO2-PAS100-b, and pPK4_CspAss-TPO2-GS8, a secretory expression plasmid for each TPO-PAS dimer peptide linker variant using the CspA signal sequence. As a result of determining the base sequence of the inserted fragment, it was confirmed that each TPO-PAS dimer peptide linker variant expression cassette as designed had been constructed. Base sequences were determined using BigDye (registered trademark) Terminator v3.1 Cycle Sequencing Kit (Applied Biosystems) and 3500xL Genetic Analyzer (Applied Biosystems).

### (47-5) Secretory expression of each TPO-PAS dimer peptide linker variant in C. glutamicum

The pPK4_CspAss-TPO2-PAS49, pPK4_CspAss-TPO2-PAS49-b, pPK4_CspAss-TPO2-PAS100, pPK4_CspAss-TPO2-PAS100-b, and pPK4_CspAss-TPO2-GS8 constructed above were used to transform the C. glutamicum YDK0107 strain described in WO2016/171224 to obtain the YDK0107/pPK4_CspAss-TPO2-PAS49 strain, YDK0107/pPK4_CspAss-TPO2-PAS49-b strain, YDK0107/pPK4_CspAss-TPO2-PAS100 strain, YDK0107/pPK4_CspAss-TPO2-PAS100-b strain, and YDK0107/pPK4_CspAss-TPO2-GS8 strain.

The obtained transformants were each cultured in MMTG liquid medium containing 25 mg/L kanamycin (glucose at 120 g, magnesium sulfate heptahydrate at 3 g, ammonium sulfate at 30 g, potassium dihydrogen phosphate at 1.5 g, iron heptahydrate at 0.03 g, manganese sulfate pentahydrate at 0.03 g, thiamine hydrochloride at 0.45 mg, biotin at 0.45 mg, DL-methionine at 0.15 g, soybean hydrochloric acid hydrolyzate (total nitrogen amount at 0.2 g), calcium carbonate at 50 g, adjusted to pH 7.0 with 1 L of water) at 30°C for 72 hours.

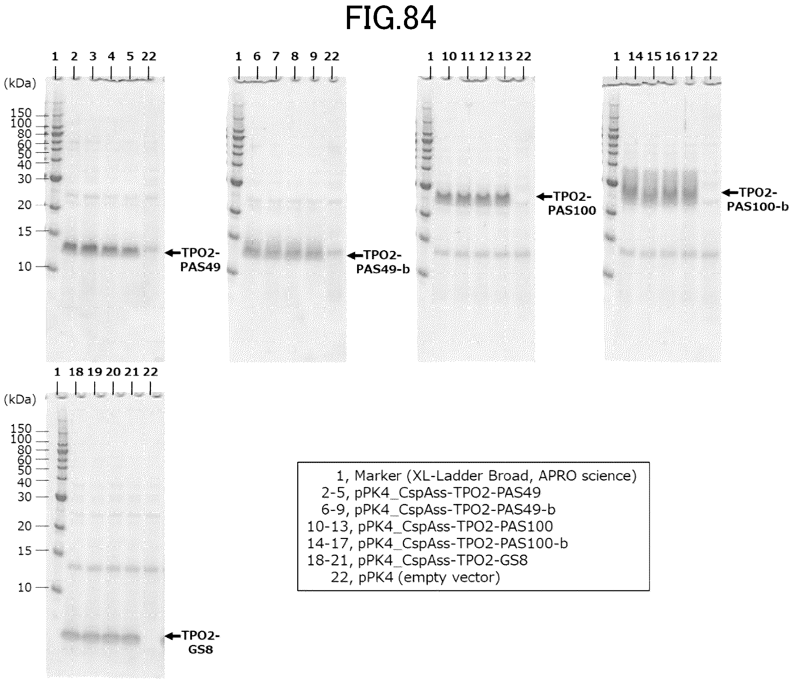

After completion of the culture, 6.5 µL of the culture supernatant obtained by centrifuging each culture solution was subjected to reduced SDS-PAGE using NuPAGE (registered trademark) 12% Bis-Tirs Gel (Thermo Fisher Scientific), and then stained with Quick-CBB (Wako). As a result, a protein band presumed to be TPO2-PAS49 was detected in the culture supernatant of the YDK0107/pPK4_CspAss-TPO2-PAS49 strain (Fig. 84, lanes 2 to 5), a protein band presumed to be TPO2-PAS49-b was detected in the culture supernatant of the YDK0107/pPK4_CspAss-TPO2-PAS49-b strain (Fig. 84, lanes 6 to 9), a protein band presumed to be TPO2-PAS100 was detected in the culture supernatant of the YDK0107/pPK4_CspAss-TPO2-PAS100 strain (Fig. 84, lanes 10 to 13), a protein band presumed to be TPO2-PAS49-b was detected in the culture supernatant of the YDK0107/pPK4_CspAss-TPO2-PAS49-b strain (Fig. 84, lanes 14 to 17), and a protein band presumed to be TPO2-GS8 was detected in the culture supernatant of the YDK0107/pPK4_CspAss-TPO2-GS8 strain (Fig. 84, lanes 18 to 21).

### Example 48: Molecular weight analysis of TPO-PAS dimer peptide linker variant

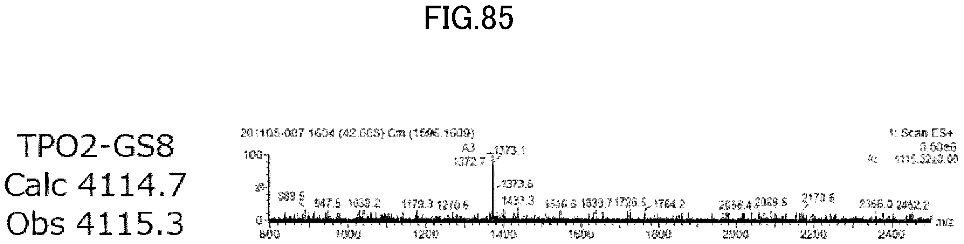

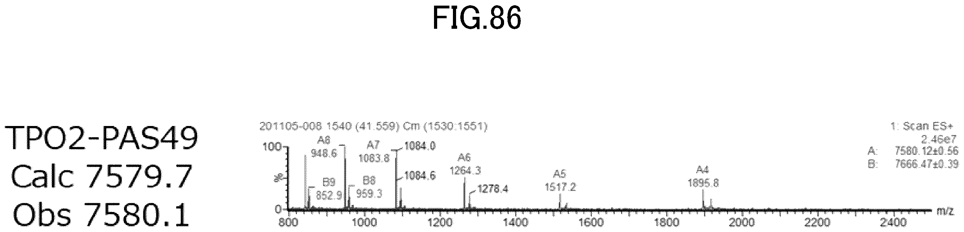

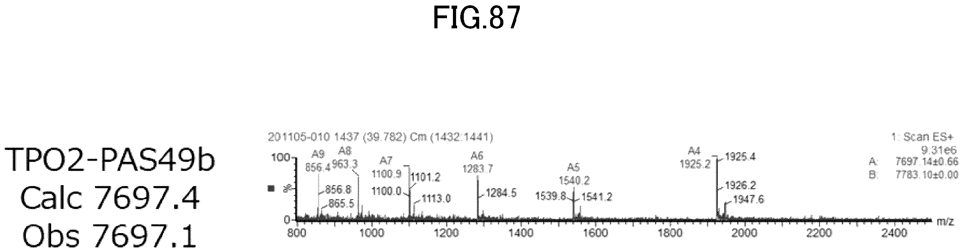

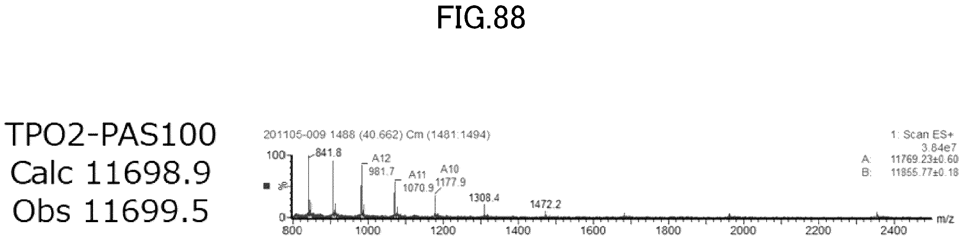

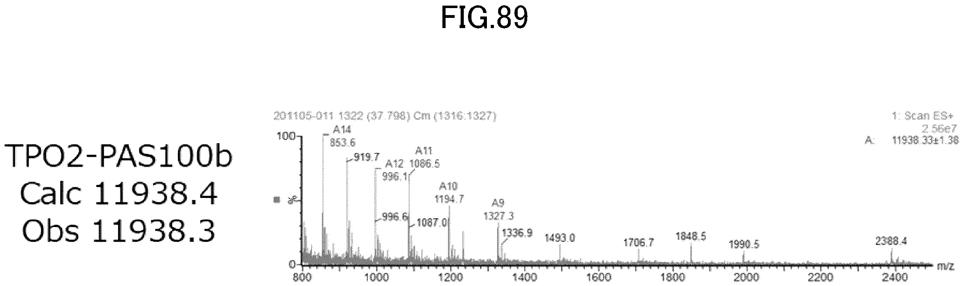

The cell filtrate of the TPO-PAS dimer peptide linker variant after culturing in Example 47 was subjected to molecular weight measurement using LC-MS (Waters, ACQUITY UPLC/SQD2). The analysis conditions are shown in Table 2. As shown in Figs. 85 to 89, almost the same value as the theoretical value of the molecular weight of each oxidized dimer peptide forming two disulfide bonds in the molecule was obtained. From this, it was confirmed that a peptide having the correct amino acid sequence containing two disulfide bonds could be expressed.

### Example 49: Activity evaluation of TPO-PAS dimer peptide linker variant

The thrombopoietin-like activity of the TPO-PAS dimer peptide variant was evaluated by quantification of CREB phosphorylation levels in HEL cells, a human leukemia-derived blood cell line. 200,000 HEL cells were cultured in RPMI 1640 medium without FBS (Thermo Fisher Scientific) for 24 hours and starved. The cells were stimulated for 20 minutes in RPMI 1640 medium supplemented with 100 ng/mL recombinant human TPO (rhTPO, Peprotech) or a culture supernatant containing each dimer peptide of TPO2-PAS8, TPO2-PAS49, TPO2-PAS100, or TPO2-GS8 diluted to a final concentration of 20-40 ng/mL. As a negative control, PBS was added, and stimulation was performed for 20 minutes (Mock group). After removing the medium and washing the cells once with PBS, the cells were lysed by adding 100 µL of Lysis buffer 17 (R&D Systems) supplemented with Protease inhibitor cocktail (Nacalai Tesque). The lysate solution was centrifuged at 14,000 G for 5 minutes to obtain the supernatant as lysate. Note that TPO2-PAS8 was obtained according to the method described in Example 23.

Phospho-CREB (Ser133) and Total CREB ELISA kit (RayBio) were used to quantify phosphorylated CREB (pCREB) levels in the lysates. Each lysate was diluted 25-fold with Assay Diluent, and 100 µL each was added to CREB Pan CREB Microplate, and shaken overnight at 4°C. After washing the Microplate 3 times with a washing buffer, 100 µL of Phospho Detection Antibody CREB (Ser133) was added, and the mixture was allowed to stand at 37°C for 30 minutes for an antigen-antibody reaction. Furthermore, after washing the Microplate 3 times with a washing buffer, 100 µL of 100 µL anti-rabbit antibody HRP conjugate was added, and the mixture was allowed to stand at 37°C for 30 minutes for an antigen-antibody reaction. After further washing the Microplate 3 times with a washing buffer, 100 µL of TMB solution was added, and a color reaction was carried out at room temperature for 30 minutes. Phosphorylation levels were quantified by adding 50 µL of Stop solution and measuring the absorbance at a wavelength of 450 nm on a microplate.

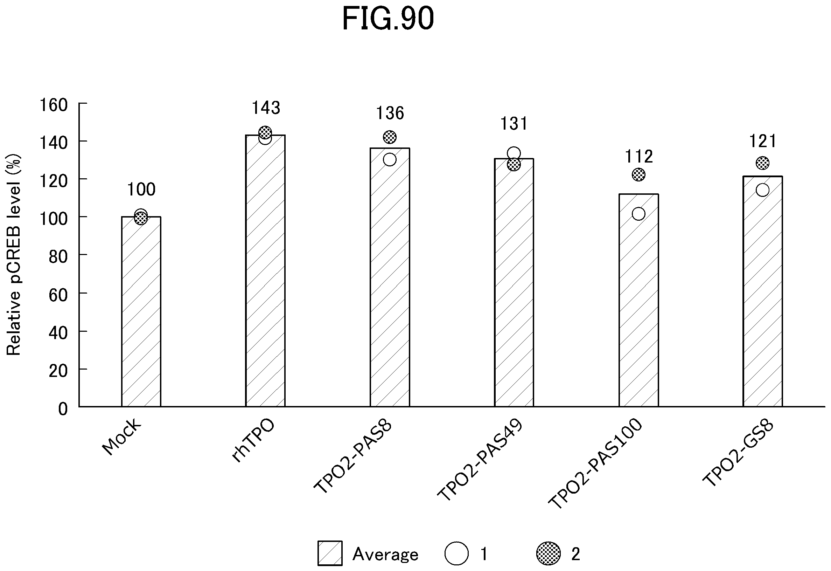

Fig. 90 shows the results. Higher pCREB levels were confirmed in all groups supplemented with dimer peptides compared to the Mock group. TPO2-PAS8 and TPO2-PAS49showed pCREB levels of 136% and 131%, respectively, close to the rhTPO stimulation group (143%), while TPO2-PAS100 had a low pCREB level of 112%. In addition, TPO2-GS8 was 121%, which was a lower pCREB level than TPO2-PAS8. From these, it was clarified that the use of the short-chain PAS linker provided higher agonist activity than the long-chain PAS linker and GS linker.

## Claims

1. A peptide molecule comprising:
(I) a first physiologically active peptide portion;
(II) a linker portion composed of 49 or less amino acid residues; and
(III) a second physiologically active peptide portion located on the opposite side of the linker portion from the first physiologically active peptide portion, wherein
the first physiologically active peptide portion and the second physiologically active peptide portion may be the same as or different from each other, and
at least 90% of an amino acid sequence of the linker portion is composed of amino acid residues selected from alanine (A), proline (P), and serine (S).

2. The peptide molecule according to claim 1, wherein the first physiologically active peptide portion constitutes a cyclic peptide and/or the second physiologically active peptide portion constitutes a cyclic peptide.

3. The peptide molecule according to claim 1 or 2, wherein the first physiologically active peptide portion constitutes a cyclic peptide having a cyclic structure formed by a disulfide bond and/or the second physiologically active peptide portion constitutes a cyclic peptide having a cyclic structure formed by a disulfide bond.

4. The peptide molecule according to any one of claims 1 to 3, wherein the first physiologically active peptide portion and the second physiologically active peptide portion each constitute a cyclic peptide that binds to a c-Met protein, and may be the same as or different from each other.

5. The peptide molecule according to claim 4, wherein the cyclic peptide has a cyclic structure formed by a disulfide bond and contains an amino acid sequence selected from the following (a) to (i):
(a) CYRQFNRRTHEVWNLDC (SEQ ID NO: 1);
(b) CRQFNRRTHEVWNLDC (SEQ ID NO: 2);
(c) CYWYYAWDQTYKAFPC (SEQ ID NO: 3);
(d) CWYYAWDQTYKAFPC (SEQ ID NO: 4);
(e) CYISWNEFNSPNWRFITC (SEQ ID NO: 5);
(f) CISWNEFNSPNWRFITC (SEQ ID NO: 6);
(g) a peptide in which one or two amino acid residues other than a cysteine residue are substituted, deleted, or added in any of the amino acid sequences (a) to (f), and which binds to a c-Met protein;
(h) a peptide which is composed of an amino acid sequence having 90% or more sequence identity with any of the amino acid sequences (a) to (f), but having cysteine residues at both ends, and which binds to a c-Met protein; and
(i) a peptide in which at least one amino acid other than the cysteine residues at both ends in any of the amino acid sequences (a) to (h) is modified (the amino acid modification is phosphorylation, methylation, acetylation, adenylylation, ADP-ribosylation, or glycosylation).

6. The peptide molecule according to any one of claims 1 to 3, wherein the first physiologically active peptide portion and the second physiologically active peptide portion each constitute a cyclic peptide that binds to an erythropoietin receptor, and may be the same as or different from each other.

7. The peptide molecule according to claim 6, wherein the cyclic peptide has a cyclic structure formed by a disulfide bond and contains an amino acid sequence selected from the following (j) to (n):
(j) GGLYACHMGPMTWVCQPLRG (SEQ ID NO: 65);
(k) CISWNEFNSPNWRFITC (SEQ ID NO: 66);
(l) a peptide in which one or two amino acid residues other than a cysteine residue are substituted, deleted, or added in the amino acid sequence of (j) or (k), and which binds to an erythropoietin receptor;
(m) a peptide which is composed of an amino acid sequence having 90% or more sequence identity with either the amino acid sequence of (j) or (k), but having a cysteine residue at the same position in (j) or (k), and which binds to an erythropoietin receptor; and
(n) a peptide in which at least one amino acid other than the cysteine residue in any of the amino acid sequences (j) to (m) is modified (the amino acid modification is phosphorylation, methylation, acetylation, adenylylation, ADP-ribosylation, or glycosylation).

8. The peptide molecule according to any one of claims 1 to 3, wherein the first physiologically active peptide portion and the second physiologically active peptide portion are each composed of a physiologically active peptide that binds to a thrombopoietin receptor, and may be the same as or different from each other.

9. The peptide molecule according to claim 8, wherein the physiologically active peptide contains an amino acid sequence selected from the following (o) to (u):
(o) IEGPTLRQWLAARA (SEQ ID NO: 67);
(p) GGCADGPTLREWISFCGG (SEQ ID NO: 68);
(q) GGCTLREWLHGGFCGG (SEQ ID NO: 69);
(r) LAIEGPTLRQWLHGNGRDT (SEQ ID NO: 70);
(s) a peptide in which one or two amino acid residues other than a cysteine residue are substituted, deleted, or added in any of the amino acid sequences (o) to (r), and which binds to a thrombopoietin receptor;
(t) a peptide which is composed of an amino acid sequence having 90% or more sequence identity with any of the amino acid sequences (o) to (r), but having a cysteine residue at the same position in (p) or (q) when based on (p) or (q), and which binds to a thrombopoietin receptor; and
(u) a peptide in which at least one amino acid other than the cysteine residue in any of the amino acid sequences (o) to (t) is modified (the amino acid modification is phosphorylation, methylation, acetylation, adenylylation, ADP-ribosylation, or glycosylation).

10. The peptide molecule according to any one of claims 1 to 9, further comprising: a functional modification portion at a position not adjacent to the linker portion (II).

11. The peptide molecule according to any one of claims 1 to 10, wherein
the linker portion (II) is a first linker portion, and
the peptide molecule further comprising:
a second linker portion on the opposite side of the second physiologically active peptide portion from the first linker portion; and
a third physiologically active peptide portion located on the opposite side of the second linker portion from the second physiologically active peptide portion, wherein
the third physiologically active peptide portion may be the same as or different from the first physiologically active peptide portion and/or second physiologically active peptide portion, and
the second linker portion is composed of 49 or less amino acid residues, and at least 90% of an amino acid sequence thereof is composed of amino acid residues selected from alanine (A), proline (P), and serine (S), which may be the same as or different from the first linker portion.

12. The peptide molecule according to any one of claims 1 to 11, wherein
the linker portion (II) repeatedly contains two or more amino acid sequences selected from the group consisting of AP, AAP, AS, ASP, and ASS, and if a second linker portion is present, the second linker portion repeatedly contains two or more amino acid sequences selected from the group consisting of AP, AAP, AS, ASP, and ASS.

13. A method for producing a peptide molecule, comprising the steps of:
producing the peptide molecule by expressing a polynucleotide in a host cell containing the polynucleotide encoding the peptide molecule or by expressing a polynucleotide encoding the peptide molecule in a cell-free expression system, wherein
the peptide molecule contains (I) a first physiologically active peptide portion, (II) a linker portion composed of an amino acid residue, and (III) a second physiologically active peptide portion and/or an additional amino acid sequence composed of at least one amino acid residue, located on the opposite side of the linker portion from the first physiologically active peptide portion,
the first physiologically active peptide portion and the second physiologically active peptide portion each have a molecular weight of 10,000 or less and are composed of 50 or less amino acid residues, and may be the same as or different from each other, and
the additional amino acid sequence has a molecular weight of 10,000 or less and is composed of 50 or less amino acid residues.

14. The production method according to claim 13, wherein the first physiologically active peptide portion constitutes a cyclic peptide and/or the second physiologically active peptide portion constitutes a cyclic peptide.

15. A method for producing a peptide molecule, comprising the steps of:
producing the peptide molecule by expressing a polynucleotide in a host cell containing the polynucleotide encoding the peptide molecule or by expressing a polynucleotide encoding the peptide molecule in a cell-free expression system, wherein
the peptide molecule contains (I) a first physiologically active peptide portion constituting a cyclic peptide, (II) a linker portion composed of an amino acid residue, and (III) a second physiologically active peptide portion and/or an additional amino acid sequence composed of at least one amino acid residue, located on the opposite side of the linker portion from the first physiologically active peptide portion,
the first physiologically active peptide portion and the second physiologically active peptide portion may be the same as or different from each other, and
the additional amino acid sequence has a molecular weight of 10,000 or less and is composed of 50 or less amino acid residues.

16. The production method according to claim 15, wherein the peptide molecule contains the second physiologically active peptide portion, and the second physiologically active peptide portion constitutes a cyclic peptide.

17. The production method according to any one of claims 13 to 16, wherein the linker portion has a molecular weight of 10,000 or less and is composed of 50 or less amino acid residues.

18. The production method according to any one of claims 13 to 16, wherein the linker portion has a molecular weight of 10,000 or less and is composed of 49 or less amino acid residues, and at least 90% of an amino acid sequence thereof is composed of amino acid residues selected from alanine (A), proline (P), and serine (S).

19. The production method according to any one of claims 13 to 18, wherein the linker portion repeatedly contains two or more amino acid sequences selected from the group consisting of AP, AAP, AS, ASP, and ASS.


SEQUENCE LISTING

<110>  Ajinomoto Co., Inc.

<120>  Method for producing peptide having physiological activity and
       peptide comprising short chain linker

<130>  OP20095

<150>  JP 2019-221040
<151>  2019-12-06

<160>  204

<170>  PatentIn version 3.5

<210>  1
<211>  17
<212>  PRT
<213>  Artificial Sequence

<220>
<223>  Met binding peptide


<220>
<221>  DISULFID
<222>  (1)..(17)
<223>  Disulfid bridge between Cys-1 and Cys-17

<400>  1

Cys Tyr Arg Gln Phe Asn Arg Arg Thr His Glu Val Trp Asn Leu Asp
1               5                   10                  15


Cys




<210>  2
<211>  16
<212>  PRT
<213>  Artificial Sequence

<220>
<223>  Met binding peptide


<220>
<221>  DISULFID
<222>  (1)..(16)
<223>  Disulfid bridge between Cys-1 and Cys-16

<400>  2

Cys Arg Gln Phe Asn Arg Arg Thr His Glu Val Trp Asn Leu Asp Cys
1               5                   10                  15


<210>  3
<211>  16
<212>  PRT

<213>  Artificial Sequence

<220>
<223>  Met binding peptide


<220>
<221>  DISULFID
<222>  (1)..(16)
<223>  Disulfid bridge between Cys-1 and Cys-16

<400>  3

Cys Tyr Trp Tyr Tyr Ala Trp Asp Gln Thr Tyr Lys Ala Phe Pro Cys
1               5                   10                  15


<210>  4
<211>  15
<212>  PRT
<213>  Artificial Sequence

<220>
<223>  Met binding peptide


<220>
<221>  DISULFID
<222>  (1)..(15)
<223>  Disulfid bridge between Cys-1 and Cys-15

<400>  4

Cys Trp Tyr Tyr Ala Trp Asp Gln Thr Tyr Lys Ala Phe Pro Cys
1               5                   10                  15


<210>  5
<211>  18
<212>  PRT
<213>  Artificial Sequence

<220>
<223>  Met binding peptide


<220>
<221>  DISULFID
<222>  (1)..(18)
<223>  Disulfid bridge between Cys-1 and Cys-18

<400>  5

Cys Tyr Ile Ser Trp Asn Glu Phe Asn Ser Pro Asn Trp Arg Phe Ile
1               5                   10                  15


Thr Cys



<210>  6
<211>  17

<212>  PRT
<213>  Artificial Sequence

<220>
<223>  Met binding peptide


<220>
<221>  DISULFID
<222>  (1)..(17)
<223>  Disulfid bridge between Cys-1 and Cys-17

<400>  6

Cys Ile Ser Trp Asn Glu Phe Asn Ser Pro Asn Trp Arg Phe Ile Thr
1               5                   10                  15


Cys




<210>  7
<211>  20
<212>  PRT
<213>  Artificial Sequence

<220>
<223>  PAS Linker

<400>  7

Ala Ala Pro Ala Ala Pro Ala Pro Ala Ala Pro Ala Ala Pro Ala Pro
1               5                   10                  15


Ala Ala Pro Ala
            20


<210>  8
<211>  22
<212>  PRT
<213>  Artificial Sequence

<220>
<223>  PAS Linker

<400>  8

Ala Ala Pro Ala Ala Pro Ala Pro Ala Ala Pro Ala Ala Pro Ala Pro
1               5                   10                  15


Ala Ala Pro Ala Ala Pro
            20


<210>  9
<211>  14
<212>  PRT
<213>  Artificial Sequence

<220>
<223>  PAS Linker

<400>  9

Ala Ala Pro Ala Ala Pro Ala Pro Ala Ala Pro Ala Ala Pro
1               5                   10


<210>  10
<211>  20
<212>  PRT
<213>  Artificial Sequence

<220>
<223>  PAS Linker

<400>  10

Ala Ala Pro Ala Ala Ala Pro Ala Pro Ala Ala Pro Ala Ala Pro Ala
1               5                   10                  15


Pro Ala Ala Pro
            20


<210>  11
<211>  20
<212>  PRT
<213>  Artificial Sequence

<220>
<223>  PAS Linker

<400>  11

Ala Ala Ala Pro Ala Ala Ala Pro Ala Ala Ala Pro Ala Ala Ala Pro
1               5                   10                  15


Ala Ala Ala Pro
            20


<210>  12
<211>  24
<212>  PRT
<213>  Artificial Sequence

<220>
<223>  PAS Linker

<400>  12

Ala Ala Pro Ala Ala Pro Ala Ala Pro Ala Ala Pro Ala Ala Pro Ala
1               5                   10                  15


Ala Pro Ala Ala Pro Ala Ala Pro
            20

<210>  13
<211>  24
<212>  PRT
<213>  Artificial Sequence

<220>
<223>  PAS Linker

<400>  13

Ala Pro Ala Ala Ala Pro Ala Pro Ala Ala Ala Pro Ala Pro Ala Ala
1               5                   10                  15

Ala Pro Ala Pro Ala Ala Ala Pro
            20

<210>  14
<211>  24
<212>  PRT
<213>  Artificial Sequence

<220>
<223>  PAS Linker

<400>  14

Ala Ala Ala Pro Ala Ala Pro Ala Ala Pro Pro Ala Ala Ala Ala Pro
1               5                   10                  15

Ala Ala Pro Ala Ala Pro Pro Ala
            20

<210>  15
<211>  20
<212>  PRT
<213>  Artificial Sequence

<220>
<223>  PAS Linker

<400>  15

Ala Pro Ala Pro Ala Pro Ala Pro Ala Pro Ala Pro Ala Pro Ala Pro
1               5                   10                  15

Ala Pro Ala Pro
            20

<210>  16
<211>  20
<212>  PRT
<213>  Artificial Sequence

<220>
<223>  PAS Linker

<400>  16

Ala Ser Pro Ala Ala Pro Ala Pro Ala Ser Pro Ala Ala Pro Ala Pro
1               5                   10                  15

Ser Ala Pro Ala
            20

<210>  17
<211>  20
<212>  PRT
<213>  Artificial Sequence

<220>
<223>  PAS Linker

<400>  17

Ala Ala Pro Ala Ser Pro Ala Pro Ala Ala Pro Ser Ala Pro Ala Pro
1               5                   10                  15

Ala Ala Pro Ser
            20

<210>  18
<211>  20
<212>  PRT
<213>  Artificial Sequence

<220>
<223>  PAS Linker

<400>  18

Ala Pro Ser Ser Pro Ser Pro Ser Ala Pro Ser Ser Pro Ser Pro Ala
1               5                   10                  15

Ser Pro Ser Ser
            20

<210>  19
<211>  20
<212>  PRT
<213>  Artificial Sequence

<220>
<223>  PAS Linker

<400>  19

Ser Ala Pro Ser Ser Pro Ser Pro Ser Ala Pro Ser Ser Pro Ser Pro
1               5                   10                  15

Ala Ser Pro Ser
            20

<210>  20
<211>  20
<212>  PRT
<213>  Artificial Sequence

<220>
<223>  PAS Linker

<400>  20

Ser Ser Pro Ser Ala Pro Ser Pro Ser Ser Pro Ala Ser Pro Ser Pro
1               5                   10                  15


Ser Ser Pro Ala
            20


<210>  21
<211>  24
<212>  PRT
<213>  Artificial Sequence

<220>
<223>  PAS Linker

<400>  21

Ala Ala Ser Pro Ala Ala Pro Ser Ala Pro Pro Ala Ala Ala Ser Pro
1               5                   10                  15


Ala Ala Pro Ser Ala Pro Pro Ala
            20


<210>  22
<211>  20
<212>  PRT
<213>  Artificial Sequence

<220>
<223>  PAS Linker

<400>  22

Ala Ser Ala Ala Ala Pro Ala Ala Ala Ser Ala Ala Ala Ser Ala Pro
1               5                   10                  15


Ser Ala Ala Ala
            20


<210>  23
<211>  20
<212>  PRT
<213>  Artificial Sequence

<220>
<223>  PAS Linker

<400>  23

Ala Pro Ala Ala Pro Ala Pro Ala Ala Pro Ala Ala Pro Ala Pro Ala
1               5                   10                  15

Ala Pro Ala Ala
            20

<210>  24
<211>  20
<212>  PRT
<213>  Artificial Sequence

<220>
<223>  PAS Linker

<400>  24

Pro Ala Ala Pro Ala Pro Ala Ala Pro Ala Ala Pro Ala Pro Ala Ala
1               5                   10                  15

Pro Ala Ala Ala
            20

<210>  25
<211>  20
<212>  PRT
<213>  Artificial Sequence

<220>
<223>  PAS Linker

<400>  25

Ala Ala Pro Ala Pro Ala Ala Pro Ala Ala Pro Ala Pro Ala Ala Pro
1               5                   10                  15

Ala Ala Ala Pro
            20

<210>  26
<211>  20
<212>  PRT
<213>  Artificial Sequence

<220>
<223>  PAS Linker

<400>  26

Ala Pro Ala Pro Ala Ala Pro Ala Ala Pro Ala Pro Ala Ala Pro Ala
1               5                   10                  15

Ala Ala Pro Ala
            20

<210>  27
<211>  20
<212>  PRT
<213>  Artificial Sequence

<220>
<223>  PAS Linker

<400>  27

Pro Ala Pro Ala Ala Pro Ala Ala Pro Ala Pro Ala Ala Pro Ala Ala
1               5                   10                  15

Ala Pro Ala Ala
            20

<210>  28
<211>  20
<212>  PRT
<213>  Artificial Sequence

<220>
<223>  PAS Linker

<400>  28

Ala Pro Ala Ala Pro Ala Ala Pro Ala Pro Ala Ala Pro Ala Ala Ala
1               5                   10                  15

Pro Ala Ala Pro
            20

<210>  29
<211>  20
<212>  PRT
<213>  Artificial Sequence

<220>
<223>  PAS Linker

<400>  29

Pro Ala Ala Pro Ala Ala Pro Ala Pro Ala Ala Pro Ala Ala Ala Pro
1               5                   10                  15

Ala Ala Pro Ala
            20

<210>  30
<211>  20
<212>  PRT
<213>  Artificial Sequence

<220>
<223>  PAS Linker

<400>  30

Ala Ala Pro Ala Ala Pro Ala Pro Ala Ala Pro Ala Ala Ala Pro Ala
1               5                   10                  15

Ala Pro Ala Pro
            20

<210>  31
<211>  20
<212>  PRT
<213>  Artificial Sequence

<220>
<223>  PAS Linker

<400>  31

Ala Pro Ala Ala Pro Ala Pro Ala Ala Pro Ala Ala Ala Pro Ala Ala
1               5                   10                  15

Pro Ala Pro Ala
            20

<210>  32
<211>  20
<212>  PRT
<213>  Artificial Sequence

<220>
<223>  PAS Linker

<400>  32

Pro Ala Ala Pro Ala Pro Ala Ala Pro Ala Ala Ala Pro Ala Ala Pro
1               5                   10                  15

Ala Pro Ala Ala
            20

<210>  33
<211>  34
<212>  DNA
<213>  Artificial Sequence

<220>
<223>  Primer

<400>  33
tcagaggaat gtcgactgct cgcaaccggc ccgt                                    34

<210>  34
<211>  33
<212>  DNA
<213>  Artificial Sequence

<220>

<223>  Primer

<400>  34
cctacattca gcgcaggttc ccctagagca cgt                                    33


<210>  35
<211>  19
<212>  DNA
<213>  Artificial Sequence

<220>
<223>  Primer

<400>  35
tgcgctgaat gtagggaag                                                    19


<210>  36
<211>  34
<212>  DNA
<213>  Artificial Sequence

<220>
<223>  Primer

<400>  36
atcgctgcag gtcgactcta gaggatcccc ggaa                                   34


<210>  37
<211>  36
<212>  DNA
<213>  Artificial Sequence

<220>
<223>  Primer

<400>  37
cttcccatac aatcgataga ttgtcgcacc tgattg                                 36


<210>  38
<211>  33
<212>  DNA
<213>  Artificial Sequence

<220>
<223>  Primer

<400>  38
cctcaggctg gtgttgggcg agctcgaatt cgt                                    33


<210>  39
<211>  18
<212>  DNA
<213>  Artificial Sequence

<220>
<223>  Primer

<400>  39

aacaccagcc tgaggcaa                                                    18


<210>  40
<211>  37
<212>  DNA
<213>  Artificial Sequence

<220>
<223>  Primer

<400>  40
gatcttcggg actagttact ttcaaatgct cagcatg                               37


<210>  41
<211>  58
<212>  PRT
<213>  Artificial Sequence

<220>
<223>  FBP-PAS



<220>
<221>  DISULFID
<222>  (4)..(15)
<223>  Disulfid bridge between Cys-4 and Cys-15

<220>
<221>  DISULFID
<222>  (44)..(55)
<223>  Disulfid bridge between Cys-44 and Cys-55

<400>  41

Arg Pro Gly Cys Gly Pro Arg Lys Pro Arg Thr Pro Lys Lys Cys Gly
1               5                   10                  15


Ser His Gly Ala Ala Pro Ala Ala Pro Ala Pro Ala Ala Pro Ala Ala
            20                  25                  30


Pro Ala Pro Ala Ala Pro Ala Gly Arg Pro Gly Cys Gly Pro Arg Lys
        35                  40                  45


Pro Arg Thr Pro Lys Lys Cys Gly Ser His
    50                  55


<210>  42
<211>  294
<212>  DNA
<213>  Artificial Sequence

<220>
<223>  TorAss-FBP-PAS

<400>  42
atgaacaata acgatctctt tcaggcatca cgtcggcgtt ttctggcaca actcggcggc      60

ttaaccgtcg ccgggatgct ggggccgtca ttgttaacgc cgcgacgtgc gactgcgcgt 120

cctggttgtg gtcctcgcaa gccccgcacc cctaagaaat gtggttctca cggcgccgct 180

cctgccgcac ccgcacccgc tgctccagca gccccagccc cagctgcacc agcaggtcgc 240

ccaggctgtg gtccccgcaa accccgcacc cctaagaaat gtggctctca ctaa 294

<210> 43
<211> 97
<212> PRT
<213> Artificial Sequence

<220>
<223> TorAss-FBP-PAS

<220>
<221> DISULFID
<222> (43)..(54)
<223> Disulfid bridge between Cys-43 and Cys-54

<220>
<221> DISULFID
<222> (83)..(94)
<223> Disulfid bridge between Cys-83 and Cys-94

<400> 43

Met Asn Asn Asn Asp Leu Phe Gln Ala Ser Arg Arg Arg Phe Leu Ala
1               5                   10                  15

Gln Leu Gly Gly Leu Thr Val Ala Gly Met Leu Gly Pro Ser Leu Leu
            20                  25                  30

Thr Pro Arg Arg Ala Thr Ala Arg Pro Gly Cys Gly Pro Arg Lys Pro
        35                  40                  45

Arg Thr Pro Lys Lys Cys Gly Ser His Gly Ala Ala Pro Ala Ala Pro
    50                  55                  60

Ala Pro Ala Ala Pro Ala Ala Pro Ala Pro Ala Ala Pro Ala Gly Arg
65                  70                  75                  80

Pro Gly Cys Gly Pro Arg Lys Pro Arg Thr Pro Lys Lys Cys Gly Ser
                85                  90                  95

His

<210> 44
<211> 58
<212> PRT
<213> Artificial Sequence

<220>
<223>  PAS-aMD4-PEG11

<220>
<221>  DISULFID
<222>  (1)..(17)
<223>  Disulfid bridge between Cys-1 and Cys-17

<220>
<221>  DISULFID
<222>  (42)..(58)
<223>  Disulfid bridge between Cys-42 and Cys-58

<400>  44

Cys Tyr Arg Gln Phe Asn Arg Arg Thr His Glu Val Trp Asn Leu Asp
1               5                   10                  15

Cys Gly Ala Ala Pro Ala Ala Pro Ala Pro Ala Ala Pro Ala Ala Pro
            20                  25                  30

Ala Pro Ala Ala Pro Ala Ala Pro Gly Cys Tyr Arg Gln Phe Asn Arg
        35                  40                  45

Arg Thr His Glu Val Trp Asn Leu Asp Cys
    50                  55

<210>  45
<211>  56
<212>  PRT
<213>  Artificial Sequence

<220>
<223>  PAS-aMD4dY-PEG11

<220>
<221>  DISULFID
<222>  (1)..(16)
<223>  Disulfid bridge between Cys-1 and Cys-16

<220>
<221>  DISULFID
<222>  (41)..(56)
<223>  Disulfid bridge between Cys-41 and Cys-56

<400>  45

Cys Arg Gln Phe Asn Arg Arg Thr His Glu Val Trp Asn Leu Asp Cys
1               5                   10                  15

Gly Ala Ala Pro Ala Ala Pro Ala Pro Ala Ala Pro Ala Ala Pro Ala
            20                  25                  30

Pro Ala Ala Pro Ala Ala Pro Gly Cys Arg Gln Phe Asn Arg Arg Thr
        35                  40                  45

His Glu Val Trp Asn Leu Asp Cys
    50                  55

<210>  46
<211>  50
<212>  PRT
<213>  Artificial Sequence

<220>
<223>  PAS-aMD4-PEG3

<220>
<221>  DISULFID
<222>  (1)..(17)
<223>  Disulfid bridge between Cys-1 and Cys-17

<220>
<221>  DISULFID
<222>  (34)..(50)
<223>  Disulfid bridge between Cys-34 and Cys-50

<400>  46

Cys Tyr Arg Gln Phe Asn Arg Arg Thr His Glu Val Trp Asn Leu Asp
1               5                   10                  15

Cys Gly Ala Ala Pro Ala Ala Pro Ala Pro Ala Ala Pro Ala Ala Pro
            20                  25                  30

Gly Cys Tyr Arg Gln Phe Asn Arg Arg Thr His Glu Val Trp Asn Leu
        35                  40                  45

Asp Cys
    50

<210>  47
<211>  61
<212>  PRT
<213>  Artificial Sequence

<220>
<223>  AET-PAS-aMD4-PEG11

<220>
<221>  DISULFID
<222>  (4)..(20)
<223>  Disulfid bridge between Cys-4 and Cys-20

<220>
<221>  DISULFID
<222>  (45)..(61)
<223>  Disulfid bridge between Cys-45 and Cys-61

<400>  47

Ala Glu Thr Cys Tyr Arg Gln Phe Asn Arg Arg Thr His Glu Val Trp
1               5                   10                  15

Asn Leu Asp Cys Gly Ala Ala Pro Ala Ala Pro Ala Pro Ala Ala Pro
            20                  25                  30

Ala Ala Pro Ala Pro Ala Ala Pro Ala Ala Pro Gly Cys Tyr Arg Gln
        35                  40                  45

Phe Asn Arg Arg Thr His Glu Val Trp Asn Leu Asp Cys
    50                  55                  60

<210>  48
<211>  59
<212>  PRT
<213>  Artificial Sequence

<220>
<223>  AET-PAS-aMD4dY-PEG11


<220>
<221>  DISULFID
<222>  (4)..(19)
<223>  Disulfid bridge between Cys-4 and Cys-19

<220>
<221>  DISULFID
<222>  (44)..(59)
<223>  Disulfid bridge between Cys-44 and Cys-59

<400>  48

Ala Glu Thr Cys Arg Gln Phe Asn Arg Arg Thr His Glu Val Trp Asn
1               5                   10                  15

Leu Asp Cys Gly Ala Ala Pro Ala Ala Pro Ala Pro Ala Ala Pro Ala
            20                  25                  30

Ala Pro Ala Pro Ala Ala Pro Ala Ala Pro Gly Cys Arg Gln Phe Asn
        35                  40                  45

Arg Arg Thr His Glu Val Trp Asn Leu Asp Cys
    50                  55

<210>  49
<211>  53
<212>  PRT
<213>  Artificial Sequence

<220>
<223>  AET-PAS-aMD4-PEG3

<220>
<221>  DISULFID
<222>  (4)..(20)
<223>  Disulfid bridge between Cys-4 and Cys-20

<220>
<221>  DISULFID
<222>  (37)..(53)
<223>  Disulfid bridge between Cys-37 and Cys-53

<400>  49

Ala Glu Thr Cys Tyr Arg Gln Phe Asn Arg Arg Thr His Glu Val Trp
1               5                   10                  15

Asn Leu Asp Cys Gly Ala Ala Pro Ala Ala Pro Ala Pro Ala Ala Pro
            20                  25                  30

Ala Ala Pro Gly Cys Tyr Arg Gln Phe Asn Arg Arg Thr His Glu Val
        35                  40                  45

Trp Asn Leu Asp Cys
    50

<210>  50
<211>  294
<212>  DNA
<213>  Artificial Sequence

<220>
<223>  TorAss-PAS-aMD4-PEG11

<400>  50
atgaacaata acgatctctt tcaggcatca cgtcggcgtt ttctggcaca actcggcggc      60

ttaaccgtcg ccgggatgct ggggccgtca ttgttaacgc cgcgacgtgc gactgcgtgt     120

taccgtcaat tcaaccgtcg cacccacgaa gtttggaacc tggactgtgg cgcagcaccc     180

gcagccccag cccccgcagc acctgcagcc ccagcaccag ctgcaccagc cgctccaggc     240

tgctaccgtc agttcaaccg ccgcacccac gaagtttgga atttggattg ctaa           294

<210>  51
<211>  97
<212>  PRT
<213>  Artificial Sequence

<220>
<223>  TorAss-PAS-aMD4-PEG11

<220>
<221>  DISULFID
<222>  (40)..(56)
<223>  Disulfid bridge between Cys-40 and Cys-56

<220>

<221>  DISULFID
<222>  (81)..(97)
<223>  Disulfid bridge between Cys-81 and Cys-97

<400>  51

Met Asn Asn Asn Asp Leu Phe Gln Ala Ser Arg Arg Arg Phe Leu Ala
1               5                   10                  15

Gln Leu Gly Gly Leu Thr Val Ala Gly Met Leu Gly Pro Ser Leu Leu
            20                  25                  30

Thr Pro Arg Arg Ala Thr Ala Cys Tyr Arg Gln Phe Asn Arg Arg Thr
        35                  40                  45

His Glu Val Trp Asn Leu Asp Cys Gly Ala Ala Pro Ala Ala Pro Ala
    50                  55                  60

Pro Ala Ala Pro Ala Ala Pro Ala Pro Ala Ala Pro Ala Ala Pro Gly
65                  70                  75                  80

Cys Tyr Arg Gln Phe Asn Arg Arg Thr His Glu Val Trp Asn Leu Asp
                85                  90                  95

Cys

<210>  52
<211>  288
<212>  DNA
<213>  Artificial Sequence

<220>
<223>  TorAss-PAS-aMD4dY-PEG11

<400>  52
atgaacaata acgatctctt tcaggcatca cgtcggcgtt ttctggcaca actcggcggc      60

ttaaccgtcg ccgggatgct ggggccgtca ttgttaacgc cgcgacgtgc gactgcgtgc     120

cgtcagttca accgtcgcac ccacgaagtt tggaatctgg actgtggcgc agcccccgca     180

gcacccgctc ccgcagcacc cgcagctcca gcaccagcag ccccagcagc accaggctgc     240

cgccagttca accgtcgcac ccacgaagtt tggaatttgg attgttag                  288

<210>  53
<211>  95
<212>  PRT
<213>  Artificial Sequence

<220>
<223>  TorAss-PAS-aMD4dY-PEG11

<220>
<221>  DISULFID
<222>  (40)..(55)
<223>  Disulfid bridge between Cys-40 and Cys-55

<220>
<221>  DISULFID
<222>  (80)..(95)
<223>  Disulfid bridge between Cys-80 and Cys-95

<400>  53

Met Asn Asn Asn Asp Leu Phe Gln Ala Ser Arg Arg Arg Phe Leu Ala
1               5                   10                  15

Gln Leu Gly Gly Leu Thr Val Ala Gly Met Leu Gly Pro Ser Leu Leu
            20                  25                  30

Thr Pro Arg Arg Ala Thr Ala Cys Arg Gln Phe Asn Arg Arg Thr His
        35                  40                  45

Glu Val Trp Asn Leu Asp Cys Gly Ala Ala Pro Ala Ala Pro Ala Pro
    50                  55                  60

Ala Ala Pro Ala Ala Pro Ala Pro Ala Ala Pro Ala Ala Pro Gly Cys
65                  70                  75                  80

Arg Gln Phe Asn Arg Arg Thr His Glu Val Trp Asn Leu Asp Cys
                85                  90                  95

<210>  54
<211>  270
<212>  DNA
<213>  Artificial Sequence

<220>
<223>  TorAss-PAS-aMD4-PEG3

<400>  54
atgaacaata acgatctctt tcaggcatca cgtcggcgtt ttctggcaca actcggcggc      60

ttaaccgtcg ccgggatgct ggggccgtca ttgttaacgc cgcgacgtgc gactgcgtgt      120

taccgtcagt tcaaccgccg tacccacgaa gtctggaacc tggattgtgg tgccgccccc      180

gccgctcccg ctcccgccgc tccggcagcc cctggctgct accgtcagtt taaccgtcgc      240

acccacgaag tctggaacct ggattgctaa                                        270

<210>  55
<211>  89
<212>  PRT
<213>  Artificial Sequence

<220>
<223>  TorAss-PAS-aMD4-PEG3

<220>
<221>  DISULFID
<222>  (40)..(56)
<223>  Disulfid bridge between Cys-40 and Cys-56

<220>
<221>  DISULFID
<222>  (73)..(89)
<223>  Disulfid bridge between Cys-73 and Cys-89

<400>  55

Met Asn Asn Asn Asp Leu Phe Gln Ala Ser Arg Arg Arg Phe Leu Ala
1               5                   10                  15

Gln Leu Gly Gly Leu Thr Val Ala Gly Met Leu Gly Pro Ser Leu Leu
            20                  25                  30

Thr Pro Arg Arg Ala Thr Ala Cys Tyr Arg Gln Phe Asn Arg Arg Thr
        35                  40                  45

His Glu Val Trp Asn Leu Asp Cys Gly Ala Ala Pro Ala Ala Pro Ala
    50                  55                  60

Pro Ala Ala Pro Ala Ala Pro Gly Cys Tyr Arg Gln Phe Asn Arg Arg
65                  70                  75                  80

Thr His Glu Val Trp Asn Leu Asp Cys
                85


<210>  56
<211>  276
<212>  DNA
<213>  Artificial Sequence

<220>
<223>  CspBss-AET-PAS-aMD4-PEG11

<400>  56
atgtttaaca accgtatccg cactgcagct ctcgctggtg caatcgcaat ctccaccgca      60

gcttccggcg tagctatccc agcattcgct gcagagacct gttaccgtca attcaaccgt     120

cgcacccacg aagtttggaa cctggactgt ggcgcagcac ccgcagcccc agcccccgca     180

gcacctgcag ccccagcacc agctgcacca gccgctccag gctgctaccg tcagttcaac     240

cgccgcaccc acgaagtttg gaatttggat tgctaa                               276


<210>  57
<211>  91
<212>  PRT
<213>  Artificial Sequence

<220>
<223>  CspBss-AET-PAS-aMD4-PEG11

<220>
<221>  DISULFID
<222>  (34)..(50)
<223>  Disulfid bridge between Cys-34 and Cys-50

<220>
<221>  DISULFID
<222>  (75)..(91)
<223>  Disulfid bridge between Cys-75 and Cys-91

<400>  57

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Tyr Arg Gln Phe Asn Arg Arg Thr His Glu Val Trp Asn Leu
        35                  40                  45

Asp Cys Gly Ala Ala Pro Ala Ala Pro Ala Pro Ala Ala Pro Ala Ala
    50                  55                  60

Pro Ala Pro Ala Ala Pro Ala Ala Pro Gly Cys Tyr Arg Gln Phe Asn
65                  70                  75                  80

Arg Arg Thr His Glu Val Trp Asn Leu Asp Cys
                85                  90

<210>  58
<211>  270
<212>  DNA
<213>  Artificial Sequence

<220>
<223>  CspBss-AET-PAS-aMD4dY-PEG11

<400>  58
atgtttaaca accgtatccg cactgcagct ctcgctggtg caatcgcaat ctccaccgca      60

gcttccggcg tagctatccc agcattcgct gcagagacct gccgtcagtt caaccgtcgc     120

acccacgaag tttggaatct ggactgtggc gcagcccccg cagcacccgc tcccgcagca     180

cccgcagctc cagcaccagc agccccagca gcaccaggct gccgccagtt caaccgtcgc     240

acccacgaag tttggaattt ggattgttag                                      270

<210>  59
<211>  89
<212>  PRT

<213>  Artificial Sequence

<220>
<223>  CspBss-AET-PAS-aMD4dY-PEG11

<220>
<221>  DISULFID
<222>  (34)..(49)
<223>  Disulfid bridge between Cys-34 and Cys-49

<220>
<221>  DISULFID
<222>  (74)..(89)
<223>  Disulfid bridge between Cys-74 and Cys-89

<400>  59

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Arg Gln Phe Asn Arg Arg Thr His Glu Val Trp Asn Leu Asp
        35                  40                  45

Cys Gly Ala Ala Pro Ala Ala Pro Ala Pro Ala Ala Pro Ala Ala Pro
    50                  55                  60

Ala Pro Ala Ala Pro Ala Ala Pro Gly Cys Arg Gln Phe Asn Arg Arg
65                  70                  75                  80

Thr His Glu Val Trp Asn Leu Asp Cys
                85

<210>  60
<211>  252
<212>  DNA
<213>  Artificial Sequence

<220>
<223>  CspBss-AET-PAS-aMD4-PEG3

<400>  60
atgtttaaca accgtatccg cactgcagct ctcgctggtg caatcgcaat ctccaccgca      60

gcttccggcg tagctatccc agcattcgct gcagagacct gttaccgtca gttcaaccgc     120

cgtacccacg aagtctggaa cctggattgt ggtgccgccc ccgccgctcc cgctcccgcc     180

gctccggcag cccctggctg ctaccgtcag tttaaccgtc gcacccacga agtctggaac     240

ctggattgct aa                                                         252

<210>  61

<211>  83
<212>  PRT
<213>  Artificial Sequence

<220>
<223>  CspBss-AET-PAS-aMD4-PEG3

<220>
<221>  DISULFID
<222>  (34)..(50)
<223>  Disulfid bridge between Cys-34 and Cys-50

<220>
<221>  DISULFID
<222>  (67)..(83)
<223>  Disulfid bridge between Cys-67 and Cys-83

<400>  61

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Tyr Arg Gln Phe Asn Arg Arg Thr His Glu Val Trp Asn Leu
        35                  40                  45

Asp Cys Gly Ala Ala Pro Ala Ala Pro Ala Pro Ala Ala Pro Ala Ala
    50                  55                  60

Pro Gly Cys Tyr Arg Gln Phe Asn Arg Arg Thr His Glu Val Trp Asn
65                  70                  75                  80

Leu Asp Cys

<210>  62
<211>  60
<212>  PRT
<213>  Artificial Sequence

<220>
<223>  AET-PAS-aMD4_aMD5-PEG11

<220>
<221>  DISULFID
<222>  (4)..(20)
<223>  Disulfid bridge between Cys-4 and Cys-20

<220>
<221>  DISULFID
<222>  (45)..(60)
<223>  Disulfid bridge between Cys-45 and Cys-60

<400>  62

Ala Glu Thr Cys Tyr Arg Gln Phe Asn Arg Arg Thr His Glu Val Trp
1               5                   10                  15

Asn Leu Asp Cys Gly Ala Ala Pro Ala Ala Pro Ala Pro Ala Ala Pro
            20                  25                  30

Ala Ala Pro Ala Pro Ala Ala Pro Ala Ala Pro Gly Cys Tyr Trp Tyr
        35                  40                  45

Tyr Ala Trp Asp Gln Thr Tyr Lys Ala Phe Pro Cys
    50                  55                  60

<210>  63
<211>  273
<212>  DNA
<213>  Artificial Sequence

<220>
<223>  CspBss-AET-PAS-aMD4_aMD5-PEG11

<400>  63
atgtttaaca accgtatccg cactgcagct ctcgctggtg caatcgcaat ctccaccgca      60

gcttccggcg tagctatccc agcattcgct gcagagacct gttaccgtca gttcaaccgt     120

cgcacccacg aagtttggaa cctggattgt ggcgcagccc ccgcagcccc cgcacccgca     180

gcccctgcag ccccagcccc agctgcacca gcagcacctg gctgttactg gtattatgcg     240

tgggatcaga cctacaaagc gtttccgtgt tag                                  273

<210>  64
<211>  90
<212>  PRT
<213>  Artificial Sequence

<220>
<223>  CspBss-AET-PAS-aMD4_aMD5-PEG11

<220>
<221>  DISULFID
<222>  (34)..(50)
<223>  Disulfid bridge between Cys-34 and Cys-50

<220>
<221>  DISULFID
<222>  (75)..(90)
<223>  Disulfid bridge between Cys-75 and Cys-90

<400>  64

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Tyr Arg Gln Phe Asn Arg Arg Thr His Glu Val Trp Asn Leu
        35                  40                  45

Asp Cys Gly Ala Ala Pro Ala Ala Pro Ala Pro Ala Ala Pro Ala Ala
    50                  55                  60

Pro Ala Pro Ala Ala Pro Ala Ala Pro Gly Cys Tyr Trp Tyr Tyr Ala
65                  70                  75                  80

Trp Asp Gln Thr Tyr Lys Ala Phe Pro Cys
                85                  90

<210>  65
<211>  20
<212>  PRT
<213>  Artificial Sequence

<220>
<223>  EpoR binding peptide

<220>
<221>  DISULFID
<222>  (6)..(15)
<223>  Disulfid bridge between Cys-6 and Cys-15

<400>  65

Gly Gly Leu Tyr Ala Cys His Met Gly Pro Met Thr Trp Val Cys Gln
1               5                   10                  15

Pro Leu Arg Gly
            20

<210>  66
<211>  17
<212>  PRT
<213>  Artificial Sequence

<220>
<223>  EpoR binding peptide

<220>
<221>  DISULFID
<222>  (1)..(17)
<223>  Disulfid bridge between Cys-1 and Cys-17

<400>  66

Cys Ile Ser Trp Asn Glu Phe Asn Ser Pro Asn Trp Arg Phe Ile Thr
1               5                   10                  15

Cys

<210> 67
<211> 14
<212> PRT
<213> Artificial Sequence

<220>
<223> TPO recepter binding peptide

<400> 67

Ile Glu Gly Pro Thr Leu Arg Gln Trp Leu Ala Ala Arg Ala
1               5                   10

<210> 68
<211> 18
<212> PRT
<213> Artificial Sequence

<220>
<223> TPO receptor binding peptide

<220>
<221> DISULFID
<222> (3)..(16)
<223> Disulfid bridge between Cys-3 and Cys-16

<400> 68

Gly Gly Cys Ala Asp Gly Pro Thr Leu Arg Glu Trp Ile Ser Phe Cys
1               5                   10                  15

Gly Gly

<210> 69
<211> 16
<212> PRT
<213> Artificial Sequence

<220>
<223> TPO receptor binding peptide

<220>
<221> DISULFID
<222> (3)..(14)
<223> Disulfid bridge between Cys-3 and Cys-14

<400> 69

Gly Gly Cys Thr Leu Arg Glu Trp Leu His Gly Gly Phe Cys Gly Gly
1               5                   10                  15

<210>  70
<211>  19
<212>  PRT
<213>  Artificial Sequence

<220>
<223>  TPO receptor binding peptide

<400>  70

Leu Ala Ile Glu Gly Pro Thr Leu Arg Gln Trp Leu His Gly Asn Gly
1               5                   10                  15

Arg Asp Thr

<210>  71
<211>  18
<212>  PRT
<213>  Artificial Sequence

<220>
<223>  VEGFR binding peptide

<220>
<221>  DISULFID
<222>  (6)..(13)
<223>  Disulfid bridge between Cys-6 and Cys-13

<400>  71

Ala Gly Pro Thr Trp Cys Glu Asp Asp Trp Tyr Tyr Cys Trp Leu Phe
1               5                   10                  15

Gly Thr

<210>  72
<211>  17
<212>  PRT
<213>  Artificial Sequence

<220>
<223>  VEGFR binding peptide

<220>
<221>  DISULFID
<222>  (2)..(12)
<223>  Disulfid bridge between Cys-2 and Cys-12

<400>  72

Val Cys Trp Glu Asp Ser Trp Gly Gly Glu Val Cys Trp Leu Phe Gly
1               5                   10                  15

Thr

<210>  73
<211>  17
<212>  PRT
<213>  Artificial Sequence

<220>
<223>  VEGFR binding peptide


<220>
<221>  DISULFID
<222>  (2)..(12)
<223>  Disulfid bridge between Cys-2 and Cys-12

<400>  73

Val Cys Trp Glu Asp Ser Trp Gly Gly Glu Val Cys Phe Arg Tyr Asp
1               5                   10                  15


Pro




<210>  74
<211>  8
<212>  PRT
<213>  Artificial Sequence

<220>
<223>  PAS Linker

<400>  74

Ala Ala Pro Ala Ala Pro Ala Pro
1               5


<210>  75
<211>  16
<212>  PRT
<213>  Artificial Sequence

<220>
<223>  PAS Linker

<400>  75

Ala Ala Pro Ala Ala Pro Ala Pro Ala Ala Pro Ala Ala Pro Ala Pro
1               5                   10                  15


<210>  76
<211>  49
<212>  PRT
<213>  Artificial Sequence

<220>
<223>  PAS Linker

<400>  76

Ala Ala Pro Ala Ala Pro Ala Pro Ala Ala Pro Ala Ala Pro Ala Pro
1               5                   10                  15

Ala Ala Pro Ala Ala Pro Ala Pro Ala Ala Pro Ala Ala Pro Ala Pro
            20                  25                  30

Ala Ala Pro Ala Ala Pro Ala Pro Ala Ala Pro Ala Ala Pro Ala Pro
        35                  40                  45

Ala

<210>  77
<211>  49
<212>  PRT
<213>  Artificial Sequence

<220>
<223>  PAS Linker

<400>  77

Ser Ala Pro Ser Pro Ser Ser Ala Pro Ala Ser Ala Ser Ala Pro Ala
1               5                   10                  15

Ser Pro Ala Ser Ala Ser Ala Ser Ala Ser Pro Ala Ser Ser Pro Ala
            20                  25                  30

Ser Ala Ser Ser Ala Ser Pro Ala Ala Ser Ser Ala Ser Pro Ala Ser
        35                  40                  45

Ser

<210>  78
<211>  100
<212>  PRT
<213>  Artificial Sequence

<220>
<223>  PAS Linker

<400>  78

Ala Ala Pro Ala Ala Pro Ala Pro Ala Ala Pro Ala Ala Pro Ala Pro
1               5                   10                  15

Ala Ala Pro Ala Ala Pro Ala Pro Ala Ala Pro Ala Ala Pro Ala Pro
            20                  25                  30

Ala Ala Pro Ala Ala Pro Ala Pro Ala Ala Pro Ala Ala Pro Ala Pro

35                  40                  45

Ala Ala Pro Ala Ala Pro Ala Pro Ala Ala Pro Ala Ala Pro Ala Pro
    50                  55                  60

Ala Ala Pro Ala Ala Pro Ala Pro Ala Ala Pro Ala Ala Pro Ala Pro
65                  70                  75                  80

Ala Ala Pro Ala Ala Pro Ala Pro Ala Ala Pro Ala Ala Pro Ala Pro
                85                  90                  95

Ala Ala Pro Ala
            100

<210>  79
<211>  100
<212>  PRT
<213>  Artificial Sequence

<220>
<223>  PAS Linker

<400>  79

Ser Ala Pro Ser Pro Ser Ser Ala Pro Ala Ser Ala Ser Ala Pro Ala
1               5                   10                  15

Ser Pro Ala Ser Ala Ser Ala Ser Ala Ser Pro Ala Ser Ser Pro Ala
            20                  25                  30

Ser Ala Ser Ser Ala Ser Pro Ala Ala Ser Ser Ala Ser Pro Ala Ser
        35                  40                  45

Ser Ser Ala Ser Pro Ser Ala Pro Ala Ala Pro Ser Ala Ser Ala Ala
    50                  55                  60

Pro Ser Ala Ser Pro Ala Ser Ser Ser Pro Ala Pro Ala Ser Ala Pro
65                  70                  75                  80

Ser Pro Ala Ala Ala Ala Ser Ser Pro Ser Pro Ala Pro Ser Ser Ala
                85                  90                  95

Ser Ser Ala Ala
            100

<210>  80
<211>  200
<212>  PRT
<213>  Artificial Sequence

<220>

<223>  PAS Linker

<400>  80

Ala Ala Pro Ala Ala Pro Ala Pro Ala Ala Pro Ala Ala Pro Ala Pro
1               5                   10                  15

Ala Ala Pro Ala Ala Pro Ala Pro Ala Ala Pro Ala Ala Pro Ala Pro
            20                  25                  30

Ala Ala Pro Ala Ala Pro Ala Pro Ala Ala Pro Ala Ala Pro Ala Pro
        35                  40                  45

Ala Ala Pro Ala Ala Pro Ala Pro Ala Ala Pro Ala Ala Pro Ala Pro
    50                  55                  60

Ala Ala Pro Ala Ala Pro Ala Pro Ala Ala Pro Ala Ala Pro Ala Pro
65                  70                  75                  80

Ala Ala Pro Ala Ala Pro Ala Pro Ala Ala Pro Ala Ala Pro Ala Pro
                85                  90                  95

Ala Ala Pro Ala Ala Pro Ala Pro Ala Ala Pro Ala Ala Pro Ala Pro
            100                 105                 110

Ala Ala Pro Ala Ala Pro Ala Pro Ala Ala Pro Ala Ala Pro Ala Pro
        115                 120                 125

Ala Ala Pro Ala Ala Pro Ala Pro Ala Ala Pro Ala Ala Pro Ala Pro
    130                 135                 140

Ala Ala Pro Ala Ala Pro Ala Pro Ala Ala Pro Ala Ala Pro Ala Pro
145                 150                 155                 160

Ala Ala Pro Ala Ala Pro Ala Pro Ala Ala Pro Ala Ala Pro Ala Pro
                165                 170                 175

Ala Ala Pro Ala Ala Pro Ala Pro Ala Ala Pro Ala Ala Pro Ala Pro
            180                 185                 190

Ala Ala Pro Ala Ala Pro Ala Pro
        195                 200


<210>  81
<211>  45
<212>  PRT
<213>  Artificial Sequence

<220>
<223>  AET-aMD4dY-PAS8

<220>
<221>  DISULFID
<222>  (4)..(19)
<223>  Disulfid bridge between Cys-4 and Cys-19

<220>
<221>  DISULFID
<222>  (30)..(45)
<223>  Disulfid bridge between Cys-30 and Cys-45

<400>  81

Ala Glu Thr Cys Arg Gln Phe Asn Arg Arg Thr His Glu Val Trp Asn
1               5                   10                  15

Leu Asp Cys Gly Ala Ala Pro Ala Ala Pro Ala Pro Gly Cys Arg Gln
            20                  25                  30

Phe Asn Arg Arg Thr His Glu Val Trp Asn Leu Asp Cys
        35                  40                  45

<210>  82
<211>  86
<212>  PRT
<213>  Artificial Sequence

<220>
<223>  AET-aMD4dY-PAS49

<220>
<221>  DISULFID
<222>  (4)..(19)
<223>  Disulfid bridge between Cys-4 and Cys-19

<220>
<221>  DISULFID
<222>  (71)..(86)
<223>  Disulfid bridge between Cys-71 and Cys-86

<400>  82

Ala Glu Thr Cys Arg Gln Phe Asn Arg Arg Thr His Glu Val Trp Asn
1               5                   10                  15

Leu Asp Cys Gly Ala Ala Pro Ala Ala Pro Ala Pro Ala Ala Pro Ala
            20                  25                  30

Ala Pro Ala Pro Ala Ala Pro Ala Ala Pro Ala Pro Ala Ala Pro Ala
        35                  40                  45

Ala Pro Ala Pro Ala Ala Pro Ala Ala Pro Ala Pro Ala Ala Pro Ala
    50                  55                  60

Ala Pro Ala Pro Ala Gly Cys Arg Gln Phe Asn Arg Arg Thr His Glu
65                  70                  75                  80

Val Trp Asn Leu Asp Cys
                85

<210>  83
<211>  137
<212>  PRT
<213>  Artificial Sequence

<220>
<223>  AET-aMD4dY-PAS100

<220>
<221>  DISULFID
<222>  (4)..(19)
<223>  Disulfid bridge between Cys-4 and Cys-19

<220>
<221>  DISULFID
<222>  (122)..(137)
<223>  Disulfid bridge between Cys-122 and Cys-137

<400>  83

Ala Glu Thr Cys Arg Gln Phe Asn Arg Arg Thr His Glu Val Trp Asn
1               5                   10                  15

Leu Asp Cys Gly Ala Ala Pro Ala Ala Pro Ala Pro Ala Ala Pro Ala
            20                  25                  30

Ala Pro Ala Pro Ala Ala Pro Ala Ala Pro Ala Pro Ala Ala Pro Ala
        35                  40                  45

Ala Pro Ala Pro Ala Ala Pro Ala Ala Pro Ala Pro Ala Ala Pro Ala
    50                  55                  60

Ala Pro Ala Pro Ala Ala Pro Ala Ala Pro Ala Pro Ala Ala Pro Ala
65                  70                  75                  80

Ala Pro Ala Pro Ala Ala Pro Ala Ala Pro Ala Pro Ala Ala Pro Ala
                85                  90                  95

Ala Pro Ala Pro Ala Ala Pro Ala Ala Pro Ala Pro Ala Ala Pro Ala
            100                 105                 110

Ala Pro Ala Pro Ala Ala Pro Ala Gly Cys Arg Gln Phe Asn Arg Arg
        115                 120                 125

Thr His Glu Val Trp Asn Leu Asp Cys
    130                 135

<210>  84
<211>  237
<212>  PRT
<213>  Artificial Sequence

<220>
<223>  AET-aMD4dY-PAS200

<220>
<221>  DISULFID
<222>  (4)..(19)
<223>  Disulfid bridge between Cys-4 and Cys-19

<220>
<221>  DISULFID
<222>  (222)..(237)
<223>  Disulfid bridge between Cys-222 and Cys-237

<400>  84

Ala Glu Thr Cys Arg Gln Phe Asn Arg Arg Thr His Glu Val Trp Asn
1               5                   10                  15

Leu Asp Cys Gly Ala Ala Pro Ala Ala Pro Ala Pro Ala Ala Pro Ala
            20                  25                  30

Ala Pro Ala Pro Ala Ala Pro Ala Ala Pro Ala Pro Ala Ala Pro Ala
        35                  40                  45

Ala Pro Ala Pro Ala Ala Pro Ala Ala Pro Ala Pro Ala Ala Pro Ala
    50                  55                  60

Ala Pro Ala Pro Ala Ala Pro Ala Ala Pro Ala Pro Ala Ala Pro Ala
65                  70                  75                  80

Ala Pro Ala Pro Ala Ala Pro Ala Ala Pro Ala Pro Ala Ala Pro Ala
                85                  90                  95

Ala Pro Ala Pro Ala Ala Pro Ala Ala Pro Ala Pro Ala Ala Pro Ala
            100                 105                 110

Ala Pro Ala Pro Ala Ala Pro Ala Ala Pro Ala Pro Ala Ala Pro Ala
        115                 120                 125

Ala Pro Ala Pro Ala Ala Pro Ala Ala Pro Ala Pro Ala Ala Pro Ala
    130                 135                 140

Ala Pro Ala Pro Ala Ala Pro Ala Ala Pro Ala Pro Ala Ala Pro Ala
145                 150                 155                 160

Ala Pro Ala Pro Ala Ala Pro Ala Ala Pro Ala Pro Ala Ala Pro Ala
                165                 170                 175

Ala Pro Ala Pro Ala Ala Pro Ala Ala Pro Ala Pro Ala Ala Pro Ala
            180                 185                 190

Ala Pro Ala Pro Ala Ala Pro Ala Ala Pro Ala Pro Ala Ala Pro Ala
        195                 200                 205

Ala Pro Ala Pro Ala Ala Pro Ala Ala Pro Ala Pro Gly Cys Arg Gln
    210                 215                 220

Phe Asn Arg Arg Thr His Glu Val Trp Asn Leu Asp Cys
225                 230                 235

<210>  85
<211>  52
<212>  PRT
<213>  Artificial Sequence

<220>
<223>  AET-aMD4dY-GS15

<220>
<221>  DISULFID
<222>  (4)..(19)
<223>  Disulfid bridge between Cys-4 and Cys-19

<220>
<221>  DISULFID
<222>  (37)..(52)
<223>  Disulfid bridge between Cys-37 and Cys-52

<400>  85

Ala Glu Thr Cys Arg Gln Phe Asn Arg Arg Thr His Glu Val Trp Asn
1               5                   10                  15

Leu Asp Cys Gly Gly Gly Gly Gly Ser Gly Gly Gly Gly Ser Gly Gly
            20                  25                  30

Gly Gly Ser Gly Cys Arg Gln Phe Asn Arg Arg Thr His Glu Val Trp
        35                  40                  45

Asn Leu Asp Cys
    50

<210>  86
<211>  59
<212>  PRT
<213>  Artificial Sequence

<220>

<223>  AET-aMD4dY-GS22

<220>
<221>  DISULFID
<222>  (4)..(19)
<223>  Disulfid bridge between Cys-4 and Cys-19

<220>
<221>  DISULFID
<222>  (44)..(59)
<223>  Disulfid bridge between Cys-44 and Cys-59

<400>  86

Ala Glu Thr Cys Arg Gln Phe Asn Arg Arg Thr His Glu Val Trp Asn
1               5                   10                  15

Leu Asp Cys Gly Gly Gly Gly Gly Ser Gly Gly Gly Gly Ser Gly Gly
            20                  25                  30

Gly Gly Ser Gly Gly Gly Gly Ser Gly Gly Gly Cys Arg Gln Phe Asn
        35                  40                  45

Arg Arg Thr His Glu Val Trp Asn Leu Asp Cys
    50                  55

<210>  87
<211>  228
<212>  DNA
<213>  Artificial Sequence

<220>
<223>  CspBss-AET-aMD4dY-PAS8

<400>  87
atgtttaaca accgtatccg cactgcagct ctcgctggtg caatcgcaat ctccaccgca      60

gcttccggcg tagctatccc agcattcgct gcagagacct gccgccagtt caaccgccgc     120

acccacgaag tgtggaacct ggattgcggc gctgcaccag ccgctccagc accaggctgc     180

cgccagttca accgccgcac ccacgaagtg tggaacctgg attgctaa                  228

<210>  88
<211>  75
<212>  PRT
<213>  Artificial Sequence

<220>
<223>  CspBss-AET-aMD4dY-PAS8

<220>
<221>  DISULFID
<222>  (34)..(49)
<223>  Disulfid bridge between Cys-34 and Cys-49

<220>
<221> DISULFID
<222> (60)..(75)
<223> Disulfid bridge between Cys-60 and Cys-75

<400> 88

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Arg Gln Phe Asn Arg Arg Thr His Glu Val Trp Asn Leu Asp
        35                  40                  45

Cys Gly Ala Ala Pro Ala Ala Pro Ala Pro Gly Cys Arg Gln Phe Asn
    50                  55                  60

Arg Arg Thr His Glu Val Trp Asn Leu Asp Cys
65                  70                  75

<210> 89
<211> 351
<212> DNA
<213> Artificial Sequence

<220>
<223> CspBss-AET-aMD4dY-PAS49

<400> 89
atgtttaaca accgtatccg cactgcagct ctcgctggtg caatcgcaat ctccaccgca 60

gcttccggcg tagctatccc agcattcgct gcagagacct gtcgtcagtt caaccgtcgc 120

acccacgaag tttggaattt ggactgtggc gcagcaccag cggcaccagc accagcagcc 180

ccagcggcac cagccccagc tgcaccagcc gctccagcac cagcagcacc cgcagccccc 240

gctcccgcag cccccgccgc accagcaccc gcagcaccag cagcacccgc tccagcaggt 300

tgtcgccaat tcaaccgccg cacccacgaa gtgtggaacc tggattgcta a 351

<210> 90
<211> 116
<212> PRT
<213> Artificial Sequence

<220>
<223> CspBss-AET-aMD4dY-PAS49

<220>
<221> DISULFID
<222> (34)..(49)
<223> Disulfid bridge between Cys-34 and Cys-49

<220>
<221>  DISULFID
<222>  (101)..(116)
<223>  Disulfid bridge between Cys-101 and Cys-116

<400>  90

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Arg Gln Phe Asn Arg Arg Thr His Glu Val Trp Asn Leu Asp
        35                  40                  45

Cys Gly Ala Ala Pro Ala Ala Pro Ala Pro Ala Ala Pro Ala Ala Pro
    50                  55                  60

Ala Pro Ala Ala Pro Ala Ala Pro Ala Pro Ala Ala Pro Ala Ala Pro
65                  70                  75                  80

Ala Pro Ala Ala Pro Ala Ala Pro Ala Pro Ala Ala Pro Ala Ala Pro
                85                  90                  95

Ala Pro Ala Gly Cys Arg Gln Phe Asn Arg Arg Thr His Glu Val Trp
            100                 105                 110

Asn Leu Asp Cys
        115


<210>  91
<211>  504
<212>  DNA
<213>  Artificial Sequence

<220>
<223>  CspBss-AET-aMD4dY-PAS100

<400>  91
atgtttaaca accgtatccg cactgcagct ctcgctggtg caatcgcaat ctccaccgca      60

gcttccggcg tagctatccc agcattcgct gcagagacct gtcgtcagtt caaccgtcgc     120

acccacgaag tttggaattt ggactgtggc gcagcaccag cggcaccagc accagcagcc     180

ccagcggcac cagccccagc tgcaccagcc gctccagcac cagcagcccc agcagccccc     240

gctccagcag cacccgcagc accagctccc gcagccccag cagccccagc cccagcagca     300

ccagcagccc cagctccagc agccccagct gcaccagctc cagccgcacc agcagctcca     360

gccccagccg ctcccgccgc cccagccccc gccgcaccag ccgcccctgc tcccgccgcc     420

ccagccgctc ccgctccagc cgctcctgcc ggttgccgcc agttcaaccg ccgcacccac     480

gaagtgtgga acctggattg ctaa                                              504

<210>  92
<211>  167
<212>  PRT
<213>  Artificial Sequence

<220>
<223>  CspBss-AET-aMD4dY-PAS100

<220>
<221>  DISULFID
<222>  (34)..(49)
<223>  Disulfid bridge between Cys-34 and Cys-49

<220>
<221>  DISULFID
<222>  (152)..(167)
<223>  Disulfid bridge between Cys-152 and Cys-167

<400>  92

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Arg Gln Phe Asn Arg Arg Thr His Glu Val Trp Asn Leu Asp
        35                  40                  45

Cys Gly Ala Ala Pro Ala Ala Pro Ala Pro Ala Ala Pro Ala Ala Pro
    50                  55                  60

Ala Pro Ala Ala Pro Ala Ala Pro Ala Pro Ala Ala Pro Ala Ala Pro
65                  70                  75                  80

Ala Pro Ala Ala Pro Ala Ala Pro Ala Pro Ala Ala Pro Ala Ala Pro
                85                  90                  95

Ala Pro Ala Ala Pro Ala Ala Pro Ala Pro Ala Ala Pro Ala Ala Pro
            100                 105                 110

Ala Pro Ala Ala Pro Ala Ala Pro Ala Pro Ala Ala Pro Ala Ala Pro
        115                 120                 125

Ala Pro Ala Ala Pro Ala Ala Pro Ala Pro Ala Ala Pro Ala Ala Pro
    130                 135                 140

Ala Pro Ala Ala Pro Ala Gly Cys Arg Gln Phe Asn Arg Arg Thr His
145                 150                 155                 160

Glu Val Trp Asn Leu Asp Cys
                165

<210>  93
<211>  804
<212>  DNA
<213>  Artificial Sequence

<220>
<223>  CspBss-AET-aMD4dY-PAS200

<400>  93
atgtttaaca accgtatccg cactgcagct ctcgctggtg caatcgcaat ctccaccgca      60

gcttccggcg tagctatccc agcattcgct gcagagacct gtcgtcagtt caaccgtcgc     120

acccacgaag tttggaattt ggactgtggc gcagcaccag cggcaccagc accagcagcc     180

ccagcggcac cagccccagc tgcaccagcc gctccagcac cagccgcccc cgccgccccc     240

gcccctgctg ctcctgctgc tcccgctccc gctgctcccg ctgcccctgc tcctgctgct     300

cccgctgcgc cagccccagc agcaccagct gccccagcac cagctgcacc agccgcacca     360

gctccagcag ccccagctgc tccagcacca gcagcaccag cagcaccagc tccagcggca     420

ccagcagcac cagcacctgc ggcaccagcg gctccagccc cagcagcacc agcagcgcca     480

gcaccagcag ctccagcagc accagcacca gcagcaccag cggcaccagc cccagctgca     540

ccagccgctc cagcaccagc tgccccagca gctccagccc cagccgcacc tgccgcacct     600

gcccctgccg ctcctgctgc ccccgctcct gccgctcctg ctgcccctgc ccctgctgct     660

cctgctgctc cagctccagc cgcaccagcc gctccagcac cagctgcacc agcagctcca     720

gccccagctg ccccagcagc tccagcccct ggttgccgcc aatttaatcg ccgcacccac     780

gaggtctgga atcttgactg ctaa                                            804

<210>  94
<211>  267
<212>  PRT
<213>  Artificial Sequence

<220>
<223>  CspBss-AET-aMD4dY-PAS200


<220>
<221>  DISULFID
<222>  (34)..(49)
<223>  Disulfid bridge between Cys-34 and Cys-49

<220>
<221>  DISULFID
<222>  (252)..(267)
<223>  Disulfid bridge between Cys-252 and Cys-267

<400>  94

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Arg Gln Phe Asn Arg Arg Thr His Glu Val Trp Asn Leu Asp
        35                  40                  45

Cys Gly Ala Ala Pro Ala Ala Pro Ala Pro Ala Ala Pro Ala Ala Pro
    50                  55                  60

Ala Pro Ala Ala Pro Ala Ala Pro Ala Pro Ala Ala Pro Ala Ala Pro
65                  70                  75                  80

Ala Pro Ala Ala Pro Ala Ala Pro Ala Pro Ala Ala Pro Ala Ala Pro
                85                  90                  95

Ala Pro Ala Ala Pro Ala Ala Pro Ala Pro Ala Ala Pro Ala Ala Pro
            100                 105                 110

Ala Pro Ala Ala Pro Ala Ala Pro Ala Pro Ala Ala Pro Ala Ala Pro
        115                 120                 125

Ala Pro Ala Ala Pro Ala Ala Pro Ala Pro Ala Ala Pro Ala Ala Pro
    130                 135                 140

Ala Pro Ala Ala Pro Ala Ala Pro Ala Pro Ala Ala Pro Ala Ala Pro
145                 150                 155                 160

Ala Pro Ala Ala Pro Ala Ala Pro Ala Pro Ala Ala Pro Ala Ala Pro
                165                 170                 175

Ala Pro Ala Ala Pro Ala Ala Pro Ala Pro Ala Ala Pro Ala Ala Pro
            180                 185                 190

Ala Pro Ala Ala Pro Ala Ala Pro Ala Pro Ala Ala Pro Ala Ala Pro
        195                 200                 205

Ala Pro Ala Ala Pro Ala Ala Pro Ala Pro Ala Ala Pro Ala Ala Pro
    210                 215                 220

Ala Pro Ala Ala Pro Ala Ala Pro Ala Pro Ala Ala Pro Ala Ala Pro
225                 230                 235                 240

Ala Pro Ala Ala Pro Ala Ala Pro Ala Pro Gly Cys Arg Gln Phe Asn

                    245                     250                     255

Arg Arg Thr His Glu Val Trp Asn Leu Asp Cys
            260                     265

<210>  95
<211>  249
<212>  DNA
<213>  Artificial Sequence

<220>
<223>  CspBss-AET-aMD4dY-GS15

<400>  95
atgtttaaca accgtatccg cactgcagct ctcgctggtg caatcgcaat ctccaccgca        60

gcttccggcg tagctatccc agcattcgct gcagagacct gccgccagtt caaccgccgc       120

acccacgaag tgtggaacct ggattgcggc ggcggcggcg gctccggcgg cggcggctcc       180

ggcggcggcg gctccggctg ccgccagttc aaccgccgca cccacgaagt gtggaacctg       240

gattgctaa                                                               249

<210>  96
<211>  82
<212>  PRT
<213>  Artificial Sequence

<220>
<223>  CspBss-AET-aMD4dY-GS15

<220>
<221>  DISULFID
<222>  (34)..(49)
<223>  Disulfid bridge between Cys-34 and Cys-49

<220>
<221>  DISULFID
<222>  (67)..(82)
<223>  Disulfid bridge between Cys-67 and Cys-82

<400>  96

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Arg Gln Phe Asn Arg Arg Thr His Glu Val Trp Asn Leu Asp
        35                  40                  45

Cys Gly Gly Gly Gly Gly Ser Gly Gly Gly Gly Ser Gly Gly Gly Gly
    50                  55                  60

Ser Gly Cys Arg Gln Phe Asn Arg Arg Thr His Glu Val Trp Asn Leu
65                  70                  75                  80

Asp Cys

<210>  97
<211>  270
<212>  DNA
<213>  Artificial Sequence

<220>
<223>  CspBss-AET-aMD4dY-GS22

<400>  97
atgtttaaca accgtatccg cactgcagct ctcgctggtg caatcgcaat ctccaccgca      60

gcttccggcg tagctatccc agcattcgct gcagagacct gccgccagtt caaccgccgc     120

acccacgaag tgtggaacct ggattgcggc ggcggcggcg gctccggcgg cggcggttct     180

ggcggtggcg gttctggcgg cggcggttct ggcggcggtt gtcgtcagtt caatcgtcgc     240

acccacgaag tctggaatct cgactgctaa                                      270

<210>  98
<211>  89
<212>  PRT
<213>  Artificial Sequence

<220>
<223>  CspBss-AET-aMD4dY-GS22

<220>
<221>  DISULFID
<222>  (34)..(49)
<223>  Disulfid bridge between Cys-34 and Cys-49

<220>
<221>  DISULFID
<222>  (74)..(89)
<223>  Disulfid bridge between Cys-74 and Cys-89

<400>  98

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Arg Gln Phe Asn Arg Arg Thr His Glu Val Trp Asn Leu Asp
        35                  40                  45

Cys Gly Gly Gly Gly Gly Ser Gly Gly Gly Gly Ser Gly Gly Gly Gly

50                  55                  60

Ser Gly Gly Gly Gly Ser Gly Gly Gly Cys Arg Gln Phe Asn Arg Arg
65                  70                  75                  80

Thr His Glu Val Trp Asn Leu Asp Cys
                85

<210>  99
<211>  259
<212>  PRT
<213>  Artificial Sequence

<220>
<223>  AET-aMD4dY-PAS22-PAS200

<220>
<221>  DISULFID
<222>  (4)..(19)
<223>  Disulfid bridge between Cys-4 and Cys-19

<220>
<221>  DISULFID
<222>  (44)..(59)
<223>  Disulfid bridge between Cys-44 and Cys-59

<400>  99

Ala Glu Thr Cys Arg Gln Phe Asn Arg Arg Thr His Glu Val Trp Asn
1               5                   10                  15

Leu Asp Cys Gly Ala Ala Pro Ala Ala Pro Ala Pro Ala Ala Pro Ala
            20                  25                  30

Ala Pro Ala Pro Ala Ala Pro Ala Ala Pro Gly Cys Arg Gln Phe Asn
        35                  40                  45

Arg Arg Thr His Glu Val Trp Asn Leu Asp Cys Ala Ala Pro Ala Ala
    50                  55                  60

Pro Ala Pro Ala Ala Pro Ala Ala Pro Ala Pro Ala Ala Pro Ala Ala
65                  70                  75                  80

Pro Ala Pro Ala Ala Pro Ala Ala Pro Ala Pro Ala Ala Pro Ala Ala
                85                  90                  95

Pro Ala Pro Ala Ala Pro Ala Ala Pro Ala Pro Ala Ala Pro Ala Ala
            100                 105                 110

Pro Ala Pro Ala Ala Pro Ala Ala Pro Ala Pro Ala Ala Pro Ala Ala
        115                 120                 125

Pro Ala Pro Ala Ala Pro Ala Ala Pro Ala Pro Ala Ala Pro Ala Ala
    130                 135                 140

Pro Ala Pro Ala Ala Pro Ala Ala Pro Ala Pro Ala Ala Pro Ala Ala
145                 150                 155                 160

Pro Ala Pro Ala Ala Pro Ala Ala Pro Ala Pro Ala Ala Pro Ala Ala
                165                 170                 175

Pro Ala Pro Ala Ala Pro Ala Ala Pro Ala Pro Ala Ala Pro Ala Ala
            180                 185                 190

Pro Ala Pro Ala Ala Pro Ala Ala Pro Ala Pro Ala Ala Pro Ala Ala
        195                 200                 205

Pro Ala Pro Ala Ala Pro Ala Ala Pro Ala Pro Ala Ala Pro Ala Ala
    210                 215                 220

Pro Ala Pro Ala Ala Pro Ala Ala Pro Ala Pro Ala Ala Pro Ala Ala
225                 230                 235                 240

Pro Ala Pro Ala Ala Pro Ala Ala Pro Ala Pro Ala Ala Pro Ala Ala
                245                 250                 255

Pro Ala Pro

<210>  100
<211>  870
<212>  DNA
<213>  Artificial Sequence

<220>
<223>  CspBss-AET-aMD4dY-PAS22-PAS200

<400>  100
atgtttaaca accgtatccg cactgcagct ctcgctggtg caatcgcaat ctccaccgca      60

gcttccggcg tagctatccc agcattcgct gcagagacct gccgtcagtt caaccgccgt     120

acccacgaag tttggaatct cgactgtggt gccgcccctg ccgctcctgc ccctgccgcc     180

ccagccgctc cagcaccagc cgctccagca gccccaggct gccgccagtt caaccgccgc     240

acccacgaag tgtggaacct ggattgcgca gctccagcag cccctgcccc cgccgcccca     300

gccgcccctg cccccgccgc ccccgccgca cccgcccctg ccgcccccgc cgcccccgcc     360

cctgctgctc ctgctgctcc cgctcccgct gctcccgctg cccctgctcc tgctgctccc     420

gctgcgccag ccccagcagc accagctgcc ccagcaccag ctgcaccagc cgcaccagct     480

ccagcagccc cagctgctcc agcaccagca gcaccagcag caccagctcc agcggcacca     540

gcagcaccag cacctgcggc accagcggct ccagccccag cagcaccagc agcgccagca 600

ccagcagctc cagcagcacc agcaccagca gcaccagcgg caccagcccc agctgcacca 660

gccgctccag caccagctgc cccagcagct ccagccccag ctgcccctgc tgcccccgct 720

cctgccgctc ccgctgcccc tgcccctgct gctcctgctg ctcccgctcc tgccgctccc 780

gctgcgccag ctccagcagc cccagctgca ccagccccag ccgcaccagc cgccccagcc 840

cccgcagcac ccgcagcacc agccccataa 870

<210> 101
<211> 289
<212> PRT
<213> Artificial Sequence

<220>
<223> CspBss-AET-aMD4dY-PAS22-PAS200

<220>
<221> DISULFID
<222> (34)..(49)
<223> Disulfid bridge between Cys-34 and Cys-49

<220>
<221> DISULFID
<222> (74)..(89)
<223> Disulfid bridge between Cys-74 and Cys-89

<400> 101

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Cys Arg Gln Phe Asn Arg Arg Thr His Glu Val Trp Asn Leu Asp
        35                  40                  45

Cys Gly Ala Ala Pro Ala Ala Pro Ala Pro Ala Ala Pro Ala Ala Pro
    50                  55                  60

Ala Pro Ala Ala Pro Ala Ala Pro Gly Cys Arg Gln Phe Asn Arg Arg
65                  70                  75                  80

Thr His Glu Val Trp Asn Leu Asp Cys Ala Ala Pro Ala Ala Pro Ala
                85                  90                  95

Pro Ala Ala Pro Ala Ala Pro Ala Pro Ala Ala Pro Ala Ala Pro Ala
            100                 105                 110

Pro Ala Ala Pro Ala Ala Pro Ala Pro Ala Ala Pro Ala Ala Pro Ala
      115                 120                 125

Pro Ala Ala Pro Ala Ala Pro Ala Pro Ala Ala Pro Ala Ala Pro Ala
    130                 135                 140

Pro Ala Ala Pro Ala Ala Pro Ala Pro Ala Ala Pro Ala Ala Pro Ala
145                 150                 155                 160

Pro Ala Ala Pro Ala Ala Pro Ala Pro Ala Ala Pro Ala Ala Pro Ala
                165                 170                 175

Pro Ala Ala Pro Ala Ala Pro Ala Pro Ala Ala Pro Ala Ala Pro Ala
            180                 185                 190

Pro Ala Ala Pro Ala Ala Pro Ala Pro Ala Ala Pro Ala Ala Pro Ala
      195                 200                 205

Pro Ala Ala Pro Ala Ala Pro Ala Pro Ala Ala Pro Ala Ala Pro Ala
    210                 215                 220

Pro Ala Ala Pro Ala Ala Pro Ala Pro Ala Ala Pro Ala Ala Pro Ala
225                 230                 235                 240

Pro Ala Ala Pro Ala Ala Pro Ala Pro Ala Ala Pro Ala Ala Pro Ala
                245                 250                 255

Pro Ala Ala Pro Ala Ala Pro Ala Pro Ala Ala Pro Ala Ala Pro Ala
            260                 265                 270

Pro Ala Ala Pro Ala Ala Pro Ala Pro Ala Ala Pro Ala Ala Pro Ala
      275                 280                 285

Pro

<210>  102
<211>  48
<212>  PRT
<213>  Artificial Sequence

<220>
<223>  EPO-PAS8

<220>
<221>  DISULFID
<222>  (6)..(15)
<223>  Disulfid bridge between Cys-6 and Cys-15

<220>

<221>  DISULFID
<222>  (34)..(43)
<223>  Disulfid bridge between Cys-34 and Cys-43

<400>  102

Gly Gly Leu Tyr Ala Cys His Met Gly Pro Met Thr Trp Val Cys Gln
1               5                   10                  15

Pro Leu Arg Gly Ala Ala Pro Ala Ala Pro Ala Pro Gly Gly Leu Tyr
            20                  25                  30

Ala Cys His Met Gly Pro Met Thr Trp Val Cys Gln Pro Leu Arg Gly
        35                  40                  45

<210>  103
<211>  62
<212>  PRT
<213>  Artificial Sequence

<220>
<223>  EPO-PAS22

<220>
<221>  DISULFID
<222>  (6)..(15)
<223>  Disulfid bridge between Cys-6 and Cys-15

<220>
<221>  DISULFID
<222>  (48)..(57)
<223>  Disulfid bridge between Cys-48 and Cys-57

<400>  103

Gly Gly Leu Tyr Ala Cys His Met Gly Pro Met Thr Trp Val Cys Gln
1               5                   10                  15

Pro Leu Arg Gly Ala Ala Pro Ala Ala Pro Ala Pro Ala Ala Pro Ala
            20                  25                  30

Ala Pro Ala Pro Ala Ala Pro Ala Ala Pro Gly Gly Leu Tyr Ala Cys
        35                  40                  45

His Met Gly Pro Met Thr Trp Val Cys Gln Pro Leu Arg Gly
    50                  55                  60

<210>  104
<211>  51
<212>  PRT
<213>  Artificial Sequence

<220>
<223>  AET-EPO-PAS8

<220>
<221>  DISULFID
<222>  (9)..(18)
<223>  Disulfid bridge between Cys-9 and Cys-18

<220>
<221>  DISULFID
<222>  (37)..(46)
<223>  Disulfid bridge between Cys-37 and Cys-46

<400>  104

Ala Glu Thr Gly Gly Leu Tyr Ala Cys His Met Gly Pro Met Thr Trp
1               5                   10                  15

Val Cys Gln Pro Leu Arg Gly Ala Ala Pro Ala Ala Pro Ala Pro Gly
            20                  25                  30

Gly Leu Tyr Ala Cys His Met Gly Pro Met Thr Trp Val Cys Gln Pro
        35                  40                  45

Leu Arg Gly
    50

<210>  105
<211>  65
<212>  PRT
<213>  Artificial Sequence

<220>
<223>  AET-EPO-PAS22

<220>
<221>  DISULFID
<222>  (9)..(18)
<223>  Disulfid bridge between Cys-9 and Cys-18

<220>
<221>  DISULFID
<222>  (51)..(60)
<223>  Disulfid bridge between Cys-51 and Cys-60

<400>  105

Ala Glu Thr Gly Gly Leu Tyr Ala Cys His Met Gly Pro Met Thr Trp
1               5                   10                  15

Val Cys Gln Pro Leu Arg Gly Ala Ala Pro Ala Ala Pro Ala Pro Ala
            20                  25                  30

Ala Pro Ala Ala Pro Ala Pro Ala Ala Pro Ala Ala Pro Gly Gly Leu
        35                  40                  45

Tyr Ala Cys His Met Gly Pro Met Thr Trp Val Cys Gln Pro Leu Arg

50                  55                  60

Gly
65

<210>  106
<211>  222
<212>  DNA
<213>  Artificial Sequence

<220>
<223>  CspAss-EPO-PAS8

<400>  106
atgaaacgca tgaaatcgct ggctgcggcg ctcaccgtcg ctggggccat gctggccgca        60

cctgtggcaa cggcaggtgg cctttacgct tgtcacatgg gtccaatgac ctgggtttgt       120

cagcccctcc gtggtgccgc tcctgccgcc cccgctcctg gtggcctgta cgcatgccat       180

atgggtccaa tgacttgggt gtgccagcca ctccgcggct aa                          222

<210>  107
<211>  73
<212>  PRT
<213>  Artificial Sequence

<220>
<223>  CspAss-EPO-PAS8

<220>
<221>  DISULFID
<222>  (31)..(40)
<223>  Disulfid bridge between Cys-31 and Cys-40

<220>
<221>  DISULFID
<222>  (59)..(68)
<223>  Disulfid bridge between Cys-59 and Cys-68

<400>  107

Met Lys Arg Met Lys Ser Leu Ala Ala Ala Leu Thr Val Ala Gly Ala
1               5                   10                  15

Met Leu Ala Ala Pro Val Ala Thr Ala Gly Gly Leu Tyr Ala Cys His
            20                  25                  30

Met Gly Pro Met Thr Trp Val Cys Gln Pro Leu Arg Gly Ala Ala Pro
        35                  40                  45

Ala Ala Pro Ala Pro Gly Gly Leu Tyr Ala Cys His Met Gly Pro Met
    50                  55                  60

Thr Trp Val Cys Gln Pro Leu Arg Gly

65                  70


<210>  108
<211>  264
<212>  DNA
<213>  Artificial Sequence

<220>
<223>  CspAss-EPO-PAS22

<400>  108
atgaaacgca tgaaatcgct ggctgcggcg ctcaccgtcg ctggggccat gctggccgca      60

cctgtggcaa cggcaggcgg cctttacgct tgtcacatgg gtccaatgac ctgggtctgt     120

caacctctcc gtggtgcagc cccggcagcc cccgctccag ccgcaccagc agccccagcc     180

ccagctgcac cagccgctcc aggcggcctc tacgcctgcc acatgggtcc tatgacctgg     240

gtgtgccaac cacttcgcgg ctaa                                            264


<210>  109
<211>  87
<212>  PRT
<213>  Artificial Sequence

<220>
<223>  CspAss-EPO-PAS22


<220>
<221>  DISULFID
<222>  (31)..(40)
<223>  Disulfid bridge between Cys-31 and Cys-40

<220>
<221>  DISULFID
<222>  (73)..(82)
<223>  Disulfid bridge between Cys-73 and Cys-82

<400>  109

Met Lys Arg Met Lys Ser Leu Ala Ala Ala Leu Thr Val Ala Gly Ala
1               5                   10                  15


Met Leu Ala Ala Pro Val Ala Thr Ala Gly Gly Leu Tyr Ala Cys His
            20                  25                  30


Met Gly Pro Met Thr Trp Val Cys Gln Pro Leu Arg Gly Ala Ala Pro
        35                  40                  45


Ala Ala Pro Ala Pro Ala Ala Pro Ala Ala Pro Ala Pro Ala Ala Pro
    50                  55                  60


Ala Ala Pro Gly Gly Leu Tyr Ala Cys His Met Gly Pro Met Thr Trp
65                  70                  75                  80

Val Cys Gln Pro Leu Arg Gly
                85

<210>  110
<211>  246
<212>  DNA
<213>  Artificial Sequence

<220>
<223>  CspBss-AET-EPO-PAS8

<400>  110
atgtttaaca accgtatccg cactgcagct ctcgctggtg caatcgcaat ctccaccgca      60

gcttccggcg tagctatccc agcattcgct gcagagaccg gtggccttta cgcttgtcac     120

atgggtccaa tgacctgggt ttgtcagccc ctccgtggtg ccgctcctgc cgcccccgct     180

cctggtggcc tgtacgcatg ccatatgggt ccaatgactt gggtgtgcca gccactccgc     240

ggctaa                                                                246

<210>  111
<211>  81
<212>  PRT
<213>  Artificial Sequence

<220>
<223>  CspBss-AET-EPO-PAS8

<220>
<221>  DISULFID
<222>  (39)..(48)
<223>  Disulfid bridge between Cys-39 and Cys-48

<220>
<221>  DISULFID
<222>  (67)..(76)
<223>  Disulfid bridge between Cys-67 and Cys-76

<400>  111

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Gly Gly Leu Tyr Ala Cys His Met Gly Pro Met Thr Trp Val Cys
        35                  40                  45

Gln Pro Leu Arg Gly Ala Ala Pro Ala Ala Pro Ala Pro Gly Gly Leu
    50                  55                  60

Tyr Ala Cys His Met Gly Pro Met Thr Trp Val Cys Gln Pro Leu Arg

65                  70                  75                  80

Gly

<210>  112
<211>  288
<212>  DNA
<213>  Artificial Sequence

<220>
<223>  CspBss-AET-EPO-PAS22

<400>  112
atgtttaaca accgtatccg cactgcagct ctcgctggtg caatcgcaat ctccaccgca      60

gcttccggcg tagctatccc agcattcgct gcagagaccg gcggccttta cgcttgtcac     120

atgggtccaa tgacctgggt ctgtcaacct ctccgtggtg cagccccggc agcccccgct     180

ccagccgcac cagcagcccc agccccagct gcaccagccg ctccaggcgg cctctacgcc     240

tgccacatgg gtcctatgac ctgggtgtgc caaccacttc gcggctaa                  288

<210>  113
<211>  95
<212>  PRT
<213>  Artificial Sequence

<220>
<223>  CspBss-AET-EPO-PAS22

<220>
<221>  DISULFID
<222>  (39)..(48)
<223>  Disulfid bridge between Cys-39 and Cys-48

<220>
<221>  DISULFID
<222>  (81)..(90)
<223>  Disulfid bridge between Cys-81 and Cys-90

<400>  113

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Gly Gly Leu Tyr Ala Cys His Met Gly Pro Met Thr Trp Val Cys
        35                  40                  45

Gln Pro Leu Arg Gly Ala Ala Pro Ala Ala Pro Ala Pro Ala Ala Pro
    50                  55                  60

Ala Ala Pro Ala Pro Ala Ala Pro Ala Ala Pro Gly Gly Leu Tyr Ala
65                  70                  75                  80

Cys His Met Gly Pro Met Thr Trp Val Cys Gln Pro Leu Arg Gly
                85                  90                  95

<210>  114
<211>  44
<212>  PRT
<213>  Artificial Sequence

<220>
<223>  TPO2-PAS8

<220>
<221>  DISULFID
<222>  (3)..(16)
<223>  Disulfid bridge between Cys-3 and Cys-16

<220>
<221>  DISULFID
<222>  (29)..(42)
<223>  Disulfid bridge between Cys-29 and Cys-42

<400>  114

Gly Gly Cys Ala Asp Gly Pro Thr Leu Arg Glu Trp Ile Ser Phe Cys
1               5                   10                  15

Gly Gly Ala Ala Pro Ala Ala Pro Ala Pro Gly Gly Cys Ala Asp Gly
            20                  25                  30

Pro Thr Leu Arg Glu Trp Ile Ser Phe Cys Gly Gly
        35                  40

<210>  115
<211>  39
<212>  PRT
<213>  Artificial Sequence

<220>
<223>  AET-TPO1-PAS8

<400>  115

Ala Glu Thr Ile Glu Gly Pro Thr Leu Arg Gln Trp Leu Ala Ala Arg
1               5                   10                  15

Ala Ala Ala Pro Ala Ala Pro Ala Pro Ile Glu Gly Pro Thr Leu Arg
            20                  25                  30

Gln Trp Leu Ala Ala Arg Ala
        35

<210>  116
<211>  47
<212>  PRT
<213>  Artificial Sequence

<220>
<223>  AET-TPO2-PAS8

<220>
<221>  DISULFID
<222>  (6)..(19)
<223>  Disulfid bridge between Cys-6 and Cys-19

<220>
<221>  DISULFID
<222>  (32)..(45)
<223>  Disulfid bridge between Cys-32 and Cys-45

<400>  116

Ala Glu Thr Gly Gly Cys Ala Asp Gly Pro Thr Leu Arg Glu Trp Ile
1               5                   10                  15

Ser Phe Cys Gly Gly Ala Ala Pro Ala Ala Pro Ala Pro Gly Gly Cys
            20                  25                  30

Ala Asp Gly Pro Thr Leu Arg Glu Trp Ile Ser Phe Cys Gly Gly
        35                  40                  45

<210>  117
<211>  210
<212>  DNA
<213>  Artificial Sequence

<220>
<223>  CspAss-TPO2-PAS8

<400>  117
atgaaacgca tgaaatcgct ggctgcggcg ctcaccgtcg ctggggccat gctggccgca      60

cctgtggcaa cggcaggtgg ttgtgctgat ggtcctacct tgcgtgaatg gatttccttc     120

tgcggcggcg ctgctccggc tgcccctgcc cctggcggtt gcgcagatgg cccaaccctg     180

cgcgaatgga tctccttctg cggcggctaa                                      210

<210>  118
<211>  69
<212>  PRT
<213>  Artificial Sequence

<220>
<223>  CspAss-TPO2-PAS8

<220>
<221>  DISULFID

<222>  (28)..(41)
<223>  Disulfid bridge between Cys-28 and Cys-41

<220>
<221>  DISULFID
<222>  (54)..(67)
<223>  Disulfid bridge between Cys-54 and Cys-67

<400>  118

Met Lys Arg Met Lys Ser Leu Ala Ala Ala Leu Thr Val Ala Gly Ala
1               5                   10                  15

Met Leu Ala Ala Pro Val Ala Thr Ala Gly Gly Cys Ala Asp Gly Pro
            20                  25                  30

Thr Leu Arg Glu Trp Ile Ser Phe Cys Gly Gly Ala Ala Pro Ala Ala
        35                  40                  45

Pro Ala Pro Gly Gly Cys Ala Asp Gly Pro Thr Leu Arg Glu Trp Ile
    50                  55                  60

Ser Phe Cys Gly Gly
65

<210>  119
<211>  210
<212>  DNA
<213>  Artificial Sequence

<220>
<223>  CspBss-AET-TPO1-PAS8

<400>  119
atgtttaaca accgtatccg cactgcagct ctcgctggtg caatcgcaat ctccaccgca      60

gcttccggcg tagctatccc agcattcgct gcagagacca tcgaaggtcc taccctccgt     120

caatggctgg ctgctcgtgc cgccgctccc gccgctcctg ctccaatcga aggtcccacc     180

ctccgtcagt ggctggcagc ccgcgcataa                                      210

<210>  120
<211>  69
<212>  PRT
<213>  Artificial Sequence

<220>
<223>  CspBss-AET-TPO1-PAS8

<400>  120

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu

20                  25                  30

Thr Ile Glu Gly Pro Thr Leu Arg Gln Trp Leu Ala Ala Arg Ala Ala
        35                  40                  45

Ala Pro Ala Ala Pro Ala Pro Ile Glu Gly Pro Thr Leu Arg Gln Trp
    50                  55                  60

Leu Ala Ala Arg Ala
65

<210>  121
<211>  234
<212>  DNA
<213>  Artificial Sequence

<220>
<223>  CspBss-AET-TPO2-PAS8

<400>  121
atgtttaaca accgtatccg cactgcagct ctcgctggtg caatcgcaat ctccaccgca        60

gcttccggcg tagctatccc agcattcgct gcagagaccg gtggttgtgc tgatggtcct       120

accttgcgtg aatggatttc cttctgcggc ggcgctgctc cggctgcccc tgcccctggc       180

ggttgcgcag atggcccaac cctgcgcgaa tggatctcct tctgcggcgg ctaa             234

<210>  122
<211>  77
<212>  PRT
<213>  Artificial Sequence

<220>
<223>  CspBss-AET-TPO2-PAS8

<220>
<221>  DISULFID
<222>  (36)..(49)
<223>  Disulfid bridge between Cys-36 and Cys-49

<220>
<221>  DISULFID
<222>  (62)..(75)
<223>  Disulfid bridge between Cys-62 and Cys-75

<400>  122

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Gly Gly Cys Ala Asp Gly Pro Thr Leu Arg Glu Trp Ile Ser Phe

35                  40                  45

Cys Gly Gly Ala Ala Pro Ala Ala Pro Ala Pro Gly Gly Cys Ala Asp
    50                  55                  60

Gly Pro Thr Leu Arg Glu Trp Ile Ser Phe Cys Gly Gly
65                  70                  75

<210>  123
<211>  54
<212>  PRT
<213>  Artificial Sequence

<220>
<223>  AET-VEGFR_D2-PAS16

<220>
<221>  DISULFID
<222>  (9)..(16)
<223>  Disulfid bridge between Cys-9 and Cys-16

<220>
<221>  DISULFID
<222>  (39)..(49)
<223>  Disulfid bridge between Cys-39 and Cys-49

<400>  123

Ala Glu Thr Ala Gly Pro Thr Trp Cys Glu Asp Asp Trp Tyr Tyr Cys
1               5                   10                  15

Trp Leu Phe Gly Thr Ala Ala Pro Ala Ala Pro Ala Pro Ala Ala Pro
            20                  25                  30

Ala Ala Pro Ala Pro Val Cys Trp Glu Asp Ser Trp Gly Gly Glu Val
        35                  40                  45

Cys Trp Leu Phe Gly Thr
    50

<210>  124
<211>  255
<212>  DNA
<213>  Artificial Sequence

<220>
<223>  CspBss-AET-VEGFR_D2-PAS16

<400>  124
atgtttaaca accgtatccg cactgcagct ctcgctggtg caatcgcaat ctccaccgca      60

gcttccggcg tagctatccc agcattcgct gcagagaccg ctggtcctac ctggtgtgaa      120

gacgactggt actactgctg gctgtttggc accgctgctc ccgctgcccc tgctcctgcc      180

gctccggcag ccccagctcc tgtgtgttgg gaagattctt ggggcggcga agtttgctgg     240

ctgtttggca cctaa                                                      255

<210>  125
<211>  84
<212>  PRT
<213>  Artificial Sequence

<220>
<223>  CspBss-AET-VEGFR_D2-PAS16

<220>
<221>  DISULFID
<222>  (39)..(46)
<223>  Disulfid bridge between Cys-39 and Cys-46

<220>
<221>  DISULFID
<222>  (69)..(79)
<223>  Disulfid bridge between Cys-69 and Cys-79

<400>  125

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Ala Gly Pro Thr Trp Cys Glu Asp Asp Trp Tyr Tyr Cys Trp Leu
        35                  40                  45

Phe Gly Thr Ala Ala Pro Ala Ala Pro Ala Pro Ala Ala Pro Ala Ala
    50                  55                  60

Pro Ala Pro Val Cys Trp Glu Asp Ser Trp Gly Gly Glu Val Cys Trp
65                  70                  75                  80

Leu Phe Gly Thr

<210>  126
<211>  92
<212>  PRT
<213>  Artificial Sequence

<220>
<223>  AET-EPO-PAS49

<220>
<221>  DISULFID
<222>  (9)..(18)
<223>  Disulfid bridge between Cys-9 and Cys-18

<220>
<221>  DISULFID
<222>  (78)..(87)
<223>  Disulfid bridge between Cys-78 and Cys-87

<400>  126

Ala Glu Thr Gly Gly Leu Tyr Ala Cys His Met Gly Pro Met Thr Trp
1               5                   10                  15

Val Cys Gln Pro Leu Arg Gly Ala Ala Pro Ala Ala Pro Ala Pro Ala
            20                  25                  30

Ala Pro Ala Ala Pro Ala Pro Ala Ala Pro Ala Ala Pro Ala Pro Ala
        35                  40                  45

Ala Pro Ala Ala Pro Ala Pro Ala Ala Pro Ala Ala Pro Ala Pro Ala
    50                  55                  60

Ala Pro Ala Ala Pro Ala Pro Ala Gly Gly Leu Tyr Ala Cys His Met
65                  70                  75                  80

Gly Pro Met Thr Trp Val Cys Gln Pro Leu Arg Gly
                85                  90


<210>  127
<211>  92
<212>  PRT
<213>  Artificial Sequence

<220>
<223>  AET-EPO-PAS49-b


<220>
<221>  DISULFID
<222>  (9)..(18)
<223>  Disulfid bridge between Cys-9 and Cys-18

<220>
<221>  DISULFID
<222>  (78)..(87)
<223>  Disulfid bridge between Cys-78 and Cys-87

<400>  127

Ala Glu Thr Gly Gly Leu Tyr Ala Cys His Met Gly Pro Met Thr Trp
1               5                   10                  15

Val Cys Gln Pro Leu Arg Gly Ser Ala Pro Ser Pro Ser Ser Ala Pro
            20                  25                  30

Ala Ser Ala Ser Ala Pro Ala Ser Pro Ala Ser Ala Ser Ala Ser Ala
        35                  40                  45

Ser Pro Ala Ser Ser Pro Ala Ser Ala Ser Ser Ala Ser Pro Ala Ala
    50                  55                  60

Ser Ser Ala Ser Pro Ala Ser Ser Gly Gly Leu Tyr Ala Cys His Met
65                  70                  75                  80

Gly Pro Met Thr Trp Val Cys Gln Pro Leu Arg Gly
                85                  90

<210>  128
<211>  143
<212>  PRT
<213>  Artificial Sequence

<220>
<223>  AET-EPO-PAS100

<220>
<221>  DISULFID
<222>  (9)..(18)
<223>  Disulfid bridge between Cys-9 and Cys-18

<220>
<221>  DISULFID
<222>  (129)..(138)
<223>  Disulfid bridge between Cys-129 and Cys-138

<400>  128

Ala Glu Thr Gly Gly Leu Tyr Ala Cys His Met Gly Pro Met Thr Trp
1               5                   10                  15

Val Cys Gln Pro Leu Arg Gly Ala Ala Pro Ala Ala Pro Ala Pro Ala
            20                  25                  30

Ala Pro Ala Ala Pro Ala Pro Ala Ala Pro Ala Ala Pro Ala Pro Ala
        35                  40                  45

Ala Pro Ala Ala Pro Ala Pro Ala Ala Pro Ala Ala Pro Ala Pro Ala
    50                  55                  60

Ala Pro Ala Ala Pro Ala Pro Ala Ala Pro Ala Ala Pro Ala Pro Ala
65                  70                  75                  80

Ala Pro Ala Ala Pro Ala Pro Ala Ala Pro Ala Ala Pro Ala Pro Ala
                85                  90                  95

Ala Pro Ala Ala Pro Ala Pro Ala Ala Pro Ala Ala Pro Ala Pro Ala
            100                 105                 110

Ala Pro Ala Ala Pro Ala Pro Ala Ala Pro Ala Gly Gly Leu Tyr Ala
        115                 120                 125

Cys His Met Gly Pro Met Thr Trp Val Cys Gln Pro Leu Arg Gly
    130                 135                 140

<210>  129
<211>  143
<212>  PRT
<213>  Artificial Sequence

<220>
<223>  AET-EPO-PAS100-b

<220>
<221>  DISULFID
<222>  (9)..(18)
<223>  Disulfid bridge between Cys-9 and Cys-18

<220>
<221>  DISULFID
<222>  (129)..(138)
<223>  Disulfid bridge between Cys-129 and Cys-138

<400>  129

Ala Glu Thr Gly Gly Leu Tyr Ala Cys His Met Gly Pro Met Thr Trp
1               5                   10                  15

Val Cys Gln Pro Leu Arg Gly Ser Ala Pro Ser Pro Ser Ser Ala Pro
            20                  25                  30

Ala Ser Ala Ser Ala Pro Ala Ser Pro Ala Ser Ala Ser Ala Ser Ala
        35                  40                  45

Ser Pro Ala Ser Ser Pro Ala Ser Ala Ser Ser Ala Ser Pro Ala Ala
    50                  55                  60

Ser Ser Ala Ser Pro Ala Ser Ser Ser Ala Ser Pro Ser Ala Pro Ala
65                  70                  75                  80

Ala Pro Ser Ala Ser Ala Ala Pro Ser Ala Ser Pro Ala Ser Ser Ser
                85                  90                  95

Pro Ala Pro Ala Ser Ala Pro Ser Pro Ala Ala Ala Ala Ser Ser Pro
            100                 105                 110

Ser Pro Ala Pro Ser Ser Ala Ser Ser Ala Ala Gly Gly Leu Tyr Ala
        115                 120                 125

Cys His Met Gly Pro Met Thr Trp Val Cys Gln Pro Leu Arg Gly
    130                 135                 140

<210>  130
<211>  51
<212>  PRT
<213>  Artificial Sequence

<220>
<223>  AET-EPO-GS8

<220>
<221>  DISULFID
<222>  (9)..(18)
<223>  Disulfid bridge between Cys-9 and Cys-18

<220>
<221>  DISULFID
<222>  (37)..(46)
<223>  Disulfid bridge between Cys-37 and Cys-46

<400>  130

Ala Glu Thr Gly Gly Leu Tyr Ala Cys His Met Gly Pro Met Thr Trp
1               5                   10                  15

Val Cys Gln Pro Leu Arg Gly Gly Gly Gly Gly Ser Gly Gly Gly Gly
            20                  25                  30

Gly Leu Tyr Ala Cys His Met Gly Pro Met Thr Trp Val Cys Gln Pro
        35                  40                  45

Leu Arg Gly
    50

<210>  131
<211>  65
<212>  PRT
<213>  Artificial Sequence

<220>
<223>  AET-EPO-GS22

<220>
<221>  DISULFID
<222>  (9)..(18)
<223>  Disulfid bridge between Cys-9 and Cys-18

<220>
<221>  DISULFID
<222>  (51)..(60)
<223>  Disulfid bridge between Cys-51 and Cys-60

<400>  131

Ala Glu Thr Gly Gly Leu Tyr Ala Cys His Met Gly Pro Met Thr Trp
1               5                   10                  15

Val Cys Gln Pro Leu Arg Gly Gly Gly Gly Gly Ser Gly Gly Gly Gly
            20                  25                  30

Ser Gly Gly Gly Gly Ser Gly Gly Gly Gly Ser Gly Gly Gly Gly Leu
        35                  40                  45

Tyr Ala Cys His Met Gly Pro Met Thr Trp Val Cys Gln Pro Leu Arg
    50                  55                  60

Gly
65

<210>  132
<211>  369
<212>  DNA
<213>  Artificial Sequence

<220>
<223>  CspBss-AET-EPO-PAS49

<400>  132
atgtttaaca accgtatccg cactgcagct ctcgctggtg caatcgcaat ctccaccgca      60

gcttccggcg tagctatccc agcattcgct gcagagaccg gtggtctcta cgcctgtcac     120

atgggtccta tgacctgggt gtgccaacct ctccgtggtg ccgcccccgc cgcccccgcc     180

cccgccgccc cagctgcacc agctccagca gccccagccg caccagcccc agcagctcca     240

gctgccccag caccagcagc accagccgct ccagcaccag ctgctccagc agctccagcc     300

ccagccggcg gcctttacgc ctgccacatg ggtcctatga cctgggtttg ccagcctctt     360

cgcggttag                                                             369

<210>  133
<211>  122
<212>  PRT
<213>  Artificial Sequence

<220>
<223>  CspBss-AET-EPO-PAS49


<220>
<221>  DISULFID
<222>  (39)..(48)
<223>  Disulfid bridge between Cys-39 and Cys-48

<220>
<221>  DISULFID
<222>  (108)..(117)
<223>  Disulfid bridge between Cys-108 and Cys-117

<400>  133

Met Phe Asn Asn Arg Ile Arg Thr Ala Ala Leu Ala Gly Ala Ile Ala

1               5                   10                  15

Ile Ser Thr Ala Ala Ser Gly Val Ala Ile Pro Ala Phe Ala Ala Glu
            20                  25                  30

Thr Gly Gly Leu Tyr Ala Cys His Met Gly Pro Met Thr Trp Val Cys
        35                  40                  45

Gln Pro Leu Arg Gly Ala Ala Pro Ala Ala Pro Ala Pro Ala Ala Pro
    50                  55                  60

Ala Ala Pro Ala Pro Ala Ala Pro Ala Ala Pro Ala Pro Ala Ala Pro
65                  70                  75                  80

Ala Ala Pro Ala Pro Ala Ala Pro Ala Ala Pro Ala Pro Ala Ala Pro
                85                  90                  95

Ala Ala Pro Ala Pro Ala Gly Gly Leu Tyr Ala Cys His Met Gly Pro
            100                 105                 110

Met Thr Trp Val Cys Gln Pro Leu Arg Gly
        115                 120

<210>  134
<211>  369
<212>  DNA
<213>  Artificial Sequence

<220>
<223>  CspBss-AET-EPO-PAS49-b

<400>  134
atgtttaaca accgtatccg cactgcagct ctcgctggtg caatcgcaat ctccaccgca      60

gcttccggcg tagctatccc agcattcgct gcagagaccg gcggactgta cgcctgccat     120

atggggccaa tgacctgggt gtgccaaccg ttgcgtggat cagcgccatc gccctcttcc     180

gctccagcct ctgcgtctgc tcccgcatcc ccagcgtcag cttccgcatc cgcttcgcct     240

gcaagctccc ctgctagcgc gtcctctgcc tcccctgcag catcctcggc ttccccggca     300

agctcaggtg gcctctatgc ctgccacatg ggtccgatga cttgggtctg tcagccactt     360

cgcggctaa                                                             369

<210>  135
<211>  122
<212>  PRT
<213>  Artificial Sequence

<220>
<223>  CspBss-AET-EPO-PAS49-b

<220>
<221>  DISULFID
<222>  (39)..(48)
<223>  Disulfid bridge between Cys-39 and Cys-48

<220>
<221>  DISULFID
<222>  (108)..(117)
<223>  Disulfid bridge between Cys-108 and Cys-117

<400>  135

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Gly Gly Leu Tyr Ala Cys His Met Gly Pro Met Thr Trp Val Cys
        35                  40                  45

Gln Pro Leu Arg Gly Ser Ala Pro Ser Pro Ser Ser Ala Pro Ala Ser
    50                  55                  60

Ala Ser Ala Pro Ala Ser Pro Ala Ser Ala Ser Ala Ser Ala Ser Pro
65                  70                  75                  80

Ala Ser Ser Pro Ala Ser Ala Ser Ser Ala Ser Pro Ala Ala Ser Ser
                85                  90                  95

Ala Ser Pro Ala Ser Ser Gly Gly Leu Tyr Ala Cys His Met Gly Pro
            100                 105                 110

Met Thr Trp Val Cys Gln Pro Leu Arg Gly
        115                 120

<210>  136
<211>  522
<212>  DNA
<213>  Artificial Sequence

<220>
<223>  CspBss-AET-EPO-PAS100

<400>  136
atgtttaaca accgtatccg cactgcagct ctcgctggtg caatcgcaat ctccaccgca      60

gcttccggcg tagctatccc agcattcgct gcagagaccg gtggtttgta cgcttgccac     120

atgggtccta tgacctgggt ttgtcagcct cttcgtggtg ccgcccccgc agcccccgct     180

cccgctgcac cagccgcacc agcaccagca gctccagcag ctccagcacc agctgcacca     240

gccgccccag caccagcagc cccagccgct ccagcaccag ctgccccagc tgctccagct     300

ccagcagcac cagctgcccc agccccagca gcaccagccg ctccagcccc agctgcccca 360

gcagctccag ctccagctgc tccagcagcc ccagcaccag ccgccccagc agccccagct 420

ccagctgcac cagctgcacc agccccagcc gcaccagccg gcggtttgta cgcttgccac 480

atgggtccga tgacctgggt ttgtcagccc cttcgcggtt ag 522

<210> 137
<211> 173
<212> PRT
<213> Artificial Sequence

<220>
<223> CspBss-AET-EPO-PAS100

<220>
<221> DISULFID
<222> (39)..(48)
<223> Disulfid bridge between Cys-39 and Cys-48

<220>
<221> DISULFID
<222> (159)..(168)
<223> Disulfid bridge between Cys-159 and Cys-168

<400> 137

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Gly Gly Leu Tyr Ala Cys His Met Gly Pro Met Thr Trp Val Cys
        35                  40                  45

Gln Pro Leu Arg Gly Ala Ala Pro Ala Ala Pro Ala Pro Ala Ala Pro
    50                  55                  60

Ala Ala Pro Ala Pro Ala Ala Pro Ala Ala Pro Ala Pro Ala Ala Pro
65                  70                  75                  80

Ala Ala Pro Ala Pro Ala Ala Pro Ala Ala Pro Ala Pro Ala Ala Pro
                85                  90                  95

Ala Ala Pro Ala Pro Ala Ala Pro Ala Ala Pro Ala Pro Ala Ala Pro
            100                 105                 110

Ala Ala Pro Ala Pro Ala Ala Pro Ala Ala Pro Ala Pro Ala Ala Pro
        115                 120                 125

Ala Ala Pro Ala Pro Ala Ala Pro Ala Ala Pro Ala Pro Ala Ala Pro
    130                 135                 140

Ala Ala Pro Ala Pro Ala Ala Pro Ala Gly Gly Leu Tyr Ala Cys His
145                 150                 155                 160

Met Gly Pro Met Thr Trp Val Cys Gln Pro Leu Arg Gly
                165                 170

<210>  138
<211>  522
<212>  DNA
<213>  Artificial Sequence

<220>
<223>  CspBss-AET-EPO-PAS100-b

<400>  138
atgtttaaca accgtatccg cactgcagct ctcgctggtg caatcgcaat ctccaccgca      60

gcttccggcg tagctatccc agcattcgct gcagagaccg gcggcttgta cgcctgccat     120

atggggccta tgacctgggt ctgccaacca ctgcgtggaa gcgcaccctc accatcctcc     180

gcaccagcta gcgcctccgc tcctgcaagc ccagcgtccg catccgcttc ggcctcacca     240

gcgtcctctc cagcttccgc ttcgagcgct agcccagcgg catcctctgc ctccccagcc     300

tcctcgtctg catccccgtc cgctccagcc gcaccttctg catctgcggc tccgtccgcc     360

tccccggcat cttcctcgcc tgctcccgcg tcagcgcctt ccccggctgc agcggcctcc     420

tctccgtcac ccgctccttc aagcgcgtcg tctgcagcag gaggcctcta tgcctgccac     480

atgggtccga tgacttgggt gtgtcagccc cttcgcggtt aa                        522

<210>  139
<211>  173
<212>  PRT
<213>  Artificial Sequence

<220>
<223>  CspBss-AET-EPO-PAS100-b


<220>
<221>  DISULFID
<222>  (39)..(48)
<223>  Disulfid bridge between Cys-39 and Cys-48

<220>
<221>  DISULFID
<222>  (159)..(168)
<223>  Disulfid bridge between Cys-159 and Cys-168

<400>  139

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Gly Gly Leu Tyr Ala Cys His Met Gly Pro Met Thr Trp Val Cys
        35                  40                  45

Gln Pro Leu Arg Gly Ser Ala Pro Ser Pro Ser Ser Ala Pro Ala Ser
    50                  55                  60

Ala Ser Ala Pro Ala Ser Pro Ala Ser Ala Ser Ala Ser Ala Ser Pro
65                  70                  75                  80

Ala Ser Ser Pro Ala Ser Ala Ser Ser Ala Ser Pro Ala Ala Ser Ser
                85                  90                  95

Ala Ser Pro Ala Ser Ser Ser Ala Ser Pro Ser Ala Pro Ala Ala Pro
            100                 105                 110

Ser Ala Ser Ala Ala Pro Ser Ala Ser Pro Ala Ser Ser Ser Pro Ala
        115                 120                 125

Pro Ala Ser Ala Pro Ser Pro Ala Ala Ala Ala Ser Ser Pro Ser Pro
    130                 135                 140

Ala Pro Ser Ser Ala Ser Ser Ala Ala Gly Gly Leu Tyr Ala Cys His
145                 150                 155                 160

Met Gly Pro Met Thr Trp Val Cys Gln Pro Leu Arg Gly
                165                 170

<210>  140
<211>  246
<212>  DNA
<213>  Artificial Sequence

<220>
<223>  CspBss-AET-EPO-GS8

<400>  140
atgtttaaca accgtatccg cactgcagct ctcgctggtg caatcgcaat ctccaccgca      60

gcttccggcg tagctatccc agcattcgct gcagagaccg gcggcctcta tgcatgccat     120

atggggccaa tgacctgggt ctgccaaccg ttgcgtggcg gaggtggcgg ttccggtgga     180

ggtggtggcc tgtacgcttg ccacatggga cccatgactt gggtgtgtca gcctcttcgc     240

ggctaa                                                                246

<210>  141

<211>  81
<212>  PRT
<213>  Artificial Sequence

<220>
<223>  CspBss-AET-EPO-GS8


<220>
<221>  DISULFID
<222>  (39)..(48)
<223>  Disulfid bridge between Cys-39 and Cys-48

<220>
<221>  DISULFID
<222>  (67)..(76)
<223>  Disulfid bridge between Cys-67 and Cys-76

<400>  141

Met Phe Asn Asn Arg Ile Arg Thr Ala Ala Leu Ala Gly Ala Ile Ala
1               5                   10                  15


Ile Ser Thr Ala Ala Ser Gly Val Ala Ile Pro Ala Phe Ala Ala Glu
            20                  25                  30


Thr Gly Gly Leu Tyr Ala Cys His Met Gly Pro Met Thr Trp Val Cys
        35                  40                  45


Gln Pro Leu Arg Gly Gly Gly Gly Gly Ser Gly Gly Gly Gly Gly Leu
    50                  55                  60


Tyr Ala Cys His Met Gly Pro Met Thr Trp Val Cys Gln Pro Leu Arg
65                  70                  75                  80


Gly




<210>  142
<211>  288
<212>  DNA
<213>  Artificial Sequence

<220>
<223>  CspBss-AET-EPO-GS22

<400>  142
atgtttaaca accgtatccg cactgcagct ctcgctggtg caatcgcaat ctccaccgca      60

gcttccggcg tagctatccc agcattcgct gcagagaccg gtgggctgta tgcttgccat     120

atggggccta tgacttgggt gtgccaaccc ttgcgtggtg gaggcggagg ctccggcggt     180

ggtgggagcg gaggaggcgg ttccggtggc ggtggctctg gcggaggcgg cctctacgca     240

tgccacatgg gtccgatgac ctgggtctgt cagccacttc gcggctaa                  288

<210>  143
<211>  95
<212>  PRT
<213>  Artificial Sequence

<220>
<223>  CspBss-AET-EPO-GS22

<220>
<221>  DISULFID
<222>  (39)..(48)
<223>  Disulfid bridge between Cys-39 and Cys-48

<220>
<221>  DISULFID
<222>  (81)..(90)
<223>  Disulfid bridge between Cys-81 and Cys-90

<400>  143

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Gly Gly Leu Tyr Ala Cys His Met Gly Pro Met Thr Trp Val Cys
        35                  40                  45

Gln Pro Leu Arg Gly Gly Gly Gly Gly Ser Gly Gly Gly Gly Ser Gly
    50                  55                  60

Gly Gly Gly Ser Gly Gly Gly Gly Ser Gly Gly Gly Gly Leu Tyr Ala
65                  70                  75                  80

Cys His Met Gly Pro Met Thr Trp Val Cys Gln Pro Leu Arg Gly
                85                  90                  95

<210>  144
<211>  265
<212>  PRT
<213>  Artificial Sequence

<220>
<223>  AET-EPO-PAS22-PAS200

<220>
<221>  DISULFID
<222>  (9)..(18)
<223>  Disulfid bridge between Cys-9 and Cys-18

<220>
<221>  DISULFID
<222>  (51)..(60)

<223>  Disulfid bridge between Cys-51 and Cys-60

<400>  144

Ala Glu Thr Gly Gly Leu Tyr Ala Cys His Met Gly Pro Met Thr Trp
1               5                   10                  15

Val Cys Gln Pro Leu Arg Gly Ala Ala Pro Ala Ala Pro Ala Pro Ala
            20                  25                  30

Ala Pro Ala Ala Pro Ala Pro Ala Ala Pro Ala Ala Pro Gly Gly Leu
        35                  40                  45

Tyr Ala Cys His Met Gly Pro Met Thr Trp Val Cys Gln Pro Leu Arg
    50                  55                  60

Gly Ala Ala Pro Ala Ala Pro Ala Pro Ala Ala Pro Ala Ala Pro Ala
65                  70                  75                  80

Pro Ala Ala Pro Ala Ala Pro Ala Pro Ala Ala Pro Ala Ala Pro Ala
                85                  90                  95

Pro Ala Ala Pro Ala Ala Pro Ala Pro Ala Ala Pro Ala Ala Pro Ala
            100                 105                 110

Pro Ala Ala Pro Ala Ala Pro Ala Pro Ala Ala Pro Ala Ala Pro Ala
        115                 120                 125

Pro Ala Ala Pro Ala Ala Pro Ala Pro Ala Ala Pro Ala Ala Pro Ala
    130                 135                 140

Pro Ala Ala Pro Ala Ala Pro Ala Pro Ala Ala Pro Ala Ala Pro Ala
145                 150                 155                 160

Pro Ala Ala Pro Ala Ala Pro Ala Pro Ala Ala Pro Ala Ala Pro Ala
                165                 170                 175

Pro Ala Ala Pro Ala Ala Pro Ala Pro Ala Ala Pro Ala Ala Pro Ala
            180                 185                 190

Pro Ala Ala Pro Ala Ala Pro Ala Pro Ala Ala Pro Ala Ala Pro Ala
        195                 200                 205

Pro Ala Ala Pro Ala Ala Pro Ala Pro Ala Ala Pro Ala Ala Pro Ala
    210                 215                 220

Pro Ala Ala Pro Ala Ala Pro Ala Pro Ala Ala Pro Ala Ala Pro Ala
225                 230                 235                 240

Pro Ala Ala Pro Ala Ala Pro Ala Pro Ala Ala Pro Ala Ala Pro Ala
                245                 250                 255

Pro Ala Ala Pro Ala Ala Pro Ala Pro
            260                 265

<210>  145
<211>  665
<212>  PRT
<213>  Artificial Sequence

<220>
<223>  AET-EPO-PAS22-PAS600

<220>
<221>  DISULFID
<222>  (9)..(18)
<223>  Disulfid bridge between Cys-9 and Cys-18

<220>
<221>  DISULFID
<222>  (51)..(60)
<223>  Disulfid bridge between Cys-51 and Cys-60

<400>  145

Ala Glu Thr Gly Gly Leu Tyr Ala Cys His Met Gly Pro Met Thr Trp
1               5                   10                  15

Val Cys Gln Pro Leu Arg Gly Ala Ala Pro Ala Ala Pro Ala Pro Ala
            20                  25                  30

Ala Pro Ala Ala Pro Ala Pro Ala Ala Pro Ala Ala Pro Gly Gly Leu
        35                  40                  45

Tyr Ala Cys His Met Gly Pro Met Thr Trp Val Cys Gln Pro Leu Arg
    50                  55                  60

Gly Ala Ala Pro Ala Ala Pro Ala Pro Ala Ala Pro Ala Ala Pro Ala
65                  70                  75                  80

Pro Ala Ala Pro Ala Ala Pro Ala Pro Ala Ala Pro Ala Ala Pro Ala
                85                  90                  95

Pro Ala Ala Pro Ala Ala Pro Ala Pro Ala Ala Pro Ala Ala Pro Ala
            100                 105                 110

Pro Ala Ala Pro Ala Ala Pro Ala Pro Ala Ala Pro Ala Ala Pro Ala
        115                 120                 125

Pro Ala Ala Pro Ala Ala Pro Ala Pro Ala Ala Pro Ala Ala Pro Ala

    130                 135                 140

Pro Ala Ala Pro Ala Ala Pro Ala Pro Ala Ala Pro Ala Ala Pro Ala
145                 150                 155                 160

Pro Ala Ala Pro Ala Ala Pro Ala Pro Ala Ala Pro Ala Ala Pro Ala
                165                 170                 175

Pro Ala Ala Pro Ala Ala Pro Ala Pro Ala Ala Pro Ala Ala Pro Ala
            180                 185                 190

Pro Ala Ala Pro Ala Ala Pro Ala Pro Ala Ala Pro Ala Ala Pro Ala
        195                 200                 205

Pro Ala Ala Pro Ala Ala Pro Ala Pro Ala Ala Pro Ala Ala Pro Ala
    210                 215                 220

Pro Ala Ala Pro Ala Ala Pro Ala Pro Ala Ala Pro Ala Ala Pro Ala
225                 230                 235                 240

Pro Ala Ala Pro Ala Ala Pro Ala Pro Ala Ala Pro Ala Ala Pro Ala
                245                 250                 255

Pro Ala Ala Pro Ala Ala Pro Ala Pro Ala Ala Pro Ala Ala Pro Ala
            260                 265                 270

Pro Ala Ala Pro Ala Ala Pro Ala Pro Ala Ala Pro Ala Ala Pro Ala
        275                 280                 285

Pro Ala Ala Pro Ala Ala Pro Ala Pro Ala Ala Pro Ala Ala Pro Ala
    290                 295                 300

Pro Ala Ala Pro Ala Ala Pro Ala Pro Ala Ala Pro Ala Ala Pro Ala
305                 310                 315                 320

Pro Ala Ala Pro Ala Ala Pro Ala Pro Ala Ala Pro Ala Ala Pro Ala
                325                 330                 335

Pro Ala Ala Pro Ala Ala Pro Ala Pro Ala Ala Pro Ala Ala Pro Ala
            340                 345                 350

Pro Ala Ala Pro Ala Ala Pro Ala Pro Ala Ala Pro Ala Ala Pro Ala
        355                 360                 365

Pro Ala Ala Pro Ala Ala Pro Ala Pro Ala Ala Pro Ala Ala Pro Ala
    370                 375                 380

Pro Ala Ala Pro Ala Ala Pro Ala Pro Ala Ala Pro Ala Ala Pro Ala
385                 390                 395                 400

Pro Ala Ala Pro Ala Ala Pro Ala Pro Ala Ala Pro Ala Ala Pro Ala
                405                 410                 415

Pro Ala Ala Pro Ala Ala Pro Ala Pro Ala Ala Pro Ala Ala Pro Ala
            420                 425                 430

Pro Ala Ala Pro Ala Ala Pro Ala Pro Ala Ala Pro Ala Ala Pro Ala
        435                 440                 445

Pro Ala Ala Pro Ala Ala Pro Ala Pro Ala Ala Pro Ala Ala Pro Ala
    450                 455                 460

Pro Ala Ala Pro Ala Ala Pro Ala Pro Ala Ala Pro Ala Ala Pro Ala
465                 470                 475                 480

Pro Ala Ala Pro Ala Ala Pro Ala Pro Ala Ala Pro Ala Ala Pro Ala
                485                 490                 495

Pro Ala Ala Pro Ala Ala Pro Ala Pro Ala Ala Pro Ala Ala Pro Ala
            500                 505                 510

Pro Ala Ala Pro Ala Ala Pro Ala Pro Ala Ala Pro Ala Ala Pro Ala
        515                 520                 525

Pro Ala Ala Pro Ala Ala Pro Ala Pro Ala Ala Pro Ala Ala Pro Ala
    530                 535                 540

Pro Ala Ala Pro Ala Ala Pro Ala Pro Ala Ala Pro Ala Ala Pro Ala
545                 550                 555                 560

Pro Ala Ala Pro Ala Ala Pro Ala Pro Ala Ala Pro Ala Ala Pro Ala
                565                 570                 575

Pro Ala Ala Pro Ala Ala Pro Ala Pro Ala Ala Pro Ala Ala Pro Ala
            580                 585                 590

Pro Ala Ala Pro Ala Ala Pro Ala Pro Ala Ala Pro Ala Ala Pro Ala
        595                 600                 605

Pro Ala Ala Pro Ala Ala Pro Ala Pro Ala Ala Pro Ala Ala Pro Ala
    610                 615                 620

Pro Ala Ala Pro Ala Ala Pro Ala Pro Ala Ala Pro Ala Ala Pro Ala
625                 630                 635                 640

Pro Ala Ala Pro Ala Ala Pro Ala Pro Ala Ala Pro Ala Ala Pro Ala
                645                 650                 655

Pro Ala Ala Pro Ala Ala Pro Ala Pro
            660                 665

<210>  146
<211>  888
<212>  DNA
<213>  Artificial Sequence

<220>
<223>  CspBss-AET-EPO-PAS22-PAS200

<400>  146
   atgttcaaca accgtattcg taccgctgcc ctggccggcg ccattgccat ctccaccgct      60
   gcgtccggcg tcgccattcc tgcgttcgca gccgaaaccg gcggcctgta cgcatgccac     120
   atgggtccaa tgacctgggt gtgccagcca ctgcgcggcg ctgcaccagc tgctccagca     180
   ccagcagccc cagctgcacc agcaccagct gctccagctg caccaggcgg cctgtacgct     240
   tgtcatatgg gtccaatgac ttgggtctgc cagccactcc gcggcgctgc accagcagct     300
   cctgctccag cagcacctgc agcaccagca ccagcagctc cagcagcacc tgccccagca     360
   gctcctgcag ctcctgcccc agcagcacct gccgctcctg ctcctgcagc tccagcagca     420
   cccgccccag cagctcccgc agctcctgcg ccagcagcac ctgccgcccc tgctcccgca     480
   gctccagcag caccggcacc cgcagctcca gcagctcctg cacctgcagc acctgccgct     540
   cccgctccgg cagctccagc agcaccagcc cctgcagctc ctgctgcacc tgctccagca     600
   gcacccgcgg ctcctgctcc ggctgctcca gcagcccctg ctccagcagc tcccgctgca     660
   cctgcaccag cagcacctgc cgcgcctgct cctgctgccc ctgctgctcc tgctcctgca     720
   gcccctgctg cacccgctcc agcagcacct gcggctcctg ctccggccgc ccctgctgct     780
   ccagcaccag ctgcccctgc agcccctgcc cctgcagccc cagctgcacc agcaccagca     840
   gcaccagcag cacccgcacc agcagcccca gcagccccag ccccttag                 888

<210>  147
<211>  295
<212>  PRT
<213>  Artificial Sequence

<220>
<223>  CspBss-AET-EPO-PAS22-PAS200

<220>
<221>  DISULFID
<222>  (39)..(48)
<223>  Disulfid bridge between Cys-39 and Cys-48

<220>
<221> DISULFID
<222> (81)..(90)
<223> Disulfid bridge between Cys-81 and Cys-90

<400> 147

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Gly Gly Leu Tyr Ala Cys His Met Gly Pro Met Thr Trp Val Cys
        35                  40                  45

Gln Pro Leu Arg Gly Ala Ala Pro Ala Ala Pro Ala Pro Ala Ala Pro
    50                  55                  60

Ala Ala Pro Ala Pro Ala Ala Pro Ala Ala Pro Gly Gly Leu Tyr Ala
65                  70                  75                  80

Cys His Met Gly Pro Met Thr Trp Val Cys Gln Pro Leu Arg Gly Ala
                85                  90                  95

Ala Pro Ala Ala Pro Ala Pro Ala Ala Pro Ala Ala Pro Ala Pro Ala
            100                 105                 110

Ala Pro Ala Ala Pro Ala Pro Ala Ala Pro Ala Ala Pro Ala Pro Ala
        115                 120                 125

Ala Pro Ala Ala Pro Ala Pro Ala Ala Pro Ala Ala Pro Ala Pro Ala
    130                 135                 140

Ala Pro Ala Ala Pro Ala Pro Ala Ala Pro Ala Ala Pro Ala Pro Ala
145                 150                 155                 160

Ala Pro Ala Ala Pro Ala Pro Ala Ala Pro Ala Ala Pro Ala Pro Ala
                165                 170                 175

Ala Pro Ala Ala Pro Ala Pro Ala Ala Pro Ala Ala Pro Ala Pro Ala
            180                 185                 190

Ala Pro Ala Ala Pro Ala Pro Ala Ala Pro Ala Ala Pro Ala Pro Ala
        195                 200                 205

Ala Pro Ala Ala Pro Ala Pro Ala Ala Pro Ala Ala Pro Ala Pro Ala
    210                 215                 220

Ala Pro Ala Ala Pro Ala Pro Ala Ala Pro Ala Ala Pro Ala Pro Ala
225                 230                 235                 240

Ala Pro Ala Ala Pro Ala Pro Ala Ala Pro Ala Ala Pro Ala Pro Ala
                245                 250                 255

Ala Pro Ala Ala Pro Ala Pro Ala Ala Pro Ala Ala Pro Ala Pro Ala
            260                 265                 270

Ala Pro Ala Ala Pro Ala Pro Ala Ala Pro Ala Ala Pro Ala Pro Ala
        275                 280                 285

Ala Pro Ala Ala Pro Ala Pro
    290                 295

<210>  148
<211>  2088
<212>  DNA
<213>  Artificial Sequence

<220>
<223>  CspBss-AET-EPO-PAS22-PAS600

<400>  148
atgtttaaca accgtattcg taccgctgcc ctcgctggtg ccattgccat ctctaccgct      60

gcgtccggcg tcgctattcc tgcctttgca gccgaaaccg gcggcctgta cgcatgccac     120

atgggtccaa tgacctgggt gtgccagcca ctccgcggcg ctgcaccagc agctccagca     180

ccagcagccc cagctgcacc agctccagct gctccagctg caccaggcgg cctgtacgct     240

tgtcatatgg gtccaatgac ttgggtctgt caacctctgc gcggcgctgc tcctgcagct     300

ccagctccag cagcaccagc tgcaccagcc cctgctgcac cagcagcacc agcaccagca     360

gcaccagctg cccctgcccc tgctgcccca gctgcaccag cccccgctgc ccctgccgct     420

cctgctcctg cagcaccagc tgcccccgcc cctgctgctc ctgcagcccc tgctcccgct     480

gcccctgccg cccccgcccc tgcagcacca gctgcgcctg cccccgctgc gccagctgca     540

ccagccccgg ctgcccctgc cgctcccgcc cccgcagcac cagctgcccc ggccccggct     600

gcgcctgctg caccagctcc tgctgcccct gccgctccgg ccccggcagc accagctgcc     660

ccagcacctg ctgcaccagc tgcaccagcg cctgctgccc ctgccgcccc ggcccccgca     720

gctcctgctg cccccgcacc tgccgcacca gctgcaccag cacctgctgc tcccgccgct     780

cctgctcccg cagcaccagc tgcgcccgcc ccggctgcgc ccgcagcccc tgctcccgcc     840

gcacctgctg ctcccgcgcc tgcagcacca gctgctcctg cacctgctgc tcctgccgct     900

ccagctcctg ctgccccggc cgctcctgct ccggcagcac cagctgcgcc ggcccccgct     960

gcccccgcag cccctgctcc ggctgcccct gccgcccctg ctccagcagc tcctgctgcc    1020

ccagcacctg ccgctcctgc agctcctgct ccagctgccc ctgccgctcc agccccagca 1080

gcaccagctg cgccagcacc tgccgctccc gcagctcctg ctccagccgc acctgctgct 1140

ccggcgcccg cagcaccagc tgctcccgca cctgcggctc cagcagcccc tgcccctgct 1200

gcgcctgccg ctcctgctcc agcagctccc gctgcacctg ccccggccgc tccagctgca 1260

ccagccccag ctgctccagc cgctcccgct ccggcagctc ctgctgctcc cgctccggct 1320

gctcctgccg caccagctcc tgcagctcca gcagcccccg ctccggcagc tcccgctgct 1380

cccgctccag ccgctcctgc agccccagca ccagctgctc cagccgcccc cgctccggca 1440

gcccctgctg ctcccgctcc cgctgctcct gcggcaccag ctcccgcagc tccagcagcc 1500

ccggctccgg cagctccggc tgctcccgct cccgccgctc ccgcagcccc tgctccagct 1560

gctccagccg cgcccgctcc agcagctcca gctgcacctg ctccggccgc ccctgccgct 1620

ccagctccgg cagctccagc agccccagcg cctgcagctc ctgctgctcc ggctccagcc 1680

gctcccgcag cccccgctcc agctgctcca gcggctcccg ctccagcagc ccccgccgca 1740

cctgctccag ccgctccggc agccccagct ccagccgctc cagccgctcc agcgcctgca 1800

gctccagcgg ctcccgcccc agccgctcct gcagcgccag ctccagcggc tcctgccgct 1860

cctgcccctg cagctccagc agcgcccgct ccagccgccc cggcagctcc tgctcctgct 1920

gctccagccg ctccagcacc tgcagctcca gcagctcctg ccccagccgc tcccgcagcg 1980

ccagctccag cagcgccggc tgcaccggcc cctgcagcac cagccgctcc cgctcccgct 2040

gctcccgccg cccccgcacc cgccgccccc gctgctcccg ctccctaa 2088

<210> 149
<211> 695
<212> PRT
<213> Artificial Sequence

<220>
<223> CspBss-AET-EPO-PAS22-PAS600


<220>
<221> DISULFID
<222> (39)..(48)
<223> Disulfid bridge between Cys-39 and Cys-48

<220>
<221> DISULFID
<222> (81)..(90)
<223> Disulfid bridge between Cys-81 and Cys-90

<400> 149

Met Phe Asn Asn Arg Ile Arg Thr Ala Ala Leu Ala Gly Ala Ile Ala
1               5                   10                  15


Ile Ser Thr Ala Ala Ser Gly Val Ala Ile Pro Ala Phe Ala Ala Glu

20                  25                  30

Thr Gly Gly Leu Tyr Ala Cys His Met Gly Pro Met Thr Trp Val Cys
        35                  40                  45

Gln Pro Leu Arg Gly Ala Ala Pro Ala Ala Pro Ala Pro Ala Ala Pro
    50                  55                  60

Ala Ala Pro Ala Pro Ala Ala Pro Ala Ala Pro Gly Gly Leu Tyr Ala
65                  70                  75                  80

Cys His Met Gly Pro Met Thr Trp Val Cys Gln Pro Leu Arg Gly Ala
                85                  90                  95

Ala Pro Ala Ala Pro Ala Pro Ala Ala Pro Ala Ala Pro Ala Pro Ala
            100                 105                 110

Ala Pro Ala Ala Pro Ala Pro Ala Ala Pro Ala Ala Pro Ala Pro Ala
        115                 120                 125

Ala Pro Ala Ala Pro Ala Pro Ala Ala Pro Ala Ala Pro Ala Pro Ala
    130                 135                 140

Ala Pro Ala Ala Pro Ala Pro Ala Ala Pro Ala Ala Pro Ala Pro Ala
145                 150                 155                 160

Ala Pro Ala Ala Pro Ala Pro Ala Ala Pro Ala Ala Pro Ala Pro Ala
                165                 170                 175

Ala Pro Ala Ala Pro Ala Pro Ala Ala Pro Ala Ala Pro Ala Pro Ala
            180                 185                 190

Ala Pro Ala Ala Pro Ala Pro Ala Ala Pro Ala Ala Pro Ala Pro Ala
        195                 200                 205

Ala Pro Ala Ala Pro Ala Pro Ala Ala Pro Ala Ala Pro Ala Pro Ala
    210                 215                 220

Ala Pro Ala Ala Pro Ala Pro Ala Ala Pro Ala Ala Pro Ala Pro Ala
225                 230                 235                 240

Ala Pro Ala Ala Pro Ala Pro Ala Ala Pro Ala Ala Pro Ala Pro Ala
                245                 250                 255

Ala Pro Ala Ala Pro Ala Pro Ala Ala Pro Ala Ala Pro Ala Pro Ala
            260                 265                 270

Ala Pro Ala Ala Pro Ala Pro Ala Ala Pro Ala Ala Pro Ala Pro Ala
        275                 280                 285

Ala Pro Ala Ala Pro Ala Pro Ala Ala Pro Ala Ala Pro Ala Pro Ala
    290                 295                 300

Ala Pro Ala Ala Pro Ala Pro Ala Ala Pro Ala Ala Pro Ala Pro Ala
305                 310                 315                 320

Ala Pro Ala Ala Pro Ala Pro Ala Ala Pro Ala Ala Pro Ala Pro Ala
                325                 330                 335

Ala Pro Ala Ala Pro Ala Pro Ala Ala Pro Ala Ala Pro Ala Pro Ala
            340                 345                 350

Ala Pro Ala Ala Pro Ala Pro Ala Ala Pro Ala Ala Pro Ala Pro Ala
        355                 360                 365

Ala Pro Ala Ala Pro Ala Pro Ala Ala Pro Ala Ala Pro Ala Pro Ala
    370                 375                 380

Ala Pro Ala Ala Pro Ala Pro Ala Ala Pro Ala Ala Pro Ala Pro Ala
385                 390                 395                 400

Ala Pro Ala Ala Pro Ala Pro Ala Ala Pro Ala Ala Pro Ala Pro Ala
                405                 410                 415

Ala Pro Ala Ala Pro Ala Pro Ala Ala Pro Ala Ala Pro Ala Pro Ala
            420                 425                 430

Ala Pro Ala Ala Pro Ala Pro Ala Ala Pro Ala Ala Pro Ala Pro Ala
        435                 440                 445

Ala Pro Ala Ala Pro Ala Pro Ala Ala Pro Ala Ala Pro Ala Pro Ala
    450                 455                 460

Ala Pro Ala Ala Pro Ala Pro Ala Ala Pro Ala Ala Pro Ala Pro Ala
465                 470                 475                 480

Ala Pro Ala Ala Pro Ala Pro Ala Ala Pro Ala Ala Pro Ala Pro Ala
                485                 490                 495

Ala Pro Ala Ala Pro Ala Pro Ala Ala Pro Ala Ala Pro Ala Pro Ala
            500                 505                 510

Ala Pro Ala Ala Pro Ala Pro Ala Ala Pro Ala Ala Pro Ala Pro Ala
        515                 520                 525

Ala Pro Ala Ala Pro Ala Pro Ala Ala Pro Ala Ala Pro Ala Pro Ala
    530                 535                 540

Ala Pro Ala Ala Pro Ala Pro Ala Ala Pro Ala Ala Pro Ala Pro Ala
545                 550                 555                 560

Ala Pro Ala Ala Pro Ala Pro Ala Ala Pro Ala Ala Pro Ala Pro Ala
                565                 570                 575

Ala Pro Ala Ala Pro Ala Pro Ala Ala Pro Ala Ala Pro Ala Pro Ala
            580                 585                 590

Ala Pro Ala Ala Pro Ala Pro Ala Ala Pro Ala Ala Pro Ala Pro Ala
        595                 600                 605

Ala Pro Ala Ala Pro Ala Pro Ala Ala Pro Ala Ala Pro Ala Pro Ala
    610                 615                 620

Ala Pro Ala Ala Pro Ala Pro Ala Ala Pro Ala Ala Pro Ala Pro Ala
625                 630                 635                 640

Ala Pro Ala Ala Pro Ala Pro Ala Ala Pro Ala Ala Pro Ala Pro Ala
                645                 650                 655

Ala Pro Ala Ala Pro Ala Pro Ala Ala Pro Ala Ala Pro Ala Pro Ala
            660                 665                 670

Ala Pro Ala Ala Pro Ala Pro Ala Ala Pro Ala Ala Pro Ala Pro Ala
        675                 680                 685

Ala Pro Ala Ala Pro Ala Pro
    690                 695

<210>  150
<211>  60
<212>  PRT
<213>  Artificial Sequence

<220>
<223>  MAET-aMD4dY-PAS22

<220>
<221>  DISULFID
<222>  (5)..(20)
<223>  Disulfid bridge between Cys-5 and Cys-20

<220>
<221>  DISULFID
<222>  (45)..(60)
<223>  Disulfid bridge between Cys-45 and Cys-60

<400>  150

Met Ala Glu Thr Cys Arg Gln Phe Asn Arg Arg Thr His Glu Val Trp
1               5                   10                  15

Asn Leu Asp Cys Gly Ala Ala Pro Ala Ala Pro Ala Pro Ala Ala Pro
            20                  25                  30

Ala Ala Pro Ala Pro Ala Ala Pro Ala Ala Pro Gly Cys Arg Gln Phe
        35                  40                  45

Asn Arg Arg Thr His Glu Val Trp Asn Leu Asp Cys
    50                  55                  60

<210>  151
<211>  46
<212>  PRT
<213>  Artificial Sequence

<220>
<223>  MAET-aMD4dY-PAS8

<220>
<221>  DISULFID
<222>  (5)..(20)
<223>  Disulfid bridge between Cys-5 and Cys-20

<220>
<221>  DISULFID
<222>  (31)..(46)
<223>  Disulfid bridge between Cys-31 and Cys-46

<400>  151

Met Ala Glu Thr Cys Arg Gln Phe Asn Arg Arg Thr His Glu Val Trp
1               5                   10                  15

Asn Leu Asp Cys Gly Ala Ala Pro Ala Ala Pro Ala Pro Gly Cys Arg
            20                  25                  30

Gln Phe Asn Arg Arg Thr His Glu Val Trp Asn Leu Asp Cys
        35                  40                  45

<210>  152
<211>  61
<212>  PRT
<213>  Artificial Sequence

<220>
<223>  MAET-aMD4-PAS22

<220>
<221>  DISULFID

<222>  (5)..(21)
<223>  Disulfid bridge between Cys-5 and Cys-21

<220>
<221>  DISULFID
<222>  (46)..(61)
<223>  Disulfid bridge between Cys-46 and Cys-61

<400>  152

Met Ala Glu Thr Cys Tyr Arg Gln Phe Asn Arg Arg Thr His Glu Val
1               5                   10                  15

Trp Asn Leu Asp Cys Gly Ala Ala Pro Ala Ala Pro Ala Pro Ala Ala
            20                  25                  30

Pro Ala Ala Pro Ala Pro Ala Ala Pro Ala Ala Pro Gly Cys Arg Gln
        35                  40                  45

Phe Asn Arg Arg Thr His Glu Val Trp Asn Leu Asp Cys
    50                  55                  60

<210>  153
<211>  52
<212>  PRT
<213>  Artificial Sequence

<220>
<223>  MAET-EPO-PAS8

<220>
<221>  DISULFID
<222>  (10)..(19)
<223>  Disulfid bridge between Cys-10 and Cys-19

<220>
<221>  DISULFID
<222>  (38)..(47)
<223>  Disulfid bridge between Cys-38 and Cys-47

<400>  153

Met Ala Glu Thr Gly Gly Leu Tyr Ala Cys His Met Gly Pro Met Thr
1               5                   10                  15

Trp Val Cys Gln Pro Leu Arg Gly Ala Ala Pro Ala Ala Pro Ala Pro
            20                  25                  30

Gly Gly Leu Tyr Ala Cys His Met Gly Pro Met Thr Trp Val Cys Gln
        35                  40                  45

Pro Leu Arg Gly
    50

<210>  154
<211>  93
<212>  PRT
<213>  Artificial Sequence

<220>
<223>  MAET-EPO-PAS49-b



<220>
<221>  DISULFID
<222>  (10)..(19)
<223>  Disulfid bridge between Cys-10 and Cys-19

<220>
<221>  DISULFID
<222>  (79)..(88)
<223>  Disulfid bridge between Cys-79 and Cys-88

<400>  154

Met Ala Glu Thr Gly Gly Leu Tyr Ala Cys His Met Gly Pro Met Thr
1               5                   10                  15


Trp Val Cys Gln Pro Leu Arg Gly Ser Ala Pro Ser Pro Ser Ser Ala
            20                  25                  30


Pro Ala Ser Ala Ser Ala Pro Ala Ser Pro Ala Ser Ala Ser Ala Ser
        35                  40                  45


Ala Ser Pro Ala Ser Ser Pro Ala Ser Ala Ser Ser Ala Ser Pro Ala
    50                  55                  60


Ala Ser Ser Ala Ser Pro Ala Ser Ser Gly Gly Leu Tyr Ala Cys His
65                  70                  75                  80


Met Gly Pro Met Thr Trp Val Cys Gln Pro Leu Arg Gly
                85                  90


<210>  155
<211>  144
<212>  PRT
<213>  Artificial Sequence

<220>
<223>  MAET-EPO-PAS100-b



<220>
<221>  DISULFID
<222>  (10)..(19)
<223>  Disulfid bridge between Cys-10 and Cys-19

<220>
<221>  DISULFID
<222>  (130)..(139)
<223>  Disulfid bridge between Cys-130 and Cys-139

<400>  155

Met Ala Glu Thr Gly Gly Leu Tyr Ala Cys His Met Gly Pro Met Thr
1               5                   10                  15

Trp Val Cys Gln Pro Leu Arg Gly Ser Ala Pro Ser Pro Ser Ser Ala
            20                  25                  30

Pro Ala Ser Ala Ser Ala Pro Ala Ser Pro Ala Ser Ala Ser Ala Ser
        35                  40                  45

Ala Ser Pro Ala Ser Ser Pro Ala Ser Ala Ser Ser Ala Ser Pro Ala
    50                  55                  60

Ala Ser Ser Ala Ser Pro Ala Ser Ser Ser Ala Ser Pro Ser Ala Pro
65                  70                  75                  80

Ala Ala Pro Ser Ala Ser Ala Ala Pro Ser Ala Ser Pro Ala Ser Ser
                85                  90                  95

Ser Pro Ala Pro Ala Ser Ala Pro Ser Pro Ala Ala Ala Ala Ser Ser
            100                 105                 110

Pro Ser Pro Ala Pro Ser Ser Ala Ser Ser Ala Ala Gly Gly Leu Tyr
        115                 120                 125

Ala Cys His Met Gly Pro Met Thr Trp Val Cys Gln Pro Leu Arg Gly
    130                 135                 140


<210>  156
<211>  55
<212>  PRT
<213>  Artificial Sequence

<220>
<223>  MAET-VEGFR_D2-PAS16


<220>
<221>  DISULFID
<222>  (10)..(17)
<223>  Disulfid bridge between Cys-10 and Cys-17

<220>
<221>  DISULFID
<222>  (40)..(50)
<223>  Disulfid bridge between Cys-40 and Cys-50

<400>  156

Met Ala Glu Thr Ala Gly Pro Thr Trp Cys Glu Asp Asp Trp Tyr Tyr
1               5                   10                  15

Cys Trp Leu Phe Gly Thr Ala Ala Pro Ala Ala Pro Ala Pro Ala Ala
            20                  25                  30

Pro Ala Ala Pro Ala Pro Val Cys Trp Glu Asp Ser Trp Gly Gly Glu
        35                  40                  45

Val Cys Trp Leu Phe Gly Thr
    50                  55

<210>  157
<211>  88
<212>  PRT
<213>  Artificial Sequence

<220>
<223>  MAET-VEGFR_D2-PAS49-b

<220>
<221>  DISULFID
<222>  (10)..(17)
<223>  Disulfid bridge between Cys-10 and Cys-17

<220>
<221>  DISULFID
<222>  (73)..(83)
<223>  Disulfid bridge between Cys-73 and Cys-83

<400>  157

Met Ala Glu Thr Ala Gly Pro Thr Trp Cys Glu Asp Asp Trp Tyr Tyr
1               5                   10                  15

Cys Trp Leu Phe Gly Thr Ser Ala Pro Ser Pro Ser Ser Ala Pro Ala
            20                  25                  30

Ser Ala Ser Ala Pro Ala Ser Pro Ala Ser Ala Ser Ala Ser Ala Ser
        35                  40                  45

Pro Ala Ser Ser Pro Ala Ser Ala Ser Ser Ala Ser Pro Ala Ala Ser
    50                  55                  60

Ser Ala Ser Pro Ala Ser Ser Val Cys Trp Glu Asp Ser Trp Gly Gly
65                  70                  75                  80

Glu Val Cys Trp Leu Phe Gly Thr
                85

<210>  158
<211>  139
<212>  PRT
<213>  Artificial Sequence

<220>
<223>  MAET-VEGFR_D2-PAS100-b

<220>
<221>  DISULFID
<222>  (10)..(17)
<223>  Disulfid bridge between Cys-10 and Cys-17

<220>
<221>  DISULFID
<222>  (124)..(134)
<223>  Disulfid bridge between Cys-124 and Cys-134

<400>  158

Met Ala Glu Thr Ala Gly Pro Thr Trp Cys Glu Asp Asp Trp Tyr Tyr
1               5                   10                  15

Cys Trp Leu Phe Gly Thr Ser Ala Pro Ser Pro Ser Ser Ala Pro Ala
            20                  25                  30

Ser Ala Ser Ala Pro Ala Ser Pro Ala Ser Ala Ser Ala Ser Ala Ser
        35                  40                  45

Pro Ala Ser Ser Pro Ala Ser Ala Ser Ser Ala Ser Pro Ala Ala Ser
    50                  55                  60

Ser Ala Ser Pro Ala Ser Ser Ser Ala Ser Pro Ser Ala Pro Ala Ala
65                  70                  75                  80

Pro Ser Ala Ser Ala Ala Pro Ser Ala Ser Pro Ala Ser Ser Ser Pro
                85                  90                  95

Ala Pro Ala Ser Ala Pro Ser Pro Ala Ala Ala Ala Ser Ser Pro Ser
            100                 105                 110

Pro Ala Pro Ser Ser Ala Ser Ser Ala Ala Val Cys Trp Glu Asp Ser
        115                 120                 125

Trp Gly Gly Glu Val Cys Trp Leu Phe Gly Thr
    130                 135

<210>  159
<211>  55
<212>  PRT
<213>  Artificial Sequence

<220>
<223>  MAET-VEGFR_D2-GS16

<220>
<221>  DISULFID

<222>  (10)..(17)
<223>  Disulfid bridge between Cys-10 and Cys-17

<220>
<221>  DISULFID
<222>  (40)..(50)
<223>  Disulfid bridge between Cys-40 and Cys-50

<400>  159

Met Ala Glu Thr Ala Gly Pro Thr Trp Cys Glu Asp Asp Trp Tyr Tyr
1               5                   10                  15

Cys Trp Leu Phe Gly Thr Gly Gly Gly Gly Ser Gly Gly Gly Gly Ser
            20                  25                  30

Gly Gly Gly Gly Ser Gly Val Cys Trp Glu Asp Ser Trp Gly Gly Glu
        35                  40                  45

Val Cys Trp Leu Phe Gly Thr
    50                  55

<210>  160
<211>  281
<212>  DNA
<213>  Artificial Sequence

<220>
<223>  MAET-aMD4dY-PAS22

<400>  160
gaaattaata cgactcacta tagggagacc acaacggttt ccctctagaa ataattttgt      60

ttaactttaa gaaggagata taccaatgca gagacctgtc gtcagtttaa ccgtcgtacg     120

cacgaagttt ggaatctgga ctgtggcgca gcccccgcag caccagctcc agcagcaccc     180

gcagctccag caccagcagc ccctgcagcc ccaggctgcc gccaattcaa tcgccgcacc     240

catgaagtgt ggaacttaga ttgctagtga ataactaatc c                         281

<210>  161
<211>  240
<212>  DNA
<213>  Artificial Sequence

<220>
<223>  MAET-aMD4dY-PAS8

<400>  161
gaaattaata cgactcacta tagggagacc acaacggttt ccctctagaa ataattttgt      60

ttaactttaa gaaggagata taccaatggc agagacctgt cgtcagttta accgtcgtac     120

gcacgaagtt tggaatctgg actgtggcgc agcacccgca gctccagcac caggctgccg     180

ccaattcaat cgccgcaccc atgaagtgtg gaacttagat tgctagtgaa taactaatcc     240

<210>  162
<211>  288
<212>  DNA
<213>  Artificial Sequence

<220>
<223>  MAET-aMD4-PAS22

<400>  162
gaaattaata cgactcacta tagggagacc acaacggttt ccctctagaa ataattttgt      60

ttaactttaa gaaggagata taccaatggc agagacctgt tatcgtcagt ttaaccgtcg     120

tacgcacgaa gtttggaatc tggactgtgg cgcagccccc gcagcaccag ctccagcagc     180

acccgcagct ccagcaccag cagcccctgc agccccaggc tgctatcgcc aattcaatcg     240

ccgcacccat gaagtgtgga acttagattg ctagtgaata actaatcc                  288


<210>  163
<211>  258
<212>  DNA
<213>  Artificial Sequence

<220>
<223>  MAET-EPO-PAS8

<400>  163
gaaattaata cgactcacta tagggagacc acaacggttt ccctctagaa ataattttgt      60

ttaactttaa gaaggagata taccaatggc agaaactggt ggtttatacg cctgtcacat     120

gggcccgatg acttgggttt gccagccatt gcgtggcgca gcacccgcag ctccagcacc     180

aggtggcctg tatgcgtgcc atatggggcc aatgacctgg gtgtgtcagc cgttacgcgg     240

ctagtgaata actaatcc                                                   258


<210>  164
<211>  381
<212>  DNA
<213>  Artificial Sequence

<220>
<223>  MAET-EPO-PAS49-b

<400>  164
gaaattaata cgactcacta tagggagacc acaacggttt ccctctagaa ataattttgt      60

ttaactttaa gaaggagata taccaatggc agaaactggt ggtttatacg cctgtcacat     120

gggcccgatg acttgggttt gccagccatt gcgtggctct gctccatcac cttcttccgc     180

accagcatca gcttctgcac cagcgtcgcc tgcttctgca tctgcgtctg cttcaccagc     240

atcctcacct gcatcagcat cgtctgcttc tccagcagct tcctcagctt cacctgcttc     300

gtctggtggc ctgtatgcgt gccatatggg gccaatgacc tgggtgtgtc agccgttacg     360

cggctagtga ataactaatc c                                               381

<210>  165
<211>  534
<212>  DNA
<213>  Artificial Sequence

<220>
<223>  MAET-EPO-PAS100-b

<400>  165
gaaattaata cgactcacta tagggagacc acaacggttt ccctctagaa ataattttgt      60

ttaactttaa gaaggagata taccaatggc agaaactggt ggtttatacg cctgtcacat     120

gggcccgatg acttgggttt gccagccatt gcgtggctct gctccatcac cttcttccgc     180

accagcatca gcttctgcac cagcgtcgcc tgcttctgca tctgcgtctg cttcaccagc     240

atcctcacct gcatcagcat cgtctgcttc tccagcagct tcctcagctt cacctgcttc     300

gtcttccgca tccccatctg ctccagcagc accttcagct tctgcagctc catctgcttc     360

acctgcctca tcgtctccag cacctgcgtc agctccatcc cctgcagcag cggcttcatc     420

gccatctcca gcaccttctt cagcgtcatc cgctgcaggt ggcctgtatg cgtgccatat     480

ggggccaatg acctgggtgt gtcagccgtt acgcggctag tgaataacta atcc           534


<210>  166
<211>  267
<212>  DNA
<213>  Artificial Sequence

<220>
<223>  MAET-VEGFR_D2-PAS16

<400>  166
gaaattaata cgactcacta tagggagacc acaacggttt ccctctagaa ataattttgt      60

ttaactttaa gaaggagata taccaatggc ggaaaccgcg ggtcccacgt ggtgtgagga     120

tgactggtac tattgctggt tattcgggac tgcggcccct gctgccccgg ctccggcagc     180

accggccgca ccagcgccgg tttgctggga agatagctgg ggtggcgaag tgtgttggct     240

gtttggcacc tagtgaataa ctaatcc                                         267


<210>  167
<211>  366
<212>  DNA
<213>  Artificial Sequence

<220>
<223>  MAET-VEGFR_D2-PAS49-b

<400>  167
gaaattaata cgactcacta tagggagacc acaacggttt ccctctagaa ataattttgt      60

ttaactttaa gaaggagata taccaatggc ggaaaccgcg ggtcccacgt ggtgtgagga     120

tgactggtac tattgctggt tattcgggac ttctgctcca tcaccttctt ccgcaccagc 180

atcagcttct gcaccagcgt cgcctgcttc tgcatctgcg tctgcttcac cagcatcctc 240

acctgcatca gcatcgtctg cttctccagc agcttcctca gcttcacctg cttcgtctgt 300

ttgctgggaa gatagctggg gtggcgaagt gtgttggctg tttggcacct agtgaataac 360

taatcc 366

<210> 168
<211> 534
<212> DNA
<213> Artificial Sequence

<220>
<223> MAET-VEGFR_D2-PAS100-b

<400> 168
gaaattaata cgactcacta tagggagacc acaacggttt ccctctagaa ataattttgt 60

ttaactttaa gaaggagata taccaatggc agaaactggt ggtttatacg cctgtcacat 120

gggcccgatg acttgggttt gccagccatt gcgtggctct gctccatcac cttcttccgc 180

accagcatca gcttctgcac cagcgtcgcc tgcttctgca tctgcgtctg cttcaccagc 240

atcctcacct gcatcagcat cgtctgcttc tccagcagct tcctcagctt cacctgcttc 300

gtcttccgca tccccatctg ctccagcagc accttcagct tctgcagctc catctgcttc 360

acctgcctca tcgtctccag cacctgcgtc agctccatcc cctgcagcag cggcttcatc 420

gccatctcca gcaccttctt cagcgtcatc cgctgcaggt ggcctgtatg cgtgccatat 480

ggggccaatg acctgggtgt gtcagccgtt acgcggctag tgaataacta atcc 534

<210> 169
<211> 267
<212> DNA
<213> Artificial Sequence

<220>
<223> MAET-VEGFR_D2-GS16

<400> 169
gaaattaata cgactcacta tagggagacc acaacggttt ccctctagaa ataattttgt 60

ttaactttaa gaaggagata taccaatggc ggaaaccgcg ggtcccacgt ggtgtgagga 120

tgactggtac tattgctggt tattcgggac tggtagcggt agcggcagcg gcagcggtag 180

tggtagtggc agcggcagcg tttgctggga agatagctgg ggtggcgaag tgtgttggct 240

gtttggcacc tagtgaataa ctaatcc 267

<210> 170
<211> 25
<212> DNA
<213> Artificial Sequence

<220>
<223>  Cell-free primer Fwd

<400>  170
gaaattaata cgactcacta taggg                                          25


<210>  171
<211>  23
<212>  DNA
<213>  Artificial Sequence

<220>
<223>  Cell-free primer Rev

<400>  171
cactgactgg attagttatt cac                                            23


<210>  172
<211>  99
<212>  PRT
<213>  Artificial Sequence

<220>
<223>  AET-aMD4dY-PAS22-Trimer



<220>
<221>  DISULFID
<222>  (4)..(19)
<223>  Disulfid bridge between Cys-4 and Cys-19

<220>
<221>  DISULFID
<222>  (44)..(59)
<223>  Disulfid bridge between Cys-44 and Cys-59

<220>
<221>  DISULFID
<222>  (84)..(99)
<223>  Disulfid bridge between Cys-84 and Cys-99

<400>  172

Ala Glu Thr Cys Arg Gln Phe Asn Arg Arg Thr His Glu Val Trp Asn
1               5                   10                  15


Leu Asp Cys Gly Ala Ala Pro Ala Ala Pro Ala Pro Ala Ala Pro Ala
            20                  25                  30


Ala Pro Ala Pro Ala Ala Pro Ala Ala Pro Gly Cys Arg Gln Phe Asn
        35                  40                  45


Arg Arg Thr His Glu Val Trp Asn Leu Asp Cys Gly Ala Ala Pro Ala
    50                  55                  60


Ala Pro Ala Pro Ala Ala Pro Ala Ala Pro Ala Pro Ala Ala Pro Ala

65                  70                  75                  80

Ala Pro Gly Cys Arg Gln Phe Asn Arg Arg Thr His Glu Val Trp Asn
                85                  90                  95

Leu Asp Cys

<210>  173
<211>  390
<212>  DNA
<213>  Artificial Sequence

<220>
<223>  CspBss-AET-aMD4dY-PAS22-Trimer

<400>  173
atgtttaaca accgtatccg cactgcagct ctcgctggtg caatcgcaat ctccaccgca      60

gcttccggcg tagctatccc agcattcgct gcagagacct gccgccagtt caatcgccgg     120

acccacgaag tctggaatct ggattgcggc gcagctcctg ccgccccagc accggctgcg     180

ccagctgctc cagcgccggc tgcccctgcg gcaccgggtt gccgtcagtt taaccgccgt     240

actcacgagg tttggaacct tgactgtgga gcggctcccg cagcaccagc cccagccgcc     300

cctgctgcac ccgcaccagc ggcacccgcc gctcctggct gtcgccaatt caaccgacgc     360

acccatgaag tgtggaacct cgattgctaa                                      390

<210>  174
<211>  129
<212>  PRT
<213>  Artificial Sequence

<220>
<223>  CspBss-AET-aMD4dY-PAS22-Trimer

<220>
<221>  DISULFID
<222>  (34)..(49)
<223>  Disulfid bridge between Cys-34 and Cys-49

<220>
<221>  DISULFID
<222>  (74)..(89)
<223>  Disulfid bridge between Cys-74 and Cys-89

<220>
<221>  DISULFID
<222>  (114)..(129)
<223>  Disulfid bridge between Cys-114 and Cys-129

<400>  174

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
                20                  25                  30

Thr Cys Arg Gln Phe Asn Arg Arg Thr His Glu Val Trp Asn Leu Asp
        35                  40                  45

Cys Gly Ala Ala Pro Ala Ala Pro Ala Pro Ala Ala Pro Ala Ala Pro
    50                  55                  60

Ala Pro Ala Ala Pro Ala Ala Pro Gly Cys Arg Gln Phe Asn Arg Arg
65                  70                  75                  80

Thr His Glu Val Trp Asn Leu Asp Cys Gly Ala Ala Pro Ala Ala Pro
                85                  90                  95

Ala Pro Ala Ala Pro Ala Ala Pro Ala Pro Ala Ala Pro Ala Ala Pro
                100                 105                 110

Gly Cys Arg Gln Phe Asn Arg Arg Thr His Glu Val Trp Asn Leu Asp
        115                 120                 125

Cys

<210>  175
<211>  87
<212>  PRT
<213>  Artificial Sequence

<220>
<223>  AET-VEGFR_D2-PAS49

<220>
<221>  DISULFID
<222>  (9)..(16)
<223>  Disulfid bridge between Cys-9 and Cys-16

<220>
<221>  DISULFID
<222>  (72)..(82)
<223>  Disulfid bridge between Cys-72 and Cys-82

<400>  175

Ala Glu Thr Ala Gly Pro Thr Trp Cys Glu Asp Asp Trp Tyr Tyr Cys
1               5                   10                  15

Trp Leu Phe Gly Thr Ala Ala Pro Ala Ala Pro Ala Pro Ala Ala Pro
                20                  25                  30

Ala Ala Pro Ala Pro Ala Ala Pro Ala Ala Pro Ala Pro Ala Ala Pro
        35                  40                  45

Ala Ala Pro Ala Pro Ala Ala Pro Ala Ala Pro Ala Pro Ala Ala Pro
    50                  55                  60

Ala Ala Pro Ala Pro Ala Val Cys Trp Glu Asp Ser Trp Gly Gly Glu
65                  70                  75                  80

Val Cys Trp Leu Phe Gly Thr
                85

<210> 176
<211> 87
<212> PRT
<213> Artificial Sequence

<220>
<223> AET-VEGFR_D2-PAS49-b

<220>
<221> DISULFID
<222> (9)..(16)
<223> Disulfid bridge between Cys-9 and Cys-16

<220>
<221> DISULFID
<222> (72)..(82)
<223> Disulfid bridge between Cys-72 and Cys-82

<400> 176

Ala Glu Thr Ala Gly Pro Thr Trp Cys Glu Asp Asp Trp Tyr Tyr Cys
1               5                   10                  15

Trp Leu Phe Gly Thr Ser Ala Pro Ser Pro Ser Ser Ala Pro Ala Ser
            20                  25                  30

Ala Ser Ala Pro Ala Ser Pro Ala Ser Ala Ser Ala Ser Ala Ser Pro
        35                  40                  45

Ala Ser Ser Pro Ala Ser Ala Ser Ser Ala Ser Pro Ala Ala Ser Ser
    50                  55                  60

Ala Ser Pro Ala Ser Ser Val Cys Trp Glu Asp Ser Trp Gly Gly Glu
65                  70                  75                  80

Val Cys Trp Leu Phe Gly Thr
                85

<210> 177
<211> 138

<212>  PRT
<213>  Artificial Sequence

<220>
<223>  AET-VEGFR_D2-PAS100


<220>
<221>  DISULFID
<222>  (9)..(16)
<223>  Disulfid bridge between Cys-9 and Cys-16

<220>
<221>  DISULFID
<222>  (123)..(133)
<223>  Disulfid bridge between Cys-123 and Cys-133

<400>  177

Ala Glu Thr Ala Gly Pro Thr Trp Cys Glu Asp Asp Trp Tyr Tyr Cys
1               5                   10                  15

Trp Leu Phe Gly Thr Ala Ala Pro Ala Ala Pro Ala Pro Ala Ala Pro
            20                  25                  30

Ala Ala Pro Ala Pro Ala Ala Pro Ala Ala Pro Ala Pro Ala Ala Pro
        35                  40                  45

Ala Ala Pro Ala Pro Ala Ala Pro Ala Ala Pro Ala Pro Ala Ala Pro
    50                  55                  60

Ala Ala Pro Ala Pro Ala Ala Pro Ala Ala Pro Ala Pro Ala Ala Pro
65                  70                  75                  80

Ala Ala Pro Ala Pro Ala Ala Pro Ala Ala Pro Ala Pro Ala Ala Pro
                85                  90                  95

Ala Ala Pro Ala Pro Ala Ala Pro Ala Ala Pro Ala Pro Ala Ala Pro
            100                 105                 110

Ala Ala Pro Ala Pro Ala Ala Pro Ala Val Cys Trp Glu Asp Ser Trp
        115                 120                 125

Gly Gly Glu Val Cys Trp Leu Phe Gly Thr
    130                 135


<210>  178
<211>  138
<212>  PRT
<213>  Artificial Sequence

<220>
<223>  AET-VEGFR_D2-PAS100-b

<220>
<221>  DISULFID
<222>  (9)..(16)
<223>  Disulfid bridge between Cys-9 and Cys-16

<220>
<221>  DISULFID
<222>  (123)..(133)
<223>  Disulfid bridge between Cys-123 and Cys-133

<400>  178

Ala Glu Thr Ala Gly Pro Thr Trp Cys Glu Asp Asp Trp Tyr Tyr Cys
1               5                   10                  15

Trp Leu Phe Gly Thr Ser Ala Pro Ser Pro Ser Ser Ala Pro Ala Ser
            20                  25                  30

Ala Ser Ala Pro Ala Ser Pro Ala Ser Ala Ser Ala Ser Ala Ser Pro
        35                  40                  45

Ala Ser Ser Pro Ala Ser Ala Ser Ser Ala Ser Pro Ala Ala Ser Ser
    50                  55                  60

Ala Ser Pro Ala Ser Ser Ser Ala Ser Pro Ser Ala Pro Ala Ala Pro
65                  70                  75                  80

Ser Ala Ser Ala Ala Pro Ser Ala Ser Pro Ala Ser Ser Ser Pro Ala
                85                  90                  95

Pro Ala Ser Ala Pro Ser Pro Ala Ala Ala Ala Ser Ser Pro Ser Pro
            100                 105                 110

Ala Pro Ser Ser Ala Ser Ser Ala Ala Val Cys Trp Glu Asp Ser Trp
        115                 120                 125

Gly Gly Glu Val Cys Trp Leu Phe Gly Thr
    130                 135


<210>  179
<211>  54
<212>  PRT
<213>  Artificial Sequence

<220>
<223>  AET-VEGFR_D2-GS16


<220>
<221>  DISULFID
<222>  (9)..(16)
<223>  Disulfid bridge between Cys-9 and Cys-16

<220>
<221>  DISULFID
<222>  (39)..(49)
<223>  Disulfid bridge between Cys-39 and Cys-49

<400>  179

Ala Glu Thr Ala Gly Pro Thr Trp Cys Glu Asp Asp Trp Tyr Tyr Cys
1               5                   10                  15

Trp Leu Phe Gly Thr Gly Gly Gly Gly Ser Gly Gly Gly Gly Ser Gly
            20                  25                  30

Gly Gly Gly Ser Gly Val Cys Trp Glu Asp Ser Trp Gly Gly Glu Val
        35                  40                  45

Cys Trp Leu Phe Gly Thr
    50

<210>  180
<211>  354
<212>  DNA
<213>  Artificial Sequence

<220>
<223>  CspBss-VEGFR_D2-PAS49

<400>  180
atgtttaaca accgtatccg cactgcagct ctcgctggtg caatcgcaat ctccaccgca      60

gcttccggcg tagctatccc agcattcgct gcagagaccg ctggccctac ctggtgtgag     120

gacgattggt actactgttg gctctttggc accgccgccc ctgctgctcc cgcccccgcc     180

gcccctgccg caccagctcc agcagcccca gctgcaccag caccagccgc tccagcagct     240

ccagcaccag ctgctccagc agcaccagcc ccagcagctc cagccgcacc agctcctgcg     300

gtttgctggg aggattcttg gggcggcgaa gtttgctggc tctttggcac ctaa           354

<210>  181
<211>  117
<212>  PRT
<213>  Artificial Sequence

<220>
<223>  CspBss-VEGFR_D2-PAS49

<220>
<221>  DISULFID
<222>  (39)..(46)
<223>  Disulfid bridge between Cys-39 and Cys-46

<220>
<221>  DISULFID
<222>  (102)..(112)
<223>  Disulfid bridge between Cys-102 and Cys-112

<400>  181

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Ala Gly Pro Thr Trp Cys Glu Asp Asp Trp Tyr Tyr Cys Trp Leu
        35                  40                  45

Phe Gly Thr Ala Ala Pro Ala Ala Pro Ala Pro Ala Ala Pro Ala Ala
    50                  55                  60

Pro Ala Pro Ala Ala Pro Ala Ala Pro Ala Pro Ala Ala Pro Ala Ala
65                  70                  75                  80

Pro Ala Pro Ala Ala Pro Ala Ala Pro Ala Pro Ala Ala Pro Ala Ala
                85                  90                  95

Pro Ala Pro Ala Val Cys Trp Glu Asp Ser Trp Gly Gly Glu Val Cys
            100                 105                 110

Trp Leu Phe Gly Thr
        115

<210>  182
<211>  354
<212>  DNA
<213>  Artificial Sequence

<220>
<223>  CspBss-VEGFR_D2-PAS49-b

<400>  182
atgtttaaca accgtatccg cactgcagct ctcgctggtg caatcgcaat ctccaccgca      60

gcttccggcg tagctatccc agcattcgct gcagagaccg caggacccac ttggtgcgaa     120

gatgactggt actattgctg gctctttggc acctcagcac ccagcccatc ctccgctcca     180

gcgtctgcga gcgctcctgc atccccagcc tctgcctccg catcggcatc accggcttcc     240

agcccagcgt cggcatcgtc agcctctccg gctgcctcct ccgcttctcc tgcgtcctcc     300

gtgtgttggg aggattcctg gggtggtgaa gtctgctggc tgttcggcac ctaa           354

<210>  183
<211>  117
<212>  PRT
<213>  Artificial Sequence

<220>

<223>  CspBss-VEGFR_D2-PAS49-b

<220>
<221>  DISULFID
<222>  (39)..(46)
<223>  Disulfid bridge between Cys-39 and Cys-46

<220>
<221>  DISULFID
<222>  (102)..(112)
<223>  Disulfid bridge between Cys-102 and Cys-112

<400>  183

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Ala Gly Pro Thr Trp Cys Glu Asp Asp Trp Tyr Tyr Cys Trp Leu
        35                  40                  45

Phe Gly Thr Ser Ala Pro Ser Pro Ser Ser Ala Pro Ala Ser Ala Ser
    50                  55                  60

Ala Pro Ala Ser Pro Ala Ser Ala Ser Ala Ser Ala Ser Pro Ala Ser
65                  70                  75                  80

Ser Pro Ala Ser Ala Ser Ser Ala Ser Pro Ala Ala Ser Ser Ala Ser
                85                  90                  95

Pro Ala Ser Ser Val Cys Trp Glu Asp Ser Trp Gly Gly Glu Val Cys
            100                 105                 110

Trp Leu Phe Gly Thr
        115

<210>  184
<211>  507
<212>  DNA
<213>  Artificial Sequence

<220>
<223>  CspBss-VEGFR_D2-PAS100

<400>  184
atgtttaaca accgtatccg cactgcagct ctcgctggtg caatcgcaat ctccaccgca      60

gcttccggcg tagctatccc agcattcgct gcagagaccg ccggtccaac ctggtgtgag     120

gatgactggt actactgctg gctctttggc accgctgctc ccgctgctcc cgcccctgct     180

gctccagctg ccccagcccc agctgcccca gctgctccag caccagccgc tccagctgcc     240

ccagcaccag ctgcaccagc agcaccagca ccagcagctc cagccgctcc agccccagcc      300

gctccagcag caccagcccc agcagctcca gctgcaccag ctccagccgc cccagcagca      360

ccagctccag cagccccagc agctccagct ccagccgcac cagctgctcc agccccagcc      420

gcaccagcag ccccagctcc agctgctcct gctgtctgtt gggaggactc ttggggcggc      480

gaagtttgtt ggttgtttgg cacctaa                                          507

<210>  185
<211>  168
<212>  PRT
<213>  Artificial Sequence

<220>
<223>  CspBss-VEGFR_D2-PAS100

<220>
<221>  DISULFID
<222>  (39)..(46)
<223>  Disulfid bridge between Cys-39 and Cys-46

<220>
<221>  DISULFID
<222>  (153)..(163)
<223>  Disulfid bridge between Cys-153 and Cys-163

<400>  185

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Ala Gly Pro Thr Trp Cys Glu Asp Asp Trp Tyr Tyr Cys Trp Leu
        35                  40                  45

Phe Gly Thr Ala Ala Pro Ala Ala Pro Ala Pro Ala Ala Pro Ala Ala
    50                  55                  60

Pro Ala Pro Ala Ala Pro Ala Ala Pro Ala Pro Ala Ala Pro Ala Ala
65                  70                  75                  80

Pro Ala Pro Ala Ala Pro Ala Ala Pro Ala Pro Ala Ala Pro Ala Ala
                85                  90                  95

Pro Ala Pro Ala Ala Pro Ala Ala Pro Ala Pro Ala Ala Pro Ala Ala
            100                 105                 110

Pro Ala Pro Ala Ala Pro Ala Ala Pro Ala Pro Ala Ala Pro Ala Ala
        115                 120                 125

Pro Ala Pro Ala Ala Pro Ala Ala Pro Ala Pro Ala Ala Pro Ala Ala
    130                 135                 140

Pro Ala Pro Ala Ala Pro Ala Val Cys Trp Glu Asp Ser Trp Gly Gly
145                 150                 155                 160

Glu Val Cys Trp Leu Phe Gly Thr
                165

<210>  186
<211>  507
<212>  DNA
<213>  Artificial Sequence

<220>
<223>  CspBss-VEGFR_D2-PAS100-b

<400>  186
atgtttaaca accgtatccg cactgcagct ctcgctggtg caatcgcaat ctccaccgca      60

gcttccggcg tagctatccc agcattcgct gcagagaccg ccggacctac ctggtgtgag     120

gatgactggt actattgctg gctcttcggc acttccgccc cttccccttc cagcgcacca     180

gcgagcgctt cggctccggc ctccccagcg tccgcatctg cgtctgcatc ccctgcgtcg     240

tcacccgctt cagcctcctc agcctcccct gcggcttcat ccgcctcgcc cgcatccagc     300

tccgcatcac cctctgcgcc agcagctcca tccgcttctg ctgccccatc ggcctctccg     360

gcttccagct ctcccgcacc ggcatccgct ccgagcccag cagcagctgc atcctcccca     420

tctccggcac catcctcggc ctctagcgcg gctgtgtgct gggaagattc ctggggtggt     480

gaagtctgct ggctgtttgg cacctaa                                         507

<210>  187
<211>  168
<212>  PRT
<213>  Artificial Sequence

<220>
<223>  CspBss-VEGFR_D2-PAS100-b

<220>
<221>  DISULFID
<222>  (39)..(46)
<223>  Disulfid bridge between Cys-39 and Cys-46

<220>
<221>  DISULFID
<222>  (153)..(163)
<223>  Disulfid bridge between Cys-153 and Cys-163

<400>  187

Met Phe Asn Asn Arg Ile Arg Thr Ala Ala Leu Ala Gly Ala Ile Ala
1               5                   10                  15

Ile Ser Thr Ala Ala Ser Gly Val Ala Ile Pro Ala Phe Ala Ala Glu
            20                  25                  30

Thr Ala Gly Pro Thr Trp Cys Glu Asp Asp Trp Tyr Tyr Cys Trp Leu
        35                  40                  45

Phe Gly Thr Ser Ala Pro Ser Pro Ser Ser Ala Pro Ala Ser Ala Ser
    50                  55                  60

Ala Pro Ala Ser Pro Ala Ser Ala Ser Ala Ser Ala Ser Pro Ala Ser
65                  70                  75                  80

Ser Pro Ala Ser Ala Ser Ser Ala Ser Pro Ala Ala Ser Ser Ala Ser
                85                  90                  95

Pro Ala Ser Ser Ser Ala Ser Pro Ser Ala Pro Ala Ala Pro Ser Ala
            100                 105                 110

Ser Ala Ala Pro Ser Ala Ser Pro Ala Ser Ser Ser Pro Ala Pro Ala
        115                 120                 125

Ser Ala Pro Ser Pro Ala Ala Ala Ala Ser Ser Pro Ser Pro Ala Pro
    130                 135                 140

Ser Ser Ala Ser Ser Ala Ala Val Cys Trp Glu Asp Ser Trp Gly Gly
145                 150                 155                 160

Glu Val Cys Trp Leu Phe Gly Thr
                165

<210>  188
<211>  255
<212>  DNA
<213>  Artificial Sequence

<220>
<223>  CspBss-VEGFR_D2-GS16

<400>  188
atgtttaaca accgtatccg cactgcagct ctcgctggtg caatcgcaat ctccaccgca      60

gcttccggcg tagctatccc agcattcgct gcagagaccg cgggtccgac ctggtgtgag     120

gatgactggt actactgttg gttgttcggc accggcggcg gcggctccgg cggcggcggc     180

tctggcggcg gcggctctgg cgtttgttgg gaggactctt ggggcggcga agtttgctgg     240

ctgtttggca cctaa                                                      255

<210>  189
<211>  84
<212>  PRT
<213>  Artificial Sequence

<220>
<223>  CspBss-VEGFR_D2-GS16



<220>
<221>  DISULFID
<222>  (39)..(46)
<223>  Disulfid bridge between Cys-39 and Cys-46

<220>
<221>  DISULFID
<222>  (69)..(79)
<223>  Disulfid bridge between Cys-69 and Cys-79

<400>  189

Met Phe Asn Asn Arg Ile Arg Thr Ala Ala Leu Ala Gly Ala Ile Ala
1               5                   10                  15


Ile Ser Thr Ala Ala Ser Gly Val Ala Ile Pro Ala Phe Ala Ala Glu
            20                  25                  30


Thr Ala Gly Pro Thr Trp Cys Glu Asp Asp Trp Tyr Tyr Cys Trp Leu
        35                  40                  45


Phe Gly Thr Gly Gly Gly Gly Ser Gly Gly Gly Gly Ser Gly Gly Gly
    50                  55                  60


Gly Ser Gly Val Cys Trp Glu Asp Ser Trp Gly Gly Glu Val Cys Trp
65                  70                  75                  80


Leu Phe Gly Thr




<210>  190
<211>  85
<212>  PRT
<213>  Artificial Sequence

<220>
<223>  TPO2-PAS49



<220>
<221>  DISULFID
<222>  (3)..(16)
<223>  Disulfid bridge between Cys-3 and Cys-16

<220>
<221>  DISULFID
<222>  (70)..(83)

<223>  Disulfid bridge between Cys-70 and Cys-83

<400>  190

Gly Gly Cys Ala Asp Gly Pro Thr Leu Arg Glu Trp Ile Ser Phe Cys
1               5                   10                  15

Gly Gly Ala Ala Pro Ala Ala Pro Ala Pro Ala Ala Pro Ala Ala Pro
            20                  25                  30

Ala Pro Ala Ala Pro Ala Ala Pro Ala Pro Ala Ala Pro Ala Ala Pro
        35                  40                  45

Ala Pro Ala Ala Pro Ala Ala Pro Ala Pro Ala Ala Pro Ala Ala Pro
    50                  55                  60

Ala Pro Ala Gly Gly Cys Ala Asp Gly Pro Thr Leu Arg Glu Trp Ile
65                  70                  75                  80

Ser Phe Cys Gly Gly
                85

<210>  191
<211>  85
<212>  PRT
<213>  Artificial Sequence

<220>
<223>  TPO2-PAS49-b

<220>
<221>  DISULFID
<222>  (3)..(16)
<223>  Disulfid bridge between Cys-3 and Cys-16

<220>
<221>  DISULFID
<222>  (70)..(83)
<223>  Disulfid bridge between Cys-70 and Cys-83

<400>  191

Gly Gly Cys Ala Asp Gly Pro Thr Leu Arg Glu Trp Ile Ser Phe Cys
1               5                   10                  15

Gly Gly Ser Ala Pro Ser Pro Ser Ser Ala Pro Ala Ser Ala Ser Ala
            20                  25                  30

Pro Ala Ser Pro Ala Ser Ala Ser Ala Ser Ala Ser Pro Ala Ser Ser
        35                  40                  45

Pro Ala Ser Ala Ser Ser Ala Ser Pro Ala Ala Ser Ser Ala Ser Pro
    50                  55                  60

Ala Ser Ser Gly Gly Cys Ala Asp Gly Pro Thr Leu Arg Glu Trp Ile
65                  70                  75                  80

Ser Phe Cys Gly Gly
                85

<210>  192
<211>  136
<212>  PRT
<213>  Artificial Sequence

<220>
<223>  TPO2-PAS100

<220>
<221>  DISULFID
<222>  (3)..(16)
<223>  Disulfid bridge between Cys-3 and Cys-16

<220>
<221>  DISULFID
<222>  (121)..(134)
<223>  Disulfid bridge between Cys-121 and Cys-134

<400>  192

Gly Gly Cys Ala Asp Gly Pro Thr Leu Arg Glu Trp Ile Ser Phe Cys
1               5                   10                  15

Gly Gly Ala Ala Pro Ala Ala Pro Ala Pro Ala Ala Pro Ala Ala Pro
            20                  25                  30

Ala Pro Ala Ala Pro Ala Ala Pro Ala Pro Ala Ala Pro Ala Ala Pro
        35                  40                  45

Ala Pro Ala Ala Pro Ala Ala Pro Ala Pro Ala Ala Pro Ala Ala Pro
    50                  55                  60

Ala Pro Ala Ala Pro Ala Ala Pro Ala Pro Ala Ala Pro Ala Ala Pro
65                  70                  75                  80

Ala Pro Ala Ala Pro Ala Ala Pro Ala Pro Ala Ala Pro Ala Ala Pro
                85                  90                  95

Ala Pro Ala Ala Pro Ala Ala Pro Ala Pro Ala Ala Pro Ala Ala Pro
            100                 105                 110

Ala Pro Ala Ala Pro Ala Gly Gly Cys Ala Asp Gly Pro Thr Leu Arg
        115                 120                 125

Glu Trp Ile Ser Phe Cys Gly Gly
    130                 135

<210>  193
<211>  136
<212>  PRT
<213>  Artificial Sequence

<220>
<223>  TPO2-PAS100-b

<220>
<221>  DISULFID
<222>  (3)..(16)
<223>  Disulfid bridge between Cys-3 and Cys-16

<220>
<221>  DISULFID
<222>  (121)..(134)
<223>  Disulfid bridge between Cys-121 and Cys-134

<400>  193

Gly Gly Cys Ala Asp Gly Pro Thr Leu Arg Glu Trp Ile Ser Phe Cys
1               5                   10                  15

Gly Gly Ser Ala Pro Ser Pro Ser Ser Ala Pro Ala Ser Ala Ser Ala
            20                  25                  30

Pro Ala Ser Pro Ala Ser Ala Ser Ala Ser Ala Ser Pro Ala Ser Ser
        35                  40                  45

Pro Ala Ser Ala Ser Ser Ala Ser Pro Ala Ala Ser Ser Ala Ser Pro
    50                  55                  60

Ala Ser Ser Ser Ala Ser Pro Ser Ala Pro Ala Ala Pro Ser Ala Ser
65                  70                  75                  80

Ala Ala Pro Ser Ala Ser Pro Ala Ser Ser Ser Pro Ala Pro Ala Ser
                85                  90                  95

Ala Pro Ser Pro Ala Ala Ala Ala Ser Ser Pro Ser Pro Ala Pro Ser
            100                 105                 110

Ser Ala Ser Ser Ala Ala Gly Gly Cys Ala Asp Gly Pro Thr Leu Arg
        115                 120                 125

Glu Trp Ile Ser Phe Cys Gly Gly
    130                 135

<210>  194
<211>  44

<212>  PRT
<213>  Artificial Sequence

<220>
<223>  TPO2-GS8


<220>
<221>  DISULFID
<222>  (3)..(16)
<223>  Disulfid bridge between Cys-3 and Cys-16

<220>
<221>  DISULFID
<222>  (29)..(42)
<223>  Disulfid bridge between Cys-29 and Cys-42

<400>  194

Gly Gly Cys Ala Asp Gly Pro Thr Leu Arg Glu Trp Ile Ser Phe Cys
1               5                   10                  15


Gly Gly Gly Gly Gly Gly Ser Gly Gly Gly Gly Gly Cys Ala Asp Gly
            20                  25                  30


Pro Thr Leu Arg Glu Trp Ile Ser Phe Cys Gly Gly
        35                  40


<210>  195
<211>  333
<212>  DNA
<213>  Artificial Sequence

<220>
<223>  CspAss-TPO2-PAS49

<400>  195
atgaaacgca tgaaatcgct ggctgcggcg ctcaccgtcg ctggggccat gctggccgca      60

cctgtggcaa cggcaggtgg ctgtgctgac ggtcctaccc ttcgtgagtg gatttctttt     120

tgcggcggtg ctgctcctgc tgcccctgct cctgctgctc ctgcagcacc agccccagca     180

gctccagccg ccccagctcc agccgcacca gctgccccag caccagccgc tccagcagca     240

ccagctccag cagccccagc tgcaccagcc ccagcgggcg gttgtgctga cggtcctacc     300

cttcgtgagt ggatttcctt ctgtggtggc taa                                  333


<210>  196
<211>  110
<212>  PRT
<213>  Artificial Sequence

<220>
<223>  CspAss-TPO2-PAS49


<220>

<221>  DISULFID
<222>  (28)..(41)
<223>  Disulfid bridge between Cys-28 and Cys-41

<220>
<221>  DISULFID
<222>  (95)..(108)
<223>  Disulfid bridge between Cys-95 and Cys-108

<400>  196

Met Lys Arg Met Lys Ser Leu Ala Ala Ala Leu Thr Val Ala Gly Ala
1               5                   10                  15

Met Leu Ala Ala Pro Val Ala Thr Ala Gly Gly Cys Ala Asp Gly Pro
            20                  25                  30

Thr Leu Arg Glu Trp Ile Ser Phe Cys Gly Gly Ala Ala Pro Ala Ala
        35                  40                  45

Pro Ala Pro Ala Ala Pro Ala Ala Pro Ala Pro Ala Ala Pro Ala Ala
    50                  55                  60

Pro Ala Pro Ala Ala Pro Ala Ala Pro Ala Pro Ala Ala Pro Ala Ala
65                  70                  75                  80

Pro Ala Pro Ala Ala Pro Ala Ala Pro Ala Pro Ala Gly Gly Cys Ala
                85                  90                  95

Asp Gly Pro Thr Leu Arg Glu Trp Ile Ser Phe Cys Gly Gly
            100                 105                 110

<210>  197
<211>  333
<212>  DNA
<213>  Artificial Sequence

<220>
<223>  CspAss-TPO2-PAS49-b

<400>  197
atgaaacgca tgaaatcgct ggctgcggcg ctcaccgtcg ctggggccat gctggccgca      60

cctgtggcaa cggcaggcgg gtgtgcagac ggacccactc tgcgcgaatg gatctccttt     120

tgcggcggtt ccgctccgtc gccatcctct gctccggcat ccgcgtctgc gccagcatcc     180

ccagccagcg cttcagcctc agcgtcccct gcctcctctc cagcatccgc cagcagcgca     240

tctcctgcgg ctagctcggc atcacccgct tcgtccggag gttgcgctga tggccctacc     300

ctccgtgagt ggatttcctt ctgcggtggc taa                                  333

<210>  198
<211>  110

<212>  PRT
<213>  Artificial Sequence

<220>
<223>  CspAss-TPO2-PAS49-b

<220>
<221>  DISULFID
<222>  (28)..(41)
<223>  Disulfid bridge between Cys-28 and Cys-41

<220>
<221>  DISULFID
<222>  (95)..(108)
<223>  Disulfid bridge between Cys-95 and Cys-108

<400>  198

Met Lys Arg Met Lys Ser Leu Ala Ala Ala Leu Thr Val Ala Gly Ala
1               5                   10                  15

Met Leu Ala Ala Pro Val Ala Thr Ala Gly Gly Cys Ala Asp Gly Pro
            20                  25                  30

Thr Leu Arg Glu Trp Ile Ser Phe Cys Gly Gly Ser Ala Pro Ser Pro
        35                  40                  45

Ser Ser Ala Pro Ala Ser Ala Ser Ala Pro Ala Ser Pro Ala Ser Ala
    50                  55                  60

Ser Ala Ser Ala Ser Pro Ala Ser Ser Pro Ala Ser Ala Ser Ser Ala
65                  70                  75                  80

Ser Pro Ala Ala Ser Ser Ala Ser Pro Ala Ser Ser Gly Gly Cys Ala
                85                  90                  95

Asp Gly Pro Thr Leu Arg Glu Trp Ile Ser Phe Cys Gly Gly
            100                 105                 110

<210>  199
<211>  486
<212>  DNA
<213>  Artificial Sequence

<220>
<223>  CspAss-TPO2-PAS100

<400>  199
atgaaacgca tgaaatcgct ggctgcggcg ctcaccgtcg ctggggccat gctggccgca      60

cctgtggcaa cggcaggcgg ttgtgctgac ggtcctacct tgcgtgaatg gatttctttc     120

tgtggtggtg cggctccggc tgctccggct cctgcggctc cggctgcacc agcaccagcc     180

gcaccagctg caccagcccc agctgcacca gccgctccag caccagctgc cccagctgca     240

ccagctccag ccgcaccagc agccccagct ccagcagccc cagcagctcc agcaccagcc 300

gctccagctg ccccagcacc agctgctcca gcagctccag caccagcagc tccagctgct 360

ccagccccag ccgccccagc cgctccagcc ccagcagcac cagccgcccc agctccagcc 420

gccccagcag gtggctgtgc tgatggtcct acccttcgtg aatggatttc cttttgtggc 480

ggttag 486

<210> 200
<211> 161
<212> PRT
<213> Artificial Sequence

<220>
<223> CspAss-TPO2-PAS100

<220>
<221> DISULFID
<222> (28)..(41)
<223> Disulfid bridge between Cys-28 and Cys-41

<220>
<221> DISULFID
<222> (146)..(159)
<223> Disulfid bridge between Cys-146 and Cys-159

<400> 200

Met Lys Arg Met Lys Ser Leu Ala Ala Ala Leu Thr Val Ala Gly Ala
1               5                   10                  15

Met Leu Ala Ala Pro Val Ala Thr Ala Gly Gly Cys Ala Asp Gly Pro
            20                  25                  30

Thr Leu Arg Glu Trp Ile Ser Phe Cys Gly Gly Ala Ala Pro Ala Ala
        35                  40                  45

Pro Ala Pro Ala Ala Pro Ala Ala Pro Ala Pro Ala Ala Pro Ala Ala
    50                  55                  60

Pro Ala Pro Ala Ala Pro Ala Ala Pro Ala Pro Ala Ala Pro Ala Ala
65                  70                  75                  80

Pro Ala Pro Ala Ala Pro Ala Ala Pro Ala Pro Ala Ala Pro Ala Ala
                85                  90                  95

Pro Ala Pro Ala Ala Pro Ala Ala Pro Ala Pro Ala Ala Pro Ala Ala
            100                 105                 110

Pro Ala Pro Ala Ala Pro Ala Ala Pro Ala Pro Ala Ala Pro Ala Ala
        115                 120                 125

Pro Ala Pro Ala Ala Pro Ala Ala Pro Ala Pro Ala Ala Pro Ala Gly
    130                 135                 140

Gly Cys Ala Asp Gly Pro Thr Leu Arg Glu Trp Ile Ser Phe Cys Gly
145                 150                 155                 160

Gly

<210>  201
<211>  486
<212>  DNA
<213>  Artificial Sequence

<220>
<223>  CspAss-TPO2-PAS100-b

<400>  201
atgaaacgca tgaaatcgct ggctgcggcg ctcaccgtcg ctggggccat gctggccgca      60

cctgtggcaa cggcaggcgg gtgtgcagac ggtcccactc tccgcgaatg gatctccttt     120

tgcggcggaa gcgcgccctc cccgtcatct gcccctgcat ctgcgtccgc accagcatcc     180

ccagcctctg cgtctgcttc tgcatcaccg gcctcgtccc cagcaagcgc atcgtccgcg     240

tcaccagctg cgtcctcagc ctcccctgct tcctcgtccg cttcgccttc ggctcctgcc     300

gctccctctg catccgctgc cccatccgcc tcaccagcca gctcctctcc ggcaccagcg     360

tccgctcctt cccctgcagc tgcggcgagc tctccaagcc cggctccctc ctccgcatcc     420

agcgctgccg gtggatgcgc agatggcccg accctgcgtg agtggattag cttctgcggc     480

ggttaa                                                                486

<210>  202
<211>  161
<212>  PRT
<213>  Artificial Sequence

<220>
<223>  CspAss-TPO2-PAS100-b

<220>
<221>  DISULFID
<222>  (28)..(41)
<223>  Disulfid bridge between Cys-28 and Cys-41

<220>
<221>  DISULFID
<222>  (146)..(159)
<223>  Disulfid bridge between Cys-146 and Cys-159

<400>  202

Met Lys Arg Met Lys Ser Leu Ala Ala Ala Leu Thr Val Ala Gly Ala
1               5                   10                  15

Met Leu Ala Ala Pro Val Ala Thr Ala Gly Gly Cys Ala Asp Gly Pro
            20                  25                  30

Thr Leu Arg Glu Trp Ile Ser Phe Cys Gly Gly Ser Ala Pro Ser Pro
        35                  40                  45

Ser Ser Ala Pro Ala Ser Ala Ser Ala Pro Ala Ser Pro Ala Ser Ala
    50                  55                  60

Ser Ala Ser Ala Ser Pro Ala Ser Ser Pro Ala Ser Ala Ser Ser Ala
65                  70                  75                  80

Ser Pro Ala Ala Ser Ser Ala Ser Pro Ala Ser Ser Ser Ala Ser Pro
                85                  90                  95

Ser Ala Pro Ala Ala Pro Ser Ala Ser Ala Ala Pro Ser Ala Ser Pro
            100                 105                 110

Ala Ser Ser Ser Pro Ala Pro Ala Ser Ala Pro Ser Pro Ala Ala Ala
        115                 120                 125

Ala Ser Ser Pro Ser Pro Ala Pro Ser Ser Ala Ser Ser Ala Ala Gly
    130                 135                 140

Gly Cys Ala Asp Gly Pro Thr Leu Arg Glu Trp Ile Ser Phe Cys Gly
145                 150                 155                 160

Gly

<210>  203
<211>  210
<212>  DNA
<213>  Artificial Sequence

<220>
<223>  CspAss-TPO2-GS8

<400>  203
atgaaacgca tgaaatcgct ggctgcggcg ctcaccgtcg ctggggccat gctggccgca      60

cctgtggcaa cggcaggtgg ctgtgcggat ggtcctacct tgcgtgagtg gatttctttc     120

tgtggcggtg gtggtggtgg ttctggtggt ggtggtggtt gcgcagatgg cccaaccctg     180

cgcgaatgga tctccttctg cggcggctaa                                      210

<210>  204

<211>  69
<212>  PRT
<213>  Artificial Sequence

<220>
<223>  CspAss-TPO2-GS8



<220>
<221>  DISULFID
<222>  (28)..(41)
<223>  Disulfid bridge between Cys-28 and Cys-41

<220>
<221>  DISULFID
<222>  (54)..(67)
<223>  Disulfid bridge between Cys-54 and Cys-67

<400>  204

Met Lys Arg Met Lys Ser Leu Ala Ala Ala Leu Thr Val Ala Gly Ala
1               5                   10                  15


Met Leu Ala Ala Pro Val Ala Thr Ala Gly Gly Cys Ala Asp Gly Pro
            20                  25                  30


Thr Leu Arg Glu Trp Ile Ser Phe Cys Gly Gly Gly Gly Gly Gly Ser
        35                  40                  45


Gly Gly Gly Gly Gly Cys Ala Asp Gly Pro Thr Leu Arg Glu Trp Ile
    50                  55                  60


Ser Phe Cys Gly Gly
65